# EUROPÄISCHE PATENTSCHRIFT

(11) **EP 2 746 399 B1**
(45) Veröffentlichungstag und Bekanntmachung des Hinweises auf die Patenterteilung: **19.07.2017**
(21) Anmeldenummer: 13194396.1
(22) Anmeldetag: 26.11.2013
(51) Int. Cl.: C12P 7/64, C12P 13/00

(54) **HERSTELLUNG VON OMEGA-AMINOFETTSÄUREN**
PRODUCTION OF OMEGA AMINO FATTY ACIDS
FABRICATION D'ACIDES GRAS OMÉGA-AMINÉS

(30) Priorität: 21.12.2012 EP 12199024
(43) Veröffentlichungstag der Anmeldung: 25.06.2014
(73) Patentinhaber: Evonik Degussa GmbH, 45128 Essen (DE)
(72) Erfinder: Schaffer, Steffen, 45699 Herten (DE); Gielen, Jasmin, 44879 Bochum (DE); Wessel, Mirja, 44799 Bochum (DE); Hennemann, Hans-Georg, 45770 Marl (DE); Häger, Harald, 59348 Lüdinghausen (DE); Haas, Thomas, 48161 Münster (DE); Blümke, Wilfried, 61137 Schöneck (DE)

(56) Entgegenhaltungen:
- WO-A1-2009/077461
- US-A1- 2007 032 428
- J. N. COPP ET AL: "The Phosphopantetheinyl Transferase Superfamily: Phylogenetic Analysis and Functional Implications in Cyanobacteria", APPLIED AND ENVIRONMENTAL MICROBIOLOGY, Bd. 72, Nr. 4, 1. April 2006 (2006-04-01), Seiten 2298-2305, XP055063071, ISSN: 0099-2240, DOI: 10.1128/AEM.72.4.2298-2305.2006

### SEQUENCE LISTING

<110> Evonik Industries AG
<120> Herstellung von omega-Aminofettsäuren
<130> 201200202
<160> 29
<170> PatentIn version 3.5
<210> 1
   <211> 669
   <212> DNA
   <213> Artificial Sequence
<220>
   <223> synthetic gene
<400> 1
   atgattgaaa ccatcttacc agccggcgtc gaatcagcag agttacttga gtaccctgaa      60
   gatctgaagg cgcatccggc tgaagagcat ctgatcgcaa agagcgtaga gaagcgtcgt     120
   cgcgacttca ttggcgcacg tcattgcgcc cgtctggcac tggcggagct gggtgaaccg     180
   ccggttgcga ttggcaaggg tgaacgtggt gcgccgattt ggccgcgtgg tgtcgtgggc     240
   tctctgaccc attgcgatgg ctatcgcgca gcggcggttg ctcacaaaat gcgttttcgc     300
   agcatcggca tcgacgccga accgcacgcg accctgccgg aaggtgtcct ggattcggtt     360
   agcctgccgc ctgagcgtga gtggctgaaa accaccgaca gcgcactgca cctggaccgt     420
   ttgctgtttt gtgcgaaaga agcaacttac aaagcgtggt ggccgctgac ggcacgttgg     480
   ctgggtttcg aagaagcgca cattaccttc gaaatcgagg atggtagcgc cgactctggt     540
   aatggcacgt ttcacagcga actgctggtg ccgggtcaga ccaatgacgg tggtaccccg     600
   ctgctgtctt tcgacggtcg ctggctgatc gctgatggct tcatcctgac ggcgattgca     660
   tacgcatga                                                             669
<210> 2
   <211> 153
   <212> DNA
   <213> Artificial Sequence
<220>
   <223> promoter
<400> 2
   ggcagtgagc gcaacgcaat taatgtaagt tagctcactc attaggcacc ccaggcttga      60
   cactttatgc ttccggctcg tataatgtgt ggaattgtga gcggataaca ataacaattt     120
   cacacaggat ctaggaacca aggagagtgg cat                                  153
<210> 3
   <211> 3507
   <212> DNA
   <213> Artificial Sequence
<220>
   <223> synthetic gene
<400> 3
   atgacaattg aaacgcgcga agatagattt aacagacgta ttgatcacct ttttgaaaca      60
   gacccgcagt tcgcagcggc acgtccggac gaagctatta gcgctgcagc agccgatccg     120
   gagctgcgcc tgccagccgc agtcaagcag attctggccg gttatgcgga ccgtccggcc     180
   ctgggtaagc gtgctgtgga gttcgttacg gacgaagaag gccgtaccac cgcaaagttg     240
   ctgccgcgtt tcgatacgat cacctaccgt caattggcag gtcgtattca ggcggtgact     300
   aacgcgtggc acaatcaccc ggtgaatgcg ggtgaccgtg tcgcaatttt gggcttcacg     360
   agcgttgact atacgaccat cgatatcgca ctgctggaac tgggtgctgt tagcgttccg     420
   ttgcaaacgt ctgctccggt tgcgcagctg caaccgattg tggccgaaac cgagccgaag     480
   gtcattgcga gcagcgtcga tttcctggca gatgcggtcg cactggttga gagcggtcca     540
   gccccgagcc gtctggtcgt ttttgattat agccacgagg tcgacgatca gcgtgaggcc     600
   ttcgaagcgg ccaagggtaa actggcgggt accggtgttg tggtggaaac gattacggat     660
   gtcttggatc gcggtcgcag cctggctgat gcgccgctgt atgtcccaga cgaaacggac     720
   ccgctgaccc tgctgatcta taccagcggt agcaccggta cgccgaaggg tgctatgtat     780
   ccggaatcca agaccgcgac gatgtggcaa gctggtagca aggcgcgttg ggatgaaacc     840
   ctgggcgtta tgccgagcat caccctgaac ttcatgccaa tgtcccacgt gatgggtcgc     900
   ggcatcctgt gtagcaccct ggcgagcggc ggtactgcgt attttgccgc acgtagcgac     960
   ttgtctacct ttctggagga cctggcgttg gtccgcccga cccaactgaa tttcgtccct    1020
   cgcatctggg atatgctgtt ccaagagtat cagagccgtc tggacaatcg tcgtgccgag    1080
   ggtagcgagg atcgcgcgga agcggccgta ctggaagagg ttcgtaccca attgctgggc    1140
   ggtcgtttcg tttccgctct gaccggtagc gctccaatca gcgcggaaat gaaaagctgg    1200
   gtcgaggacc tgctggacat gcatttgctg gaaggttacg gttccaccga ggcgggtgcc    1260
   gtgttcattg acggtcaaat ccaacgtcca cctgttatcg attacaaact ggttgatgtt    1320
   ccggacttgg gttactttgc gaccgatcgt ccataccctc gcggcgagct gctggtgaag    1380
   agcgagcaga tgttcccggg ctactataag cgtccggaga ttacggcgga gatgtttgac    1440
   gaggacggtt attaccgtac cggcgacatc gtggccgaac tgggtccgga tcatctggag    1500
   tatctggacc gccgtaacaa cgtcctgaag ctgagccagg gtgagtttgt taccgtttcc    1560
   aaactggaag cggtctttgg cgatagccca ctggtgcgcc agatttatgt ctacggtaac    1620
   tctgcacgca gctatctgct ggcggttgtc gttccgaccg aagaggcact gagccgctgg    1680
   gacggcgatg aactgaagag ccgcatcagc gacagcttgc aagatgcggc acgtgccgca 1740
   ggtttgcaaa gctatgagat cccgcgtgac tttctggttg aaaccacgcc gttcacgttg 1800
   gagaacggtc tgctgaccgg cattcgcaaa ctggcgcgtc cgaagctgaa agcgcattat 1860
   ggcgaacgtc tggaacaact gtacacggat ctggctgaag gccaagccaa tgagctgcgt 1920
   gaactgcgcc gtaatggtgc ggatcgtccg gttgttgaaa cggtttcgcg tgccgcggtc 1980
   gccttgctgg gtgcgtctgt gacggacctg cgttctgacg cccactttac ggacctgggc 2040
   ggtgattccc tgagcgccct gagcttcagc aacctgctgc acgagatttt tgacgtcgat 2100
   gtcccggtgg gcgtgattgt cagcccggca accgacctgg ccggtgttgc cgcctatatt 2160
   gagggtgagc tgcgtggtag caaacgtccg acctacgcaa gcgtccacgg tcgtgacgca 2220
   accgaggtgc gtgctcgcga tctggcgctg ggcaaattca tcgacgcaaa aacgctgagc 2280
   gcagcaccgg gtctgccgcg ctccggtacg gagatccgta cggtgctgct gacgggtgcg 2340
   accggttttc tgggtcgcta cttggccttg gagtggctgg aacgtatgga cctggttgac 2400
   ggcaaggtga tctgtttggt tcgtgcgcgt agcgacgacg aagcacgtgc gcgtctggac 2460
   gcaaccttcg ataccggcga tgctaccttg ctggagcact accgtgccct ggcggctgac 2520
   catctggaag ttattgcggg tgataagggt gaagccgact tgggcctgga tcacgatacc 2580
   tggcagcgtc tggcagatac ggtcgacctg atcgttgacc cggctgcgct ggttaaccac 2640
   gttttgccgt acagccagat gttcggccca aacgctctgg gcactgcaga gctgattcgc 2700
   attgcactga ccaccaccat taaaccgtac gtctacgtga gcaccattgg tgttggtcag 2760
   ggcatcagcc cagaagcctt tgttgaagat gcggacatcc gcgaaatcag cgccacgcgt 2820
   cgcgttgacg acagctacgc gaatggttat ggtaacagca aatgggcagg tgaggttctg 2880
   ctgcgcgaag cgcacgactg gtgcggcctg ccggtgagcg tgtttcgttg tgacatgatc 2940
   ttggccgaca ccacctactc cggccagctg aatctgccgg atatgttcac ccgtctgatg 3000
   ttgagcctgg tggcaactgg catcgcaccg ggcagctttt atgagctgga cgcggacggt 3060
   aatcgtcaac gtgcacatta tgatggtctg ccggtggagt ttatcgcgga agcaatcagc 3120
   acgatcggct ctcaagtgac tgacggtttc gaaacgttcc atgtcatgaa tccttatgat 3180
   gatggtatcg gcctggacga gtacgtcgac tggctgattg aggctggcta cccggttcac 3240
   cgtgttgatg attatgcgac gtggttgagc cgttttgaaa ccgccttgcg tgcgctgccg 3300
   gagcgtcaac gtcaggcatc cctgctgccg ctgttgcaca actaccagca gccttctccg 3360
   ccggtttgcg gtgctatggc tcctaccgac cgtttccgtg cagctgtcca ggacgccaaa 3420
   attggcccag ataaagacat cccacatgta acggcggacg tgatcgtgaa atacattagc 3480
   aacttgcaga tgctgggtct gctgtaa 3507
<210> 4
   <211> 3525
   <212> DNA
   <213> Artificial Sequence
<220>
   <223> synthetic gene
<400> 4
   atgactagcg atgtacatga cgcgactgac ggtgtgacag agacagcatt agatgacgag 60
   caaagcaccc gtcgcattgc ggaactgtat gcaacggatc cggagtttgc agctgccgca 120
   ccgctgccgg ctgttgtcga cgctgcacat aaaccgggtc tgcgtctggc ggagattctg 180
   cagaccctgt ttaccggtta cggcgatcgt ccggccctgg gctaccgtgc tcgtgaactg 240
   gcgacggatg agggtggccg taccgtgacc cgtttgttgc cgcgttttga cacgctgacg 300
   tacgcgcaag tctggagccg tgtccaggct gtggcagcag cgctgcgcca taacttcgcg 360
   cagcctatct atccaggtga cgcggttgcg accatcggct ttgcaagccc agactatctg 420
   acgctggatc tggtctgcgc gtatttgggc ctggtcagcg tgccactgca gcacaacgct 480
   cctgtgagcc gtttggcgcc gattctggcc gaggttgagc cgcgtatcct gacggtcagc 540
   gccgaatacc tggacctggc ggtcgagagc gtgcgtgatg tgaattctgt ctcgcaattg 600
   gtcgttttcg accaccaccc ggaagtcgat gaccaccgcg atgcgctggc acgcgcacgt 660
   gaacagctgg ccggtaaagg tattgccgtt acgactctgg acgcgattgc ggacgaaggc 720
   gctggtctgc cagcggagcc gatctatacc gccgatcacg atcaacgcct ggcgatgatc 780
   ctgtacacca gcggttctac cggcgctccg aagggtgcga tgtatactga ggccatgctg 840
   gcgcgtctgt ggaccatgtc ctttatcacc ggcgacccga ctcctgtgat taatgtgaac 900
   tttatgccgc tgaatcacct gggtggtcgt attccgatta gcaccgcggt ccagaacggc 960
   ggtacgagct acttcgttcc ggagagcgac atgtctaccc tgtttgagga cctggccttg 1020
   gttcgcccga cggaactggg tctggttccg cgtgtggcgg acatgttgta ccagcatcac 1080
   ctggcaactg ttgaccgtct ggttacccag ggcgcagacg agctgaccgc agagaagcag 1140
   gcaggcgcag aactgcgtga acaggtgctg ggcggtcgtg tgattaccgg cttcgtgagc 1200
   acggcgccac tggcggctga gatgcgtgcc ttcctggata tcacgctggg tgcccacatc 1260
   gttgatggtt acggtttgac cgaaaccggt gcggtgacgc gtgatggcgt tatcgtccgt 1320
   ccgccggtca ttgattacaa gctgattgat gttccagagc tgggctactt cagcaccgat 1380
   aaaccgtacc cacgtggcga actgttggtt cgtagccaaa cgttgacccc gggttactat 1440
   aaacgtccgg aagttaccgc atcggtgttt gatcgcgacg gctattacca taccggtgac 1500
   gttatggcag aaaccgctcc agatcacctg gtatatgtgg accgccgcaa taacgttctg 1560
   aaactggcgc agggtgaatt tgtcgcagtc gcaaacctgg aagccgtttt cagcggtgcc 1620
   gcgctggttc gccaaatctt cgtgtacggc aacagcgagc gcagcttcct gttggcggtg 1680
   gtggtgccga cgccagaagc cctggaacaa tacgatccgg cagccctgaa ggcggccttg 1740
   gcagatagcc tgcagcgcac ggctcgtgat gcggagctgc aatcgtatga agttccggcc 1800
   gatttcattg tggaaaccga gccgttcagc gccgcaaatg gtctgttgag cggtgtcggc 1860
   aagttgctgc gtccgaacct gaaagatcgt tatggtcaac gtctggagca aatgtatgcc 1920
   gatattgccg cgacccaggc gaatcagctg cgcgagctgc gtcgtgccgc agccacccag 1980
   ccggtgattg ataccctgac gcaggctgct gcgacgattt tgggtaccgg tagcgaagtg 2040
   gcgtctgatg cccactttac ggatctgggt ggcgacagcc tgtccgctct gaccttgtcc 2100
   aatttgctga gcgatttctt tggttttgag gttccggttg gtaccattgt taacccggcg 2160
   actaatctgg cgcagctggc acagcacatc gaagcacagc gcaccgccgg tgaccgccgt 2220
   ccgagcttta ccacggtgca tggtgcggac gcgacggaaa ttcgtgcgag cgaactgacc 2280
   ctggataagt ttattgacgc agaaaccctg caggcagcac caggcctgcc gaaggtgacc 2340
   accgagccgc gcaccgtcct gctgagcggt gccaatggtt ggttgggtcg cttcctgacc 2400
   ctgcaatggc tggaacgtct ggcgcctgtt ggtggtacgc tgatcaccat tgtccgcggt 2460
   cgtgacgacg ctgcggctcg tgctcgcctg acccaagctt acgacaccga cccagagttg 2520
   tctcgccgct tcgcagaact ggcggaccgc catctgcgtg ttgtagcagg cgatatcggt 2580
   gatccgaatc tgggcctgac cccagaaatc tggcaccgtc tggctgctga ggtggatttg 2640
   gttgtgcatc cagcggcgtt ggtgaaccac gtcttgccgt atcgtcagct gttcggcccg 2700
   aacgtcgtgg gcaccgccga agtgattaag ctggcgctga cggagcgcat caagccagtt 2760
   acctacctga gcaccgtcag cgttgcaatg ggtatcccgg actttgaaga ggatggtgat 2820
   atccgtaccg tttcccctgt tcgtccgctg gacggcggct atgctaatgg ctacggcaac 2880
   tctaagtggg ctggtgaggt cctgctgcgt gaggcccacg acctgtgtgg cctgccggtg 2940
   gccacgttcc gtagcgacat gattttggca cacccgcgct accgtggtca ggttaatgtg 3000
   ccggatatgt tcactcgtct gttgttgtcg ctgctgatca ctggcgtggc tccgcgctct 3060
   ttctacattg gtgatggtga acgtcctcgt gcgcattatc cgggtctgac tgtcgacttt 3120
   gtggccgagg ccgtgacgac cctgggtgca cagcagcgtg aaggctacgt tagctacgat 3180
   gtcatgaacc cgcatgacga tggcatcagc ctggatgttt tcgtggactg gctgatccgc 3240
   gcaggccatc cgattgaccg tgtggacgat tacgacgatt gggtgcgtcg cttcgagact 3300
   gcactgacgg cgctgccgga aaaacgtcgc gcacaaaccg ttctgccgtt gctgcacgcg 3360
   ttccgtgccc cgcaagcgcc gctgcgtggt gccccggagc caaccgaggt tttccatgcg 3420
   gcggttcgta ccgcaaaagt cggtccaggc gacattccgc acttggatga agcgctgatt 3480
   gataagtaca tccgtgatct gcgtgagttt ggcctgatct aataa 3525
<210> 5
   <211> 3525
   <212> DNA
   <213> Artificial Sequence
<220>
   <223> synthetic gene
<400> 5
   atgagcactg caattcacga tgaacacttg gatcgccgca ttgaagaact gattgcaaac      60
   gatccgcaat tcgctgcggc acgcccggac ccggcgatta ccgcagcgac cgaggcccca     120
   ggtctgcgtc tgccgcagat catccgtacg gtcctggacg gttatgccga tcgtccggcg     180
   ctggcgcaac gcgtggttga attcgttacc gacgccaaaa ccggtcgtac caccgcagag     240
   ttgctgccac gttttgaaac catcacgtac ggtgagctgg gtgagcgtgt gagcgcactg     300
   ggtcgcgcat gggccggtga cgccgtgcgc cctggcgacc gcgtttgcgt cctgggtttc     360
   aactccgtgg attacgcgac gattgacatc gcgctgggta ccatcggtgc ggtgagcgtg     420
   ccgctgcaaa ccagcgcagc gattagcagc ctgcagccga ttgtggcgga aaccgagccg     480
   agcctgattg cgagcagcgt gaatcagctg ccggacgccg tcgagctgat tctggcaggt     540
   gaccatgttc cgggtaagct ggtcgtattt gattatcaac cgcaagttga tgaccagcgt     600
   gaggcggttg aggcagcggc tgcgcgcctg gcagacagcg gcgtcgcagt ggaagcgctg     660
   gcggatgttc tgcgtcgtgg taaagacctg ccggcagttg aaccgccagc gtccgatgag     720
   gatagcctgg ctctgttgat ttacacctct ggcagcaccg gtgcgccgaa gggcgcgatg     780
   tacccgcaga gcaacgtggg taaaatgtgg cgtcgcggta gcaagaactg gtttggtgag     840
   agcgccgcaa gcatcaccct gaatttcatg ccgatgagcc atgttatggg tcgcggtatt     900
   ttgtatggca cgttgggtaa cggcggcacc gcgtatttcg cggctcgtag cgatttgtct     960
   acgctgctgg aagatctgga gttggtgcgc cctacggaga tgaattttgt cccgcgtatt    1020
   tgggaaacgc tgtatggtga gttccagcgt caggtggagc gccgtctggc ggacggtgac    1080
   gctggcccgg aagcccgtga aaccgtcgag gcggcagtgc tggaagagca gcgtcaatat    1140
   ctgctgggtg gccgttttat cttcgccatg accggctctg cgccgacgag cccggagctg    1200
   aaagcatggg cggagagctt gctgcaaatg cacctgatgg acggttacgg cagcaccgag    1260
   gcgggcatgg tgctgttcga cggtgagatc cagcgtccgc cggtcatcga ttacaaactg    1320
   gtggatgtcc cggatctggg ttacttttcg acggaccgtc cgcatccgcg tggtgagctg    1380
   ttgctgcgta ccgagaacat gttcccgggt tactataaac gcgcggaaac caccgcaaat    1440
   gttttcgacg aggatggtta ctaccgcacc ggcgatgtgt tcgcggagat cgcaccggac    1500
   cgtttggttt acgtcgatcg tcgcaataac gtcttgaaac tggcccaggg tgagtttgta    1560
   accctggcca aactggaagc ggttttcggt aactcgccgc tgatccgcca gatctacgtg 1620
   tacggcaaca gcagccagcc gtacctgctg gcggtggtgg tcccgaccga agaggcgctg 1680
   gccgataatg acttggaaag cttgaagccg aaaatcgcag actccctgca aaaagttgca 1740
   aaagaaactg gcctgcagtc ctatgaggtt ccgcgtgatt tcatcatcga gactacgccg 1800
   ttcaccctgg agaacggttt gctgaccggc attcgtaagc tggcgtggcc taagctgaag 1860
   gcccactatg gcgatcgtct ggagcaaatg tacgctgagc tggctgcggg tcaggcgaat 1920
   gagctggcgg agctgcgccg tagcggtgcg gcagcgccgg tggcccagac cgtgtcccgc 1980
   gcagcagcgg cgctgctggg cgctgctgct ggtgatctga gcgcggatgc ccactttact 2040
   gacctgggtg gtgattccct gtccgcactg acctttggta acctgctgcg tgagattttt 2100
   gacgtcgatg ttccggttgg cgttattgtc tcccctgcta acgacttggc gggcattgcc 2160
   gcatacattg aagcagagcg tcaaggtagc aaacgtccga cgttcgcagc agtgcatggt 2220
   cgcggtgcca cgatggtgca tgcgagcgac ttgactctgg ataagtttct ggacgaagcc 2280
   accctggcag cagccccaag cctgccgaaa ccggcgacgg aagttcgtac ggttctgctg 2340
   accggtgcga cgggttttct gggccgttat ctggcactgg actggctgga gcgcatggat 2400
   atggttgacg gcaaagttat tgcgctggtc cgtgcgcgca ccgatgaaga agcgcgtgcg 2460
   cgtttggaca agacgtttga cagcggcgac ccgaaactgc tggctcacta ccaacgtctg 2520
   gcagcggacc acttggaagt tatcgcgggt gacaaaggtg aggctaatct gggcttggac 2580
   ccgcaaacct ggcagcgtct ggccgaagaa gtggacgtga tcgttgaccc ggcagcgctg 2640
   gtgaaccatg tcctgccgta ctctgaattg tttggcccga acgcactggg cacggccgaa 2700
   ctgatccgca tcgcgctgac ctctaagcaa aaaccgtata cgtatgttag caccattggt 2760
   gttggcgatc agattcaacc gggcgagttc gttgaaaatg ctgatatccg tcagatcagc 2820
   gcgacgcgtg agatcaacga tggctacgct aatggttatg gcaatagcaa gtgggcgggt 2880
   gaggttctgc tgcgcgaggc gcacgacctg tgtggcctgc cggtcaccgt gttccgttgc 2940
   gatatgatcc tggctgacac cacgtacgct ggccagctga atctgccgga catgtttacc 3000
   cgtttgatgc tgagcctggt cgcgaccggt atcgcaccgg gcagctttta cgaactggac 3060
   acggatggca atcgccagcg tgtccactat gatggtctgc cagtcgagtt cattgctgcg 3120
   gccatcagca ccctgggcac ccaaatcacg gatagcgaca ccggtttcca gacttatcat 3180
   gtaatgaatc catacgatga cggtattggt ctggatgagt acattgactg gctgattgag 3240
   gctggttaca gcatcgaacg tattgcggac tatagcgaat ggctgcgtcg cttcgaaacg 3300
   tcgctgcgtg ccctgcctga ccgtcaacgc cagtattcgc tgctgccgct gttgcacaat 3360
   tatcaaaagc cggagaagcc gattaacggt agcatggcgc caactgatgt ctttcgtgca 3420
   gccgttcaag aagcgaagat cggtccggat aaggacattc cgcacgtctc tgcgccggtg 3480
   attgtcaagt acatcaccga cctggagctg ctgggtttgc tgtaa 3525
<210> 6
   <211> 3471
   <212> DNA
   <213> Artificial Sequence
<220>
   <223> synthetic gene
<400> 6
   atgacgaccg aaaccagaga agatcgctta cagagacgca ttgcccacct gtacgaggca 60
   gacagccaat ttgcggctgc gcgtcctagc gaggcggtga ataccgccgt ggcggaacca 120
   gagctgcgtc tgccggctgt cgtcaaaggt gtctttgcgg gctatgcgga tcgtccggcg 180
   ctgggtcaac gcgccgtgga gtatgttacc gacgcggacg gtcgtacctc tgcccaattg 240
   ctgccgcgtt ttgacacgat cacgtaccgt caactgggtg accgcgtcca agcagttacg 300
   aacgcgtggc acaaccatcc ggtcaaacct ggcgaccgtg tagcgatcct gggctttact 360
   agcgttgatt acaccaccgt tgatacggcc ctgatcgaat tgggcgctgt gagcgtcccg 420
   ctgcagactt ctgcaccggt caccacgctg cgcccgatcg tcgcagagac ggagccgacc 480
   gtgattgcgg cttctattga tttcctggat gatgcggtgg aactggtcaa aagcggtccg 540
   gcaccgcgtc gtctggttgt gttcgactac cgcccacgtg tcgacgccca gcgcgaggca 600
   tttgaagcag ccaaagcggc actggcgggt actgacgttg tagttgagcc gctggcggat 660
   gtcctggacc gcggtcgtag cttggcggat gcgccgctgt acaccccggg tcagccggac 720
   ccgctgacca tgctgattta cacgagcggt tccacgggca ccccgaaggg tgcgatgtat 780
   ccggagagca aagtcgctaa catgtggcaa ctggcgacga aggcgacgtg ggatgagaac 840
   caagcggcgc tgccggccat caccctgaac tttatgccga tgagccatgt gatgggccgt 900
   ggcatcctga ttggtaccct gagcagcggt ggcaccgcat acttcgcggc tcgcagcgat 960
   ctgagcacct tcctggaaga tctggcattg gttcgtccga cccagttgag ctttgttccg 1020
   cgtatttggg atatgctgtt ccaggaatat cagagccgtc tggaccgttc tggtgcgcca 1080
   gaggacgagg tgctggccga ggtccgccaa gacctgctgg gcggtcgttt cgtgagcgcg 1140
   atgacgggtt ctgcgccgat tagcgcggaa atgaagaatt gggtggaacg tctgctggac 1200
   atgcatctgc tggaaggcta tggttccacc gaagcaggct ccgtgtttgt ggacggtcat 1260
   attcaacgtc cgccggttat tgactataaa ctggttgatg tcccggacct gggttatttc 1320
   ttgacggacc gtccgcatcc gcgtggcgag ctgctggtga agagcgagca aatgttccca 1380
   ggttactaca agcgtccgga gattaccgct gaaatgttcg atgaggatgg ctactaccgc 1440
   accggcgaca tcgtggcgga attgggtccg gatcaagttg agtatctgga ccgtcgtaat 1500
   aacgttctga aattgtctca gggtgagttt gtgacggtta gcaaactgga agcggtgttc 1560
   ggcgacagcc cgctggttcg tcagatcttc gtttatggta acagcgcacg ttcctacctg 1620
   ctggcagttg tggttccgac cgacccgtcg ctgagcaagc aggcgatcgg cgattcgttg 1680
   caggacgcgg cacgcgctgc aggtctgcaa tcctacgaga ttccgcgtga ctttatcgtc 1740
   gaaacgaccc cttttagcct ggagaacggc ctgctgacgg gcattcgcaa actggctcgc 1800
   ccaaacctga aggcctacta cggcgatcgt ctggagcagc tgtacaccga gctggcagag 1860
   ggccaagcga atgagttgag cgagctgcgt cgcaatggcg ctcaagcccc ggtcctggat 1920
   accgtgagcc gtgcagcggg tgctctgttg ggcgcagcgg ctagcgatct ggctccagag 1980
   gcgcatttta ccgacttggg tggcgatagc ttgagcgcgc tgacctttgg taacctgctg 2040
   caagagatct ttgatgtcga agtcccggtg agcgcaattg tttcgccggc atccgacttg 2100
   cgtacgatcg cagagtatat cgaagctcaa cgttccggcg cagacgtccg cccgaccttc 2160
   acgtccgtgc acggtcgcaa tgcgaccgag gtgcatgcga gcgatttgac gctggataag 2220
   ttcattgatg ccgccacgct ggcggcagcc ccgagcctgc cgggtccggt gagcgagatt 2280
   cgcactgtct tgctgaccgg tgcgacgggt ttcttgggcc gctatctggc gctggaatgg 2340
   ctggaacgta tggatctggt tgatggtaaa gtgatctgct tggttcgtgc gaagagcgac 2400
   gaagaggcgc gtgcccgcct ggacaaaact ttcgacagcg gcgatccgaa gctgtgggcc 2460
   cactatcaga agctggcagc cgatcatctg gaagtgatcg cgggtgacaa gggtgaggca 2520
   gacctgggcc tggatcaggt tacctggcag cgtctggcgg ataccgtgga tttcatcgtg 2580
   gatccggcag cgctggttaa tcacgttctg ccttatagcg aactgtttgg tccgaatgct 2640
   ctgggtactg ccgagctgat ccgtattgcg ctgaccaccc gtatcaaacc gttcgcgtac 2700
   gtgagcacga tcggcgtggg tggtggtatc gagccgggta agttcgtcga ggcgggtgac 2760
   attcgtgcga tctctcctgt ccgtcgcgtt gacgatggtt acgcaaatgg ctacggcaac 2820
   agcaaatggg ccggtgaagt cctgctgcgc gaagcccacg atctggcggg tctgccagtg 2880
   accgtttttc gctgtgatat gatcttggcc gacaccacct acgcaggtca gttgaatctg 2940
   cctgacatgt tcacgcgtat gatgttttcc ctggttgcga ccggtgtggc gccgaagagc 3000
   tttaatcaac tggatgcaga cggcaaccgc cagcgtagcc actatgacgg tctgccggtc 3060
   gagtttattg cagaagctat cagcaccctg ggcgcacacg ttcaggacgg cttcgaaact 3120
   tatcacgtta tgaatccgca cgacgacggt attggtatgg atgagttcgt tgattggctg 3180
   atcgaagccg gttacccgat tcagcgcgtc gaggactatc aggagtggct ggcgcgcttc 3240
   gaaaccaccc tgcgtgccct gccggataaa cagcgtcagg cgagcctgct gccgctgctg 3300
   cacaactacc agcaaccggg tgtcccggtt aacggcgcca tggcaccgac cgacgttttc 3360
   cgtacggccg tccaagacgc gaaaattggt ccggacaagg atattccgca cgtcagccgt 3420
   gaagttattg tgaagtacat cagcgatttg aaactgctgg gtttgctgta a 3471
<210> 7
   <211> 3522
   <212> DNA
   <213> Artificial Sequence
<220>
   <223> synthetic gene
<400> 7
   atgagcactg caacgcatga tgagagactg gatagacgcg tacatgaact gatcgcaacc 60
   gacccgcaat ttgcggcagc acagccggac ccggctatta cggcggcatt ggagcaaccg 120
   ggtctgcgtc tgccgcagat cattcgtacc gtcctggacg gctatgcgga tcgtccggcg 180
   ttgggtcaac gcgtcgttga gtttgttacc gacgcgaaaa ccggtcgcac gagcgcgcag 240
   ctgctgccgc gttttgaaac cattacctac tccgaagtgg cacaacgcgt tagcgcgctg 300
   ggtcgtgcgc tgtccgacga cgcagttcat ccgggtgatc gtgtgtgtgt cctgggtttt 360
   aacagcgttg attacgcgac catcgatatg gcactgggtg cgattggcgc ggtgtcggtt 420
   ccgctgcaaa cctctgcagc gattagctcc ctgcagccta ttgtcgcgga aaccgaaccg 480
   acgctgatcg cgagcagcgt taatcagctg agcgatgccg tgcagctgat caccggtgca 540
   gagcaggccc ctacccgttt ggtggtgttc gattatcacc cgcaagtcga tgaccagcgc 600
   gaggctgttc aggacgcggc tgcgcgcttg tccagcaccg gcgttgccgt gcaaaccctg 660
   gccgagctgc tggagcgcgg taaagacctg ccggctgtgg cggagccgcc agcggatgag 720
   gacagcctgg cgttgctgat ttacaccagc ggcagcacgg gtgctccgaa aggtgcgatg 780
   tacccacaga gcaatgttgg taagatgtgg cgtcgtggct ctaaaaactg gttcggcgaa 840
   agcgctgcta gcattaccct gaacttcatg ccgatgagcc atgtgatggg ccgtagcatc 900
   ttgtacggca ccctgggtaa cggcggtacg gcctatttcg ctgctcgtag cgatctgagc 960
   accctgctgg aagatctgga gctggtgcgt ccgacggaac tgaattttgt tccacgcatt 1020
   tgggaaacct tgtatggtga gttccaacgt caagtggaac gccgcctgag cgaggctggc 1080
   gacgcgggtg agcgtcgtgc tgtggaagcg gaagtgctgg cggagcagcg ccaatatctg 1140
   ctgggcggtc gtttcacctt tgcgatgacg ggcagcgccc ctatttcgcc tgagctgcgc 1200
   aactgggtcg agtccctgct ggagatgcat ctgatggatg gttacggtag cacggaagcg 1260
   ggcatggtcc tgtttgacgg tgagatccaa cgtccgccgg ttattgatta caagttggtg 1320
   gacgttccgg atttgggtta ctttagcacc gatcgcccgc acccgcgtgg tgagctgctg 1380
   ctgcgtacgg agaatatgtt cccgggttac tataagcgtg ccgaaactac cgcgggtgtt 1440
   tttgatgagg acggttacta ccgcacgggc gatgtgtttg ccgaaatcgc accggatcgc 1500
   ctggtgtatg ttgaccgtcg caacaacgta ttgaaactgg cacaaggcga gttcgtcacg 1560
   ttggccaagc tggaagcggt gtttggtaat tccccgctga ttcgtcagat ctatgtctat 1620
   ggtaacagcg cgcaaccgta tctgctggcc gttgtcgttc cgactgaaga agccctggcc 1680
   agcggtgatc ctgagacgct gaagccgaaa atcgcagata gcctgcagca agtggcgaaa 1740
   gaagcaggtc tgcagagcta cgaagttccg cgcgacttta tcatcgagac gacgccgttt 1800
   agcctggaaa acggcctgtt gacgggcatc cgcaaactgg cttggccgaa actgaagcaa 1860
   cactacggcg agcgtctgga gcaaatgtac gccgacctgg cagcgggcca agcaaatgaa 1920
   ctggcagagc tgcgtcgtaa tggcgcccaa gccccggtgc tgcagacggt tagccgtgca 1980
   gcgggtgcaa tgttgggctc tgccgcgagc gatttgagcc cagatgcgca cttcaccgac 2040
   ctgggtggtg acagcctgtc tgctctgacc ttcggtaacc tgctgcgtga gatcttcgac 2100
   gtcgatgtcc cggtcggcgt gatcgtcagc ccggcaaatg atctggcggc gattgcgagc 2160
   tatattgaag cagaacgtca gggtagcaaa cgtccgacct ttgcgagcgt ccatggtcgc 2220
   gacgcgaccg ttgttcgtgc tgccgacttg accctggaca agtttttgga cgctgaaacg 2280
   ttggctgcgg caccgaacct gccgaagccg gcgaccgagg ttcgcaccgt tctgctgacc 2340
   ggtgctactg gtttcctggg tcgttacctg gctctggagt ggctggagcg tatggatatg 2400
   gttgacggta aggtcattgc attggtgcgt gcgcgttctg atgaagaggc ccgtgcccgt 2460
   ctggataaga cctttgacag cggtgacccg aaactgctgg cgcactatca gcagctggcg 2520
   gcagaccacc tggaagtgat cgccggtgac aagggtgagg ccaatctggg tctgggtcag 2580
   gacgtgtggc aacgtctggc ggacaccgtt gatgtcattg tggacccggc agcgctggtg 2640
   aaccacgtcc tgccgtactc cgaactgttc ggtccgaatg cgttgggcac tgcagagctg 2700
   atccgtctgg cactgaccag caagcagaaa ccgtatactt acgttagcac catcggcgtc 2760
   ggcgaccaaa tcgaaccggg taaattcgtt gaaaatgcag atattcgtca aatgtcggcg 2820
   acccgtgcga ttaatgacag ctacgcgaac ggctatggca actccaagtg ggcgggtgag 2880
   gttctgctgc gcgaggcgca tgatctgtgc ggtctgccgg tagcggtgtt ccgttgcgac 2940
   atgatcctgg cagacacgac ctacgcaggt cagttgaatc tgccggatat gttcacgcgc 3000
   ctgatgttga gcctggtagc gacgggtatt gcaccgggtt ctttctatga gttggatgcc 3060
   gatggcaatc gccagcgcgc acactatgac ggcctgccgg tcgagtttat cgcagcggcg 3120
   attagcacgc tgggttccca gatcaccgat agcgataccg gcttccagac gtaccacgtc 3180
   atgaatcctt acgacgacgg cgtcggcctg gacgagtacg tcgactggct ggttgatgcg 3240
   ggttattcga ttgagcgtat tgccgactac tctgaatggt tgcgtcgctt cgaaaccagc 3300
   ctgcgtgcgc tgccggatcg tcaacgccag tatagcctgc tgccactgtt gcacaactac 3360
   cgtaccccgg agaaaccgat taacggcagc atcgccccga cggatgtttt ccgtgcggca     3420
   gtccaggaag cgaagatcgg tccagacaag gacatcccgc acgtgtcccc gccagtgatc     3480
   gtgaaataca ttaccgacct gcagctgttg ggcctgctgt aa                        3522
<210> 8
   <211> 3435
   <212> DNA
   <213> Artificial Sequence
<220>
   <223> synthetic gene
<400> 8
   atgaatgaaa gcagcgcaga ccagagcagc ggtaatgtat cagagggttg gccggacgcg        60
   agcgtcaccg cgcgtgcgct gcaagcgcac ctgcgttacg aacagatcat cgacgctatc       120
   ctgagcggct atgcggaacg tccggcactg gcggagcgca gctacctggt ccgtccagat       180
   ccgagcacgg gccaaaccgt tcgcgtccac gagcaggcgt ttcgttctat cagctaccgt       240
   acgctgcaag aacgcgtcca cgccctgacc atggcgtggc gtctgcaccc ggactccccg       300
   gtacaagcgg gtgcattcgt cgtgctggtt ggctttgcca gcatcgatta tgcggttctg       360
   gacctggccc tggcctatac caagggtgtc ccggttccgc tgagcccgaa ccactctagc       420
   gaggacgatg acgcaatctt gggcaccgtg caaccggtga ccctggccgt ttctatctct       480
   gagttctccg gctgcgtgga tctgattgct cgtagcacca gcatccgtac ggttattgtg       540
   tttgatttgg atccggcggt ggattgtgag cgtgcagccc tggagtccgg tattcgtgcg       600
   ctgaacgaga agggtagcga tgtcgttgtg caaaccctgc aagacctgat cgatgtcggc       660
   cgtgatgcgg agtttagctt tctgcctatt caggcgcaag accaggatga tttggctctg       720
   ctgattcata ccagcggtag caccggcacg ccgaagggcg cttgcatcag cagccgtgcc       780
   ctgatcaaca cctggcgtca cgtcagcggt ccgtatccta aagtgaccgt cgttctggcg       840
   ccgtttcacc acatgatggg tcgtgacagc atgattaccg ccctgggtgc aggcggcacc       900
   gcgtacttca cgttgcgtcc ggatctgagc actgttattg aggacatccg cctggcacgc       960
   ccgaccggcc tggtgctgtt cccgcgtctg tgcgaggtga tcgagcacca tctgacgacc      1020
   gctccggagt actctggtaa cgagatcctg ggtggccgtc tgcagtccat tgtcgtagcg      1080
   agcgctccga ttactccgcg tctgaaagcg agcttggagt gtttgctggg cgttccggtg      1140
   agcgagggtt attccagcac ggagacggca agcggtggtt tggcaatgaa cggcctgttg      1200
   aatcgcaaca atattctggc gtaccgcttg cgtgacgttc cggaagcggg ttactctgtt      1260
   aacgaccgcc cgtttccgcg tggcgaactg tgtgttaaga cgcgctttgg tattagcggc      1320
   tattttcgca atccggaagc gaccgcggaa ctgttcgacg atgacggttt ctactgcacg      1380
   ggtgacattg ttgaagaacg tgcaccagat cagatcgcga tcattgaccg ccgcaaaaac      1440
   gtcattaaac tggctcaggg cgagtacgtg gctgtgggtc gtctggagca attgtttcaa 1500
   gaaggctgcg gttgcgtgca acagattcat ctgcatggtg acagcacccg tgcatacctg 1560
   ctggcggtcg ttgttccgga ccgcaatacc ttggcgccac ctggttcgcg tcaggcgtct 1620
   gaggcagagt tgaaagcgcg cgtccgcgaa gagattctga ccctggcaaa tcagcgcgag 1680
   ctgcgtggct ttgaaatccc gcgtgacctg attctggcgg aagaaccgtt cagccaacag 1740
   aatggcctgc tgagcagctt gggtaaaccg atccgtccgg ctatccgtgc acgctaccgt 1800
   agccgtctgg aaagcctgta tgcgagccat gaagccaccc gtggtaccga gctggaagca 1860
   atccgtgcgt ccgcaggtgc cgttgatgtg gaaaccacgc tgttggccct gctgtctagc 1920
   actctgggcg tggtctgtgg tgccgcggat cgccagacct cttttcgcga gctgggtggc 1980
   gacagcctgg ccgcagtgca gctggccatg gagatcaaaa agcagttcgg tgtgggcctg 2040
   gaaggtagcc aaatcctggg tccgggtggt acggtcgagg cgtgggcccg tcgcatccac 2100
   accgcgagca ttcagcaggc accgcaccaa cgcgtgggca gcccgctggc tgcaattccg 2160
   gccgaaggct ggctgaaacc ggatcactat cgtctggaga acttgatcgg cattccgatt 2220
   ggtaccccgt ccgcagaggt tgctcgcccg actggcggtc cgccgaccgt gctgctgacg 2280
   ggtgcgactg gtttcttggg tggccgcctg tgcctggaat ggctgcaacg tctggcgggt 2340
   cagggtggtc gcctgatctg tctggtgcgt cctagcaaca gccacagcgc ctgggaacgt 2400
   ctgcgcaacc gcttcagcca cctggagccg gagcaggttg cgcgttttcg tgaactggca 2460
   ggccgccatc tggaagttat tccggcagat atcggcgagc cgggtttggg cctggagcct 2520
   ggttgtcaag agcgtctggc caccgaggtc gacgcgatct gtcattgtgc agccgaggtt 2580
   aaccatcgtc tgccgtaccg tcacctgtat cgtccgaatg tgattggtac cgcagagatc 2640
   attcatctgg ctattactac ccgtctgaag agcgtggact tcatttcgag catcggtgtg 2700
   gcgagcctgc cgcgtcgtcc gggtggctcg attccggtcg agggcggtta cgcacgcggc 2760
   tatttcgctt ccaagtgggc gtgcgaacaa ctgctgcgta gcacccatga ttgcaccggt 2820
   gttccggtgc gtgtcattcg tccgagcctg attctgccag accgtgtcct ggccggtgaa 2880
   atgaatcctg acgacctgct gtctcgcttg ctgtactcga ttctggttac gggtatcgcg 2940
   ccaggctgct tcggtgaaga gagccagaac agcggtcgca gcggtttcag cgttcaaggt 3000
   ctgccggtgg atcagctggc gcagacgatc ctggcactgg gcgaggcacg cacggaaggc 3060
   ttccacgttt tgaatctgaa tgcggactcc ggtagcggtg tcccgttgga tgcgattttg 3120
   caagacatcg cggcgaaagg tattcgtctg cgtcgtgtcg agggttacga tctgtggctg 3180
   gacgcaatta ccacgcgcct gcgtcgtttg ccagcggaac agcgtgcgcg cagcctgttg 3240
   gatgtggcag aggcgtacgc cggcagcgct ggccagacga cgcaatccag cggtgagatg 3300
   caagcgggta gctcgtcctg cccagaagaa atcaccagcc tgcagccgga cttcagccgt 3360
   gcctatcgtc gtaagatcgt tgacgatctg gcgcgctggg gtctgatcga accaccgggt 3420
   ccggttgatc agtaa 3435
<210> 9
   <211> 3492
   <212> DNA
   <213> Artificial Sequence
<220>
   <223> synthetic gene
<400> 9
   atgacggcag gcgcagcagc aagagtggct aaattatttg aatccgaccc tcaatttcgt      60
   gcggcgatgc cggacccggc agtgatggat agcctgctgg caccgggttt gcgcctgtcc     120
   caggtgctgc acgccctgct gtcgggttac gcggagcgtc cggtgatggg tttccgtagc     180
   cgtgaaagcg ttgtagatac ggccaccggc cgtactgtcg accgtctgct gccggcattt     240
   gaaacgatta cgtatggcca gttgctggaa gatatcagcg ctatcctggc cgagtggcag     300
   cacggtgata tcccgatggg tgctggtgac ttcatcgcga cgattggctt tagcagcccg     360
   gactatgtca ctctggacct ggcgaccctg atgaacggca gcgtcagcat cccgctgcaa     420
   cataacacca gcgtcgcgca gctgcgtatg atgctggaag agacgagccc gcgtctggtt     480
   gccgcgagcg cggactgttt ggatttggct gttgaagcgg ctgttggctt gacggacctg     540
   cgccgtgtgg ttgtgttcga ctaccgtgcg gagacggacg accatcgtga gaaactggcg     600
   accgcgcgtg aacgtctgca cgcagcgggt atggacgtgg tcgtagagcc gctggcggaa     660
   gtcatcggcc gtggtcgcga tctgccggag ccggttctgt acaccgccgg tgatgaccaa     720
   cgcacggcgt tgattatgta cacgagcggt tccacgggtg ccccgaaagg cgcgatgttt     780
   acggagtgga ccgttacccg tttttggagc tccggtgcgg caccgaaccg cgatacgccg     840
   attatcaatg tcaatttcct gccgctgaac catctggcgg gtcgcgtcgg tctgctgacc     900
   gcgttcattc cgggtggcac ctgctacttc gtcccggaga gcgatttgtc caccctgttt     960
   gaggattggc agctggctcg cccgacgcac atgggcgtcg tgcctcgcgt cgtggacatg    1020
   ctgttccagc actatcagac ccgtgtggac gcgctgatgg ccggtggtac cgatgttgac    1080
   accgccgacc gtctggctaa gaccgaattg cgcgaggatg ttttgggtgg ccgtgtagtt    1140
   gcgggtatgt tggcgaccgc tccgctgagc cctgagatga aagcgttcct ggagtcctcg    1200
   ttggactttc acctgctgga cctgtatggt ttgaccgagg ttggcggcgt tttccgtgat    1260
   ggcaagatca gccgtccgcc ggtgctggat tacaaactgg tcgacgttcc ggaactgggt    1320
   tactatacga cggataaacc gcatccacgt ggcgaactgc tggtgaagtc tgcgaccgcg    1380
   accccaggct attacaaacg tccagacgtt acggccgagg tctttgacgc cgatggttac    1440
   tatcgtactg gcgacgtgat ggcagaggtc gcgccagacc agctggtgta cgttgatcgt 1500
   cgcaataatg tgattaagtt ggctcagggc gagttcgttg cggttgcgaa tctggagact 1560
   gtttacgtgg gtgcgccgct ggtgcgtcag atttttgttt acggtaacag cgaacgtgcc 1620
   tatctgctgg ccgttgtggt gccgaccgaa gaggcgctgc gcgcacaccc ggatccggtt 1680
   gagctgaaga acagcattcg tgaatccctg cagcgtaccg cgcgtagcaa ccacctgcac 1740
   tcttatgagc tgccggcaga cttcattatt gaaaccaccc cgttcacgat cgaaagcggc 1800
   atgctggctg cggtgggcaa accgatccgc ccgaagatga ttgaacatta tggtgatcgc 1860
   ctggagcagc tgtacgtgga tctggcagag gcccgtgttc aggagttgcg tcagctgcgt 1920
   gacaccgcac aacaacgccc ggttttggat accgttactg aggcagcgca ggcactgctg 1980
   ggcatgagcg ccgacgctgt gcgtccggat catcatttca tcgatctggg tggtgacagc 2040
   ctgagcgcgc tgaccttcag caatctgctg cgcgacctgt tcgacgtcga ggtgccggtg 2100
   ggcgtgatta ccggtccggc agcggatttg cgtaaactgg cggcgtatat tcaacatgaa 2160
   cgcgaacact ctaccgctac cgcagcgagc gttcacggtt tggacactac cgttatcagc 2220
   gcaaccgagc tgacgttgga taagttcatc gatgctgaaa ccttgcacaa cgcgagccaa 2280
   ctggacgtcc ctgccggtgc ggtcgcaacg gtcctgctga ccggtgcgaa tggttatctg 2340
   ggtcgctttc tgtgcctgga atggctgcag cgcctgagcc aaactggtgg tcaactgatc 2400
   tgtctggttc gtggtgacaa cgcggatcag gccctggcgc gtctggtcgc ggcatatggc 2460
   gacaccgacc gtaccctgct ggaagaattt cacaccctgg cacgtcgcca tctgcgtgtt 2520
   attgcggcag acattgcgca accgcgtttc ggcgttgacg atgctacgtg ggagcaactg 2580
   gcacgcgatg tggataagat cgttcacccg gctgccctgg tgaatcacgt gctgccgtac 2640
   aatcaattgt ttggcccaaa cgtatttggt accgccgagg tgatccgtct ggcgttgacc 2700
   acgcgcatta agccggttac gtacctgagc accatggccg tggcgatgac cgtcccggac 2760
   ttcgatgagg acggtgacat ccgcacggtg tctccgacgc gtcatatcga cccgggctat 2820
   gccaatggct acgcaaattc gaaatgggca ggtgaagtcc tgctgcgtga ggcacatgac 2880
   atttgcggcc tgccggttag cgtttttcgt agcgatatga ttctgaccca ccgtcgttat 2940
   agcggtcagc tgaacgtcac cgatgcgttt acgcgtatgc tgctgtctct ggttctgacc 3000
   ggtatcgccc ctcgcagctt ttatcaaggt gatggttccg gtgcgcgtcc acgcgcccac 3060
   tacgaaggtc tgccggtgga ctttgtcacc gaggccatta ccagcctggg tctgagcagc 3120
   tccgagggct tccgtagcta cgatgtcatg aatccgcacg atgatggtat tagcgtcgat 3180
   accttcgtcg attggctgat ggaagatggt cacagcattg acatcattga caactacgac 3240
   gagtggctgt ctcgcttcga aactgcactg cgcggcttgc cggatgagca acgtcgcgcg 3300
   agcgtcctgc cgttgctgga cgcgtaccgc attccgggca accctcgccg tgctgcagcg 3360
   accccgaacc acgtgttccg caaggcagtg caggagaaca atatcggcgg tgacggtgca 3420
   gacatcccgc aaattgatcg cgcgctgatc gcaaaataca tcgcggatct gcgtgcgcat 3480
   cgtctgctgt aa 3492
<210> 10
   <211> 3453
   <212> DNA
   <213> Artificial Sequence
<220>
   <223> synthetic gene
<400> 10
   atgtttgcag aggatgaaca agtgaaggcg gcggtaccag atcaggaagt tgtggaagcg      60
   atccgcgcac cgggtctgcg cctggctcaa atcatggcga cggtgatgga gcgctacgcc     120
   gaccgtccgg ccgttggtca acgtgcttct gagccggtta ccgagagcgg ccgtacgacg     180
   tttcgtctgc tgccggagtt tgagacgttg acctaccgtg aactgtgggc gcgtgtccgt     240
   gcagtcgccg cagcttggca cggtgacgct gagcgtccac tgcgcgcagg tgacttcgtc     300
   gcgctgctgg gctttgccgg tatcgactac ggcaccctgg acttggcgaa catccacctg     360
   ggcttggtta ccgtcccgct gcagtctggc gcgaccgctc cgcaactggc ggcgatcctg     420
   gccgaaacga ccccgcgtgt cttggcggcg acgccggatc atctggatat tgcggttgag     480
   ttgctgactg gtggcgcgag cccggaacgc ttggttgtgt tcgactaccg tccggcagat     540
   gatgaccacc gtgcagcatt ggagagcgca cgtcgccgtt tgagcgatgc aggcagcgcg     600
   gtcgttgttg aaaccttgga cgcggtgcgt gctcgtggtt ccgaactgcc ggcagcaccg     660
   ctgttcgtcc cggctgcgga tgaagatccg ttggcgctgc tgatttacac gagcggtagc     720
   accggcaccc cgaaaggcgc aatgtatacc gagcgcctga atcgtacgac ctggctgagc     780
   ggtgcgaagg gcgtgggcct gacgttgggt tatatgccga tgagccatat cgcgggtcgt     840
   gcgagcttcg cgggtgtgct ggcgcgtggt ggtaccgtct actttaccgc ccgtagcgat     900
   atgagcactc tgtttgagga tttggcgctg gtccgtccga cggagatgtt cttcgttccg     960
   cgcgtgtgcg acatgatctt tcagcgttat caagcggagt tgtcgcgtcg tgcgccagcc    1020
   gcagcggcaa gccctgaact ggaacaggaa ctgaaaaccg agctgcgtct gtccgcggtt    1080
   ggcgaccgcc tgctgggtgc gattgcgggt tctgcaccgc tgagcgcgga gatgcgtgag    1140
   tttatggagt ccttgctgga tctggagttg catgacggct acggttccac cgaggccggc    1200
   atcggtgtcc tgcaagacaa tatcgttcaa cgtccgccgg ttattgatta caagctggtg    1260
   gacgttccgg agttgggcta tttccgtacc gaccagccgc accctcgcgg tgaactgctg    1320
   ctgaaaacgg aaggtatgat tccgggttat ttccgtcgcc cggaagttac cgcggagatt    1380
   ttcgatgagg acggcttcta tcgtacgggt gatatcgttg cggaactgga gccagaccgc 1440
   ctgatctatc tggaccgtcg caataacgta ctgaagctgg cgcaaggcga gtttgtcacg 1500
   gtggcccatc tggaagcagt gttcgcgacg tctccgctga tccgtcagat ctacatctat 1560
   ggtaacagcg agcgtagctt tttgctggca gtcattgtgc cgaccgcgga cgcgctggcg 1620
   gacggtgtga ccgatgccct gaacaccgct ctgactgagt cgctgcgcca gttggcgaaa 1680
   gaagccggtc tgcagagcta tgaactgccg cgtgagttcc tggtcgagac ggagccgttc 1740
   accgttgaga atggtctgct gagcggtatt gcgaaactgc tgcgtccgaa gctgaaagaa 1800
   cactacggtg aacgcctgga gcagttgtat cgcgacattg aagccaatcg caacgatgaa 1860
   ctgatcgagc tgcgccgtac cgcagcggag ttgccggttc tggaaactgt gacgcgtgcg 1920
   gcacgcagca tgctgggtct ggccgcgagc gaactgcgtc cagacgcgca ttttaccgac 1980
   ctgggtggtg actctctgtc ggcgctgtcc tttagcaccc tgctgcaaga tatgttggaa 2040
   gttgaggtgc cggtgggcgt tattgtgtct ccggcaaaca gcctggctga cctggccaag 2100
   tatattgagg cggaacgtca ctccggtgtc cgccgtccga gcctgatcag cgtgcacggc 2160
   ccaggtaccg agattcgcgc tgcagatctg acgctggaca aattcattga cgagcgcacc 2220
   ctggcagcgg cgaaggcagt tccggctgca cctgcccaag cacagaccgt gctgctgacc 2280
   ggtgcaaatg gttacctggg tcgtttcttg tgcctggaat ggctgcagcg tctggatcaa 2340
   accggcggta cgctggtctg tattgtgcgc ggcaccgatg cggcagcagc gcgtaagcgc 2400
   ctggatgcgg ttttcgacag cggtgatcct gagctgctgg atcactaccg taagctggca 2460
   gccgagcacc tggaagttct ggcgggtgac atcggtgacc cgaacctggg tctggatgag 2520
   gccacgtggc agcgcctggc cgcgaccgtg gatctgattg tgcatccggc tgctttggtt 2580
   aatcacgtgc tgccgtacag ccagctgttc ggtccgaacg tcgtcggcac cgcagagatt 2640
   attcgcctgg cgattaccga gcgtcgtaaa ccggtgacct acctgagcac tgtcgcggtt 2700
   gccgcccaag tggaccctgc gggcttcgat gaagaacgtg acatccgcga gatgagcgcg 2760
   gtccgtagca ttgacgcggg ctacgcgaac ggctatggta atagcaaatg ggctggtgag 2820
   gttctgctgc gcgaggcaca cgatctgtgt ggtctgccgg tggccgtatt ccgcagcgat 2880
   atgattctgg cacactccaa atacgttggc cagctgaacg tgccggacgt ttttacgcgt 2940
   ctgattctga gcctggccct gacgggcatc gctccgtata gcttttacgg tactgacagc 3000
   gccggtcaac gtcgtcgtgc acactatgac ggcctgccgg cagatttcgt cgccgaagct 3060
   attaccacgc tgggcgctcg cgcagaaagc ggctttcaca cctacgacgt ttggaacccg 3120
   tacgacgacg gtatttccct ggacgaattt gtggactggc tgggcgattt tggcgttccg 3180
   attcaacgta tcgatgatta tgacgagtgg ttccgccgtt tcgaaaccgc gatccgtgcg 3240
   ctgccggaga aacagcgcga cgcgagcctg ctgccgctgc tggatgccca tcgtcgtcca 3300
   ttgcgtgcag tgcgtggtag cctgttgccg gcgaagaatt ttcaggctgc ggtccagtcg 3360
   gcacgcatcg gtccggacca ggatatcccg catctgagcc cgcaactgat cgataagtac 3420
   gtcaccgatt tgcgtcattt gggcctgctg taa 3453
<210> 11
   <211> 3567
   <212> DNA
   <213> Artificial Sequence
<220>
   <223> synthetic gene
<400> 11
   atgacggata cggttacgga cagcggtcgc gagcagagac tgacggaaag agttgaacag 60
   ctgtatgcga atgacccgca gtttcgtgca gccgctccga gccctgaggt taccgaggca 120
   gcgcaccgtg ccggtctgcg tctggcagag gttgtggaca tttacctgtc cggttatgcg 180
   gaccgtccgg cgctgggcca acgtgcgtgc gaagtggcac gcgatccggc gacgggtcgt 240
   gctgccacca gcctgttgag cggttttgag actatcacct accgcgaact gggtgatcgt 300
   gtcgccgcct tggcggctgc gtggcgttcc ggcctgccgg gtggtttgcg tccgggtgat 360
   ttcgtcggtg ttctgggctt tacgtccatc gattatgtcg tgcactacct ggcttgcatc 420
   cgtctgggtg cggtgtttgt gccgctgcaa acgagcagca ccgccgcaca actggcgccg 480
   attgtggcgg agacggcgcc tcgcatcctg gcggtatctg tggagtccct ggcgaccgca 540
   gttgacgtcg ttctggaagc gccgagcgtt cagcgtctgg tggtctttga ttacacctct 600
   gatgacgatg agcagcgcgg tcgttacgat gatgcacgcg ctcgtctgcg cgacgcaggt 660
   cacggtgcgg agatggtggc gttggctgct gagttggcaa gcggccgtga acgtcctgcg 720
   ccggaagcgc acgtcccagg tcagggtgaa aacccgctgg caactctgat ctataccagc 780
   ggtagcaccg gcaccccaaa gggtgcaatg tacaccgcgg acatgatgac gcgtatctgg 840
   caacgtcctc actccccgag cgtcgacatc ggtcgtgtca ttccggcgat ccacctgcag 900
   tatatgccgc tgtcccacgt ctacggtctg gagtggctga tcgcgaccct gagctccggt 960
   ggcattggtt atttcgcagc caaaagcgac atgagcaccc tgttcgacga cattggtctg 1020
   gtccgtccga cggcgctgaa cctggttccg cgcgtgtgtg atatgttttt ccgtcgctac 1080
   cgtaaagagc tggaccaacg tgcaggtgac ggtctgaccg cagagcagcg cgatgaagcc 1140
   gttaaagccg aactgcgcca ggatttcatt ggtggccgtg tcattagcgc gatgtgcggt 1200
   agcgcaccgt tgagcaagca gatgcatgcg ttcatggaga gcctgctgga tgtgaccgtt 1260
   gcggatggct atggcgccac cgaaacgggt ggcggcatta tgcgttcggg tcgcatccgt 1320
   cgtccgccgg tcactgatta caagctggtg gacgtgccgg aactgggtta cctgacgacc 1380
   gacaagccgt acccgcgtgg cgagttgcac ctgaaggcga gcaacgttat cccgggttac 1440
   tttaagcatc cggaactgag cgcgcagatt tttgatgatg aaggtttcta caaaacgggt 1500
   gacattatgg cggaactggg ccctgaccat ctgatgtacc tggaccgtag caacaacgtg 1560
   atcaagctga gccagggcga gtttgtggcg gtgagccagc tggaagccac gttttcgacc 1620
   agcccgtaca ttcgtcagat tttcctgtat ggcagctctg aacaaccgtt tctgctggcg 1680
   gttatcgtcc cgaatgttga tgcagtcggt ggtggcgatg cgcgtgcgtt gatcgccgag 1740
   agcctgcagc agattgcggc ggacagctac ctgcacccgt atgaggtgcc acgcgacttc 1800
   ctgctggagc cgcaacgttt tacccgcgat aacggcttgc tgtctggtgt tggtaaactg 1860
   ctgcgtccgg cactgaaagc gcgttacggc gaacgtttgg acgcaatgta tgatgagatt 1920
   gaggcatctc acggtaatca actggacgaa ttgcgtagcg cgagccgcga gttgccgacg 1980
   attgacaccg ttcgccgtgc cgctgccgcg accttgggcc tgccggctga tgccgccctg 2040
   cgtggcgacg cgaagtttat tgagctgggt ggcgattccc tgagcgcatt cagctttgcg 2100
   accctgctgt cggaaatctt tcacatcgac gtgccggtgc aaactatcgt tagcccgacc 2160
   gccaccttgg cgaccatcgc gaattatgtt gacggtgaac gcactagcga atcgacgcgt 2220
   ccgaccttcg ctagcgtgca tggtcgtggt gcaaccgtgg cacgtgccgc tgacttgacg 2280
   ctggcgaagt tcatcgatga cgatacgctg gcagcagcgc cgcacctgcc ggctccaacg 2340
   ggtgcggtca acactgtgct gctgaccggc gcaaatggct acctgggccg cttcctgtgc 2400
   ttggactggc tggaacgcct ggccccgacc ggtggcaccg taatttgtct ggcgcgtggc 2460
   gccgatccga cggcaggtcg ccaacgtatt gaggccgcta tcgacagcgg cgatgcggaa 2520
   ttgagccgtc gtttctgtca gctggcggac aaacacctgc aagttctggt gggtgatgtg 2580
   ggtgctgcga acctgggcct ggacaccccg acgtatcaac gcctggcgcg cagcgttgat 2640
   ttggtcgtcc acagcgcagc cctggttaat catgtgctgc cgtatagcca attgttcggt 2700
   ccgaacgtgg ttggtaccgc agagatcgtc aagctggcga tttccgaacg cctgaaaccg 2760
   attaactaca tttccacggt tgcggtcacc accctgccgg acggcagctt catcggcgag 2820
   gacgcggacg tccgttctgc atctccgagc cgtagcctgg acgagagcta cgctagcggt 2880
   tatgcgacca gcaaatgggc aggcgaggtt ctgctgcgcg aggcgcacga cctgtgcggt 2940
   gtgccggttg cagtttttcg tagcgacatg attctggccc atagcgactt cgcgggtcag 3000
   ttgaacgtgc cggatatgtt cacccgcctg atcttgagcc tggttgcgac gggtatcgca 3060
   ccacgtagct tctatcagct ggacgcgagc ggtaatcgtc agcgtgcgca ttacgacggc 3120
   ctgccagcgg atttcactgc tgaggcaatc acgacgctgg gtgcacgtac ccgccaaggc 3180
   taccatacct ataatgtgct gaatacccac gatgatggcg tctccctgga cacctttgtt 3240
   gattggctga ttgccgatgg ccacaaaatc gagcgcgtgg acgactacga cgagtggctg      3300
   gcgcgtttta ccgctgcgat gaaatctctg ccggataatc agcgcaaaag cagcctgctg      3360
   ccactgatga gcgcgtacgc gaaaccgggc caacctaccc atggcaccgg tatgccggct      3420
   gagaagttcc gcgcagcggt ccaaagcgct ggtattggtg ccactcgtga tgtcccgcat      3480
   gttacccagg ccctgattga caagtatgtt gcggatctgc aacgtttggg cctgctgcgt      3540
   acccgcgctg gtgcccatgc aggctaa                                          3567
<210> 12
   <211> 3468
   <212> DNA
   <213> Artificial Sequence
<220>
   <223> synthetic gene
<400> 12
   atggagagca ctagagcaac ccgcctgaga cagagaatcg cagccctgta cgcagatgac 60
   gcgcaggtcc gtgacgcacg tccagacgaa gctatctcta ccgcgctgcg cgaaccgggc 120
   ttgcgtttgc gcgagctggt cgcgaccgtc gttgatggtt atcgtgaccg tccggctttg 180
   gcggcacgta gcgtccaacc ggctgtcgac gcggcgaccg gtgcgtgcgt ggcgcgtctg 240
   ttgccggagt acaccacgat gagctacggt gagctgggcc tgcgcctgcg tgccgttgcg 300
   gcagcgtggc agcacgacga tgagacgcgc ttgcgtccag gcgaatttgt tgcgacgttg 360
   ggttttacgt ccccggatta cgcagtggtg gacctggcgt gcgtgtgggc gggtgcggtt 420
   gcagtaccgt tgcaagcgag cgccagcgtg acccagctga cggcaattct ggcagaaacc 480
   gcaccagcga tcctggccac cggtctggat accctgccgc atgccgtgga ttgtgtcctg 540
   gcgggtgcaa cgccgcgtgc gctgcacgtg ttcgacttcg atccggcaat tgatgcgcag 600
   cgcaccgttt acgaagcagc atgcgcacgc ttggcgggca ccggcgtccg cgttcgcacg 660
   ttggcggaag ttgaggaccg tggtcgtgcc ctgccaccgg cggttattga tgatggtccg 720
   ggtgacgacc gcctggcgtt gctgatctat acgagcggta gcaccggtac gccgaagggc 780
   gcaatgtata ctgagcgctt ggttgctctg atgtggctgg gccagccgca agttgcagcg 840
   ctgacggtca attatctgcc gctgagccac gtcgcaggcc gtctggcgct gttcggcctg 900
   ctggcgcgtg gcggcaccgc gtatttcacc gcccgtgcag acatgtccac cctgtttgag 960
   gatctggctc tggcgcgtcc gaccgagctg tttgtcgtac cgcgtgtgtg cgaaatggtt 1020
   ctgcaacgtt tccaaactga gcgtctgcgt cgccaagcgg acgatgatcg cgtgaaggca 1080
   gatctgcgcc tggagctgtt cggtgaccgc ctgttgagcg ttgtttgtgg tagcgcacca 1140
   ctggctcctg aactgaaagc gttcatggag agcgtgctgg acctgacctt gcacgacggt 1200
   tatggctcca ccgaagcagg tggtagcgtc gtcatcgaca ccaccgtgcg tcgtccgccg 1260
   gttctggact atcgtctggc ggacgtgccg gagctgggtt actttcgtac ggataaaccg 1320
   catccgcgtg gcgagctgct gctgaaaacg accaccatga tcccgggcta ctaccgtcgc 1380
   ccggagctga atgctcaaat ctttgatgag gacggttttt accgcaccgg cgacgtggtt 1440
   gcggagctgg caccggatcg cctggtgtac gttgaccgtc gcaataacgt tctgaagctg 1500
   gcgcagggtg aattcgtgac gattgcacgt ttggaagcga ttttcgcgaa cagcccgttg 1560
   gtccgccaaa tctttgttta cggtaacagc gaacgtgcct atctgctggc cgtgattgtt 1620
   ccgtctcgtc aagcaatggc tggcgacccg gcgaccctga aaacccgtat cgcggagagc 1680
   ctgcagctga ttggtcgcga cgcggagctg gaagcgtacg agatccctcg cgatttcctg 1740
   atcgagacgg agccgttcac cacggaatcc ggcctgctgt ccggtattgg taaaatcctg 1800
   cgtccggcgg tcgaagcacg ctatcgcgat cgtctggagc agctgtacgc ggacctggca 1860
   gcagcgcagc aagacgagtt ggcagccctg cgtcgcgagg caggtcagcg tccggtcctg 1920
   gagactgtga cccgtgccgc agcggcgatt ctgggcggta ccgctagcga cctgtcccct 1980
   gcggcccact ttaccgattt gggtggtgat agcctggcgg cgttggcgct gtcgaatctg 2040
   ctgcgcgaga ttttcgccgt agaggttccg gtcggtgtga ttaccggtcc ggcgaccgat 2100
   ctgcgtggcc tggccgcaca tatcgcggca gaacgtgaaa accgtacgga aacgccgctg 2160
   ttcgatcgtg tccatccgga tcagattttg atccgtgcga ccgacctggc gctggaaaag 2220
   tttttcgatg cggaagagct ggcggcagcc gcgaccgcag caccgccggt agccgagccg 2280
   cgtgtggttt tgctgacggg tgcgaacggt tacctgggtc gcttcctgtg cctggagtgg 2340
   ctggagcgtc tggaccgtgt cgacggccgt ctgatttgtc tggttcgtgg cgcggacgaa 2400
   gcagcggcac tggctcgtct ggaagcggcg ttcgacagcg gcgatcctga gttggtgcgt 2460
   cgctttaaag aactggccca acgtcgtctg accgtggtgg cgggtgatat cggcgagcct 2520
   ggcctgggtc tggcaaccgc cacctggcgc cgtctggccg ctgaggttga acatatcgtt 2580
   caccctgctg ccctggtgaa ccatgtcctg ccgtatcgcc agctgtttgg cccgaacgtt 2640
   gcgggcacgg cggagatcct gcgtctggcc ctgacggagc gccgcaaacc gatcgatttc 2700
   ttgagcacgg ttgccgtcgc tgcgcagatc ccggcagacc gcttcgccga agatggtgac 2760
   attcgtgtga tcagccctac tcgcactgtc gatcgtggct atgcaaatgg ttacggcaat 2820
   agcaagtggg cggcagaagt tctgctgcgt gcggcgcatg accgcttcga tctgccggtg 2880
   gcggtgttcc gtagcgatat gattctggcc catggcagct ttgccggtca attgaatatt 2940
   ccggatgtct ttacccgtct gttgctgtct ctgttggtga ccggcattgc cccagcatct 3000
   tttcacgccg ctacggtcac tggtgaacgt ccgcgtgctc actatgatgg tctgccggct 3060
   gatttcacgg ctgccgcgat caccgccctg ggtgcccgta cggctggctt tcacacgtac 3120
   gacgttctga acccgcacga tgacggtatc agcctggaca ccttcgtgga ctggctgatt 3180
   gaagcgggtc acccgatcga acgtattccg gagcacagcg aatgggtcac ccgctttgag 3240
   acggccctgc acgcgctgcc ggagcgtcag cgtaagcatt cgctgctgcc gctgctgcac 3300
   gcgtatcgtc gtccggttcc ggccctgcgt ggtagcgcgt tgccagctgc agagtttcgt 3360
   gcagcggtgc gtgctgctgg tattaccgcg gacggtgaca ttccgcacct gacccgcgct 3420
   ctgattgaaa agtacgtcgc cgacctgcgc ttgcatggtc tgctgtaa 3468
<210> 13
   <211> 3525
   <212> DNA
   <213> Artificial Sequence
<220>
   <223> synthetic gene
<400> 13
   atgtccccaa ttacccgtga agaacgcctg gagcgccgta ttcaagacct gtacgcgaat     60
   gacccgcagt tcgctgcggc gaagccggtg actgcgatta cggcagcaat tgagcgccca    120
   ggtctgccgc tgccgcagat tatcgaaacc gtgatgaccg gctacgcaga tcgcccagcg    180
   ttggcgcagc gctccgtcga gttcgttacc gacgcgggta ccggtcacac gactctgcgt    240
   ctgctgccgc actttgagac gattagctat ggcgagctgt gggatcgtat tagcgctttg    300
   gcagacgtgc tgagcacgga gcaaaccgtt aaaccgtcgg atcgtgtctg cctgctgggt    360
   ttcaacagcg tcgattacgc cacgatcgac atgaccttgg cgcgcttggg tgcggtcgcc    420
   gtgccgctgc aaacgtccgc agcgatcacg caactgcagc cgatcgtggc tgagacgcag    480
   cctaccatga ttgccgcgag cgtggacgca ttggcagatg caaccgagct ggcactgagc    540
   ggtcaaaccg cgacgcgtgt cctggttttt gaccaccacc gtcaggtgga cgcgcatcgt    600
   gccgcggtcg agtcggcccg tgaacgcctg gcgggcagcg cagtggttga gactctggcg    660
   gaagcaattg cacgtggcga cgttccgcgt ggcgcgagcg cgggttctgc acctggcacg    720
   gatgttagcg atgacagcct ggcactgctg atctacacga gcggttctac cggtgctccg    780
   aaaggtgcga tgtatccgcg tcgcaacgtt gccactttct ggcgtaaacg tacgtggttt    840
   gaaggcggtt atgagccgag cattaccctg aatttcatgc cgatgagcca cgttatgggc    900
   cgtcagatcc tgtatggtac cctgtgtaat ggcggtacgg cgtatttcgt tgttaagagc    960
   gacctgagca cgctgtttga agatctggcc ctggttcgcc cgaccgagct gacctttgtt   1020
   ccgcgcgtct gggatatggt gttcgacgag tttcagagcg aggtggatcg tcgtttggtg   1080
   gatggtgctg accgtgtggc actggaagcg caggttaagg cagagatccg caatgatgtc   1140
   ctgggtggcc gttacaccag cgcgctgacc ggtagcgcgc ctatttccga cgagatgaaa   1200
   gcgtgggttg aagaactgct ggatatgcac ctggtggaag gctacggtag caccgaagcg   1260
   ggcatgattt tgatcgatgg tgcgattcgt cgcccagccg ttctggacta caaactggtt 1320
   gacgtcccag acttgggtta cttcctgacg gatcgtccgc acccgcgtgg tgagctgctg 1380
   gttaagacgg acagcctgtt cccgggctac taccaacgcg ctgaagtcac cgcagacgtc 1440
   tttgacgcgg atggtttcta ccgcaccggt gacatcatgg ccgaggttgg cccggagcaa 1500
   ttcgtgtatt tggaccgccg taataacgtc ctgaaactga gccagggcga attcgtgacc 1560
   gtttccaagc tggaagcggt ctttggcgac agcccgctgg ttcgccaaat ctatatctac 1620
   ggtaactccg cgcgtgcgta tctgctggcg gtgattgtgc cgacccaaga ggccctggac 1680
   gccgtcccgg tggaagagct gaaagcacgt ctgggcgaca gcttgcagga agtggccaag 1740
   gctgcgggcc tgcaatccta cgagatcccg cgtgacttca tcattgagac gaccccgtgg 1800
   actctgcaga atggtctgct gacgggtatt cgcaagctgg cgcgtccgca gctgaagaag 1860
   cactatggtg aattgctgga gcagatctac acggacctgg cgcacggcca agcggacgag 1920
   ctgcgtagcc tgcgtcagag cggtgcagac gcgcctgttc tggtcaccgt ctgccgcgct 1980
   gctgcagcgt tgctgggtgg cagcgcaagc gacgtgcagc cggatgcgca ttttaccgac 2040
   ctgggcggtg atagcctgtc tgcgctgagc tttaccaatt tgttgcacga gatttttgat 2100
   atcgatgttc cggtgggcgt catcgtgtcc ccggcaaacg acttgcaggc gctggcagac 2160
   tacgtagagg ccgcacgcaa accgggtagc agccgtccga ccttcgcttc tgtgcatggt 2220
   gccagcaatg agcaggtcac cgaagtccat gcgggtgatc tgagcctgga caaattcatc 2280
   gatgccgcta ccctggcaga agctccgcgt ttgccggcag caaacaccca agtgcgcacg 2340
   gtgctgttga ccggtgcaac cggttttctg ggtcgttatt tggcgctgga gtggctggag 2400
   cgtatggacc tggtggatgg caaattgatc tgcctggtgc gcgccaagtc cgacactgaa 2460
   gcgcgtgcgc gtctggagaa aaccttcgac agcggtgcgc cggaactgtt ggcgcattat 2520
   cgcgccctgg ctggcgatca cctggaagtt ctggcgggtg acaagggcga ggcggacctg 2580
   ggcctggatc gtcaaacgtg gcagcgtctg gccgacaccg ttgatctgat tgttgatccg 2640
   gcagcgctgg tcaaccatgt cctgccgtac agccagctgt ttggtccgaa tgcgctgggc 2700
   accgcggagc tgttgcgtct ggccctgacc agcaagatca aaccgtacag ctatacgagc 2760
   accattggtg ttgctgacca gatcccgcct agcgcgttca ccgaagatgc agacatccgt 2820
   gttatcagcg ccacccgtgc cgtcgacgat agctatgcca acggctacag caactctaag 2880
   tgggcaggtg aagtactgct gcgtgaagct catgtgctgt gtggtctgcc ggttgccgtt 2940
   tttcgctgtg acatgatctt ggctgatacc acctgggcgg gtcaactgaa cgtgccggat 3000
   atgttcacgc gcatgattct gagcttggcg gcgacgggca ttgcaccggg ttcgttctat 3060
   gagctggctg cggacggtgc ccgtcaacgt gcgcattacg atggcctgcc ggtcgagttt 3120
   attgcagaag ccatctctac cctgggtgcg cagagccagg atggtttcca cacgtatcac      3180
   gtgatgaacc cgtacgatga tggtatcggt ctggatgagt tcgtggattg gctgaacgag      3240
   tctggctgcc ctattcaacg cattgcggat tatggtgatt ggttgcagcg ctttgagact      3300
   gcgctgcgtg ccctgccgga tcgtcaacgt catagctcgc tgctgccgct gctgcacaat      3360
   taccgccaac cggaacgtcc ggtgcgtggt tccattgctc cgaccgaccg ttttcgtgcg      3420
   gcggttcaag aagccaagat cggcccagac aaagatatcc cgcacgttgg tgcgccgatt      3480
   atcgtcaaat acgtcagcga cctgcgcctg ctgggcttgc tgtaa                      3525
<210> 14
   <211> 3483
   <212> DNA
   <213> Artificial Sequence
<220>
   <223> synthetic gene
<400> 14
   atgactgtaa tgcctgcaaa accaccagcc gatgtatccc cacttagccg cgcagcacgt 60
   ctggtcgccg agttgagcgc gcacgatccg cagtatcgtg ccgcaatgcc gctgccggca 120
   gttcgtgaag cggttcgcga ggccgcacgc gaccaagttc tgagccgcac cgttgcaacc 180
   gtcatggcag gttatgcgga ccgtccggcg ctggcacgcc gtgccaccga gccggtgacc 240
   gatccggtta gcggccgtac tagcttgcgt cgtctgccgg agtttacgac ggtcacgtac 300
   ggtgaactgt gggcgcgtgc aggtgcggtg agcgcggagt gggcagccga cacccgcctg 360
   ccgctgagcc caggcgactt tgttgccatc tacggtttca cgagcggcga ctatgttact 420
   gtcgatttgg cctgtctgcg ccatggtgcg gttagcgtcc cgctgcagtc gggtgcgccg 480
   gttgccggcc tggccccgat tctggcgaaa acgggtccga aagtcctggc ggtgtccttg 540
   gaattgctgg accgtggtgt tgaactggcg ttgagcgccg aggttgctcc gcgtctggtg 600
   gtctttgact tccacgccgg tgacgacgct cagcgcgagg cctttgaagc ggcaagcgca 660
   cgcctgaccg cggcaggcca tgcggctccg ctgccgctgg aagcggtgat tgaacgcggt 720
   cgtgcactgc cgccagcgcc actgttcgtt ccgggtccgg acgaagatcc ggttcgtctg 780
   ctgatttaca ccagcggtag caccggtacc ccgaaaggcg ccattcaaac ggagcgcatg 840
   ctgcatcgcg catgggcagg cgcggtgccg attccggacg acgtcgcgtc catcgtcgtg 900
   aattacctgc cgctgtccca cgtcgctggt cgtagctctc tggttgagac gctgcgtcgt 960
   ggcggcatta gctatttcac ggcacatagc gacctgtctg atctgttcga ggacatcgcc 1020
   ctggcgcgtc ctaccgcgct gttgtttgtt ccgcgtgtgt gcgatttgct gttccaagaa 1080
   tatcaggctg aattggcacg tcgcgcaggt gagtttgcgg acgcgggtgc gctggacgcg 1140
   gctgttaagg cagatctgcg tgaacgtttc gttggtggcc gcctgattca agcgctgtac 1200
   ggcagcgcac cgctgtccgc cgaactgcgt gagtttatgc acacctgtct ggacctgcct 1260
   gtgctggacg gttatggtag caccgaaacg ggttccgtgt tgttgaacac ccgcgtgcaa 1320
   cgtccgccgg tcaccgatat caagctggtg gatgtcccgg aactgggtta cttcgccacc 1380
   gattccccgt acccacgcgg cgaattggtg ctgaaaagcg cgaccctgac gcctggctat 1440
   taccgtcgcc cagaggtcac cgcagctgct ttcgacgcgg atggctttta ccgtacgggc 1500
   gacattatgg cggaagttgg cccggaccag tacgtgtacg ttgatcgtcg taataacgtt 1560
   gtgaagctgg cccagggtga gttcgttgcg ctgtctcgtc tggaaggtgt gtatgtgact 1620
   cacccgctga ttcgccagat ttacgtgtac ggcaatagcg agcgtgcaca cttgctggct 1680
   gtgatcgtgc cgacgcgcga gaattgtacg cacgaggatc tggcagccgc gctgcagcag 1740
   gcagcccgtg agagcgagtt gaactcttac gagatcccgc gtgcgttcct ggttgaaacc 1800
   gagccgttct ccctggccaa cggtttgctg agcgacaccc gtaagaatct gccgccacgt 1860
   ctgaaggcgc gctatggcga gcgtctggaa gcgctgtatg aagagctggc acgtgaacaa 1920
   gaggatgcgg ttcgtgtgct gcgcgatgaa ggtacgggtc gccctgtttc ggaaacggtc 1980
   gagcgtgcag cgcgtgcgct gctgggtagc agcgcagcgg acgcgcgttt taccgacttg 2040
   ggcggtgatt ctctgtcggc gttgtccttc agcaccctgc tggcagagat cttcggtgtc 2100
   gaggtgccgg tgggtactgt cctgagcccg gcaaacgacc tgcgtgcatt ggcagcgcac 2160
   atcgaagctc gtcgtgcgtc tggtgcgagc cgtccgacct tcgctagcgt tcatggcgtg 2220
   ggccgtacgg tcgtgcgtgc gggcgacctg gcactggaga agtttctgga tgccggtgcg 2280
   ctggcggaag cggcgcagct gccggcaccg ggtcgtgaaa cgccgcgtac ggtcctgctg 2340
   accggtgcga acggttatct gggtcgtttc atgtgcctgg actggctgga acgtctggct 2400
   tcgggcggtg gccgtctggt gtgcgtggtt cgcggtaaag acgacgccga tgcccgtgca 2460
   cgtctggatg cggcgttcga cagcggtgat ccggagctgc tgcgtcgcta ccgcgaactg 2520
   gcagcgggtc gcctggatgt cttggctggc gatatcggtg cggagcgtct gggtctggcg 2580
   ggtgagacgt ggcgtcgcct ggccgaggat gtggatctga tcgcccaccc ggctgccctg 2640
   gtcaaccacg tcttgccgta cgagcaactg tttggtccga acgttgtagg caccgctgaa 2700
   ctgattcgcc tggcactgac cgcgcgtgtt aaaccgttcg tgtatgttag caccaccgcg 2760
   attagcacca cgctggacga gacgagcgat attcgtgaga gcatcccgga acgcgccctg 2820
   accgatgcat atgcagcggg ctacggtacc agcaaatggg cgggtgaggt gctgctgcgc 2880
   gaggcgcacg ctcgcctggc gctgccggtt gcggtctttc gcagcgactt gattctggcg 2940
   cacccgcatc acaccggcca actgaatccg gccgacgtac tgacccgtct gctgtttagc 3000
   atcctgagca ctggcttggc gccgactagc ttttactctg aagagggtcg cgcgcacttc 3060
   gacggtctgc cggtggattt taccgctgag gctatcaaca ccctgggcgc acagccgatt      3120
   agcgcgcacc gtacctataa tgcggtaaat ccgcatgacg acggcgtgag cctggatacc      3180
   ttcatcactt ggctggaaga ggctggtcac ccgctgcgcc gtttgccgca tacgacctgg      3240
   agcccgcgtc tggaaaccgc cttgcgcagc ctgccggaac atcaccgtcc gcacacgttg      3300
   ctgcctctgc tgcatgcgtt tgcaacccct cagccgccga ccccgaccag cccggttccg      3360
   gccacccatt ttcatgaggc agtgcgtgag gctggcatcg gcccagataa tgatatcccg      3420
   cacatcacgc aaaacttgat taccaagtac gcgacggatc tgcgccaact gggtctgcgc      3480
   taa                                                                    3483
<210> 15
   <211> 3582
   <212> DNA
   <213> Artificial Sequence
<220>
   <223> synthetic gene
<400> 15
   atgactgacg acgcaaagag agcaaaagta tccagcaccg gtccgatttc tgcagcacgt      60
   cagcaagttg cggaccgcat tcgcgatctg gacgcgcgtg acgaagaatt tcgcacgacc     120
   aaaccggact ctgcgctgca gctggcggca cgtgaaccgg gtctgcgtct gccgcaaatc     180
   ctggaaatct ttgccgaagg ttacgcggat cgtccggcgc tgggctggcg tgcgcgcagc     240
   ctgaccaccg atgcggcgac tggccgtacc agcgctcagt tgctgccgcg cttcgacacg     300
   atgacgtacc gtgaactgtg ggcgaatgtc cgcgcaatcg ccggtgcttg gcgccacgat     360
   gctaccaacc cggtggcacc gggtgatgtt gtggcaactg ttggtttcgc gagcgcagaa     420
   tacctgacca tcgatctggt ctgtgcgtat ctgggcctgg ttgccgtgcc gctgcaacat     480
   aacgcgaccg catcccgtct gcgtccgatc gtcgaagagg ttgagccgtc gacgttggcg     540
   gctggcgtgg gctacctgga cttggccgtc gaggcggcct tgggcagctc cagcctgcgc     600
   cgtgtggttg tgtttgatta tcgtccggaa gttgacgaac agcgcgaggc ggttgaccgt     660
   gcgcgtagca aactggcggg tgcaggtatc gcggtcaccg ttgagacgct gggtgacgtc     720
   attgagcgtg gccgtaccct gccgcctgag ccgatgttta ccggcgatac ggacgagcgt     780
   ctggcaatga ttatgtatac cagcggttcc accggtttgc cgaaaggcgc catgtatacc     840
   gagcgtatgc tgtgccgtct gtggaccact gaactgatgc cggatttcgc cgacaccccg     900
   gttatcaatg tcaacttcat gccgctgaat cacctgggtg gtcgtatccc gctgagcacc     960
   gcgttccagg ccggtggtac ctcttatttc gttccggaga gcgatctgag caccctgttc    1020
   gatgattgga atctggtccg tccgaccgag atgggcctgg tgccgcgtgt ggcagaaatg    1080
   ttgtaccagc gttaccagag cgcggtcgat cgctttgaag ttgctggcac ggacacggcg    1140
   acggcacaag cgcgtgctca ggccgaactg cgtgagcagg tgctgggcgg tcgcattgtt 1200
   actgccttct gtggcaccgc accgctggct gccgagatgc gcagcttcat tgagacgtgc 1260
   ctgagcgtgc atgtgctgga cggttacggt ctgacggaag ttggtatggt caccaaggat 1320
   ggtttgatta cgcaaccgcc ggttttggac tataagctga tcgatgttcc ggagctgggt 1380
   tacttcctga ccgacaaacc gtatccgcgt ggcgagctgc tggttaaatc cctgaccgca 1440
   accccgggtt acttcaagcg tccagatgtc accgccaatg cattcgaccc agaaggctat 1500
   taccgtacgg gcgacgtgat ggcggagctg gaacctagcc gtctggccta cgtggatcgt 1560
   cgcaataacg tcctgaagct ggcacaaggt gagttcgttg ccgtcgctcg tctggaagcc 1620
   gtttttagca gcgccgcact ggtgcgtcaa atctttgtgt acggcaacag cgagcgcccg 1680
   tacttgctgg cggtcgtggt accgacggat gacgcagccc aaaagtatgc gggtgacccg 1740
   ggtggtctga aaagcgcgtt gggtgagagc ctgcgccaag cggctaagct ggcagaactg 1800
   caaagctacg aagttccggt tgattttgtt gtggaaaccg agccgtttag cgaggataat 1860
   ggcttgctga gcggtgtcgg caaactgctg cgtccaaaac tgaaagagca ctacggcgca 1920
   cgcctggaac agctgtatgc agacctggct gaaagccgcg ttacggagct gcgtgccctg 1980
   cgtgagggtg ctgctgatca ccctgtgatc ttcacgttga cccgtgcggc ggaagcgctg 2040
   ctgggtttgg ccggtggtcc gcctgcgccg gatgcgttgt tcattgaact gggtggtgat 2100
   agcttgagcg cgctgacgtt tagcaatctg ttgcgtgaca tcttcgacgt tgaggtgccg 2160
   gtcggtatga ttaccggtcc ggcgaccgac ctgggtcagc tggcagagta tgtggaatcc 2220
   gaacgtgcgt ccggcagccg tcgtccgacc tttgcgaccg tccacggtcg cggtgccacg 2280
   caggtgcgcg ctgcagactt gacgttggat aagttcatcg acgcaaccac cctggcagaa 2340
   gcaccggcat tgccgcgtgc gaccggtacc ccgcacactg tgctgctgac cggtgcgaac 2400
   ggctacctgg gccgtttctt gattctggag tggttggaac gtctggcgga gacgggtggc 2460
   aagctgatta gcattattcg tgcagcggac gctgcggcag cagcgaaacg cctggagagc 2520
   gtgttcgaca gcggcgatcc gcaactgctg gagcgtttcc gcacgctggc agcggatcat 2580
   ctggaagtta tcgtcggtga tattggtgag ccaaacctgg gtctgcaaca gggcacctgg 2640
   gagcgcctgg cgcagtcggt cgatttgatt gtccatccag cagcactggt caaccatgtg 2700
   ctgccgtacg accagctgtt cggtccgaat gtcgtcggca ctgcggagct gattcgtttg 2760
   gccatcacca ctcgcattaa acctgtcacg tacatgtcca ccgtggctgt cgcgctgagc 2820
   gttgacccgg ctgccttcgc cgaggacggc gacatccgca cggtttcggc tctgcgtccg 2880
   gttgatggtg gctatgcgaa tggttacgca aactcgaagt gggcgggtga agtactgctg 2940
   cgcgaggcac acgatctgtg cggcctgccg gttgcggttt ttcgcagcga catgatcctg 3000
   gcacatagcc gttacgcagg ccaactgaac gtgccggacg cgtttacgcg tctgatcttt 3060
   agcctgctga cgaccggcat cgcaccgggt agcttttatc aaaccgaccc tcgcggcaac 3120
   cgtgctattg cgcactacga cggtctgcca gcggattttg tcgctgaagc ggttaccacg 3180
   ctgggcgagc agatcgcgac tgccgcgcag gattctggtg catatcgcag ctttgatgtt 3240
   atgaacccgc acgacgatgg catctctctg gacgtgttcg tggattggct gattgcgggt 3300
   ggtcacgata ttcgccgtat cgatgactat gacgagtggc tgagccgctt taccaccgcc 3360
   ctgcgcgcac tgccggacaa acaacgtcag cactccgtcc tgccgctgct ggacgcttat 3420
   cgtaagccgg aaaccccgct gcgtggtgcg cctgcgccta ccgatgtgtt tcgtagcgcg 3480
   gttcgtgagg cgaagattgg tgcggccgag gatatcccac atctgagcgc ggcactgatt 3540
   gacaaatacg tggccgactt gcgtttgctg ggtctggtgt aa 3582
<210> 16
   <211> 9269
   <212> DNA
   <213> Artificial Sequence
<220>
   <223> vector
<400> 16
   ttctcatgtt tgacagctta tcatcgataa gctttaatgc ggtagtttat cacagttaaa      60
   ttgctaacgc agtcaggcac cgtgtatgaa atctaacaat gcgctcatcg tcatcctcgg     120
   caccgtcacc ctggatgctg taggcatagg cttggttatg ccggtactgc cgggcctctt     180
   gcgggatatc gtccattccg acagcatcgc cagtcactat ggcgtgctgc tagcgctata     240
   tgcgttgatg caatttctat gcgcacccgt tctcggagca ctgtccgacc gctttggccg     300
   ccgcccagtc ctgctcgctt cgctacttgg agccactatc gactacgcga tcatggcgac     360
   cacacccgtc ctgtggatcc tctacgccgg acgcatcgtg gccggcatca ccggcgccac     420
   aggtgcggtt gctggcgcct atatcgccga catcaccgat ggggaagatc gggctcgcca     480
   cttcgggctc atgagcgctt gtttcggcgt gggtatggtg gcaggccccg tggccggggg     540
   actgttgggc gccatctcct tgcatgcacc attccttgcg gcggcggtgc tcaacggcct     600
   caacctacta ctgggctgct tcctaatgca ggagtcgcat aagggagagc gtcgaccgat     660
   gcccttgaga gccttcaacc cagtcagctc cttccggtgg gcgcggggca tgactatcgt     720
   cgccgcactt atgactgtct tctttatcat gcaactcgta ggacaggtgc cggcagcgct     780
   ctgggtcatt ttcggcgagg accgctttcg ctggagcgcg acgatgatcg gcctgtcgct     840
   tgcggtattc ggaatcttgc acgccctcgc tcaagccttc gtcactggtc ccgccaccaa     900
   acgtttcggc gagaagcagg ccattatcgc cggcatggcg gccgacgcgc tgggctacgt     960
   cttgctggcg ttcgcgacgc gaggctggat ggccttcccc attatgattc ttctcgcttc    1020
   cggcggcatc gggatgcccg cgttgcaggc catgctgtcc aggcaggtag atgacgacca 1080
   tcagggacag cttcaaggat cgctcgcggc tcttaccagc ctaacttcga tcattggacc 1140
   gctgatcgtc acggcgattt atgccgcctc ggcgagcaca tggaacgggt tggcatggat 1200
   tgtaggcgcc gccctatacc ttgtctgcct ccccgcgttg cgtcgcggtg catggagccg 1260
   ggccacctcg acctgaatgg aagccggcgg cacctcgcta acggattcac cactccaaga 1320
   attggagcca atcaattctt gcggagaact gtgaatgcgc aaaccaaccc ttggcagaac 1380
   atatccatcg cgtccgccat ctccagcagc cgcacgcggc gcatctcggg cagcgttggg 1440
   tcctggccac gggtgcgcat gatcgtgctc ctgtcgttga ggacccggct aggctggcgg 1500
   ggttgcctta ctggttagca gaatgaatca ccgatacgcg agcgaacgtg aagcgactgc 1560
   tgctgcaaaa cgtctgcgac ctgagcaaca acatgaatgg tcttcggttt ccgtgtttcg 1620
   taaagtctgg aaacgcggaa gtcccctacg tgctgctgaa gttgcccgca acagagagtg 1680
   gaaccaaccg gtgataccac gatactatga ctgagagtca acgccatgag cggcctcatt 1740
   tcttattctg agttacaaca gtccgcaccg ctgccggtag ctattgacta tccggctgca 1800
   ctagccctgc gtcagatggc tctgatccaa ggcaaactgc caaaatatct gctggcaccg 1860
   gaagtcagcg ccctgcacca ttatgttccg gatctgcatc gcaggatgct gctggctacc 1920
   ctgtggaaca cctacatctg tattaacgaa gcgctggcat tgaccctgag tgatttttct 1980
   ctggtgccgc cctatccctt tgtgcagctt gccacgctca aaggggtttg aggtccaacc 2040
   gtacgaaaac gtacggtaag aggaaaatta tcgtctgaaa aatcgattag tagacaagaa 2100
   agtccgttaa gtgccaattt tcgattaaaa agacaccgtt ttgatggcgt tttccaatgt 2160
   acattatgtt tcgatatatc agacagttac ttcactaacg tacgttttcg ttctattggc 2220
   cttcagaccc catatcctta atgtccttta tttgctgggg ttatcagatc cccccgacac 2280
   gtttaattaa tgctttctcc gccggagatc gacgcacagc gttctgtgct ctatgatgtt 2340
   atttcttaat aatcatccag gtattctctt tatcaccata cgtagtgcga gtgtccacct 2400
   taacgcaggg ctttccgtca cagcgcgata tgtcagccag cggggctttc ttttgccaga 2460
   ccgcttccat cctctgcatt tcagcaatct ggctataccc gtcattcata aaccacgtaa 2520
   atgccgtcac gcaggaagcc aggacgaaga atatcgtcag tacaagataa atcgcggatt 2580
   tccacgtata gcgtgacatc tcacgacgca tttcatggat catcgctttc gccgtatcgg 2640
   cagcctgatt cagcgcttct gtcgccggtt tctgctgtgc taatccggct tgtttcagtt 2700
   ctttctcaac ctgagtgagc gcggaactca ccgatttcct gacggtgtca gtcatattac 2760
   cggacgcgct gtccagctca cgaatgaccc tgctcagcgt ttcactttgc tgctgtaatt 2820
   gtgatgaggc ggcctgaaac tgttctgtca gagaagtaac acgcttttcc agcgcctgat 2880
   gatgcccgat aagggcggca atttgtttaa tttcgtcgct catacaaaat cctgcctatc 2940
   gtgagaatga ccagccttta tccggcttct gtcgtatctg ttcggcgagt cgctgtcgtt 3000
   ctttctcctg ctgacgctgt ttttccgcca gacgttcgcg ctctctctgc ctttccatct 3060
   cctgatgtat cccctggaac tccgccatcg catcgttaac aagggactga agatcgattt 3120
   cttcctgtat atccttcatg gcatcactga ccagtgcgtt cagcttgtca ggctcttttt 3180
   caaaatcaaa cgttctgccg gaatgggatt cctgctcagg ctctgacttc agctcctgtt 3240
   ttagcgtcag agtatccctc tcgctgaggg cttcccgtaa cgaggtagtc acgtcaatta 3300
   cgctgtcacg ttcatcacgg gactgctgca cctgcctttc agcctccctg cgctcaagaa 3360
   tggcctgtag ctgctcagta tcgaatcgct gaacctgacc cgcgcccaga tgccgctcag 3420
   gctcacggtc aatgccctgc gccttcaggg aacgggaatc aacccggtca gcgtgctgat 3480
   accgttcaag gtgcttattc tggaggtcag cccagcgttc cctctgggca acaaggtatt 3540
   ctttgcgttc ggtcggtgtt tccccgaaac gtgccttttt tgcgccaccg cgctccggct 3600
   ctttggtgtt agcccgttta aaatactgct cagggtcacg gtgaataccg tcattaatgc 3660
   gttcagagaa catgatatgg gcgtggggct gctcgccacc ggctatcgct gctttcggat 3720
   tatggatagc gaactgatag gcatggcggt cgccaatttc ctgttggaca aaatcgcgga 3780
   caagctcaag acgttgttcg ggttttaact cacgcggcag ggcaatctcg atttcacggt 3840
   aggtacagcc gttggcacgt tcagacgtgt cagcggcttt ccagaactcg gacggtttat 3900
   gcgctgccca cgccggcata ttgccggact ccttgtgctc aaggtcggag tctttttcac 3960
   gggcatactt tccctcacgc gcaatataat cggcatgagg agaggcactg ccttttccgc 4020
   cggtttttac gctgagatga taggatgcca tcgtgtttta tcccgctgaa ggcgcgcacc 4080
   gtttctgaac gaagtgaaga aacgtctaag tgcgccctga taaataaaag agttatcagg 4140
   gattgtagtg ggatttgacc tcctctgcca tcactgagca taatcattcc gttagcattc 4200
   aggaggtaaa cagcatgaat aaaagcgaaa aacaggaaca atgggcagca gaaagagtgc 4260
   agtatattcg cggcttaaag tcgccgaatg agcaacagaa acttatgctg atactgacgg 4320
   ataaagcaga taaaacagca caggatatca aaacgctgtc cctgctgatg aaggctgaac 4380
   aggcagcaga gaaagcgcag gaagccagag cgaaagtcat gaacctgata caggcagaaa 4440
   agcgagccga agccagagcc gcccgtaaag cccgtgacca tgctctgtac cagtctgccg 4500
   gattgcttat cctggcgggt ctggttgaca gtaagacggg taagcctgtt gatgataccg 4560
   ctgccttact gggtgcatta gccagtctga atgacctgtc acgggataat ccgaagtggt 4620
   cagactggaa aatcagaggg caggaactgc tgaacagcaa aaagtcagat agcaccacat 4680
   agcagacccg ccataaaacg ccctgagaag cccgtgacgg gcttttcttg tattatgggt 4740
   agtttccttg catgaatcca taaaaggcgc ctgtagtgcc atttaccccc attcactgcc 4800
   agagccgtga gcgcagcgaa ctgaatgtca cgaaaaagac agcgactcag gtgcctgatg 4860
   gtcggagaca aaaggaatat tcagcgattt gcccgagctt gcgagggtgc tacttaagcc 4920
   tttagggttt taaggtctgt tttgtagagg agcaaacagc gtttgcgaca tccttttgta 4980
   atactgcgga actgactaaa gtagtgagtt atacacaggg ctgggatcta ttctttttat 5040
   ctttttttat tctttcttta ttctataaat tataaccact tgaatataaa caaaaaaaac 5100
   acacaaaggt ctagcggaat ttacagaggg tctagcagaa tttacaagtt ttccagcaaa 5160
   ggtctagcag aatttacaga tacccacaac tcaaaggaaa aggactagta attatcattg 5220
   actagcccat ctcaattggt atagtgatta aaatcaccta gaccaattga gatgtatgtc 5280
   tgaattagtt gttttcaaag caaatgaact agcgattagt cgctatgact taacggagca 5340
   tgaaaccaag ctaattttat gctgtgtggc actactcaac cccacgattg aaaaccctac 5400
   aaggaaagaa cggacggtat cgttcactta taaccaatac gctcagatga tgaacatcag 5460
   tagggaaaat gcttatggtg tattagctaa agcaaccaga gagctgatga cgagaactgt 5520
   ggaaatcagg aatcctttgg ttaaaggctt tgagattttc cagtggacaa actatgccaa 5580
   gttctcaagc gaaaaattag aattagtttt tagtgaagag atattgcctt atcttttcca 5640
   gttaaaaaaa ttcataaaat ataatctgga acatgttaag tcttttgaaa acaaatactc 5700
   tatgaggatt tatgagtggt tattaaaaga actaacacaa aagaaaactc acaaggcaaa 5760
   tatagagatt agccttgatg aatttaagtt catgttaatg cttgaaaata actaccatga 5820
   gtttaaaagg cttaaccaat gggttttgaa accaataagt aaagatttaa acacttacag 5880
   caatatgaaa ttggtggttg ataagcgagg ccgcccgact gatacgttga ttttccaagt 5940
   tgaactagat agacaaatgg atctcgtaac cgaacttgag aacaaccaga taaaaatgaa 6000
   tggtgacaaa ataccaacaa ccattacatc agattcctac ctacataacg gactaagaaa 6060
   aacactacac gatgctttaa ctgcaaaaat tcagctcacc agttttgagg caaaattttt 6120
   gagtgacatg caaagtaagt atgatctcaa tggttcgttc tcatggctca cgcaaaaaca 6180
   acgaaccaca ctagagaaca tactggctaa atacggaagg atctgaggtt cttatggctc 6240
   ttgtatctat cagtgaagca tcaagactaa caaacaaaag tagaacaact gttcaccgtt 6300
   acatatcaaa gggaaaactg tccatatgca cagatgaaaa cggtgtaaaa aagatagata 6360
   catcagagct tttacgagtt tttggtgcat tcaaagctgt tcaccatgaa cagatcgaca 6420
   atgtaacaga tgaacagcat gtaacaccta atagaacagg tgaaaccagt aaaacaaagc 6480
   aactagaaca tgaaattgaa cacctgagac aacttgttac agctcaacag tcacacatag 6540
   acagcctgaa acaggcgatg ctgcttatcg aatcaaagct gccgacaaca cgggagccag 6600
   tgacgcctcc cgtggggaaa aaatcatggc aattctggaa gaaatagcgc tttcagccgg 6660
   caaaccggct gaagccggat ctgcgattct gataacaaac tagcaacacc agaacagccc 6720
   gtttgcgggc agcaaaaccc gtacttttgg acgttccggc ggttttttgt ggcgagtggt 6780
   gttcgggcgg tgcgcgcaag atccattatg ttaaacgggc gagtttacat ctcaaaaccg 6840
   cccgcttaac accatcagaa atcctcagcg cgattttaag caccaacccc cccccgtaac 6900
   acccaaatcc atactgaaag tggctttgtt gaataaatcg aacttttgct gagttgaagg 6960
   atcagatcac gcatcctccc gacaacacag accattccgt ggcaaagcaa aagttcagaa 7020
   tcaccaactg gtccacctac aacaaagctc tcatcaaccg tggctccctc actttctggc 7080
   tggatgatga ggcgattcag gcctggtatg agtcggcaac accttcatca cgaggaaggc 7140
   cccagcgcta ttctgatctc gccatcacca ccgttctggt gattaaacgc gtattccggc 7200
   tgaccctgcg ggctgcgcag ggttttattg attccatttt tgccctgatg aacgttccgt 7260
   tgcgctgccc ggattacacc agtgtcagta agcgggcaaa gtcggttaat gtcagtttca 7320
   aaacgtccac ccggggtgaa atcgcacacc tggtgattga ttccaccggg ctgaaggtct 7380
   ttggtgaagg cgaatggaaa gtcagaaagc acggcaaaga gcgccgtcgt atctggcgaa 7440
   agttgcatct tgctgttgac agcaacacac atgaagttgt ctgtgcagac ctgtcgctga 7500
   ataacgtcac ggactcagaa gccttcccgg gccttatccg gcagactcac agaaaaatca 7560
   gggcagccgc ggcagacggg gcttacgata cccggctctg tcacgatgaa ctgcgccgca 7620
   aaaaaatcag cgcgcttatt cctccccgaa aaggtgcggg ttactggccc ggtgaatatg 7680
   cagaccgtaa ccgtgcagtg gctaatcagc gaatgaccgg gagtaatgcg cggtggaaat 7740
   ggacaacaga ttacaaccgt cgctcgatag cggaaacggc gatgtaccgg gtaaaacagc 7800
   tgttcggggg ttcactgacg ctgcgtgact acgatggtca ggttgcggag gctatggccc 7860
   tggtacgagc gctgaacaaa atgacgaaag caggtatgcc tgaaagcgtg cgtattgcct 7920
   gaaaacacaa cccgctacgg gggagactta cccgaaatct gatttattca acaaagccgg 7980
   gtgtggtgaa ctacaaagca gacccgttga ggttatcagt tcgatgcaca atcagcagcg 8040
   cataaaatat gcacaagaac aggagcaccc ttcgcattaa gctgtggtgg taacaagtag 8100
   tgccgggcta ccatcagcga gcatgatgcg ctcccacagc attcgccttg gcagtatgga 8160
   agttcctcgc tccagttcgg gccggtatcc acctcgagtg accccagccg cccctcatgc 8220
   caaccgagcc cattaagcgc agagtcggcc gcaactatct cggggacgca taaacacgca 8280
   gcgatttacc aaggagttcg gctaggtcaa tccgcaattc cctttgcctg gccggagatg 8340
   ccgcgagcaa acaatgtgtc cggtcgctac cagatggact ttgtgggcgg aggattgcac 8400
   cagacgcttc agcgcagcgt cgtacgatga atcggaaggc ccggtaagca attgaagaac 8460
   cgctgaccac ctcccatccg acaatgcaag ggtgccccgc tcccagcgcg acacggttgc 8520
   ctgatccaca cccattaatt cagctaggtg actttgcttc atatgacgaa gcaaccgcgc 8580
   cctccgcacc tgccgccccc tgtcattcgt caacattctt cagcacctca atgtcgttcg 8640
   tagtgagcct cattttttca agaccgccga tgatgagagc caggcctcgc tcgaatgccg 8700
   cgtccggacc gccttcgtag acgattttca tcgcgctctg taggcgcgcc ggcatcgtag 8760
   acgctgaggt ggtcaactga tcttcgcccc gctcctcggc gtctgcctcg ctagcttgct 8820
   gctcaagaac agcgccgacg gtgaagtagc tgattgccat caacgcatag gtcgcgtcac 8880
   ctgccgaaaa gccagcatcg caaaggaagc gaagctgcgc gtcggctttt tccatctgcg 8940
   gcgcggctgg ccgcgtcccg gcatgaatac gcgcgccatc gcgataagcg agcaacgccc 9000
   gtcgaaaact gcatgcattg cccttcagga acgaacgcca gtcgtcgtca tcccttggcg 9060
   tcgaatgcgt gtgatttatc gtcagcatgg cttcggcaag tgcgtcgagc aacgcacgct 9120
   tgttcttgaa atgccagtag agcgctggct gttgcacccc gaggcgctca gccagtcggc 9180
   gcgtcgttag accttccatg cccacgtcgt taagcagttc gagcgcggtt cggatcacgg 9240
   cctcgcgttg gagcttgttc attcgcgaa 9269
<210> 17
   <211> 13712
   <212> DNA
   <213> Artificial Sequence
<220>
   <223> vector
<400> 17
   ttctcatgtt tgacagctta tcatcgataa gctttaatgc ggtagtttat cacagttaaa        60
   ttgctaacgc agtcaggcac cgtgtatgaa atctaacaat gcgctcatcg tcatcctcgg       120
   caccgtcacc ctggatgctg taggcatagg cttggttatg ccggtactgc cgggcctctt       180
   gcgggatatc gtccattccg acagcatcgc cagtcactat ggcgtgctgc tagcgctata       240
   tgcgttgatg caatttctat gcgcacccgt tctcggagca ctgtccgacc gctttggccg       300
   ccgcccagtc ctgctcgctt cgctacttgg agccactatc gactacgcga tcatggcgac       360
   cacacccgtc ctgtggatcc tctacgccgg acgcatcgtg gccggcatca ccggcgccac       420
   aggtgcggtt gctggcgcct atatcgccga catcaccgat ggggaagatc gggctcgcca       480
   cttcgggctc atgagcgctt gtttcggcgt gggtatggtg gcaggccccg tggccggggg       540
   actgttgggc gccatctcct tgcatgcacc attccttgcg gcggcggtgc tcaacggcct       600
   caacctacta ctgggctgct tcctaatgca ggagtcgcat aagggagagc gtcgaccgat       660
   gcccttgaga gccttcaacc cagtcagctc cttccggtgg gcgcggggca tgactatcgt       720
   cgccgcactt atgactgtct tctttatcat gcaactcgta ggacaggtgc cggcagcgct       780
   ctgggtcatt ttcggcgagg accgctttcg ctggagcgcg acgatgatcg gcctgtcgct       840
   tgcggtattc ggaatcttgc acgccctcgc tcaagccttc gtcactggtc ccgccaccaa 900
   acgtttcggc gagaagcagg ccattatcgc cggcatggcg gccgacgcgc tgggctacgt 960
   cttgctggcg ttcgcgacgc gaggctggat ggccttcccc attatgattc ttctcgcttc 1020
   cggcggcatc gggatgcccg cgttgcaggc catgctgtcc aggcaggtag atgacgacca 1080
   tcagggacag cttcaaggat cgctcgcggc tcttaccagc ctaacttcga tcactggacc 1140
   gctgatcgtc acggcgattt atgccgcctc ggcgagcaca tggaacgggt tggcatggat 1200
   tgtaggcgcc gccctatacc ttgtctgcct ccccgcgttg cgtcgcggtg catggagccg 1260
   ggccacctcg acctgaatgg aagccggcgg cacctcgcta acggattcac cactccaaga 1320
   attggagcca atcaattctt gcggagaact gtgaatgcgc aaaccaaccc ttggcagaac 1380
   atatccatcg cgtccgccat ctccagcagc cgcacgcggc gcatctcggg cagcgttggg 1440
   tcctggccac gggtgcgcat gatcgtgctc ctgtcgttga ggacccggct aggctggcgg 1500
   ggttgcctta ctggttagca gaatgaatca ccgatacgcg agcgaacgtg aagcgactgc 1560
   tgctgcaaaa cgtctgcgac ctgagcaaca acatgaatgg tcttcggttt ccgtgtttcg 1620
   taaagtctgg aaacgcggaa gtcccctacg tgctgctgaa gttgcccgca acagagagtg 1680
   gaaccaaccg gtgataccac gatactatga ctgagagtca acgccatgag cggcctcatt 1740
   tcttattctg agttacaaca gtccgcaccg ctgccggtag ctacttgact atccggctgc 1800
   actagccctg cgtcagatgg ctctgatcca aggcaaactg ccaaaatatc tgctggcacc 1860
   ggaagtcagc gccctgcacc attatgttcc ggatctgcat cgcaggatgc tgctggctac 1920
   cctgtggaac acctacatct gtattaacga agcgctggca ttgaccctga gtgatttttc 1980
   tctggtgccg ccctatccct ttgtgcagct tgccacgctc aaaggggttt gaggtccaac 2040
   cgtacgaaaa cgtacggtaa gaggaaaatt atcgtctgaa aaatcgatta gtagacaaga 2100
   aagtccgtta agtgccaatt ttcgattaaa aagacaccgt tttgatggcg ttttccaatg 2160
   tacattatgt ttcgatatat cagacagtta cttcactaac gtacgttttc gttctattgg 2220
   ccttcagacc ccatatcctt aatgtccttt atttgctggg gttatcagat ccccccgaca 2280
   cgtttaatta atgctttctc cgccggagat cgacgcacag gcttctgtgt ctatgatgtt 2340
   atttcttaat aatcatccag gtattctctt tatcaccata cgtagtgcga gtgtccacct 2400
   taacgcaggg ctttccgtca cagcgcgata tgtcagccag cggggctttc ttttgccaga 2460
   ccgcttccat cctctgcatt tcagcaatct ggctataccc gtcattcata aaccacgtaa 2520
   atgccgtcac gcaggaagcc aggacgaaga atatcgtcag tacaagataa atcgcggatt 2580
   tccacgtata gcgtgacatc tcacgacgca tttcatggat catcgctttc gccgtatcgg 2640
   cagcctgatt cagcgcttct gtcgccggtt tctgctgtgc taatccggct tgtttcagtt 2700
   ctttctcaac ctgagtgagc gcggaactca ccgatttcct gacggtgtca gtcatattac 2760
   cggacgcgct gtccagctca cgaatgaccc tgctcagcgt ttcactttgc tgctgtaatt 2820
   gtgatgaggc ggcctgaaac tgttctgtca gagaagtaac acgcttttcc agcgcctgat 2880
   gatgcccgat aagggcggca atttgtttaa tttcgtcgct catacaaaat cctgcctatc 2940
   gtgagaatga ccagccttta tccggcttct gtcgtatctg ttcggcgagt cgctgtcgtt 3000
   ctttctcctg ctgacgctgt ttttccgcca gacgttcgcg ctctctctgc ctttccatct 3060
   cctgatgtat cccctggaac tccgccatcg catcgttaac aagggactga agatcgattt 3120
   cttcctgtat atccttcatg gcatcactga ccagtgcgtt cagcttgtca ggctcttttt 3180
   caaaatcaaa cgttctgccg gaatgggatt cctgctcagg ctctgacttc agctcctgtt 3240
   ttagcgtcag agtatccctc tcgctgaggg cttcccgtaa cgaggtagtc acgtcaatta 3300
   cgctgtcacg ttcatcacgg gactgctgca cctgcctttc agcctccctg cgctcaagaa 3360
   tggcctgtag ctgctcagta tcgaatcgct gaacctgacc cgcgcccaga tgccgctcag 3420
   gctcacggtc aatgccctgc gccttcaggg aacgggaatc aacccggtca gcgtgctgat 3480
   accgttcaag gtgcttattc tggaggtcag cccagcgtct ccctctgggc aacaaggtat 3540
   tctttgcgtt cggtcggtgt ttccccgaaa cgtgcctttt ttgcgccacc gcgtccggct 3600
   ctttggtgtt agcccgttta aaatactgct cagggtcacg gtgaataccg tcattaatgc 3660
   gttcagagaa catgatatgg gcgtggggct gctcgccacc ggctatcgct gctttcggat 3720
   tatggatagc gaactgatag gcatggcggt cgccaatttc ctgttggaca aaatcgcgga 3780
   caagctcaag acgttgttcg ggttttaact cacgcggcag ggcaatctcg atttcacggt 3840
   aggtacagcc gttggcacgt tcagacgtgt cagcggcttt ccagaactcg gacggtttat 3900
   gcgctgccca cgccggcata ttgccggact ccttgtgctc aaggtcggag tctttttcac 3960
   gggcatactt tccctcacgc gcaatataat cggcatgagg agaggcactg ccttttccgc 4020
   cggtttttac gctgagatga taggatgcca tcgtgtttta tcccgctgaa gggcgcacgt 4080
   ttctgaacga agtgaagaaa gtctaagtgc gccctgataa ataaaagagt tatcagggat 4140
   tgtagtggga tttgacctcc tctgccatca tgagcgtaat cattccgtta gcattcagga 4200
   ggtaaacagc atgaataaaa gcgaaaaaac aggaacaatg ggcagcagaa agagtgcagt 4260
   atattcgcgg cttaaagtcg ccgaatgagc aacagaaact tatgctgata ctgacggata 4320
   aagcagataa aacagcacag gatatcaaaa cgctgtccct gctgatgaag gctgaacagg 4380
   cagcagagaa agcgcaggaa gccagagcga aagtcatgaa cctgatacag gcagaaaagc 4440
   gagccgaagc cagagccgcc cgtaaagccc gtgaccatgc tctgtaccag tctgccggat 4500
   tgcttatcct ggcgggtctg gttgacagta agacgggtaa gcctgttgat gataccgctg 4560
   ccttactggg tgcattagcc agtctgaatg acctgtcacg ggataatccg aagtggtcag 4620
   actggaaaat cagagggcag gaactgctga acagcaaaaa gtcagatagc accacatagc 4680
   agacccgcca taaaacgccc tgagaagccc gtgacgggct tttcttgtat tatgggtagt 4740
   ttccttgcat gaatccataa aaggcgcctg tagtgccatt tacccccatt cactgccaga 4800
   gccgtgagcg cagcgaactg aatgtcacga aaaagacagc gactcaggtg cctgatggtc 4860
   ggagacaaaa ggaatattca gcgatttgcc cgagcttgcg agggtgctac ttaagccttt 4920
   agggttttaa ggtctgtttt gtagaggagc aaacagcgtt tgcgacatcc ttttgtaata 4980
   ctgcggaact gactaaagta gtgagttata cacagggctg ggatctattc tttttatctt 5040
   tttttattct ttctttattc tataaattat aaccacttga atataaacaa aaaaaacaca 5100
   caaaggtcta gcggaattta cagagggtct agcagaattt acaagttttc cagcaaaggt 5160
   ctagcagaat ttacagatac ccacaactca aaggaaaagg actagtaatt atcattgact 5220
   agcccatctc aattggtata gtgattaaaa tcacctagac caattgagat gtatgtctga 5280
   attagttgtt ttcaaagcaa atgaactagc gattagtcgc tatgacttaa cggagcatga 5340
   aaccaagcta attttatgct gtgtggcact actcaacccc acgattgaaa accctacaag 5400
   gaaagaacgg acggtatcgt tcacttataa ccaatacgct cagatgatga acatcagtag 5460
   ggaaaatgct tatggtgtat tagctaaagc aaccagagag ctgatgacga gaactgtgga 5520
   aatcaggaat cctttggtta aaggctttga gattttccag tggacaaact atgccaagtt 5580
   ctcaagcgaa aaattagaat tagtttttag tgaagagata ttgccttatc ttttccagtt 5640
   aaaaaaattc ataaaatata atctggaaca tgttaagtct tttgaaaaca aatactctat 5700
   gaggatttat gagtggttat taaaagaact aacacaaaag aaaactcaca aggcaaatat 5760
   agagattagc cttgatgaat ttaagttcat gttaatgctt gaaaataact accatgagtt 5820
   taaaaggctt aaccaatggg ttttgaaacc aataagtaaa gatttaaaca cttacagcaa 5880
   tatgaaattg gtggttgata agcgaggccg cccgactgat acgttgattt tccaagttga 5940
   actagataga caaatggatc tcgtaaccga acttgagaac aaccagataa aaatgaatgg 6000
   tgacaaaata ccaacaacca ttacatcaga ttcctaccta cgtaacggac taagaaaaac 6060
   actacacgat gctttaactg caaaaattca gctcaccagt tttgaggcaa aatttttgag 6120
   tgacatgcaa agtaagcatg atctcaatgg ttcgttctca tggctcacgc aaaaacaacg 6180
   aaccacacta gagaacatac tggctaaata cggaaggatc tgaggttctt atggctcttg 6240
   tatctatcag tgaagcatca agactaacaa acaaaagtag aacaactgtt caccgttaga 6300
   tatcaaaggg aaaactgtcc atatgcacag atgaaaacgg tgtaaaaaag atagatacat 6360
   cagagctttt acgagttttt ggtgcattta aagctgttca ccatgaacag atcgacaatg 6420
   taacagatga acagcatgta acacctaata gaacaggtga aaccagtaaa acaaagcaac 6480
   tagaacatga aattgaacac ctgagacaac ttgttacagc tcaacagtca cacatagaca 6540
   gcctgaaaca ggcgatgctg cttatcgaat caaagctgcc gacaacacgg gagccagtga 6600
   cgcctcccgt ggggaaaaaa tcatggcaat tctggaagaa atagcgcttt cagccggcaa 6660
   acctgaagcc ggatctgcga ttctgataac aaactagcaa caccagaaca gcccgtttgc 6720
   gggcagcaaa acccgtactt ttggacgttc cggcggtttt ttgtggcgag tggtgttcgg 6780
   gcggtgcgcg caagatccat tatgttaaac gggcgagttt acatctcaaa accgcccgct 6840
   taacaccatc agaaatcctc agcgcgattt taagcaccaa cccccccccg taacacccaa 6900
   atccatactg aaagtggctt tgttgaataa atcgaacttt tgctgagttg aaggatcaga 6960
   tcacgcatcc tcccgacaac acagaccatt ccgtggcaaa gcaaaagttc agaatcacca 7020
   actggtccac ctacaacaaa gctctcatca accgtggctc cctcactttc tggctggatg 7080
   atgaggcgat tcaggcctgg tatgagtcgg caacaccttc atcacgagga aggccccagc 7140
   gctattctga tctcgccatc accaccgttc tggtgattaa acgcgtattc cggctgaccc 7200
   tgcgggctgc gcagggtttt attgattcca tttttgccct gatgaacgtt ccgttgcgct 7260
   gcccggatta caccagtgtc agtaagcggg caaagtcggt taatgtcagt ttcaaaacgt 7320
   ccacccgggg tgaaatcgca cacctggtga ttgattccac cgggctgaag gtctttggtg 7380
   aaggcgaatg gaaagtcaga aagcacggca aagagcgccg tcgtatctgg cgaaagttgc 7440
   atcttgctgt tgacagcaac acacatgaag ttgtctgtgc agacctgtcg ctgaataacg 7500
   tcacggactc agaagccttc ccgggcctta tccggcagac tcacagaaaa atcagggcag 7560
   ccgcggcaga cggggcttac gatacccggc tctgtcacga tgaactgcgc cgcaaaaaaa 7620
   tcagcgcgct tattcctccc cgaaaaggtg cgggttactg gcccggtgaa tatgcagacc 7680
   gtaaccgtgc agtggctaat cagcgaatga ccgggagtaa tgcgcggtgg aaatggacaa 7740
   cagattacaa ccgtcgctcg atagcggaaa cggcgatgta ccgggtaaaa cagctgttcg 7800
   ggggttcact gacgctgcgt gactacgatg gtcaggttgc ggaggctatg gccctggtac 7860
   gagcgctgaa caaaatgacg aaagcaggta tgcctgaaag cgtgcgtatt gcctgaaaac 7920
   acaacccgct acgggggaga cttacccgaa atctgattta ttcaacaaag ccgggtgtgg 7980
   tgaactacaa agcagacccg ttgaggttat cagttcgatg cacaatcagc agcgcataaa 8040
   atatgcacaa gaacaggagc acccttcgca ttaagctgtg gtggtaacaa gtagtgccgg 8100
   gctaccatca gcgagcatga tgcgctccca cagcattcgc cttggcagta tggaagttcc 8160
   tcgctccagt tcgggccggt atccacctcg agtggcagtg agcgcaacgc aattaatgta 8220
   agttagctca ctcattaggc accccaggct tgacacttta tgcttccggc tcgtataatg 8280
   tgtggaattg tgagcggata acaataacaa tttcacacag gatctaggaa ccaaggagag 8340
   tggcatatga caattgaaac gcgcgaagat agatttaaca gacgtattga tcaccttttt 8400
   gaaacagacc cgcagttcgc agcggcacgt ccggacgaag ctattagcgc tgcagcagcc 8460
   gatccggagc tgcgcctgcc agccgcagtc aagcagattc tggccggtta tgcggaccgt 8520
   ccggccctgg gtaagcgtgc tgtggagttc gttacggacg aagaaggccg taccaccgca 8580
   aagttgctgc cgcgtttcga tacgatcacc taccgtcaat tggcaggtcg tattcaggcg 8640
   gtgactaacg cgtggcacaa tcacccggtg aatgcgggtg accgtgtcgc aattttgggc 8700
   ttcacgagcg ttgactatac gaccatcgat atcgcactgc tggaactggg tgctgttagc 8760
   gttccgttgc aaacgtctgc tccggttgcg cagctgcaac cgattgtggc cgaaaccgag 8820
   ccgaaggtca ttgcgagcag cgtcgatttc ctggcagatg cggtcgcact ggttgagagc 8880
   ggtccagccc cgagccgtct ggtcgttttt gattatagcc acgaggtcga cgatcagcgt 8940
   gaggccttcg aagcggccaa gggtaaactg gcgggtaccg gtgttgtggt ggaaacgatt 9000
   acggatgtct tggatcgcgg tcgcagcctg gctgatgcgc cgctgtatgt cccagacgaa 9060
   acggacccgc tgaccctgct gatctatacc agcggtagca ccggtacgcc gaagggtgct 9120
   atgtatccgg aatccaagac cgcgacgatg tggcaagctg gtagcaaggc gcgttgggat 9180
   gaaaccctgg gcgttatgcc gagcatcacc ctgaacttca tgccaatgtc ccacgtgatg 9240
   ggtcgcggca tcctgtgtag caccctggcg agcggcggta ctgcgtattt tgccgcacgt 9300
   agcgacttgt ctacctttct ggaggacctg gcgttggtcc gcccgaccca actgaatttc 9360
   gtccctcgca tctgggatat gctgttccaa gagtatcaga gccgtctgga caatcgtcgt 9420
   gccgagggta gcgaggatcg cgcggaagcg gccgtactgg aagaggttcg tacccaattg 9480
   ctgggcggtc gtttcgtttc cgctctgacc ggtagcgctc caatcagcgc ggaaatgaaa 9540
   agctgggtcg aggacctgct ggacatgcat ttgctggaag gttacggttc caccgaggcg 9600
   ggtgccgtgt tcattgacgg tcaaatccaa cgtccacctg ttatcgatta caaactggtt 9660
   gatgttccgg acttgggtta ctttgcgacc gatcgtccat accctcgcgg cgagctgctg 9720
   gtgaagagcg agcagatgtt cccgggctac tataagcgtc cggagattac ggcggagatg 9780
   tttgacgagg acggttatta ccgtaccggc gacatcgtgg ccgaactggg tccggatcat 9840
   ctggagtatc tggaccgccg taacaacgtc ctgaagctga gccagggtga gtttgttacc 9900
   gtttccaaac tggaagcggt ctttggcgat agcccactgg tgcgccagat ttatgtctac 9960
   ggtaactctg cacgcagcta tctgctggcg gttgtcgttc cgaccgaaga ggcactgagc 10020
   cgctgggacg gcgatgaact gaagagccgc atcagcgaca gcttgcaaga tgcggcacgt 10080
   gccgcaggtt tgcaaagcta tgagatcccg cgtgactttc tggttgaaac cacgccgttc 10140
   acgttggaga acggtctgct gaccggcatt cgcaaactgg cgcgtccgaa gctgaaagcg 10200
   cattatggcg aacgtctgga acaactgtac acggatctgg ctgaaggcca agccaatgag 10260
   ctgcgtgaac tgcgccgtaa tggtgcggat cgtccggttg ttgaaacggt ttcgcgtgcc 10320
   gcggtcgcct tgctgggtgc gtctgtgacg gacctgcgtt ctgacgccca ctttacggac 10380
   ctgggcggtg attccctgag cgccctgagc ttcagcaacc tgctgcacga gatttttgac 10440
   gtcgatgtcc cggtgggcgt gattgtcagc ccggcaaccg acctggccgg tgttgccgcc 10500
   tatattgagg gtgagctgcg tggtagcaaa cgtccgacct acgcaagcgt ccacggtcgt 10560
   gacgcaaccg aggtgcgtgc tcgcgatctg gcgctgggca aattcatcga cgcaaaaacg 10620
   ctgagcgcag caccgggtct gccgcgctcc ggtacggaga tccgtacggt gctgctgacg 10680
   ggtgcgaccg gttttctggg tcgctacttg gccttggagt ggctggaacg tatggacctg 10740
   gttgacggca aggtgatctg tttggttcgt gcgcgtagcg acgacgaagc acgtgcgcgt 10800
   ctggacgcaa ccttcgatac cggcgatgct accttgctgg agcactaccg tgccctggcg 10860
   gctgaccatc tggaagttat tgcgggtgat aagggtgaag ccgacttggg cctggatcac 10920
   gatacctggc agcgtctggc agatacggtc gacctgatcg ttgacccggc tgcgctggtt 10980
   aaccacgttt tgccgtacag ccagatgttc ggcccaaacg ctctgggcac tgcagagctg 11040
   attcgcattg cactgaccac caccattaaa ccgtacgtct acgtgagcac cattggtgtt 11100
   ggtcagggca tcagcccaga agcctttgtt gaagatgcgg acatccgcga aatcagcgcc 11160
   acgcgtcgcg ttgacgacag ctacgcgaat ggttatggta acagcaaatg ggcaggtgag 11220
   gttctgctgc gcgaagcgca cgactggtgc ggcctgccgg tgagcgtgtt tcgttgtgac 11280
   atgatcttgg ccgacaccac ctactccggc cagctgaatc tgccggatat gttcacccgt 11340
   ctgatgttga gcctggtggc aactggcatc gcaccgggca gcttttatga gctggacgcg 11400
   gacggtaatc gtcaacgtgc acattatgat ggtctgccgg tggagtttat cgcggaagca 11460
   atcagcacga tcggctctca agtgactgac ggtttcgaaa cgttccatgt catgaatcct 11520
   tatgatgatg gtatcggcct ggacgagtac gtcgactggc tgattgaggc tggctacccg 11580
   gttcaccgtg ttgatgatta tgcgacgtgg ttgagccgtt ttgaaaccgc cttgcgtgcg 11640
   ctgccggagc gtcaacgtca ggcatccctg ctgccgctgt tgcacaacta ccagcagcct 11700
   tctccgccgg tttgcggtgc tatggctcct accgaccgtt tccgtgcagc tgtccaggac 11760
   gccaaaattg gcccagataa agacatccca catgtaacgg cggacgtgat cgtgaaatac 11820
   attagcaact tgcagatgct gggtctgctg taagtctaag agaaggagtt ctatcgtcta 11880
   tgattgaaac catcttacca gccggcgtcg aatcagcaga gttacttgag taccctgaag 11940
   atctgaaggc gcatccggct gaagagcatc tgatcgcaaa gagcgtagag aagcgtcgtc 12000
   gcgacttcat tggcgcacgt cattgcgccc gtctggcact ggcggagctg ggtgaaccgc 12060
   cggttgcgat tggcaagggt gaacgtggtg cgccgatttg gccgcgtggt gtcgtgggct 12120
   ctctgaccca ttgcgatggc tatcgcgcag cggcggttgc tcacaaaatg cgttttcgca 12180
   gcatcggcat cgacgccgaa ccgcacgcga ccctgccgga aggtgtcctg gattcggtta 12240
   gcctgccgcc tgagcgtgag tggctgaaaa ccaccgacag cgcactgcac ctggaccgtt 12300
   tgctgttttg tgcgaaagaa gcaacttaca aagcgtggtg gccgctgacg gcacgttggc 12360
   tgggtttcga agaagcgcac attaccttcg aaatcgagga tggtagcgcc gactctggta 12420
   atggcacgtt tcacagcgaa ctgctggtgc cgggtcagac caatgacggt ggtaccccgc 12480
   tgctgtcttt cgacggtcgc tggctgatcg ctgatggctt catcctgacg gcgattgcat 12540
   acgcatgata atgataacct aggctgctgc caccgctgag caataactag cataacccct 12600
   tggggcctct aaacgggtct tgaggggttt tttgcctcga gtgaccccag ccgcccctca 12660
   tgccaaccga gcccattaag cgcagagtcg gccgcaacta tctcggggac gcataaacac 12720
   gcagcgattt accaaggagt tcggctaggt caatccgcaa ttccctttgc ctggccggag 12780
   atgccgcgag caaacaatgt gtccggtcgc taccagatgg actttgtggg cggaggattg 12840
   caccagacgc ttcagcgcag cgtcgtacga tgaatcggaa ggcccggtaa gcaattgaag 12900
   aaccgctgac cacctcccat ccgacaatgc aagggtgccc cgctcccagc gcgacacggt 12960
   tgcctgatcc acacccatta attcagctag gtgactttgc ttcatatgac gaagcaaccg 13020
   cgccctccgc acctgccgcc ccctgtcatt cgtcaacatt cttcagcacc tcaatgtcgt 13080
   tcgtagtgag cctcattttt tcaagaccgc cgatgatgag agccaggcct cgctcgaatg 13140
   ccgcgtccgg accgccttcg tagacgattt tcatcgcgct ctgtaggcgc gccggcatcg 13200
   tagacgctga ggtggtcaac tgatcttcgc cccgctcctc ggcgtctgcc tcgctagctt 13260
   gctgctcaag aacagcgccg acggtgaagt agctgattgc catcaacgca taggtcgcgt 13320
   cacctgccga aaagccagca tcgcaaagga agcgaagctg cgcgtcggct ttttccatct 13380
   gcggcgcggc tggccgcgtc ccggcatgaa tacgcgcgcc atcgcgataa gcgagcaacg 13440
   cccgtcgaaa actgcatgca ttgcccttca ggaacgaacg ccagtcgtcg tcatcccttg 13500
   gcgtcgaatg cgtgtgattt atcgtcagca tggcttcggc aagtgcgtcg agcaacgcac 13560
   gcttgttctt gaaatgccag tagagcgctg gctgttgcac cccgaggcgc tcagccagtc 13620
   ggcgcgtcgt tagaccttcc atgcccacgt cgttaagcag ttcgagcgcg gttcggatca 13680
   cggcctcgcg ttggagcttg ttcattcgcg aa 13712
<210> 18
   <211> 13719
   <212> DNA
   <213> Artificial Sequence
<220>
   <223> vector
<400> 18
   ttctcatgtt tgacagctta tcatcgataa gctttaatgc ggtagtttat cacagttaaa 60
   ttgctaacgc agtcaggcac cgtgtatgaa atctaacaat gcgctcatcg tcatcctcgg 120
   caccgtcacc ctggatgctg taggcatagg cttggttatg ccggtactgc cgggcctctt 180
   gcgggatatc gtccattccg acagcatcgc cagtcactat ggcgtgctgc tagcgctata 240
   tgcgttgatg caatttctat gcgcacccgt tctcggagca ctgtccgacc gctttggccg 300
   ccgcccagtc ctgctcgctt cgctacttgg agccactatc gactacgcga tcatggcgac 360
   cacacccgtc ctgtggatcc tctacgccgg acgcatcgtg gccggcatca ccggcgccac 420
   aggtgcggtt gctggcgcct atatcgccga catcaccgat ggggaagatc gggctcgcca 480
   cttcgggctc atgagcgctt gtttcggcgt gggtatggtg gcaggccccg tggccggggg 540
   actgttgggc gccatctcct tgcatgcacc attccttgcg gcggcggtgc tcaacggcct 600
   caacctacta ctgggctgct tcctaatgca ggagtcgcat aagggagagc gtcgaccgat 660
   gcccttgaga gccttcaacc cagtcagctc cttccggtgg gcgcggggca tgactatcgt 720
   cgccgcactt atgactgtct tctttatcat gcaactcgta ggacaggtgc cggcagcgct 780
   ctgggtcatt ttcggcgagg accgctttcg ctggagcgcg acgatgatcg gcctgtcgct 840
   tgcggtattc ggaatcttgc acgccctcgc tcaagccttc gtcactggtc ccgccaccaa 900
   acgtttcggc gagaagcagg ccattatcgc cggcatggcg gccgacgcgc tgggctacgt 960
   cttgctggcg ttcgcgacgc gaggctggat ggccttcccc attatgattc ttctcgcttc 1020
   cggcggcatc gggatgcccg cgttgcaggc catgctgtcc aggcaggtag atgacgacca 1080
   tcagggacag cttcaaggat cgctcgcggc tcttaccagc ctaacttcga tcactggacc 1140
   gctgatcgtc acggcgattt atgccgcctc ggcgagcaca tggaacgggt tggcatggat 1200
   tgtaggcgcc gccctatacc ttgtctgcct ccccgcgttg cgtcgcggtg catggagccg 1260
   ggccacctcg acctgaatgg aagccggcgg cacctcgcta acggattcac cactccaaga 1320
   attggagcca atcaattctt gcggagaact gtgaatgcgc aaaccaaccc ttggcagaac 1380
   atatccatcg cgtccgccat ctccagcagc cgcacgcggc gcatctcggg cagcgttggg 1440
   tcctggccac gggtgcgcat gatcgtgctc ctgtcgttga ggacccggct aggctggcgg 1500
   ggttgcctta ctggttagca gaatgaatca ccgatacgcg agcgaacgtg aagcgactgc 1560
   tgctgcaaaa cgtctgcgac ctgagcaaca acatgaatgg tcttcggttt ccgtgtttcg 1620
   taaagtctgg aaacgcggaa gtcccctacg tgctgctgaa gttgcccgca acagagagtg 1680
   gaaccaaccg gtgataccac gatactatga ctgagagtca acgccatgag cggcctcatt 1740
   tcttattctg agttacaaca gtccgcaccg ctgccggtag ctacttgact atccggctgc 1800
   actagccctg cgtcagatgg ctctgatcca aggcaaactg ccaaaatatc tgctggcacc 1860
   ggaagtcagc gccctgcacc attatgttcc ggatctgcat cgcaggatgc tgctggctac 1920
   cctgtggaac acctacatct gtattaacga agcgctggca ttgaccctga gtgatttttc 1980
   tctggtgccg ccctatccct ttgtgcagct tgccacgctc aaaggggttt gaggtccaac 2040
   cgtacgaaaa cgtacggtaa gaggaaaatt atcgtctgaa aaatcgatta gtagacaaga 2100
   aagtccgtta agtgccaatt ttcgattaaa aagacaccgt tttgatggcg ttttccaatg 2160
   tacattatgt ttcgatatat cagacagtta cttcactaac gtacgttttc gttctattgg 2220
   ccttcagacc ccatatcctt aatgtccttt atttgctggg gttatcagat ccccccgaca 2280
   cgtttaatta atgctttctc cgccggagat cgacgcacag gcttctgtgt ctatgatgtt 2340
   atttcttaat aatcatccag gtattctctt tatcaccata cgtagtgcga gtgtccacct 2400
   taacgcaggg ctttccgtca cagcgcgata tgtcagccag cggggctttc ttttgccaga 2460
   ccgcttccat cctctgcatt tcagcaatct ggctataccc gtcattcata aaccacgtaa 2520
   atgccgtcac gcaggaagcc aggacgaaga atatcgtcag tacaagataa atcgcggatt 2580
   tccacgtata gcgtgacatc tcacgacgca tttcatggat catcgctttc gccgtatcgg 2640
   cagcctgatt cagcgcttct gtcgccggtt tctgctgtgc taatccggct tgtttcagtt 2700
   ctttctcaac ctgagtgagc gcggaactca ccgatttcct gacggtgtca gtcatattac 2760
   cggacgcgct gtccagctca cgaatgaccc tgctcagcgt ttcactttgc tgctgtaatt 2820
   gtgatgaggc ggcctgaaac tgttctgtca gagaagtaac acgcttttcc agcgcctgat 2880
   gatgcccgat aagggcggca atttgtttaa tttcgtcgct catacaaaat cctgcctatc 2940
   gtgagaatga ccagccttta tccggcttct gtcgtatctg ttcggcgagt cgctgtcgtt 3000
   ctttctcctg ctgacgctgt ttttccgcca gacgttcgcg ctctctctgc ctttccatct 3060
   cctgatgtat cccctggaac tccgccatcg catcgttaac aagggactga agatcgattt 3120
   cttcctgtat atccttcatg gcatcactga ccagtgcgtt cagcttgtca ggctcttttt 3180
   caaaatcaaa cgttctgccg gaatgggatt cctgctcagg ctctgacttc agctcctgtt 3240
   ttagcgtcag agtatccctc tcgctgaggg cttcccgtaa cgaggtagtc acgtcaatta 3300
   cgctgtcacg ttcatcacgg gactgctgca cctgcctttc agcctccctg cgctcaagaa 3360
   tggcctgtag ctgctcagta tcgaatcgct gaacctgacc cgcgcccaga tgccgctcag 3420
   gctcacggtc aatgccctgc gccttcaggg aacgggaatc aacccggtca gcgtgctgat 3480
   accgttcaag gtgcttattc tggaggtcag cccagcgtct ccctctgggc aacaaggtat 3540
   tctttgcgtt cggtcggtgt ttccccgaaa cgtgcctttt ttgcgccacc gcgtccggct 3600
   ctttggtgtt agcccgttta aaatactgct cagggtcacg gtgaataccg tcattaatgc 3660
   gttcagagaa catgatatgg gcgtggggct gctcgccacc ggctatcgct gctttcggat 3720
   tatggatagc gaactgatag gcatggcggt cgccaatttc ctgttggaca aaatcgcgga 3780
   caagctcaag acgttgttcg ggttttaact cacgcggcag ggcaatctcg atttcacggt 3840
   aggtacagcc gttggcacgt tcagacgtgt cagcggcttt ccagaactcg gacggtttat 3900
   gcgctgccca cgccggcata ttgccggact ccttgtgctc aaggtcggag tctttttcac 3960
   gggcatactt tccctcacgc gcaatataat cggcatgagg agaggcactg ccttttccgc 4020
   cggtttttac gctgagatga taggatgcca tcgtgtttta tcccgctgaa gggcgcacgt 4080
   ttctgaacga agtgaagaaa gtctaagtgc gccctgataa ataaaagagt tatcagggat 4140
   tgtagtggga tttgacctcc tctgccatca tgagcgtaat cattccgtta gcattcagga 4200
   ggtaaacagc atgaataaaa gcgaaaaaac aggaacaatg ggcagcagaa agagtgcagt 4260
   atattcgcgg cttaaagtcg ccgaatgagc aacagaaact tatgctgata ctgacggata 4320
   aagcagataa aacagcacag gatatcaaaa cgctgtccct gctgatgaag gctgaacagg 4380
   cagcagagaa agcgcaggaa gccagagcga aagtcatgaa cctgatacag gcagaaaagc 4440
   gagccgaagc cagagccgcc cgtaaagccc gtgaccatgc tctgtaccag tctgccggat 4500
   tgcttatcct ggcgggtctg gttgacagta agacgggtaa gcctgttgat gataccgctg 4560
   ccttactggg tgcattagcc agtctgaatg acctgtcacg ggataatccg aagtggtcag 4620
   actggaaaat cagagggcag gaactgctga acagcaaaaa gtcagatagc accacatagc 4680
   agacccgcca taaaacgccc tgagaagccc gtgacgggct tttcttgtat tatgggtagt 4740
   ttccttgcat gaatccataa aaggcgcctg tagtgccatt tacccccatt cactgccaga 4800
   gccgtgagcg cagcgaactg aatgtcacga aaaagacagc gactcaggtg cctgatggtc 4860
   ggagacaaaa ggaatattca gcgatttgcc cgagcttgcg agggtgctac ttaagccttt 4920
   agggttttaa ggtctgtttt gtagaggagc aaacagcgtt tgcgacatcc ttttgtaata 4980
   ctgcggaact gactaaagta gtgagttata cacagggctg ggatctattc tttttatctt 5040
   tttttattct ttctttattc tataaattat aaccacttga atataaacaa aaaaaacaca 5100
   caaaggtcta gcggaattta cagagggtct agcagaattt acaagttttc cagcaaaggt 5160
   ctagcagaat ttacagatac ccacaactca aaggaaaagg actagtaatt atcattgact 5220
   agcccatctc aattggtata gtgattaaaa tcacctagac caattgagat gtatgtctga 5280
   attagttgtt ttcaaagcaa atgaactagc gattagtcgc tatgacttaa cggagcatga 5340
   aaccaagcta attttatgct gtgtggcact actcaacccc acgattgaaa accctacaag 5400
   gaaagaacgg acggtatcgt tcacttataa ccaatacgct cagatgatga acatcagtag 5460
   ggaaaatgct tatggtgtat tagctaaagc aaccagagag ctgatgacga gaactgtgga 5520
   aatcaggaat cctttggtta aaggctttga gattttccag tggacaaact atgccaagtt 5580
   ctcaagcgaa aaattagaat tagtttttag tgaagagata ttgccttatc ttttccagtt 5640
   aaaaaaattc ataaaatata atctggaaca tgttaagtct tttgaaaaca aatactctat 5700
   gaggatttat gagtggttat taaaagaact aacacaaaag aaaactcaca aggcaaatat 5760
   agagattagc cttgatgaat ttaagttcat gttaatgctt gaaaataact accatgagtt 5820
   taaaaggctt aaccaatggg ttttgaaacc aataagtaaa gatttaaaca cttacagcaa 5880
   tatgaaattg gtggttgata agcgaggccg cccgactgat acgttgattt tccaagttga 5940
   actagataga caaatggatc tcgtaaccga acttgagaac aaccagataa aaatgaatgg 6000
   tgacaaaata ccaacaacca ttacatcaga ttcctaccta cgtaacggac taagaaaaac 6060
   actacacgat gctttaactg caaaaattca gctcaccagt tttgaggcaa aatttttgag 6120
   tgacatgcaa agtaagcatg atctcaatgg ttcgttctca tggctcacgc aaaaacaacg 6180
   aaccacacta gagaacatac tggctaaata cggaaggatc tgaggttctt atggctcttg 6240
   tatctatcag tgaagcatca agactaacaa acaaaagtag aacaactgtt caccgttaga 6300
   tatcaaaggg aaaactgtcc atatgcacag atgaaaacgg tgtaaaaaag atagatacat 6360
   cagagctttt acgagttttt ggtgcattta aagctgttca ccatgaacag atcgacaatg 6420
   taacagatga acagcatgta acacctaata gaacaggtga aaccagtaaa acaaagcaac 6480
   tagaacatga aattgaacac ctgagacaac ttgttacagc tcaacagtca cacatagaca 6540
   gcctgaaaca ggcgatgctg cttatcgaat caaagctgcc gacaacacgg gagccagtga 6600
   cgcctcccgt ggggaaaaaa tcatggcaat tctggaagaa atagcgcttt cagccggcaa 6660
   acctgaagcc ggatctgcga ttctgataac aaactagcaa caccagaaca gcccgtttgc 6720
   gggcagcaaa acccgtactt ttggacgttc cggcggtttt ttgtggcgag tggtgttcgg 6780
   gcggtgcgcg caagatccat tatgttaaac gggcgagttt acatctcaaa accgcccgct 6840
   taacaccatc agaaatcctc agcgcgattt taagcaccaa cccccccccg taacacccaa 6900
   atccatactg aaagtggctt tgttgaataa atcgaacttt tgctgagttg aaggatcaga 6960
   tcacgcatcc tcccgacaac acagaccatt ccgtggcaaa gcaaaagttc agaatcacca 7020
   actggtccac ctacaacaaa gctctcatca accgtggctc cctcactttc tggctggatg 7080
   atgaggcgat tcaggcctgg tatgagtcgg caacaccttc atcacgagga aggccccagc 7140
   gctattctga tctcgccatc accaccgttc tggtgattaa acgcgtattc cggctgaccc 7200
   tgcgggctgc gcagggtttt attgattcca tttttgccct gatgaacgtt ccgttgcgct 7260
   gcccggatta caccagtgtc agtaagcggg caaagtcggt taatgtcagt ttcaaaacgt 7320
   ccacccgggg tgaaatcgca cacctggtga ttgattccac cgggctgaag gtctttggtg 7380
   aaggcgaatg gaaagtcaga aagcacggca aagagcgccg tcgtatctgg cgaaagttgc 7440
   atcttgctgt tgacagcaac acacatgaag ttgtctgtgc agacctgtcg ctgaataacg 7500
   tcacggactc agaagccttc ccgggcctta tccggcagac tcacagaaaa atcagggcag 7560
   ccgcggcaga cggggcttac gatacccggc tctgtcacga tgaactgcgc cgcaaaaaaa 7620
   tcagcgcgct tattcctccc cgaaaaggtg cgggttactg gcccggtgaa tatgcagacc 7680
   gtaaccgtgc agtggctaat cagcgaatga ccgggagtaa tgcgcggtgg aaatggacaa 7740
   cagattacaa ccgtcgctcg atagcggaaa cggcgatgta ccgggtaaaa cagctgttcg 7800
   ggggttcact gacgctgcgt gactacgatg gtcaggttgc ggaggctatg gccctggtac 7860
   gagcgctgaa caaaatgacg aaagcaggta tgcctgaaag cgtgcgtatt gcctgaaaac 7920
   acaacccgct acgggggaga cttacccgaa atctgattta ttcaacaaag ccgggtgtgg 7980
   tgaactacaa agcagacccg ttgaggttat cagttcgatg cacaatcagc agcgcataaa 8040
   atatgcacaa gaacaggagc acccttcgca ttaagctgtg gtggtaacaa gtagtgccgg 8100
   gctaccatca gcgagcatga tgcgctccca cagcattcgc cttggcagta tggaagttcc 8160
   tcgctccagt tcgggccggt atccacctcg agtggcagtg agcgcaacgc aattaatgta 8220
   agttagctca ctcattaggc accccaggct tgacacttta tgcttccggc tcgtataatg 8280
   tgtggaattg tgagcggata acaataacaa tttcacacag gatctaggaa ccaaggagag 8340
   tggcatatga ctagcgatgt acatgacgcg actgacggtg tgacagagac agcattagat 8400
   gacgagcaaa gcacccgtcg cattgcggaa ctgtatgcaa cggatccgga gtttgcagct 8460
   gccgcaccgc tgccggctgt tgtcgacgct gcacataaac cgggtctgcg tctggcggag 8520
   attctgcaga ccctgtttac cggttacggc gatcgtccgg ccctgggcta ccgtgctcgt 8580
   gaactggcga cggatgaggg tggccgtacc gtgacccgtt tgttgccgcg ttttgacacg 8640
   ctgacgtacg cgcaagtctg gagccgtgtc caggctgtgg cagcagcgct gcgccataac 8700
   ttcgcgcagc ctatctatcc aggtgacgcg gttgcgacca tcggctttgc aagcccagac 8760
   tatctgacgc tggatctggt ctgcgcgtat ttgggcctgg tcagcgtgcc actgcagcac 8820
   aacgctcctg tgagccgttt ggcgccgatt ctggccgagg ttgagccgcg tatcctgacg 8880
   gtcagcgccg aatacctgga cctggcggtc gagagcgtgc gtgatgtgaa ttctgtctcg 8940
   caattggtcg ttttcgacca ccacccggaa gtcgatgacc accgcgatgc gctggcacgc 9000
   gcacgtgaac agctggccgg taaaggtatt gccgttacga ctctggacgc gattgcggac 9060
   gaaggcgctg gtctgccagc ggagccgatc tataccgccg atcacgatca acgcctggcg 9120
   atgatcctgt acaccagcgg ttctaccggc gctccgaagg gtgcgatgta tactgaggcc 9180
   atgctggcgc gtctgtggac catgtccttt atcaccggcg acccgactcc tgtgattaat 9240
   gtgaacttta tgccgctgaa tcacctgggt ggtcgtattc cgattagcac cgcggtccag 9300
   aacggcggta cgagctactt cgttccggag agcgacatgt ctaccctgtt tgaggacctg 9360
   gccttggttc gcccgacgga actgggtctg gttccgcgtg tggcggacat gttgtaccag 9420
   catcacctgg caactgttga ccgtctggtt acccagggcg cagacgagct gaccgcagag 9480
   aagcaggcag gcgcagaact gcgtgaacag gtgctgggcg gtcgtgtgat taccggcttc 9540
   gtgagcacgg cgccactggc ggctgagatg cgtgccttcc tggatatcac gctgggtgcc 9600
   cacatcgttg atggttacgg tttgaccgaa accggtgcgg tgacgcgtga tggcgttatc 9660
   gtccgtccgc cggtcattga ttacaagctg attgatgttc cagagctggg ctacttcagc 9720
   accgataaac cgtacccacg tggcgaactg ttggttcgta gccaaacgtt gaccccgggt 9780
   tactataaac gtccggaagt taccgcatcg gtgtttgatc gcgacggcta ttaccatacc 9840
   ggtgacgtta tggcagaaac cgctccagat cacctggtat atgtggaccg ccgcaataac 9900
   gttctgaaac tggcgcaggg tgaatttgtc gcagtcgcaa acctggaagc cgttttcagc 9960
   ggtgccgcgc tggttcgcca aatcttcgtg tacggcaaca gcgagcgcag cttcctgttg 10020
   gcggtggtgg tgccgacgcc agaagccctg gaacaatacg atccggcagc cctgaaggcg 10080
   gccttggcag atagcctgca gcgcacggct cgtgatgcgg agctgcaatc gtatgaagtt 10140
   ccggccgatt tcattgtgga aaccgagccg ttcagcgccg caaatggtct gttgagcggt 10200
   gtcggcaagt tgctgcgtcc gaacctgaaa gatcgttatg gtcaacgtct ggagcaaatg 10260
   tatgccgata ttgccgcgac ccaggcgaat cagctgcgcg agctgcgtcg tgccgcagcc 10320
   acccagccgg tgattgatac cctgacgcag gctgctgcga cgattttggg taccggtagc 10380
   gaagtggcgt ctgatgccca ctttacggat ctgggtggcg acagcctgtc cgctctgacc 10440
   ttgtccaatt tgctgagcga tttctttggt tttgaggttc cggttggtac cattgttaac 10500
   ccggcgacta atctggcgca gctggcacag cacatcgaag cacagcgcac cgccggtgac 10560
   cgccgtccga gctttaccac ggtgcatggt gcggacgcga cggaaattcg tgcgagcgaa 10620
   ctgaccctgg ataagtttat tgacgcagaa accctgcagg cagcaccagg cctgccgaag 10680
   gtgaccaccg agccgcgcac cgtcctgctg agcggtgcca atggttggtt gggtcgcttc 10740
   ctgaccctgc aatggctgga acgtctggcg cctgttggtg gtacgctgat caccattgtc 10800
   cgcggtcgtg acgacgctgc ggctcgtgct cgcctgaccc aagcttacga caccgaccca 10860
   gagttgtctc gccgcttcgc agaactggcg gaccgccatc tgcgtgttgt agcaggcgat 10920
   atcggtgatc cgaatctggg cctgacccca gaaatctggc accgtctggc tgctgaggtg 10980
   gatttggttg tgcatccagc ggcgttggtg aaccacgtct tgccgtatcg tcagctgttc 11040
   ggcccgaacg tcgtgggcac cgccgaagtg attaagctgg cgctgacgga gcgcatcaag 11100
   ccagttacct acctgagcac cgtcagcgtt gcaatgggta tcccggactt tgaagaggat 11160
   ggtgatatcc gtaccgtttc ccctgttcgt ccgctggacg gcggctatgc taatggctac 11220
   ggcaactcta agtgggctgg tgaggtcctg ctgcgtgagg cccacgacct gtgtggcctg 11280
   ccggtggcca cgttccgtag cgacatgatt ttggcacacc cgcgctaccg tggtcaggtt 11340
   aatgtgccgg atatgttcac tcgtctgttg ttgtcgctgc tgatcactgg cgtggctccg 11400
   cgctctttct acattggtga tggtgaacgt cctcgtgcgc attatccggg tctgactgtc 11460
   gactttgtgg ccgaggccgt gacgaccctg ggtgcacagc agcgtgaagg ctacgttagc 11520
   tacgatgtca tgaacccgca tgacgatggc atcagcctgg atgttttcgt ggactggctg 11580
   atccgcgcag gccatccgat tgaccgtgtg gacgattacg acgattgggt gcgtcgcttc 11640
   gagactgcac tgacggcgct gccggaaaaa cgtcgcgcac aaaccgttct gccgttgctg 11700
   cacgcgttcc gtgccccgca agcgccgctg cgtggtgccc cggagccaac cgaggttttc 11760
   catgcggcgg ttcgtaccgc aaaagtcggt ccaggcgaca ttccgcactt ggatgaagcg 11820
   ctgattgata agtacatccg tgatctgcgt gagtttggcc tgatctaata agagaaggag 11880
   ttctatcatg attgaaacca tcttaccagc cggcgtcgaa tcagcagagt tacttgagta 11940
   ccctgaagat ctgaaggcgc atccggctga agagcatctg atcgcaaaga gcgtagagaa 12000
   gcgtcgtcgc gacttcattg gcgcacgtca ttgcgcccgt ctggcactgg cggagctggg 12060
   tgaaccgccg gttgcgattg gcaagggtga acgtggtgcg ccgatttggc cgcgtggtgt 12120
   cgtgggctct ctgacccatt gcgatggcta tcgcgcagcg gcggttgctc acaaaatgcg 12180
   ttttcgcagc atcggcatcg acgccgaacc gcacgcgacc ctgccggaag gtgtcctgga 12240
   ttcggttagc ctgccgcctg agcgtgagtg gctgaaaacc accgacagcg cactgcacct 12300
   ggaccgtttg ctgttttgtg cgaaagaagc aacttacaaa gcgtggtggc cgctgacggc 12360
   acgttggctg ggtttcgaag aagcgcacat taccttcgaa atcgaggatg gtagcgccga 12420
   ctctggtaat ggcacgtttc acagcgaact gctggtgccg ggtcagacca atgacggtgg 12480
   taccccgctg ctgtctttcg acggtcgctg gctgatcgct gatggcttca tcctgacggc 12540
   gattgcatac gcatgataaa ttaacctagg ctgctgccac cgctgagcaa taactagcat 12600
   aaccccttgg ggcctctaaa cgggtcttga ggggtttttt gcctcgagtg accccagccg 12660
   cccctcatgc caaccgagcc cattaagcgc agagtcggcc gcaactatct cggggacgca 12720
   taaacacgca gcgatttacc aaggagttcg gctaggtcaa tccgcaattc cctttgcctg 12780
   gccggagatg ccgcgagcaa acaatgtgtc cggtcgctac cagatggact ttgtgggcgg 12840
   aggattgcac cagacgcttc agcgcagcgt cgtacgatga atcggaaggc ccggtaagca 12900
   attgaagaac cgctgaccac ctcccatccg acaatgcaag ggtgccccgc tcccagcgcg 12960
   acacggttgc ctgatccaca cccattaatt cagctaggtg actttgcttc atatgacgaa 13020
   gcaaccgcgc cctccgcacc tgccgccccc tgtcattcgt caacattctt cagcacctca 13080
   atgtcgttcg tagtgagcct cattttttca agaccgccga tgatgagagc caggcctcgc 13140
   tcgaatgccg cgtccggacc gccttcgtag acgattttca tcgcgctctg taggcgcgcc 13200
   ggcatcgtag acgctgaggt ggtcaactga tcttcgcccc gctcctcggc gtctgcctcg 13260
   ctagcttgct gctcaagaac agcgccgacg gtgaagtagc tgattgccat caacgcatag    13320
   gtcgcgtcac ctgccgaaaa gccagcatcg caaaggaagc gaagctgcgc gtcggctttt    13380
   tccatctgcg gcgcggctgg ccgcgtcccg gcatgaatac gcgcgccatc gcgataagcg    13440
   agcaacgccc gtcgaaaact gcatgcattg cccttcagga acgaacgcca gtcgtcgtca    13500
   tcccttggcg tcgaatgcgt gtgatttatc gtcagcatgg cttcggcaag tgcgtcgagc    13560
   aacgcacgct tgttcttgaa atgccagtag agcgctggct gttgcacccc gaggcgctca    13620
   gccagtcggc gcgtcgttag accttccatg cccacgtcgt taagcagttc gagcgcggtt    13680
   cggatcacgg cctcgcgttg gagcttgttc attcgcgaa                           13719
<210> 19
   <211> 13785
   <212> DNA
   <213> Artificial Sequence
<220>
   <223> vector
<400> 19
   ttctcatgtt tgacagctta tcatcgataa gctttaatgc ggtagtttat cacagttaaa 60
   ttgctaacgc agtcaggcac cgtgtatgaa atctaacaat gcgctcatcg tcatcctcgg 120
   caccgtcacc ctggatgctg taggcatagg cttggttatg ccggtactgc cgggcctctt 180
   gcgggatatc gtccattccg acagcatcgc cagtcactat ggcgtgctgc tagcgctata 240
   tgcgttgatg caatttctat gcgcacccgt tctcggagca ctgtccgacc gctttggccg 300
   ccgcccagtc ctgctcgctt cgctacttgg agccactatc gactacgcga tcatggcgac 360
   cacacccgtc ctgtggatcc tctacgccgg acgcatcgtg gccggcatca ccggcgccac 420
   aggtgcggtt gctggcgcct atatcgccga catcaccgat ggggaagatc gggctcgcca 480
   cttcgggctc atgagcgctt gtttcggcgt gggtatggtg gcaggccccg tggccggggg 540
   actgttgggc gccatctcct tgcatgcacc attccttgcg gcggcggtgc tcaacggcct 600
   caacctacta ctgggctgct tcctaatgca ggagtcgcat aagggagagc gtcgaccgat 660
   gcccttgaga gccttcaacc cagtcagctc cttccggtgg gcgcggggca tgactatcgt 720
   cgccgcactt atgactgtct tctttatcat gcaactcgta ggacaggtgc cggcagcgct 780
   ctgggtcatt ttcggcgagg accgctttcg ctggagcgcg acgatgatcg gcctgtcgct 840
   tgcggtattc ggaatcttgc acgccctcgc tcaagccttc gtcactggtc ccgccaccaa 900
   acgtttcggc gagaagcagg ccattatcgc cggcatggcg gccgacgcgc tgggctacgt 960
   cttgctggcg ttcgcgacgc gaggctggat ggccttcccc attatgattc ttctcgcttc 1020
   cggcggcatc gggatgcccg cgttgcaggc catgctgtcc aggcaggtag atgacgacca 1080
   tcagggacag cttcaaggat cgctcgcggc tcttaccagc ctaacttcga tcactggacc 1140
   gctgatcgtc acggcgattt atgccgcctc ggcgagcaca tggaacgggt tggcatggat 1200
   tgtaggcgcc gccctatacc ttgtctgcct ccccgcgttg cgtcgcggtg catggagccg 1260
   ggccacctcg acctgaatgg aagccggcgg cacctcgcta acggattcac cactccaaga 1320
   attggagcca atcaattctt gcggagaact gtgaatgcgc aaaccaaccc ttggcagaac 1380
   atatccatcg cgtccgccat ctccagcagc cgcacgcggc gcatctcggg cagcgttggg 1440
   tcctggccac gggtgcgcat gatcgtgctc ctgtcgttga ggacccggct aggctggcgg 1500
   ggttgcctta ctggttagca gaatgaatca ccgatacgcg agcgaacgtg aagcgactgc 1560
   tgctgcaaaa cgtctgcgac ctgagcaaca acatgaatgg tcttcggttt ccgtgtttcg 1620
   taaagtctgg aaacgcggaa gtcccctacg tgctgctgaa gttgcccgca acagagagtg 1680
   gaaccaaccg gtgataccac gatactatga ctgagagtca acgccatgag cggcctcatt 1740
   tcttattctg agttacaaca gtccgcaccg ctgccggtag ctacttgact atccggctgc 1800
   actagccctg cgtcagatgg ctctgatcca aggcaaactg ccaaaatatc tgctggcacc 1860
   ggaagtcagc gccctgcacc attatgttcc ggatctgcat cgcaggatgc tgctggctac 1920
   cctgtggaac acctacatct gtattaacga agcgctggca ttgaccctga gtgatttttc 1980
   tctggtgccg ccctatccct ttgtgcagct tgccacgctc aaaggggttt gaggtccaac 2040
   cgtacgaaaa cgtacggtaa gaggaaaatt atcgtctgaa aaatcgatta gtagacaaga 2100
   aagtccgtta agtgccaatt ttcgattaaa aagacaccgt tttgatggcg ttttccaatg 2160
   tacattatgt ttcgatatat cagacagtta cttcactaac gtacgttttc gttctattgg 2220
   ccttcagacc ccatatcctt aatgtccttt atttgctggg gttatcagat ccccccgaca 2280
   cgtttaatta atgctttctc cgccggagat cgacgcacag gcttctgtgt ctatgatgtt 2340
   atttcttaat aatcatccag gtattctctt tatcaccata cgtagtgcga gtgtccacct 2400
   taacgcaggg ctttccgtca cagcgcgata tgtcagccag cggggctttc ttttgccaga 2460
   ccgcttccat cctctgcatt tcagcaatct ggctataccc gtcattcata aaccacgtaa 2520
   atgccgtcac gcaggaagcc aggacgaaga atatcgtcag tacaagataa atcgcggatt 2580
   tccacgtata gcgtgacatc tcacgacgca tttcatggat catcgctttc gccgtatcgg 2640
   cagcctgatt cagcgcttct gtcgccggtt tctgctgtgc taatccggct tgtttcagtt 2700
   ctttctcaac ctgagtgagc gcggaactca ccgatttcct gacggtgtca gtcatattac 2760
   cggacgcgct gtccagctca cgaatgaccc tgctcagcgt ttcactttgc tgctgtaatt 2820
   gtgatgaggc ggcctgaaac tgttctgtca gagaagtaac acgcttttcc agcgcctgat 2880
   gatgcccgat aagggcggca atttgtttaa tttcgtcgct catacaaaat cctgcctatc 2940
   gtgagaatga ccagccttta tccggcttct gtcgtatctg ttcggcgagt cgctgtcgtt 3000
   ctttctcctg ctgacgctgt ttttccgcca gacgttcgcg ctctctctgc ctttccatct 3060
   cctgatgtat cccctggaac tccgccatcg catcgttaac aagggactga agatcgattt 3120
   cttcctgtat atccttcatg gcatcactga ccagtgcgtt cagcttgtca ggctcttttt 3180
   caaaatcaaa cgttctgccg gaatgggatt cctgctcagg ctctgacttc agctcctgtt 3240
   ttagcgtcag agtatccctc tcgctgaggg cttcccgtaa cgaggtagtc acgtcaatta 3300
   cgctgtcacg ttcatcacgg gactgctgca cctgcctttc agcctccctg cgctcaagaa 3360
   tggcctgtag ctgctcagta tcgaatcgct gaacctgacc cgcgcccaga tgccgctcag 3420
   gctcacggtc aatgccctgc gccttcaggg aacgggaatc aacccggtca gcgtgctgat 3480
   accgttcaag gtgcttattc tggaggtcag cccagcgtct ccctctgggc aacaaggtat 3540
   tctttgcgtt cggtcggtgt ttccccgaaa cgtgcctttt ttgcgccacc gcgtccggct 3600
   ctttggtgtt agcccgttta aaatactgct cagggtcacg gtgaataccg tcattaatgc 3660
   gttcagagaa catgatatgg gcgtggggct gctcgccacc ggctatcgct gctttcggat 3720
   tatggatagc gaactgatag gcatggcggt cgccaatttc ctgttggaca aaatcgcgga 3780
   caagctcaag acgttgttcg ggttttaact cacgcggcag ggcaatctcg atttcacggt 3840
   aggtacagcc gttggcacgt tcagacgtgt cagcggcttt ccagaactcg gacggtttat 3900
   gcgctgccca cgccggcata ttgccggact ccttgtgctc aaggtcggag tctttttcac 3960
   gggcatactt tccctcacgc gcaatataat cggcatgagg agaggcactg ccttttccgc 4020
   cggtttttac gctgagatga taggatgcca tcgtgtttta tcccgctgaa gggcgcacgt 4080
   ttctgaacga agtgaagaaa gtctaagtgc gccctgataa ataaaagagt tatcagggat 4140
   tgtagtggga tttgacctcc tctgccatca tgagcgtaat cattccgtta gcattcagga 4200
   ggtaaacagc atgaataaaa gcgaaaaaac aggaacaatg ggcagcagaa agagtgcagt 4260
   atattcgcgg cttaaagtcg ccgaatgagc aacagaaact tatgctgata ctgacggata 4320
   aagcagataa aacagcacag gatatcaaaa cgctgtccct gctgatgaag gctgaacagg 4380
   cagcagagaa agcgcaggaa gccagagcga aagtcatgaa cctgatacag gcagaaaagc 4440
   gagccgaagc cagagccgcc cgtaaagccc gtgaccatgc tctgtaccag tctgccggat 4500
   tgcttatcct ggcgggtctg gttgacagta agacgggtaa gcctgttgat gataccgctg 4560
   ccttactggg tgcattagcc agtctgaatg acctgtcacg ggataatccg aagtggtcag 4620
   actggaaaat cagagggcag gaactgctga acagcaaaaa gtcagatagc accacatagc 4680
   agacccgcca taaaacgccc tgagaagccc gtgacgggct tttcttgtat tatgggtagt 4740
   ttccttgcat gaatccataa aaggcgcctg tagtgccatt tacccccatt cactgccaga 4800
   gccgtgagcg cagcgaactg aatgtcacga aaaagacagc gactcaggtg cctgatggtc 4860
   ggagacaaaa ggaatattca gcgatttgcc cgagcttgcg agggtgctac ttaagccttt 4920
   agggttttaa ggtctgtttt gtagaggagc aaacagcgtt tgcgacatcc ttttgtaata     4980
   ctgcggaact gactaaagta gtgagttata cacagggctg ggatctattc tttttatctt     5040
   tttttattct ttctttattc tataaattat aaccacttga atataaacaa aaaaaacaca     5100
   caaaggtcta gcggaattta cagagggtct agcagaattt acaagttttc cagcaaaggt     5160
   ctagcagaat ttacagatac ccacaactca aaggaaaagg actagtaatt atcattgact     5220
   agcccatctc aattggtata gtgattaaaa tcacctagac caattgagat gtatgtctga     5280
   attagttgtt ttcaaagcaa atgaactagc gattagtcgc tatgacttaa cggagcatga     5340
   aaccaagcta attttatgct gtgtggcact actcaacccc acgattgaaa accctacaag     5400
   gaaagaacgg acggtatcgt tcacttataa ccaatacgct cagatgatga acatcagtag     5460
   ggaaaatgct tatggtgtat tagctaaagc aaccagagag ctgatgacga gaactgtgga     5520
   aatcaggaat cctttggtta aaggctttga gattttccag tggacaaact atgccaagtt     5580
   ctcaagcgaa aaattagaat tagtttttag tgaagagata ttgccttatc ttttccagtt     5640
   aaaaaaattc ataaaatata atctggaaca tgttaagtct tttgaaaaca aatactctat     5700
   gaggatttat gagtggttat taaaagaact aacacaaaag aaaactcaca aggcaaatat     5760
   agagattagc cttgatgaat ttaagttcat gttaatgctt gaaaataact accatgagtt     5820
   taaaaggctt aaccaatggg ttttgaaacc aataagtaaa gatttaaaca cttacagcaa     5880
   tatgaaattg gtggttgata agcgaggccg cccgactgat acgttgattt tccaagttga     5940
   actagataga caaatggatc tcgtaaccga acttgagaac aaccagataa aaatgaatgg     6000
   tgacaaaata ccaacaacca ttacatcaga ttcctaccta cgtaacggac taagaaaaac     6060
   actacacgat gctttaactg caaaaattca gctcaccagt tttgaggcaa aatttttgag     6120
   tgacatgcaa agtaagcatg atctcaatgg ttcgttctca tggctcacgc aaaaacaacg     6180
   aaccacacta gagaacatac tggctaaata cggaaggatc tgaggttctt atggctcttg     6240
   tatctatcag tgaagcatca agactaacaa acaaaagtag aacaactgtt caccgttaga     6300
   tatcaaaggg aaaactgtcc atatgcacag atgaaaacgg tgtaaaaaag atagatacat     6360
   cagagctttt acgagttttt ggtgcattta aagctgttca ccatgaacag atcgacaatg     6420
   taacagatga acagcatgta acacctaata gaacaggtga aaccagtaaa acaaagcaac     6480
   tagaacatga aattgaacac ctgagacaac ttgttacagc tcaacagtca cacatagaca     6540
   gcctgaaaca ggcgatgctg cttatcgaat caaagctgcc gacaacacgg gagccagtga     6600
   cgcctcccgt ggggaaaaaa tcatggcaat tctggaagaa atagcgcttt cagccggcaa     6660
   acctgaagcc ggatctgcga ttctgataac aaactagcaa caccagaaca gcccgtttgc     6720
   gggcagcaaa acccgtactt ttggacgttc cggcggtttt ttgtggcgag tggtgttcgg     6780
   gcggtgcgcg caagatccat tatgttaaac gggcgagttt acatctcaaa accgcccgct 6840
   taacaccatc agaaatcctc agcgcgattt taagcaccaa cccccccccg taacacccaa 6900
   atccatactg aaagtggctt tgttgaataa atcgaacttt tgctgagttg aaggatcaga 6960
   tcacgcatcc tcccgacaac acagaccatt ccgtggcaaa gcaaaagttc agaatcacca 7020
   actggtccac ctacaacaaa gctctcatca accgtggctc cctcactttc tggctggatg 7080
   atgaggcgat tcaggcctgg tatgagtcgg caacaccttc atcacgagga aggccccagc 7140
   gctattctga tctcgccatc accaccgttc tggtgattaa acgcgtattc cggctgaccc 7200
   tgcgggctgc gcagggtttt attgattcca tttttgccct gatgaacgtt ccgttgcgct 7260
   gcccggatta caccagtgtc agtaagcggg caaagtcggt taatgtcagt ttcaaaacgt 7320
   ccacccgggg tgaaatcgca cacctggtga ttgattccac cgggctgaag gtctttggtg 7380
   aaggcgaatg gaaagtcaga aagcacggca aagagcgccg tcgtatctgg cgaaagttgc 7440
   atcttgctgt tgacagcaac acacatgaag ttgtctgtgc agacctgtcg ctgaataacg 7500
   tcacggactc agaagccttc ccgggcctta tccggcagac tcacagaaaa atcagggcag 7560
   ccgcggcaga cggggcttac gatacccggc tctgtcacga tgaactgcgc cgcaaaaaaa 7620
   tcagcgcgct tattcctccc cgaaaaggtg cgggttactg gcccggtgaa tatgcagacc 7680
   gtaaccgtgc agtggctaat cagcgaatga ccgggagtaa tgcgcggtgg aaatggacaa 7740
   cagattacaa ccgtcgctcg atagcggaaa cggcgatgta ccgggtaaaa cagctgttcg 7800
   ggggttcact gacgctgcgt gactacgatg gtcaggttgc ggaggctatg gccctggtac 7860
   gagcgctgaa caaaatgacg aaagcaggta tgcctgaaag cgtgcgtatt gcctgaaaac 7920
   acaacccgct acgggggaga cttacccgaa atctgattta ttcaacaaag ccgggtgtgg 7980
   tgaactacaa agcagacccg ttgaggttat cagttcgatg cacaatcagc agcgcataaa 8040
   atatgcacaa gaacaggagc acccttcgca ttaagctgtg gtggtaacaa gtagtgccgg 8100
   gctaccatca gcgagcatga tgcgctccca cagcattcgc cttggcagta tggaagttcc 8160
   tcgctccagt tcgggccggt atccacctcg cacctggtgt ttaaacggat ttaaattgca 8220
   ggggcagtga gcgcaacgca attaatgtaa gttagctcac tcattaggca ccccaggctt 8280
   gacactttat gcttccggct cgtataatgt gtggaattgt gagcggataa caataacaat 8340
   ttaaatcagg atctaggaac caaggagagt ggcatatgag cactgcaatt cacgatgaac 8400
   acttggatcg ccgcattgaa gaactgattg caaacgatcc gcaattcgct gcggcacgcc 8460
   cggacccggc gattaccgca gcgaccgagg ccccaggtct gcgtctgccg cagatcatcc 8520
   gtacggtcct ggacggttat gccgatcgtc cggcgctggc gcaacgcgtg gttgaattcg 8580
   ttaccgacgc caaaaccggt cgtaccaccg cagagttgct gccacgtttt gaaaccatca 8640
   cgtacggtga gctgggtgag cgtgtgagcg cactgggtcg cgcatgggcc ggtgacgccg 8700
   tgcgccctgg cgaccgcgtt tgcgtcctgg gtttcaactc cgtggattac gcgacgattg 8760
   acatcgcgct gggtaccatc ggtgcggtga gcgtgccgct gcaaaccagc gcagcgatta 8820
   gcagcctgca gccgattgtg gcggaaaccg agccgagcct gattgcgagc agcgtgaatc 8880
   agctgccgga cgccgtcgag ctgattctgg caggtgacca tgttccgggt aagctggtcg 8940
   tatttgatta tcaaccgcaa gttgatgacc agcgtgaggc ggttgaggca gcggctgcgc 9000
   gcctggcaga cagcggcgtc gcagtggaag cgctggcgga tgttctgcgt cgtggtaaag 9060
   acctgccggc agttgaaccg ccagcgtccg atgaggatag cctggctctg ttgatttaca 9120
   cctctggcag caccggtgcg ccgaagggcg cgatgtaccc gcagagcaac gtgggtaaaa 9180
   tgtggcgtcg cggtagcaag aactggtttg gtgagagcgc cgcaagcatc accctgaatt 9240
   tcatgccgat gagccatgtt atgggtcgcg gtattttgta tggcacgttg ggtaacggcg 9300
   gcaccgcgta tttcgcggct cgtagcgatt tgtctacgct gctggaagat ctggagttgg 9360
   tgcgccctac ggagatgaat tttgtcccgc gtatttggga aacgctgtat ggtgagttcc 9420
   agcgtcaggt ggagcgccgt ctggcggacg gtgacgctgg cccggaagcc cgtgaaaccg 9480
   tcgaggcggc agtgctggaa gagcagcgtc aatatctgct gggtggccgt tttatcttcg 9540
   ccatgaccgg ctctgcgccg acgagcccgg agctgaaagc atgggcggag agcttgctgc 9600
   aaatgcacct gatggacggt tacggcagca ccgaggcggg catggtgctg ttcgacggtg 9660
   agatccagcg tccgccggtc atcgattaca aactggtgga tgtcccggat ctgggttact 9720
   tttcgacgga ccgtccgcat ccgcgtggtg agctgttgct gcgtaccgag aacatgttcc 9780
   cgggttacta taaacgcgcg gaaaccaccg caaatgtttt cgacgaggat ggttactacc 9840
   gcaccggcga tgtgttcgcg gagatcgcac cggaccgttt ggtttacgtc gatcgtcgca 9900
   ataacgtctt gaaactggcc cagggtgagt ttgtaaccct ggccaaactg gaagcggttt 9960
   tcggtaactc gccgctgatc cgccagatct acgtgtacgg caacagcagc cagccgtacc 10020
   tgctggcggt ggtggtcccg accgaagagg cgctggccga taatgacttg gaaagcttga 10080
   agccgaaaat cgcagactcc ctgcaaaaag ttgcaaaaga aactggcctg cagtcctatg 10140
   aggttccgcg tgatttcatc atcgagacta cgccgttcac cctggagaac ggtttgctga 10200
   ccggcattcg taagctggcg tggcctaagc tgaaggccca ctatggcgat cgtctggagc 10260
   aaatgtacgc tgagctggct gcgggtcagg cgaatgagct ggcggagctg cgccgtagcg 10320
   gtgcggcagc gccggtggcc cagaccgtgt cccgcgcagc agcggcgctg ctgggcgctg 10380
   ctgctggtga tctgagcgcg gatgcccact ttactgacct gggtggtgat tccctgtccg 10440
   cactgacctt tggtaacctg ctgcgtgaga tttttgacgt cgatgttccg gttggcgtta 10500
   ttgtctcccc tgctaacgac ttggcgggca ttgccgcata cattgaagca gagcgtcaag 10560
   gtagcaaacg tccgacgttc gcagcagtgc atggtcgcgg tgccacgatg gtgcatgcga 10620
   gcgacttgac tctggataag tttctggacg aagccaccct ggcagcagcc ccaagcctgc 10680
   cgaaaccggc gacggaagtt cgtacggttc tgctgaccgg tgcgacgggt tttctgggcc 10740
   gttatctggc actggactgg ctggagcgca tggatatggt tgacggcaaa gttattgcgc 10800
   tggtccgtgc gcgcaccgat gaagaagcgc gtgcgcgttt ggacaagacg tttgacagcg 10860
   gcgacccgaa actgctggct cactaccaac gtctggcagc ggaccacttg gaagttatcg 10920
   cgggtgacaa aggtgaggct aatctgggct tggacccgca aacctggcag cgtctggccg 10980
   aagaagtgga cgtgatcgtt gacccggcag cgctggtgaa ccatgtcctg ccgtactctg 11040
   aattgtttgg cccgaacgca ctgggcacgg ccgaactgat ccgcatcgcg ctgacctcta 11100
   agcaaaaacc gtatacgtat gttagcacca ttggtgttgg cgatcagatt caaccgggtg 11160
   agttcgttga aaatgctgat atccgtcaga tcagcgcgac gcgtgagatc aacgatggct 11220
   acgctaatgg ttatggcaat agcaagtggg cgggtgaggt tctgctgcgc gaggcgcacg 11280
   acctgtgtgg cctgccggtc accgtgttcc gttgcgatat gatcctggct gacaccacgt 11340
   acgctggcca gctgaatctg ccggacatgt ttacccgttt gatgctgagc ctggtcgcga 11400
   ccggtatcgc accgggcagc ttttacgaac tggacacgga tggcaatcgc cagcgtgtcc 11460
   actatgatgg tctgccagtc gagttcattg ctgcggccat cagcaccctg ggcacccaaa 11520
   tcacggatag cgacaccggt ttccagactt atcatgtaat gaatccatac gatgacggta 11580
   ttggtctgga tgagtacatt gactggctga ttgaggctgg ttacagcatc gaacgtattg 11640
   cggactatag cgaatggctg cgtcgcttcg aaacgtcgct gcgtgccctg cctgaccgtc 11700
   aacgccagta ttcgctgctg ccgctgttgc acaattatca aaagccggag aagccgatta 11760
   acggtagcat ggcgccaact gatgtctttc gtgcagccgt tcaagaagcg aagatcggtc 11820
   cggataagga cattccgcac gtctctgcgc cggtgattgt caagtacatc accgacctgg 11880
   agctgctggg tttgctgtaa taagtttaaa cagagaagga gttctatcat gattgaaacc 11940
   atcttaccag ccggcgtcga atcagcagag ttacttgagt accctgaaga tctgaaggcg 12000
   catccggctg aagagcatct gatcgcaaag agcgtagaga agcgtcgtcg cgacttcatt 12060
   ggcgcacgtc attgcgcccg tctggcactg gcggagctgg gtgaaccgcc ggttgcgatt 12120
   ggcaagggtg aacgtggtgc gccgatttgg ccgcgtggtg tcgtgggctc tctgacccat 12180
   tgcgatggct atcgcgcagc ggcggttgct cacaaaatgc gttttcgcag catcggcatc 12240
   gacgccgaac cgcacgcgac cctgccggaa ggtgtcctgg attcggttag cctgccgcct 12300
   gagcgtgagt ggctgaaaac caccgacagc gcactgcacc tggaccgttt gctgttttgt 12360
   gcgaaagaag caacttacaa agcgtggtgg ccgctgacgg cacgttggct gggtttcgaa 12420
   gaagcgcaca ttaccttcga aatcgaggat ggtagcgccg actctggtaa tggcacgttt 12480
   cacagcgaac tgctggtgcc gggtcagacc aatgacggtg gtaccccgct gctgtctttc    12540
   gacggtcgct ggctgatcgc tgatggcttc atcctgacgg cgattgcata cgcatgataa    12600
   attaacctag gctgctgcca ccgctgagca ataactagca taaccccttg gggcctctaa    12660
   acgggtcttg aggggttttt tgctgaaacc tcaggcattt gagaagccct cgagtgaccc    12720
   cagccgcccc tcatgccaac cgagcccatt aagcgcagag tcggccgcaa ctatctcggg    12780
   gacgcataaa cacgcagcga tttaccaagg agttcggcta ggtcaatccg caattccctt    12840
   tgcctggccg gagatgccgc gagcaaacaa tgtgtccggt cgctaccaga tggactttgt    12900
   gggcggagga ttgcaccaga cgcttcagcg cagcgtcgta cgatgaatcg gaaggcccgg    12960
   taagcaattg aagaaccgct gaccacctcc catccgacaa tgcaagggtg ccccgctccc    13020
   agcgcgacac ggttgcctga tccacaccca ttaattcagc taggtgactt tgcttcatat    13080
   gacgaagcaa ccgcgccctc cgcacctgcc gccccctgtc attcgtcaac attcttcagc    13140
   acctcaatgt cgttcgtagt gagcctcatt ttttcaagac cgccgatgat gagagccagg    13200
   cctcgctcga atgccgcgtc cggaccgcct tcgtagacga ttttcatcgc gctctgtagg    13260
   cgcgccggca tcgtagacgc tgaggtggtc aactgatctt cgccccgctc ctcggcgtct    13320
   gcctcgctag cttgctgctc aagaacagcg ccgacggtga agtagctgat tgccatcaac    13380
   gcataggtcg cgtcacctgc cgaaaagcca gcatcgcaaa ggaagcgaag ctgcgcgtcg    13440
   gctttttcca tctgcggcgc ggctggccgc gtcccggcat gaatacgcgc gccatcgcga    13500
   taagcgagca acgcccgtcg aaaactgcat gcattgccct tcaggaacga acgccagtcg    13560
   tcgtcatccc ttggcgtcga atgcgtgtga tttatcgtca gcatggcttc ggcaagtgcg    13620
   tcgagcaacg cacgcttgtt cttgaaatgc cagtagagcg ctggctgttg caccccgagg    13680
   cgctcagcca gtcggcgcgt cgttagacct tccatgccca cgtcgttaag cagttcgagc    13740
   gcggttcgga tcacggcctc gcgttggagc ttgttcattc gcgaa                    13785
<210> 20
   <211> 13731
   <212> DNA
   <213> Artificial Sequence
<220>
   <223> vector
<400> 20
   ttctcatgtt tgacagctta tcatcgataa gctttaatgc ggtagtttat cacagttaaa       60
   ttgctaacgc agtcaggcac cgtgtatgaa atctaacaat gcgctcatcg tcatcctcgg      120
   caccgtcacc ctggatgctg taggcatagg cttggttatg ccggtactgc cgggcctctt      180
   gcgggatatc gtccattccg acagcatcgc cagtcactat ggcgtgctgc tagcgctata      240
   tgcgttgatg caatttctat gcgcacccgt tctcggagca ctgtccgacc gctttggccg 300
   ccgcccagtc ctgctcgctt cgctacttgg agccactatc gactacgcga tcatggcgac 360
   cacacccgtc ctgtggatcc tctacgccgg acgcatcgtg gccggcatca ccggcgccac 420
   aggtgcggtt gctggcgcct atatcgccga catcaccgat ggggaagatc gggctcgcca 480
   cttcgggctc atgagcgctt gtttcggcgt gggtatggtg gcaggccccg tggccggggg 540
   actgttgggc gccatctcct tgcatgcacc attccttgcg gcggcggtgc tcaacggcct 600
   caacctacta ctgggctgct tcctaatgca ggagtcgcat aagggagagc gtcgaccgat 660
   gcccttgaga gccttcaacc cagtcagctc cttccggtgg gcgcggggca tgactatcgt 720
   cgccgcactt atgactgtct tctttatcat gcaactcgta ggacaggtgc cggcagcgct 780
   ctgggtcatt ttcggcgagg accgctttcg ctggagcgcg acgatgatcg gcctgtcgct 840
   tgcggtattc ggaatcttgc acgccctcgc tcaagccttc gtcactggtc ccgccaccaa 900
   acgtttcggc gagaagcagg ccattatcgc cggcatggcg gccgacgcgc tgggctacgt 960
   cttgctggcg ttcgcgacgc gaggctggat ggccttcccc attatgattc ttctcgcttc 1020
   cggcggcatc gggatgcccg cgttgcaggc catgctgtcc aggcaggtag atgacgacca 1080
   tcagggacag cttcaaggat cgctcgcggc tcttaccagc ctaacttcga tcactggacc 1140
   gctgatcgtc acggcgattt atgccgcctc ggcgagcaca tggaacgggt tggcatggat 1200
   tgtaggcgcc gccctatacc ttgtctgcct ccccgcgttg cgtcgcggtg catggagccg 1260
   ggccacctcg acctgaatgg aagccggcgg cacctcgcta acggattcac cactccaaga 1320
   attggagcca atcaattctt gcggagaact gtgaatgcgc aaaccaaccc ttggcagaac 1380
   atatccatcg cgtccgccat ctccagcagc cgcacgcggc gcatctcggg cagcgttggg 1440
   tcctggccac gggtgcgcat gatcgtgctc ctgtcgttga ggacccggct aggctggcgg 1500
   ggttgcctta ctggttagca gaatgaatca ccgatacgcg agcgaacgtg aagcgactgc 1560
   tgctgcaaaa cgtctgcgac ctgagcaaca acatgaatgg tcttcggttt ccgtgtttcg 1620
   taaagtctgg aaacgcggaa gtcccctacg tgctgctgaa gttgcccgca acagagagtg 1680
   gaaccaaccg gtgataccac gatactatga ctgagagtca acgccatgag cggcctcatt 1740
   tcttattctg agttacaaca gtccgcaccg ctgccggtag ctacttgact atccggctgc 1800
   actagccctg cgtcagatgg ctctgatcca aggcaaactg ccaaaatatc tgctggcacc 1860
   ggaagtcagc gccctgcacc attatgttcc ggatctgcat cgcaggatgc tgctggctac 1920
   cctgtggaac acctacatct gtattaacga agcgctggca ttgaccctga gtgatttttc 1980
   tctggtgccg ccctatccct ttgtgcagct tgccacgctc aaaggggttt gaggtccaac 2040
   cgtacgaaaa cgtacggtaa gaggaaaatt atcgtctgaa aaatcgatta gtagacaaga 2100
   aagtccgtta agtgccaatt ttcgattaaa aagacaccgt tttgatggcg ttttccaatg 2160
   tacattatgt ttcgatatat cagacagtta cttcactaac gtacgttttc gttctattgg 2220
   ccttcagacc ccatatcctt aatgtccttt atttgctggg gttatcagat ccccccgaca 2280
   cgtttaatta atgctttctc cgccggagat cgacgcacag gcttctgtgt ctatgatgtt 2340
   atttcttaat aatcatccag gtattctctt tatcaccata cgtagtgcga gtgtccacct 2400
   taacgcaggg ctttccgtca cagcgcgata tgtcagccag cggggctttc ttttgccaga 2460
   ccgcttccat cctctgcatt tcagcaatct ggctataccc gtcattcata aaccacgtaa 2520
   atgccgtcac gcaggaagcc aggacgaaga atatcgtcag tacaagataa atcgcggatt 2580
   tccacgtata gcgtgacatc tcacgacgca tttcatggat catcgctttc gccgtatcgg 2640
   cagcctgatt cagcgcttct gtcgccggtt tctgctgtgc taatccggct tgtttcagtt 2700
   ctttctcaac ctgagtgagc gcggaactca ccgatttcct gacggtgtca gtcatattac 2760
   cggacgcgct gtccagctca cgaatgaccc tgctcagcgt ttcactttgc tgctgtaatt 2820
   gtgatgaggc ggcctgaaac tgttctgtca gagaagtaac acgcttttcc agcgcctgat 2880
   gatgcccgat aagggcggca atttgtttaa tttcgtcgct catacaaaat cctgcctatc 2940
   gtgagaatga ccagccttta tccggcttct gtcgtatctg ttcggcgagt cgctgtcgtt 3000
   ctttctcctg ctgacgctgt ttttccgcca gacgttcgcg ctctctctgc ctttccatct 3060
   cctgatgtat cccctggaac tccgccatcg catcgttaac aagggactga agatcgattt 3120
   cttcctgtat atccttcatg gcatcactga ccagtgcgtt cagcttgtca ggctcttttt 3180
   caaaatcaaa cgttctgccg gaatgggatt cctgctcagg ctctgacttc agctcctgtt 3240
   ttagcgtcag agtatccctc tcgctgaggg cttcccgtaa cgaggtagtc acgtcaatta 3300
   cgctgtcacg ttcatcacgg gactgctgca cctgcctttc agcctccctg cgctcaagaa 3360
   tggcctgtag ctgctcagta tcgaatcgct gaacctgacc cgcgcccaga tgccgctcag 3420
   gctcacggtc aatgccctgc gccttcaggg aacgggaatc aacccggtca gcgtgctgat 3480
   accgttcaag gtgcttattc tggaggtcag cccagcgtct ccctctgggc aacaaggtat 3540
   tctttgcgtt cggtcggtgt ttccccgaaa cgtgcctttt ttgcgccacc gcgtccggct 3600
   ctttggtgtt agcccgttta aaatactgct cagggtcacg gtgaataccg tcattaatgc 3660
   gttcagagaa catgatatgg gcgtggggct gctcgccacc ggctatcgct gctttcggat 3720
   tatggatagc gaactgatag gcatggcggt cgccaatttc ctgttggaca aaatcgcgga 3780
   caagctcaag acgttgttcg ggttttaact cacgcggcag ggcaatctcg atttcacggt 3840
   aggtacagcc gttggcacgt tcagacgtgt cagcggcttt ccagaactcg gacggtttat 3900
   gcgctgccca cgccggcata ttgccggact ccttgtgctc aaggtcggag tctttttcac 3960
   gggcatactt tccctcacgc gcaatataat cggcatgagg agaggcactg ccttttccgc 4020
   cggtttttac gctgagatga taggatgcca tcgtgtttta tcccgctgaa gggcgcacgt 4080
   ttctgaacga agtgaagaaa gtctaagtgc gccctgataa ataaaagagt tatcagggat 4140
   tgtagtggga tttgacctcc tctgccatca tgagcgtaat cattccgtta gcattcagga 4200
   ggtaaacagc atgaataaaa gcgaaaaaac aggaacaatg ggcagcagaa agagtgcagt 4260
   atattcgcgg cttaaagtcg ccgaatgagc aacagaaact tatgctgata ctgacggata 4320
   aagcagataa aacagcacag gatatcaaaa cgctgtccct gctgatgaag gctgaacagg 4380
   cagcagagaa agcgcaggaa gccagagcga aagtcatgaa cctgatacag gcagaaaagc 4440
   gagccgaagc cagagccgcc cgtaaagccc gtgaccatgc tctgtaccag tctgccggat 4500
   tgcttatcct ggcgggtctg gttgacagta agacgggtaa gcctgttgat gataccgctg 4560
   ccttactggg tgcattagcc agtctgaatg acctgtcacg ggataatccg aagtggtcag 4620
   actggaaaat cagagggcag gaactgctga acagcaaaaa gtcagatagc accacatagc 4680
   agacccgcca taaaacgccc tgagaagccc gtgacgggct tttcttgtat tatgggtagt 4740
   ttccttgcat gaatccataa aaggcgcctg tagtgccatt tacccccatt cactgccaga 4800
   gccgtgagcg cagcgaactg aatgtcacga aaaagacagc gactcaggtg cctgatggtc 4860
   ggagacaaaa ggaatattca gcgatttgcc cgagcttgcg agggtgctac ttaagccttt 4920
   agggttttaa ggtctgtttt gtagaggagc aaacagcgtt tgcgacatcc ttttgtaata 4980
   ctgcggaact gactaaagta gtgagttata cacagggctg ggatctattc tttttatctt 5040
   tttttattct ttctttattc tataaattat aaccacttga atataaacaa aaaaaacaca 5100
   caaaggtcta gcggaattta cagagggtct agcagaattt acaagttttc cagcaaaggt 5160
   ctagcagaat ttacagatac ccacaactca aaggaaaagg actagtaatt atcattgact 5220
   agcccatctc aattggtata gtgattaaaa tcacctagac caattgagat gtatgtctga 5280
   attagttgtt ttcaaagcaa atgaactagc gattagtcgc tatgacttaa cggagcatga 5340
   aaccaagcta attttatgct gtgtggcact actcaacccc acgattgaaa accctacaag 5400
   gaaagaacgg acggtatcgt tcacttataa ccaatacgct cagatgatga acatcagtag 5460
   ggaaaatgct tatggtgtat tagctaaagc aaccagagag ctgatgacga gaactgtgga 5520
   aatcaggaat cctttggtta aaggctttga gattttccag tggacaaact atgccaagtt 5580
   ctcaagcgaa aaattagaat tagtttttag tgaagagata ttgccttatc ttttccagtt 5640
   aaaaaaattc ataaaatata atctggaaca tgttaagtct tttgaaaaca aatactctat 5700
   gaggatttat gagtggttat taaaagaact aacacaaaag aaaactcaca aggcaaatat 5760
   agagattagc cttgatgaat ttaagttcat gttaatgctt gaaaataact accatgagtt 5820
   taaaaggctt aaccaatggg ttttgaaacc aataagtaaa gatttaaaca cttacagcaa 5880
   tatgaaattg gtggttgata agcgaggccg cccgactgat acgttgattt tccaagttga 5940
   actagataga caaatggatc tcgtaaccga acttgagaac aaccagataa aaatgaatgg 6000
   tgacaaaata ccaacaacca ttacatcaga ttcctaccta cgtaacggac taagaaaaac 6060
   actacacgat gctttaactg caaaaattca gctcaccagt tttgaggcaa aatttttgag 6120
   tgacatgcaa agtaagcatg atctcaatgg ttcgttctca tggctcacgc aaaaacaacg 6180
   aaccacacta gagaacatac tggctaaata cggaaggatc tgaggttctt atggctcttg 6240
   tatctatcag tgaagcatca agactaacaa acaaaagtag aacaactgtt caccgttaga 6300
   tatcaaaggg aaaactgtcc atatgcacag atgaaaacgg tgtaaaaaag atagatacat 6360
   cagagctttt acgagttttt ggtgcattta aagctgttca ccatgaacag atcgacaatg 6420
   taacagatga acagcatgta acacctaata gaacaggtga aaccagtaaa acaaagcaac 6480
   tagaacatga aattgaacac ctgagacaac ttgttacagc tcaacagtca cacatagaca 6540
   gcctgaaaca ggcgatgctg cttatcgaat caaagctgcc gacaacacgg gagccagtga 6600
   cgcctcccgt ggggaaaaaa tcatggcaat tctggaagaa atagcgcttt cagccggcaa 6660
   acctgaagcc ggatctgcga ttctgataac aaactagcaa caccagaaca gcccgtttgc 6720
   gggcagcaaa acccgtactt ttggacgttc cggcggtttt ttgtggcgag tggtgttcgg 6780
   gcggtgcgcg caagatccat tatgttaaac gggcgagttt acatctcaaa accgcccgct 6840
   taacaccatc agaaatcctc agcgcgattt taagcaccaa cccccccccg taacacccaa 6900
   atccatactg aaagtggctt tgttgaataa atcgaacttt tgctgagttg aaggatcaga 6960
   tcacgcatcc tcccgacaac acagaccatt ccgtggcaaa gcaaaagttc agaatcacca 7020
   actggtccac ctacaacaaa gctctcatca accgtggctc cctcactttc tggctggatg 7080
   atgaggcgat tcaggcctgg tatgagtcgg caacaccttc atcacgagga aggccccagc 7140
   gctattctga tctcgccatc accaccgttc tggtgattaa acgcgtattc cggctgaccc 7200
   tgcgggctgc gcagggtttt attgattcca tttttgccct gatgaacgtt ccgttgcgct 7260
   gcccggatta caccagtgtc agtaagcggg caaagtcggt taatgtcagt ttcaaaacgt 7320
   ccacccgggg tgaaatcgca cacctggtga ttgattccac cgggctgaag gtctttggtg 7380
   aaggcgaatg gaaagtcaga aagcacggca aagagcgccg tcgtatctgg cgaaagttgc 7440
   atcttgctgt tgacagcaac acacatgaag ttgtctgtgc agacctgtcg ctgaataacg 7500
   tcacggactc agaagccttc ccgggcctta tccggcagac tcacagaaaa atcagggcag 7560
   ccgcggcaga cggggcttac gatacccggc tctgtcacga tgaactgcgc cgcaaaaaaa 7620
   tcagcgcgct tattcctccc cgaaaaggtg cgggttactg gcccggtgaa tatgcagacc 7680
   gtaaccgtgc agtggctaat cagcgaatga ccgggagtaa tgcgcggtgg aaatggacaa 7740
   cagattacaa ccgtcgctcg atagcggaaa cggcgatgta ccgggtaaaa cagctgttcg 7800
   ggggttcact gacgctgcgt gactacgatg gtcaggttgc ggaggctatg gccctggtac 7860
   gagcgctgaa caaaatgacg aaagcaggta tgcctgaaag cgtgcgtatt gcctgaaaac 7920
   acaacccgct acgggggaga cttacccgaa atctgattta ttcaacaaag ccgggtgtgg 7980
   tgaactacaa agcagacccg ttgaggttat cagttcgatg cacaatcagc agcgcataaa 8040
   atatgcacaa gaacaggagc acccttcgca ttaagctgtg gtggtaacaa gtagtgccgg 8100
   gctaccatca gcgagcatga tgcgctccca cagcattcgc cttggcagta tggaagttcc 8160
   tcgctccagt tcgggccggt atccacctcg cacctggtgt ttaaacggat ttaaattgca 8220
   ggggcagtga gcgcaacgca attaatgtaa gttagctcac tcattaggca ccccaggctt 8280
   gacactttat gcttccggct cgtataatgt gtggaattgt gagcggataa caataacaat 8340
   ttaaatcagg atctaggaac caaggagagt ggcatatgac gaccgaaacc agagaagatc 8400
   gcttacagag acgcattgcc cacctgtacg aggcagacag ccaatttgcg gctgcgcgtc 8460
   ctagcgaggc ggtgaatacc gccgtggcgg aaccagagct gcgtctgccg gctgtcgtca 8520
   aaggtgtctt tgcgggctat gcggatcgtc cggcgctggg tcaacgcgcc gtggagtatg 8580
   ttaccgacgc ggacggtcgt acctctgccc aattgctgcc gcgttttgac acgatcacgt 8640
   accgtcaact gggtgaccgc gtccaagcag ttacgaacgc gtggcacaac catccggtca 8700
   aacctggcga ccgtgtagcg atcctgggct ttactagcgt tgattacacc accgttgata 8760
   cggccctgat cgaattgggc gctgtgagcg tcccgctgca gacttctgca ccggtcacca 8820
   cgctgcgccc gatcgtcgca gagacggagc cgaccgtgat tgcggcttct attgatttcc 8880
   tggatgatgc ggtggaactg gtcaaaagcg gtccggcacc gcgtcgtctg gttgtgttcg 8940
   actaccgccc acgtgtcgac gcccagcgcg aggcatttga agcagccaaa gcggcactgg 9000
   cgggtactga cgttgtagtt gagccgctgg cggatgtcct ggaccgcggt cgtagcttgg 9060
   cggatgcgcc gctgtacacc ccgggtcagc cggacccgct gaccatgctg atttacacga 9120
   gcggttccac gggcaccccg aagggtgcga tgtatccgga gagcaaagtc gctaacatgt 9180
   ggcaactggc gacgaaggcg acgtgggatg agaaccaagc ggcgctgccg gccatcaccc 9240
   tgaactttat gccgatgagc catgtgatgg gccgtggcat cctgattggt accctgagca 9300
   gcggtggcac cgcatacttc gcggctcgca gcgatctgag caccttcctg gaagatctgg 9360
   cattggttcg tccgacccag ttgagctttg ttccgcgtat ttgggatatg ctgttccagg 9420
   aatatcagag ccgtctggac cgttctggtg cgccagagga cgaggtgctg gccgaggtcc 9480
   gccaagacct gctgggcggt cgtttcgtga gcgcgatgac gggttctgcg ccgattagcg 9540
   cggaaatgaa gaattgggtg gaacgtctgc tggacatgca tctgctggaa ggctatggtt 9600
   ccaccgaagc aggctccgtg tttgtggacg gtcatattca acgtccgccg gttattgact 9660
   ataaactggt tgatgtcccg gacctgggtt atttcttgac ggaccgtccg catccgcgtg 9720
   gcgagctgct ggtgaagagc gagcaaatgt tcccaggtta ctacaagcgt ccggagatta 9780
   ccgctgaaat gttcgatgag gatggctact accgcaccgg cgacatcgtg gcggaattgg 9840
   gtccggatca agttgagtat ctggaccgtc gtaataacgt tctgaaattg tctcagggtg 9900
   agtttgtgac ggttagcaaa ctggaagcgg tgttcggcga cagcccgctg gttcgtcaga 9960
   tcttcgttta tggtaacagc gcacgttcct acctgctggc agttgtggtt ccgaccgacc 10020
   cgtcgctgag caagcaggcg atcggcgatt cgttgcagga cgcggcacgc gctgcaggtc 10080
   tgcaatccta cgagattccg cgtgacttta tcgtcgaaac gacccctttt agcctggaga 10140
   acggcctgct gacgggcatt cgcaaactgg ctcgcccaaa cctgaaggcc tactacggcg 10200
   atcgtctgga gcagctgtac accgagctgg cagagggcca agcgaatgag ttgagcgagc 10260
   tgcgtcgcaa tggcgctcaa gccccggtcc tggataccgt gagccgtgca gcgggtgctc 10320
   tgttgggcgc agcggctagc gatctggctc cagaggcgca ttttaccgac ttgggtggcg 10380
   atagcttgag cgcgctgacc tttggtaacc tgctgcaaga gatctttgat gtcgaagtcc 10440
   cggtgagcgc aattgtttcg ccggcatccg acttgcgtac gatcgcagag tatatcgaag 10500
   ctcaacgttc cggcgcagac gtccgcccga ccttcacgtc cgtgcacggt cgcaatgcga 10560
   ccgaggtgca tgcgagcgat ttgacgctgg ataagttcat tgatgccgcc acgctggcgg 10620
   cagccccgag cctgccgggt ccggtgagcg agattcgcac tgtcttgctg accggtgcga 10680
   cgggtttctt gggccgctat ctggcgctgg aatggctgga acgtatggat ctggttgatg 10740
   gtaaagtgat ctgcttggtt cgtgcgaaga gcgacgaaga ggcgcgtgcc cgcctggaca 10800
   aaactttcga cagcggcgat ccgaagctgt gggcccacta tcagaagctg gcagccgatc 10860
   atctggaagt gatcgcgggt gacaagggtg aggcagacct gggcctggat caggttacct 10920
   ggcagcgtct ggcggatacc gtggatttca tcgtggatcc ggcagcgctg gttaatcacg 10980
   ttctgcctta tagcgaactg tttggtccga atgctctggg tactgccgag ctgatccgta 11040
   ttgcgctgac cacccgtatc aaaccgttcg cgtacgtgag cacgatcggc gtgggtggtg 11100
   gtatcgagcc gggtaagttc gtcgaggcgg gtgacattcg tgcgatctct cctgtccgtc 11160
   gcgttgacga tggttacgca aatggctacg gcaacagcaa atgggccggt gaagtcctgc 11220
   tgcgcgaagc ccacgatctg gcgggtctgc cagtgaccgt ttttcgctgt gatatgatct 11280
   tggccgacac cacctacgca ggtcagttga atctgcctga catgttcacg cgtatgatgt 11340
   tttccctggt tgcgaccggt gtggcgccga agagctttaa tcaactggat gcagacggca 11400
   accgccagcg tagccactat gacggtctgc cggtcgagtt tattgcagaa gctatcagca 11460
   ccctgggcgc acacgttcag gacggcttcg aaacttatca cgttatgaat ccgcacgacg 11520
   acggtattgg tatggatgag ttcgttgatt ggctgatcga agccggttac ccgattcagc 11580
   gcgtcgagga ctatcaggag tggctggcgc gcttcgaaac caccctgcgt gccctgccgg 11640
   ataaacagcg tcaggcgagc ctgctgccgc tgctgcacaa ctaccagcaa ccgggtgtcc 11700
   cggttaacgg cgccatggca ccgaccgacg ttttccgtac ggccgtccaa gacgcgaaaa 11760
   ttggtccgga caaggatatt ccgcacgtca gccgtgaagt tattgtgaag tacatcagcg 11820
   atttgaaact gctgggtttg ctgtaataag tttaaacaga gaaggagttc tatcatgatt 11880
   gaaaccatct taccagccgg cgtcgaatca gcagagttac ttgagtaccc tgaagatctg 11940
   aaggcgcatc cggctgaaga gcatctgatc gcaaagagcg tagagaagcg tcgtcgcgac 12000
   ttcattggcg cacgtcattg cgcccgtctg gcactggcgg agctgggtga accgccggtt 12060
   gcgattggca agggtgaacg tggtgcgccg atttggccgc gtggtgtcgt gggctctctg 12120
   acccattgcg atggctatcg cgcagcggcg gttgctcaca aatgcgtttt tcgcagcatc 12180
   ggcatcgacg ccgaaccgca cgcgaccctg ccggaaggtg tcctggattc ggttagcctg 12240
   ccgcctgagc gtgagtggct gaaaaccacc gacagcgcac tgcacctgga ccgtttgctg 12300
   ttttgtgcga aagaagcaac ttacaaagcg tggtggccgc tgacggcacg ttggctgggt 12360
   ttcgaagaag cgcacattac cttcgaaatc gaggatggta gcgccgactc tggtaatggc 12420
   acgtttcaca gcgaactgct ggtgccgggt cagaccaatg acggtggtac cccgctgctg 12480
   tctttcgacg gtcgctggct gatcgctgat ggcttcatcc tgacggcgat tgcatacgca 12540
   tgataaatta acctaggctg ctgccaccgc tgagcaataa ctagcataac cccttggggc 12600
   ctctaaacgg gtcttgaggg gttttttgct gaaacctcag gcatttgaga agccctcgag 12660
   tgaccccagc cgcccctcat gccaaccgag cccattaagc gcagagtcgg ccgcaactat 12720
   ctcggggacg cataaacacg cagcgattta ccaaggagtt cggctaggtc aatccgcaat 12780
   tccctttgcc tggccggaga tgccgcgagc aaacaatgtg tccggtcgct accagatgga 12840
   ctttgtgggc ggaggattgc accagacgct tcagcgcagc gtcgtacgat gaatcggaag 12900
   gcccggtaag caattgaaga accgctgacc acctcccatc cgacaatgca agggtgcccc 12960
   gctcccagcg cgacacggtt gcctgatcca cacccattaa ttcagctagg tgactttgct 13020
   tcatatgacg aagcaaccgc gccctccgca cctgccgccc cctgtcattc gtcaacattc 13080
   ttcagcacct caatgtcgtt cgtagtgagc ctcatttttt caagaccgcc gatgatgaga 13140
   gccaggcctc gctcgaatgc cgcgtccgga ccgccttcgt agacgatttt catcgcgctc 13200
   tgtaggcgcg ccggcatcgt agacgctgag gtggtcaact gatcttcgcc ccgctcctcg 13260
   gcgtctgcct cgctagcttg ctgctcaaga acagcgccga cggtgaagta gctgattgcc 13320
   atcaacgcat aggtcgcgtc acctgccgaa aagccagcat cgcaaaggaa gcgaagctgc 13380
   gcgtcggctt tttccatctg cggcgcggct ggccgcgtcc cggcatgaat acgcgcgcca 13440
   tcgcgataag cgagcaacgc ccgtcgaaaa ctgcatgcat tgcccttcag gaacgaacgc 13500
   cagtcgtcgt catcccttgg cgtcgaatgc gtgtgattta tcgtcagcat ggcttcggca 13560
   agtgcgtcga gcaacgcacg cttgttcttg aaatgccagt agagcgctgg ctgttgcacc 13620
   ccgaggcgct cagccagtcg gcgcgtcgtt agaccttcca tgcccacgtc gttaagcagt 13680
   tcgagcgcgg ttcggatcac ggcctcgcgt tggagcttgt tcattcgcga a 13731
<210> 21
   <211> 13782
   <212> DNA
   <213> Artificial Sequence
<220>
   <223> vector
<400> 21
   ttctcatgtt tgacagctta tcatcgataa gctttaatgc ggtagtttat cacagttaaa      60
   ttgctaacgc agtcaggcac cgtgtatgaa atctaacaat gcgctcatcg tcatcctcgg     120
   caccgtcacc ctggatgctg taggcatagg cttggttatg ccggtactgc cgggcctctt     180
   gcgggatatc gtccattccg acagcatcgc cagtcactat ggcgtgctgc tagcgctata     240
   tgcgttgatg caatttctat gcgcacccgt tctcggagca ctgtccgacc gctttggccg     300
   ccgcccagtc ctgctcgctt cgctacttgg agccactatc gactacgcga tcatggcgac     360
   cacacccgtc ctgtggatcc tctacgccgg acgcatcgtg gccggcatca ccggcgccac     420
   aggtgcggtt gctggcgcct atatcgccga catcaccgat ggggaagatc gggctcgcca     480
   cttcgggctc atgagcgctt gtttcggcgt gggtatggtg gcaggccccg tggccggggg     540
   actgttgggc gccatctcct tgcatgcacc attccttgcg gcggcggtgc tcaacggcct     600
   caacctacta ctgggctgct tcctaatgca ggagtcgcat aagggagagc gtcgaccgat     660
   gcccttgaga gccttcaacc cagtcagctc cttccggtgg gcgcggggca tgactatcgt     720
   cgccgcactt atgactgtct tctttatcat gcaactcgta ggacaggtgc cggcagcgct     780
   ctgggtcatt ttcggcgagg accgctttcg ctggagcgcg acgatgatcg gcctgtcgct     840
   tgcggtattc ggaatcttgc acgccctcgc tcaagccttc gtcactggtc ccgccaccaa     900
   acgtttcggc gagaagcagg ccattatcgc cggcatggcg gccgacgcgc tgggctacgt     960
   cttgctggcg ttcgcgacgc gaggctggat ggccttcccc attatgattc ttctcgcttc    1020
   cggcggcatc gggatgcccg cgttgcaggc catgctgtcc aggcaggtag atgacgacca    1080
   tcagggacag cttcaaggat cgctcgcggc tcttaccagc ctaacttcga tcactggacc    1140
   gctgatcgtc acggcgattt atgccgcctc ggcgagcaca tggaacgggt tggcatggat    1200
   tgtaggcgcc gccctatacc ttgtctgcct ccccgcgttg cgtcgcggtg catggagccg    1260
   ggccacctcg acctgaatgg aagccggcgg cacctcgcta acggattcac cactccaaga    1320
   attggagcca atcaattctt gcggagaact gtgaatgcgc aaaccaaccc ttggcagaac 1380
   atatccatcg cgtccgccat ctccagcagc cgcacgcggc gcatctcggg cagcgttggg 1440
   tcctggccac gggtgcgcat gatcgtgctc ctgtcgttga ggacccggct aggctggcgg 1500
   ggttgcctta ctggttagca gaatgaatca ccgatacgcg agcgaacgtg aagcgactgc 1560
   tgctgcaaaa cgtctgcgac ctgagcaaca acatgaatgg tcttcggttt ccgtgtttcg 1620
   taaagtctgg aaacgcggaa gtcccctacg tgctgctgaa gttgcccgca acagagagtg 1680
   gaaccaaccg gtgataccac gatactatga ctgagagtca acgccatgag cggcctcatt 1740
   tcttattctg agttacaaca gtccgcaccg ctgccggtag ctacttgact atccggctgc 1800
   actagccctg cgtcagatgg ctctgatcca aggcaaactg ccaaaatatc tgctggcacc 1860
   ggaagtcagc gccctgcacc attatgttcc ggatctgcat cgcaggatgc tgctggctac 1920
   cctgtggaac acctacatct gtattaacga agcgctggca ttgaccctga gtgatttttc 1980
   tctggtgccg ccctatccct ttgtgcagct tgccacgctc aaaggggttt gaggtccaac 2040
   cgtacgaaaa cgtacggtaa gaggaaaatt atcgtctgaa aaatcgatta gtagacaaga 2100
   aagtccgtta agtgccaatt ttcgattaaa aagacaccgt tttgatggcg ttttccaatg 2160
   tacattatgt ttcgatatat cagacagtta cttcactaac gtacgttttc gttctattgg 2220
   ccttcagacc ccatatcctt aatgtccttt atttgctggg gttatcagat ccccccgaca 2280
   cgtttaatta atgctttctc cgccggagat cgacgcacag gcttctgtgt ctatgatgtt 2340
   atttcttaat aatcatccag gtattctctt tatcaccata cgtagtgcga gtgtccacct 2400
   taacgcaggg ctttccgtca cagcgcgata tgtcagccag cggggctttc ttttgccaga 2460
   ccgcttccat cctctgcatt tcagcaatct ggctataccc gtcattcata aaccacgtaa 2520
   atgccgtcac gcaggaagcc aggacgaaga atatcgtcag tacaagataa atcgcggatt 2580
   tccacgtata gcgtgacatc tcacgacgca tttcatggat catcgctttc gccgtatcgg 2640
   cagcctgatt cagcgcttct gtcgccggtt tctgctgtgc taatccggct tgtttcagtt 2700
   ctttctcaac ctgagtgagc gcggaactca ccgatttcct gacggtgtca gtcatattac 2760
   cggacgcgct gtccagctca cgaatgaccc tgctcagcgt ttcactttgc tgctgtaatt 2820
   gtgatgaggc ggcctgaaac tgttctgtca gagaagtaac acgcttttcc agcgcctgat 2880
   gatgcccgat aagggcggca atttgtttaa tttcgtcgct catacaaaat cctgcctatc 2940
   gtgagaatga ccagccttta tccggcttct gtcgtatctg ttcggcgagt cgctgtcgtt 3000
   ctttctcctg ctgacgctgt tttccgccca gacgttcgcg ctctctctgc ctttccatct 3060
   cctgatgtat cccctggaac tccgccatcg catcgttaac aagggactga agatcgattt 3120
   cttcctgtat atccttcatg gcatcactga ccagtgcgtt cagcttgtca ggctcttttt 3180
   caaaatcaaa cgttctgccg gaatgggatt cctgctcagg ctctgacttc agctcctgtt 3240
   ttagcgtcag agtatccctc tcgctgaggg cttcccgtaa cgaggtagtc acgtcaatta 3300
   cgctgtcacg ttcatcacgg gactgctgca cctgcctttc agcctccctg cgctcaagaa 3360
   tggcctgtag ctgctcagta tcgaatcgct gaacctgacc cgcgcccaga tgccgctcag 3420
   gctcacggtc aatgccctgc gccttcaggg aacgggaatc aacccggtca gcgtgctgat 3480
   accgttcaag gtgcttattc tggaggtcag cccagcgtct ccctctgggc aacaaggtat 3540
   tctttgcgtt cggtcggtgt ttccccgaaa cgtgcctttt ttgcgccacc gcgtccggct 3600
   ctttggtgtt agcccgttta aaatactgct cagggtcacg gtgaataccg tcattaatgc 3660
   gttcagagaa catgatatgg gcgtggggct gctcgccacc ggctatcgct gctttcggat 3720
   tatggatagc gaactgatag gcatggcggt cgccaatttc ctgttggaca aaatcgcgga 3780
   caagctcaag acgttgttcg ggttttaact cacgcggcag ggcaatctcg atttcacggt 3840
   aggtacagcc gttggcacgt tcagacgtgt cagcggcttt ccagaactcg gacggtttat 3900
   gcgctgccca cgccggcata ttgccggact ccttgtgctc aaggtcggag tctttttcac 3960
   gggcatactt tccctcacgc gcaatataat cggcatgagg agaggcactg ccttttccgc 4020
   cggtttttac gctgagatga taggatgcca tcgtgtttta tcccgctgaa gggcgcacgt 4080
   ttctgaacga agtgaagaaa gtctaagtgc gccctgataa ataaaagagt tatcagggat 4140
   tgtagtggga tttgacctcc tctgccatca tgagcgtaat cattccgtta gcattcagga 4200
   ggtaaacagc atgaataaaa gcgaaaaaac aggaacaatg ggcagcagaa agagtgcagt 4260
   atattcgcgg cttaaagtcg ccgaatgagc aacagaaact tatgctgata ctgacggata 4320
   aagcagataa aacagcacag gatatcaaaa cgctgtccct gctgatgaag gctgaacagg 4380
   cagcagagaa agcgcaggaa gccagagcga aagtcatgaa cctgatacag gcagaaaagc 4440
   gagccgaagc cagagccgcc cgtaaagccc gtgaccatgc tctgtaccag tctgccggat 4500
   tgcttatcct ggcgggtctg gttgacagta agacgggtaa gcctgttgat gataccgctg 4560
   ccttactggg tgcattagcc agtctgaatg acctgtcacg ggataatccg aagtggtcag 4620
   actggaaaat cagagggcag gaactgctga acagcaaaaa gtcagatagc accacatagc 4680
   agacccgcca taaaacgccc tgagaagccc gtgacgggct tttcttgtat tatgggtagt 4740
   ttccttgcat gaatccataa aaggcgcctg tagtgccatt tacccccatt cactgccaga 4800
   gccgtgagcg cagcgaactg aatgtcacga aaaagacagc gactcaggtg cctgatggtc 4860
   ggagacaaaa ggaatattca gcgatttgcc cgagcttgcg agggtgctac ttaagccttt 4920
   agggttttaa ggtctgtttt gtagaggagc aaacagcgtt tgcgacatcc ttttgtaata 4980
   ctgcggaact gactaaagta gtgagttata cacagggctg ggatctattc tttttatctt 5040
   tttttattct ttctttattc tataaattat aaccacttga atataaacaa aaaaaacaca 5100
   caaaggtcta gcggaattta cagagggtct agcagaattt acaagttttc cagcaaaggt 5160
   ctagcagaat ttacagatac ccacaactca aaggaaaagg actagtaatt atcattgact 5220
   agcccatctc aattggtata gtgattaaaa tcacctagac caattgagat gtatgtctga 5280
   attagttgtt ttcaaagcaa atgaactagc gattagtcgc tatgacttaa cggagcatga 5340
   aaccaagcta attttatgct gtgtggcact actcaacccc acgattgaaa accctacaag 5400
   gaaagaacgg acggtatcgt tcacttataa ccaatacgct cagatgatga acatcagtag 5460
   ggaaaatgct tatggtgtat tagctaaagc aaccagagag ctgatgacga gaactgtgga 5520
   aatcaggaat cctttggtta aaggctttga gattttccag tggacaaact atgccaagtt 5580
   ctcaagcgaa aaattagaat tagtttttag tgaagagata ttgccttatc ttttccagtt 5640
   aaaaaaattc ataaaatata atctggaaca tgttaagtct tttgaaaaca aatactctat 5700
   gaggatttat gagtggttat taaaagaact aacacaaaag aaaactcaca aggcaaatat 5760
   agagattagc cttgatgaat ttaagttcat gttaatgctt gaaaataact accatgagtt 5820
   taaaaggctt aaccaatggg ttttgaaacc aataagtaaa gatttaaaca cttacagcaa 5880
   tatgaaattg gtggttgata agcgaggccg cccgactgat acgttgattt tccaagttga 5940
   actagataga caaatggatc tcgtaaccga acttgagaac aaccagataa aaatgaatgg 6000
   tgacaaaata ccaacaacca ttacatcaga ttcctaccta cgtaacggac taagaaaaac 6060
   actacacgat gctttaactg caaaaattca gctcaccagt tttgaggcaa aatttttgag 6120
   tgacatgcaa agtaagcatg atctcaatgg ttcgttctca tggctcacgc aaaaacaacg 6180
   aaccacacta gagaacatac tggctaaata cggaaggatc tgaggttctt atggctcttg 6240
   tatctatcag tgaagcatca agactaacaa acaaaagtag aacaactgtt caccgttaga 6300
   tatcaaaggg aaaactgtcc atatgcacag atgaaaacgg tgtaaaaaag atagatacat 6360
   cagagctttt acgagttttt ggtgcattta aagctgttca ccatgaacag atcgacaatg 6420
   taacagatga acagcatgta acacctaata gaacaggtga aaccagtaaa acaaagcaac 6480
   tagaacatga aattgaacac ctgagacaac ttgttacagc tcaacagtca cacatagaca 6540
   gcctgaaaca ggcgatgctg cttatcgaat caaagctgcc gacaacacgg gagccagtga 6600
   cgcctcccgt ggggaaaaaa tcatggcaat tctggaagaa atagcgcttt cagccggcaa 6660
   acctgaagcc ggatctgcga ttctgataac aaactagcaa caccagaaca gcccgtttgc 6720
   gggcagcaaa acccgtactt ttggacgttc cggcggtttt ttgtggcgag tggtgttcgg 6780
   gcggtgcgcg caagatccat tatgttaaac gggcgagttt acatctcaaa accgcccgct 6840
   taacaccatc agaaatcctc agcgcgattt taagcaccaa cccccccccg taacacccaa 6900
   atccatactg aaagtggctt tgttgaataa atcgaacttt tgctgagttg aaggatcaga 6960
   tcacgcatcc tcccgacaac acagaccatt ccgtggcaaa gcaaaagttc agaatcacca 7020
   actggtccac ctacaacaaa gctctcatca accgtggctc cctcactttc tggctggatg 7080
   atgaggcgat tcaggcctgg tatgagtcgg caacaccttc atcacgagga aggccccagc 7140
   gctattctga tctcgccatc accaccgttc tggtgattaa acgcgtattc cggctgaccc 7200
   tgcgggctgc gcagggtttt attgattcca tttttgccct gatgaacgtt ccgttgcgct 7260
   gcccggatta caccagtgtc agtaagcggg caaagtcggt taatgtcagt ttcaaaacgt 7320
   ccacccgggg tgaaatcgca cacctggtga ttgattccac cgggctgaag gtctttggtg 7380
   aaggcgaatg gaaagtcaga aagcacggca aagagcgccg tcgtatctgg cgaaagttgc 7440
   atcttgctgt tgacagcaac acacatgaag ttgtctgtgc agacctgtcg ctgaataacg 7500
   tcacggactc agaagccttc ccgggcctta tccggcagac tcacagaaaa atcagggcag 7560
   ccgcggcaga cggggcttac gatacccggc tctgtcacga tgaactgcgc cgcaaaaaaa 7620
   tcagcgcgct tattcctccc cgaaaaggtg cgggttactg gcccggtgaa tatgcagacc 7680
   gtaaccgtgc agtggctaat cagcgaatga ccgggagtaa tgcgcggtgg aaatggacaa 7740
   cagattacaa ccgtcgctcg atagcggaaa cggcgatgta ccgggtaaaa cagctgttcg 7800
   ggggttcact gacgctgcgt gactacgatg gtcaggttgc ggaggctatg gccctggtac 7860
   gagcgctgaa caaaatgacg aaagcaggta tgcctgaaag cgtgcgtatt gcctgaaaac 7920
   acaacccgct acgggggaga cttacccgaa atctgattta ttcaacaaag ccgggtgtgg 7980
   tgaactacaa agcagacccg ttgaggttat cagttcgatg cacaatcagc agcgcataaa 8040
   atatgcacaa gaacaggagc acccttcgca ttaagctgtg gtggtaacaa gtagtgccgg 8100
   gctaccatca gcgagcatga tgcgctccca cagcattcgc cttggcagta tggaagttcc 8160
   tcgctccagt tcgggccggt atccacctcg cacctggtgt ttaaacggat ttaaattgca 8220
   ggggcagtga gcgcaacgca attaatgtaa gttagctcac tcattaggca ccccaggctt 8280
   gacactttat gcttccggct cgtataatgt gtggaattgt gagcggataa caataacaat 8340
   ttaaatcagg atctaggaac caaggagagt ggcatatgag cactgcaacg catgatgaga 8400
   gactggatag acgcgtacat gaactgatcg caaccgaccc gcaatttgcg gcagcacagc 8460
   cggacccggc tattacggcg gcattggagc aaccgggtct gcgtctgccg cagatcattc 8520
   gtaccgtcct ggacggctat gcggatcgtc cggcgttggg tcaacgcgtc gttgagtttg 8580
   ttaccgacgc gaaaaccggt cgcacgagcg cgcagctgct gccgcgtttt gaaaccatta 8640
   cctactccga agtggcacaa cgcgttagcg cgctgggtcg tgcgctgtcc gacgacgcag 8700
   ttcatccggg tgatcgtgtg tgtgtcctgg gttttaacag cgttgattac gcgaccatcg 8760
   atatggcact gggtgcgatt ggcgcggtgt cggttccgct gcaaacctct gcagcgatta 8820
   gctccctgca gcctattgtc gcggaaaccg aaccgacgct gatcgcgagc agcgttaatc 8880
   agctgagcga tgccgtgcag ctgatcaccg gtgcagagca ggcccctacc cgtttggtgg 8940
   tgttcgatta tcacccgcaa gtcgatgacc agcgcgaggc tgttcaggac gcggctgcgc 9000
   gcttgtccag caccggcgtt gccgtgcaaa ccctggccga gctgctggag cgcggtaaag 9060
   acctgccggc tgtggcggag ccgccagcgg atgaggacag cctggcgttg ctgatttaca 9120
   ccagcggcag cacgggtgct ccgaaaggtg cgatgtaccc acagagcaat gttggtaaga 9180
   tgtggcgtcg tggctctaaa aactggttcg gcgaaagcgc tgctagcatt accctgaact 9240
   tcatgccgat gagccatgtg atgggccgta gcatcttgta cggcaccctg ggtaacggcg 9300
   gtacggccta tttcgctgct cgtagcgatc tgagcaccct gctggaagat ctggagctgg 9360
   tgcgtccgac ggaactgaat tttgttccac gcatttggga aaccttgtat ggtgagttcc 9420
   aacgtcaagt ggaacgccgc ctgagcgagg ctggcgacgc gggtgagcgt cgtgctgtgg 9480
   aagcggaagt gctggcggag cagcgccaat atctgctggg cggtcgtttc acctttgcga 9540
   tgacgggcag cgcccctatt tcgcctgagc tgcgcaactg ggtcgagtcc ctgctggaga 9600
   tgcatctgat ggatggttac ggtagcacgg aagcgggcat ggtcctgttt gacggtgaga 9660
   tccaacgtcc gccggttatt gattacaagt tggtggacgt tccggatttg ggttacttta 9720
   gcaccgatcg cccgcacccg cgtggtgagc tgctgctgcg tacggagaat atgttcccgg 9780
   gttactataa gcgtgccgaa actaccgcgg gtgtttttga tgaggacggt tactaccgca 9840
   cgggcgatgt gtttgccgaa atcgcaccgg atcgcctggt gtatgttgac cgtcgcaaca 9900
   acgtattgaa actggcacaa ggcgagttcg tcacgttggc caagctggaa gcggtgtttg 9960
   gtaattcccc gctgattcgt cagatctatg tctatggtaa cagcgcgcaa ccgtatctgc 10020
   tggccgttgt cgttccgact gaagaagccc tggccagcgg tgatcctgag acgctgaagc 10080
   cgaaaatcgc agatagcctg cagcaagtgg cgaaagaagc aggtctgcag agctacgaag 10140
   ttccgcgcga ctttatcatc gagacgacgc cgtttagcct ggaaaacggc ctgttgacgg 10200
   gcatccgcaa actggcttgg ccgaaactga agcaacacta cggcgagcgt ctggagcaaa 10260
   tgtacgccga cctggcagcg ggccaagcaa atgaactggc agagctgcgt cgtaatggcg 10320
   cccaagcccc ggtgctgcag acggttagcc gtgcagcggg tgcaatgttg ggctctgccg 10380
   cgagcgattt gagcccagat gcgcacttca ccgacctggg tggtgacagc ctgtctgctc 10440
   tgaccttcgg taacctgctg cgtgagatct tcgacgtcga tgtcccggtc ggcgtgatcg 10500
   tcagcccggc aaatgatctg gcggcgattg cgagctatat tgaagcagaa cgtcagggta 10560
   gcaaacgtcc gacctttgcg agcgtccatg gtcgcgacgc gaccgttgtt cgtgctgccg 10620
   acttgaccct ggacaagttt ttggacgctg aaacgttggc tgcggcaccg aacctgccga 10680
   agccggcgac cgaggttcgc accgttctgc tgaccggtgc tactggtttc ctgggtcgtt 10740
   acctggctct ggagtggctg gagcgtatgg atatggttga cggtaaggtc attgcattgg 10800
   tgcgtgcgcg ttctgatgaa gaggcccgtg cccgtctgga taagaccttt gacagcggtg 10860
   acccgaaact gctggcgcac tatcagcagc tggcggcaga ccacctggaa gtgatcgccg 10920
   gtgacaaggg tgaggccaat ctgggtctgg gtcaggacgt gtggcaacgt ctggcggaca 10980
   ccgttgatgt cattgtggac ccggcagcgc tggtgaacca cgtcctgccg tactccgaac 11040
   tgttcggtcc gaatgcgttg ggcactgcag agctgatccg tctggcactg accagcaagc 11100
   agaaaccgta tacttacgtt agcaccatcg gcgtcggcga ccaaatcgaa ccgggtaaat 11160
   tcgttgaaaa tgcagatatt cgtcaaatgt cggcgacccg tgcgattaat gacagctacg 11220
   cgaacggcta tggcaactcc aagtgggcgg gtgaggttct gctgcgcgag gcgcatgatc 11280
   tgtgcggtct gccggtagcg gtgttccgtt gcgacatgat cctggcagac acgacctacg 11340
   caggtcagtt gaatctgccg gatatgttca cgcgcctgat gttgagcctg gtagcgacgg 11400
   gtattgcacc gggttctttc tatgagttgg atgccgatgg caatcgccag cgcgcacact 11460
   atgacggcct gccggtcgag tttatcgcag cggcgattag cacgctgggt tcccagatca 11520
   ccgatagcga taccggcttc cagacgtacc acgtcatgaa tccttacgac gacggcgtcg 11580
   gcctggacga gtacgtcgac tggctggttg atgcgggtta ttcgattgag cgtattgccg 11640
   actactctga atggttgcgt cgcttcgaaa ccagcctgcg tgcgctgccg gatcgtcaac 11700
   gccagtatag cctgctgcca ctgttgcaca actaccgtac cccggagaaa ccgattaacg 11760
   gcagcatcgc cccgacggat gttttccgtg cggcagtcca ggaagcgaag atcggtccag 11820
   acaaggacat cccgcacgtg tccccgccag tgatcgtgaa atacattacc gacctgcagc 11880
   tgttgggcct gctgtaataa gtttaaacag agaaggagtt ctatcatgat tgaaaccatc 11940
   ttaccagccg gcgtcgaatc agcagagtta cttgagtacc ctgaagatct gaaggcgcat 12000
   ccggctgaag agcatctgat cgcaaagagc gtagagaagc gtcgtcgcga cttcattggc 12060
   gcacgtcatt gcgcccgtct ggcactggcg gagctgggtg aaccgccggt tgcgattggc 12120
   aagggtgaac gtggtgcgcc gatttggccg cgtggtgtcg tgggctctct gacccattgc 12180
   gatggctatc gcgcagcggc ggttgctcac aaaatgcgtt ttcgcagcat cggcatcgac 12240
   gccgaaccgc acgcgaccct gccggaaggt gtcctggatt cggttagcct gccgcctgag 12300
   cgtgagtggc tgaaaaccac cgacagcgca ctgcacctgg accgtttgct gttttgtgcg 12360
   aaagaagcaa cttacaaagc gtggtggccg ctgacggcac gttggctggg tttcgaagaa 12420
   gcgcacatta ccttcgaaat cgaggatggt agcgccgact ctggtaatgg cacgtttcac 12480
   agcgaactgc tggtgccggg tcagaccaat gacggtggta ccccgctgct gtctttcgac 12540
   ggtcgctggc tgatcgctga tggcttcatc ctgacggcga ttgcatacgc atgataaatt 12600
   aacctaggct gctgccaccg ctgagcaata actagcataa ccccttgggg cctctaaacg 12660
   ggtcttgagg ggttttttgc tgaaacctca ggcatttgag aagccctcga gtgaccccag 12720
   ccgcccctca tgccaaccga gcccattaag cgcagagtcg gccgcaacta tctcggggac 12780
   gcataaacac gcagcgattt accaaggagt tcggctaggt caatccgcaa ttccctttgc 12840
   ctggccggag atgccgcgag caaacaatgt gtccggtcgc taccagatgg actttgtggg 12900
   cggaggattg caccagacgc ttcagcgcag cgtcgtacga tgaatcggaa ggcccggtaa 12960
   gcaattgaag aaccgctgac cacctcccat ccgacaatgc aagggtgccc cgctcccagc 13020
   gcgacacggt tgcctgatcc acacccatta attcagctag gtgactttgc ttcatatgac 13080
   gaagcaaccg cgccctccgc acctgccgcc ccctgtcatt cgtcaacatt cttcagcacc 13140
   tcaatgtcgt tcgtagtgag cctcattttt tcaagaccgc cgatgatgag agccaggcct 13200
   cgctcgaatg ccgcgtccgg accgccttcg tagacgattt tcatcgcgct ctgtaggcgc 13260
   gccggcatcg tagacgctga ggtggtcaac tgatcttcgc cccgctcctc ggcgtctgcc 13320
   tcgctagctt gctgctcaag aacagcgccg acggtgaagt agctgattgc catcaacgca 13380
   taggtcgcgt cacctgccga aaagccagca tcgcaaagga agcgaagctg cgcgtcggct 13440
   ttttccatct gcggcgcggc tggccgcgtc ccggcatgaa tacgcgcgcc atcgcgataa 13500
   gcgagcaacg cccgtcgaaa actgcatgca ttgcccttca ggaacgaacg ccagtcgtcg 13560
   tcatcccttg gcgtcgaatg cgtgtgattt atcgtcagca tggcttcggc aagtgcgtcg 13620
   agcaacgcac gcttgttctt gaaatgccag tagagcgctg gctgttgcac cccgaggcgc 13680
   tcagccagtc ggcgcgtcgt tagaccttcc atgcccacgt cgttaagcag ttcgagcgcg 13740
   gttcggatca cggcctcgcg ttggagcttg ttcattcgcg aa 13782
<210> 22
   <211> 13695
   <212> DNA
   <213> Artificial Sequence
<220>
   <223> vector
<400> 22
   ttctcatgtt tgacagctta tcatcgataa gctttaatgc ggtagtttat cacagttaaa      60
   ttgctaacgc agtcaggcac cgtgtatgaa atctaacaat gcgctcatcg tcatcctcgg     120
   caccgtcacc ctggatgctg taggcatagg cttggttatg ccggtactgc cgggcctctt     180
   gcgggatatc gtccattccg acagcatcgc cagtcactat ggcgtgctgc tagcgctata     240
   tgcgttgatg caatttctat gcgcacccgt tctcggagca ctgtccgacc gctttggccg     300
   ccgcccagtc ctgctcgctt cgctacttgg agccactatc gactacgcga tcatggcgac     360
   cacacccgtc ctgtggatcc tctacgccgg acgcatcgtg gccggcatca ccggcgccac     420
   aggtgcggtt gctggcgcct atatcgccga catcaccgat ggggaagatc gggctcgcca     480
   cttcgggctc atgagcgctt gtttcggcgt gggtatggtg gcaggccccg tggccggggg 540
   actgttgggc gccatctcct tgcatgcacc attccttgcg gcggcggtgc tcaacggcct 600
   caacctacta ctgggctgct tcctaatgca ggagtcgcat aagggagagc gtcgaccgat 660
   gcccttgaga gccttcaacc cagtcagctc cttccggtgg gcgcggggca tgactatcgt 720
   cgccgcactt atgactgtct tctttatcat gcaactcgta ggacaggtgc cggcagcgct 780
   ctgggtcatt ttcggcgagg accgctttcg ctggagcgcg acgatgatcg gcctgtcgct 840
   tgcggtattc ggaatcttgc acgccctcgc tcaagccttc gtcactggtc ccgccaccaa 900
   acgtttcggc gagaagcagg ccattatcgc cggcatggcg gccgacgcgc tgggctacgt 960
   cttgctggcg ttcgcgacgc gaggctggat ggccttcccc attatgattc ttctcgcttc 1020
   cggcggcatc gggatgcccg cgttgcaggc catgctgtcc aggcaggtag atgacgacca 1080
   tcagggacag cttcaaggat cgctcgcggc tcttaccagc ctaacttcga tcactggacc 1140
   gctgatcgtc acggcgattt atgccgcctc ggcgagcaca tggaacgggt tggcatggat 1200
   tgtaggcgcc gccctatacc ttgtctgcct ccccgcgttg cgtcgcggtg catggagccg 1260
   ggccacctcg acctgaatgg aagccggcgg cacctcgcta acggattcac cactccaaga 1320
   attggagcca atcaattctt gcggagaact gtgaatgcgc aaaccaaccc ttggcagaac 1380
   atatccatcg cgtccgccat ctccagcagc cgcacgcggc gcatctcggg cagcgttggg 1440
   tcctggccac gggtgcgcat gatcgtgctc ctgtcgttga ggacccggct aggctggcgg 1500
   ggttgcctta ctggttagca gaatgaatca ccgatacgcg agcgaacgtg aagcgactgc 1560
   tgctgcaaaa cgtctgcgac ctgagcaaca acatgaatgg tcttcggttt ccgtgtttcg 1620
   taaagtctgg aaacgcggaa gtcccctacg tgctgctgaa gttgcccgca acagagagtg 1680
   gaaccaaccg gtgataccac gatactatga ctgagagtca acgccatgag cggcctcatt 1740
   tcttattctg agttacaaca gtccgcaccg ctgccggtag ctacttgact atccggctgc 1800
   actagccctg cgtcagatgg ctctgatcca aggcaaactg ccaaaatatc tgctggcacc 1860
   ggaagtcagc gccctgcacc attatgttcc ggatctgcat cgcaggatgc tgctggctac 1920
   cctgtggaac acctacatct gtattaacga agcgctggca ttgaccctga gtgatttttc 1980
   tctggtgccg ccctatccct ttgtgcagct tgccacgctc aaaggggttt gaggtccaac 2040
   cgtacgaaaa cgtacggtaa gaggaaaatt atcgtctgaa aaatcgatta gtagacaaga 2100
   aagtccgtta agtgccaatt ttcgattaaa aagacaccgt tttgatggcg ttttccaatg 2160
   tacattatgt ttcgatatat cagacagtta cttcactaac gtacgttttc gttctattgg 2220
   ccttcagacc ccatatcctt aatgtccttt atttgctggg gttatcagat ccccccgaca 2280
   cgtttaatta atgctttctc cgccggagat cgacgcacag gcttctgtgt ctatgatgtt 2340
   atttcttaat aatcatccag gtattctctt tatcaccata cgtagtgcga gtgtccacct 2400
   taacgcaggg ctttccgtca cagcgcgata tgtcagccag cggggctttc ttttgccaga 2460
   ccgcttccat cctctgcatt tcagcaatct ggctataccc gtcattcata aaccacgtaa 2520
   atgccgtcac gcaggaagcc aggacgaaga atatcgtcag tacaagataa atcgcggatt 2580
   tccacgtata gcgtgacatc tcacgacgca tttcatggat catcgctttc gccgtatcgg 2640
   cagcctgatt cagcgcttct gtcgccggtt tctgctgtgc taatccggct tgtttcagtt 2700
   ctttctcaac ctgagtgagc gcggaactca ccgatttcct gacggtgtca gtcatattac 2760
   cggacgcgct gtccagctca cgaatgaccc tgctcagcgt ttcactttgc tgctgtaatt 2820
   gtgatgaggc ggcctgaaac tgttctgtca gagaagtaac acgcttttcc agcgcctgat 2880
   gatgcccgat aagggcggca atttgtttaa tttcgtcgct catacaaaat cctgcctatc 2940
   gtgagaatga ccagccttta tccggcttct gtcgtatctg ttcggcgagt cgctgtcgtt 3000
   ctttctcctg ctgacgctgt ttttccgcca gacgttcgcg ctctctctgc ctttccatct 3060
   cctgatgtat cccctggaac tccgccatcg catcgttaac aagggactga agatcgattt 3120
   cttcctgtat atccttcatg gcatcactga ccagtgcgtt cagcttgtca ggctcttttt 3180
   caaaatcaaa cgttctgccg gaatgggatt cctgctcagg ctctgacttc agctcctgtt 3240
   ttagcgtcag agtatccctc tcgctgaggg cttcccgtaa cgaggtagtc acgtcaatta 3300
   cgctgtcacg ttcatcacgg gactgctgca cctgcctttc agcctccctg cgctcaagaa 3360
   tggcctgtag ctgctcagta tcgaatcgct gaacctgacc cgcgcccaga tgccgctcag 3420
   gctcacggtc aatgccctgc gccttcaggg aacgggaatc aacccggtca gcgtgctgat 3480
   accgttcaag gtgcttattc tggaggtcag cccagcgtct ccctctgggc aacaaggtat 3540
   tctttgcgtt cggtcggtgt ttccccgaaa cgtgcctttt ttgcgccacc gcgtccggct 3600
   ctttggtgtt agcccgttta aaatactgct cagggtcacg gtgaataccg tcattaatgc 3660
   gttcagagaa catgatatgg gcgtggggct gctcgccacc ggctatcgct gctttcggat 3720
   tatggatagc gaactgatag gcatggcggt cgccaatttc ctgttggaca aaatcgcgga 3780
   caagctcaag acgttgttcg ggttttaact cacgcggcag ggcaatctcg atttcacggt 3840
   aggtacagcc gttggcacgt tcagacgtgt cagcggcttt ccagaactcg gacggtttat 3900
   gcgctgccca cgccggcata ttgccggact ccttgtgctc aaggtcggag tctttttcac 3960
   gggcatactt tccctcacgc gcaatataat cggcatgagg agaggcactg ccttttccgc 4020
   cggtttttac gctgagatga taggatgcca tcgtgtttta tcccgctgaa gggcgcacgt 4080
   ttctgaacga agtgaagaaa gtctaagtgc gccctgataa ataaaagagt tatcagggat 4140
   tgtagtggga tttgacctcc tctgccatca tgagcgtaat cattccgtta gcattcagga 4200
   ggtaaacagc atgaataaaa gcgaaaaaac aggaacaatg ggcagcagaa agagtgcagt 4260
   atattcgcgg cttaaagtcg ccgaatgagc aacagaaact tatgctgata ctgacggata 4320
   aagcagataa aacagcacag gatatcaaaa cgctgtccct gctgatgaag gctgaacagg 4380
   cagcagagaa agcgcaggaa gccagagcga aagtcatgaa cctgatacag gcagaaaagc 4440
   gagccgaagc cagagccgcc cgtaaagccc gtgaccatgc tctgtaccag tctgccggat 4500
   tgcttatcct ggcgggtctg gttgacagta agacgggtaa gcctgttgat gataccgctg 4560
   ccttactggg tgcattagcc agtctgaatg acctgtcacg ggataatccg aagtggtcag 4620
   actggaaaat cagagggcag gaactgctga acagcaaaaa gtcagatagc accacatagc 4680
   agacccgcca taaaacgccc tgagaagccc gtgacgggct tttcttgtat tatgggtagt 4740
   ttccttgcat gaatccataa aaggcgcctg tagtgccatt tacccccatt cactgccaga 4800
   gccgtgagcg cagcgaactg aatgtcacga aaaagacagc gactcaggtg cctgatggtc 4860
   ggagacaaaa ggaatattca gcgatttgcc cgagcttgcg agggtgctac ttaagccttt 4920
   agggttttaa ggtctgtttt gtagaggagc aaacagcgtt tgcgacatcc ttttgtaata 4980
   ctgcggaact gactaaagta gtgagttata cacagggctg ggatctattc tttttatctt 5040
   tttttattct ttctttattc tataaattat aaccacttga atataaacaa aaaaaacaca 5100
   caaaggtcta gcggaattta cagagggtct agcagaattt acaagttttc cagcaaaggt 5160
   ctagcagaat ttacagatac ccacaactca aaggaaaagg actagtaatt atcattgact 5220
   agcccatctc aattggtata gtgattaaaa tcacctagac caattgagat gtatgtctga 5280
   attagttgtt ttcaaagcaa atgaactagc gattagtcgc tatgacttaa cggagcatga 5340
   aaccaagcta attttatgct gtgtggcact actcaacccc acgattgaaa accctacaag 5400
   gaaagaacgg acggtatcgt tcacttataa ccaatacgct cagatgatga acatcagtag 5460
   ggaaaatgct tatggtgtat tagctaaagc aaccagagag ctgatgacga gaactgtgga 5520
   aatcaggaat cctttggtta aaggctttga gattttccag tggacaaact atgccaagtt 5580
   ctcaagcgaa aaattagaat tagtttttag tgaagagata ttgccttatc ttttccagtt 5640
   aaaaaaattc ataaaatata atctggaaca tgttaagtct tttgaaaaca aatactctat 5700
   gaggatttat gagtggttat taaaagaact aacacaaaag aaaactcaca aggcaaatat 5760
   agagattagc cttgatgaat ttaagttcat gttaatgctt gaaaataact accatgagtt 5820
   taaaaggctt aaccaatggg ttttgaaacc aataagtaaa gatttaaaca cttacagcaa 5880
   tatgaaattg gtggttgata agcgaggccg cccgactgat acgttgattt tccaagttga 5940
   actagataga caaatggatc tcgtaaccga acttgagaac aaccagataa aaatgaatgg 6000
   tgacaaaata ccaacaacca ttacatcaga ttcctaccta cgtaacggac taagaaaaac 6060
   actacacgat gctttaactg caaaaattca gctcaccagt tttgaggcaa aatttttgag 6120
   tgacatgcaa agtaagcatg atctcaatgg ttcgttctca tggctcacgc aaaaacaacg 6180
   aaccacacta gagaacatac tggctaaata cggaaggatc tgaggttctt atggctcttg 6240
   tatctatcag tgaagcatca agactaacaa acaaaagtag aacaactgtt caccgttaga 6300
   tatcaaaggg aaaactgtcc atatgcacag atgaaaacgg tgtaaaaaag atagatacat 6360
   cagagctttt acgagttttt ggtgcattta aagctgttca ccatgaacag atcgacaatg 6420
   taacagatga acagcatgta acacctaata gaacaggtga aaccagtaaa acaaagcaac 6480
   tagaacatga aattgaacac ctgagacaac ttgttacagc tcaacagtca cacatagaca 6540
   gcctgaaaca ggcgatgctg cttatcgaat caaagctgcc gacaacacgg gagccagtga 6600
   cgcctcccgt ggggaaaaaa tcatggcaat tctggaagaa atagcgcttt cagccggcaa 6660
   acctgaagcc ggatctgcga ttctgataac aaactagcaa caccagaaca gcccgtttgc 6720
   gggcagcaaa acccgtactt ttggacgttc cggcggtttt ttgtggcgag tggtgttcgg 6780
   gcggtgcgcg caagatccat tatgttaaac gggcgagttt acatctcaaa accgcccgct 6840
   taacaccatc agaaatcctc agcgcgattt taagcaccaa cccccccccg taacacccaa 6900
   atccatactg aaagtggctt tgttgaataa atcgaacttt tgctgagttg aaggatcaga 6960
   tcacgcatcc tcccgacaac acagaccatt ccgtggcaaa gcaaaagttc agaatcacca 7020
   actggtccac ctacaacaaa gctctcatca accgtggctc cctcactttc tggctggatg 7080
   atgaggcgat tcaggcctgg tatgagtcgg caacaccttc atcacgagga aggccccagc 7140
   gctattctga tctcgccatc accaccgttc tggtgattaa acgcgtattc cggctgaccc 7200
   tgcgggctgc gcagggtttt attgattcca tttttgccct gatgaacgtt ccgttgcgct 7260
   gcccggatta caccagtgtc agtaagcggg caaagtcggt taatgtcagt ttcaaaacgt 7320
   ccacccgggg tgaaatcgca cacctggtga ttgattccac cgggctgaag gtctttggtg 7380
   aaggcgaatg gaaagtcaga aagcacggca aagagcgccg tcgtatctgg cgaaagttgc 7440
   atcttgctgt tgacagcaac acacatgaag ttgtctgtgc agacctgtcg ctgaataacg 7500
   tcacggactc agaagccttc ccgggcctta tccggcagac tcacagaaaa atcagggcag 7560
   ccgcggcaga cggggcttac gatacccggc tctgtcacga tgaactgcgc cgcaaaaaaa 7620
   tcagcgcgct tattcctccc cgaaaaggtg cgggttactg gcccggtgaa tatgcagacc 7680
   gtaaccgtgc agtggctaat cagcgaatga ccgggagtaa tgcgcggtgg aaatggacaa 7740
   cagattacaa ccgtcgctcg atagcggaaa cggcgatgta ccgggtaaaa cagctgttcg 7800
   ggggttcact gacgctgcgt gactacgatg gtcaggttgc ggaggctatg gccctggtac 7860
   gagcgctgaa caaaatgacg aaagcaggta tgcctgaaag cgtgcgtatt gcctgaaaac 7920
   acaacccgct acgggggaga cttacccgaa atctgattta ttcaacaaag ccgggtgtgg 7980
   tgaactacaa agcagacccg ttgaggttat cagttcgatg cacaatcagc agcgcataaa 8040
   atatgcacaa gaacaggagc acccttcgca ttaagctgtg gtggtaacaa gtagtgccgg 8100
   gctaccatca gcgagcatga tgcgctccca cagcattcgc cttggcagta tggaagttcc 8160
   tcgctccagt tcgggccggt atccacctcg cacctggtgt ttaaacggat ttaaattgca 8220
   ggggcagtga gcgcaacgca attaatgtaa gttagctcac tcattaggca ccccaggctt 8280
   gacactttat gcttccggct cgtataatgt gtggaattgt gagcggataa caataacaat 8340
   ttaaatcagg atctaggaac caaggagagt ggcatatgaa tgaaagcagc gcagaccaga 8400
   gcagcggtaa tgtatcagag ggttggccgg acgcgagcgt caccgcgcgt gcgctgcaag 8460
   cgcacctgcg ttacgaacag atcatcgacg ctatcctgag cggctatgcg gaacgtccgg 8520
   cactggcgga gcgcagctac ctggtccgtc cagatccgag cacgggccaa accgttcgcg 8580
   tccacgagca ggcgtttcgt tctatcagct accgtacgct gcaagaacgc gtccacgccc 8640
   tgaccatggc gtggcgtctg cacccggact ccccggtaca agcgggtgca ttcgtcgtgc 8700
   tggttggctt tgccagcatc gattatgcgg ttctggacct ggccctggcc tataccaagg 8760
   gtgtcccggt tccgctgagc ccgaaccact ctagcgagga cgatgacgca atcttgggca 8820
   ccgtgcaacc ggtgaccctg gccgtttcta tctctgagtt ctccggctgc gtggatctga 8880
   ttgctcgtag caccagcatc cgtacggtta ttgtgtttga tttggatccg gcggtggatt 8940
   gtgagcgtgc agccctggag tccggtattc gtgcgctgaa cgagaagggt agcgatgtcg 9000
   ttgtgcaaac cctgcaagac ctgatcgatg tcggccgtga tgcggagttt agctttctgc 9060
   ctattcaggc gcaagaccag gatgatttgg ctctgctgat tcataccagc ggtagcaccg 9120
   gcacgccgaa gggcgcttgc atcagcagcc gtgccctgat caacacctgg cgtcacgtca 9180
   gcggtccgta tcctaaagtg accgtcgttc tggcgccgtt tcaccacatg atgggtcgtg 9240
   acagcatgat taccgccctg ggtgcaggcg gcaccgcgta cttcacgttg cgtccggatc 9300
   tgagcactgt tattgaggac atccgcctgg cacgcccgac cggcctggtg ctgttcccgc 9360
   gtctgtgcga ggtgatcgag caccatctga cgaccgctcc ggagtactct ggtaacgaga 9420
   tcctgggtgg ccgtctgcag tccattgtcg tagcgagcgc tccgattact ccgcgtctga 9480
   aagcgagctt ggagtgtttg ctgggcgttc cggtgagcga gggttattcc agcacggaga 9540
   cggcaagcgg tggtttggca atgaacggcc tgttgaatcg caacaatatt ctggcgtacc 9600
   gcttgcgtga cgttccggaa gcgggttact ctgttaacga ccgcccgttt ccgcgtggcg 9660
   aactgtgtgt taagacgcgc tttggtatta gcggctattt tcgcaatccg gaagcgaccg 9720
   cggaactgtt cgacgatgac ggtttctact gcacgggtga cattgttgaa gaacgtgcac 9780
   cagatcagat cgcgatcatt gaccgccgca aaaacgtcat taaactggct cagggcgagt 9840
   acgtggctgt gggtcgtctg gagcaattgt ttcaagaagg ctgcggttgc gtgcaacaga 9900
   ttcatctgca tggtgacagc acccgtgcat acctgctggc ggtcgttgtt ccggaccgca 9960
   ataccttggc gccacctggt tcgcgtcagg cgtctgaggc agagttgaaa gcgcgcgtcc 10020
   gcgaagagat tctgaccctg gcaaatcagc gcgagctgcg tggctttgaa atcccgcgtg 10080
   acctgattct ggcggaagaa ccgttcagcc aacagaatgg cctgctgagc agcttgggta 10140
   aaccgatccg tccggctatc cgtgcacgct accgtagccg tctggaaagc ctgtatgcga 10200
   gccatgaagc cacccgtggt accgagctgg aagcaatccg tgcgtccgca ggtgccgttg 10260
   atgtggaaac cacgctgttg gccctgctgt ctagcactct gggcgtggtc tgtggtgccg 10320
   cggatcgcca gacctctttt cgcgagctgg gtggcgacag cctggccgca gtgcagctgg 10380
   ccatggagat caaaaagcag ttcggtgtgg gcctggaagg tagccaaatc ctgggtccgg 10440
   gtggtacggt cgaggcgtgg gcccgtcgca tccacaccgc gagcattcag caggcaccgc 10500
   accaacgcgt gggcagcccg ctggctgcaa ttccggccga aggctggctg aaaccggatc 10560
   actatcgtct ggagaacttg atcggcattc cgattggtac cccgtccgca gaggttgctc 10620
   gcccgactgg cggtccgccg accgtgctgc tgacgggtgc gactggtttc ttgggtggcc 10680
   gcctgtgcct ggaatggctg caacgtctgg cgggtcaggg tggtcgcctg atctgtctgg 10740
   tgcgtcctag caacagccac agcgcctggg aacgtctgcg caaccgcttc agccacctgg 10800
   agccggagca ggttgcgcgt tttcgtgaac tggcaggccg ccatctggaa gttattccgg 10860
   cagatatcgg cgagccgggt ttgggcctgg agcctggttg tcaagagcgt ctggccaccg 10920
   aggtcgacgc gatctgtcat tgtgcagccg aggttaacca tcgtctgccg taccgtcacc 10980
   tgtatcgtcc gaatgtgatt ggtaccgcag agatcattca tctggctatt actacccgtc 11040
   tgaagagcgt ggacttcatt tcgagcatcg gtgtggcgag cctgccgcgt cgtccgggtg 11100
   gctcgattcc ggtcgagggc ggttacgcac gcggctattt cgcttccaag tgggcgtgcg 11160
   aacaactgct gcgtagcacc catgattgca ccggtgttcc ggtgcgtgtc attcgtccga 11220
   gcctgattct gccagaccgt gtcctggccg gtgaaatgaa tcctgacgac ctgctgtctc 11280
   gcttgctgta ctcgattctg gttacgggta tcgcgccagg ctgcttcggt gaagagagcc 11340
   agaacagcgg tcgcagcggt ttcagcgttc aaggtctgcc ggtggatcag ctggcgcaga 11400
   cgatcctggc actgggcgag gcacgcacgg aaggcttcca cgttttgaat ctgaatgcgg 11460
   actccggtag cggtgtcccg ttggatgcga ttttgcaaga catcgcggcg aaaggtattc 11520
   gtctgcgtcg tgtcgagggt tacgatctgt ggctggacgc aattaccacg cgcctgcgtc 11580
   gtttgccagc ggaacagcgt gcgcgcagcc tgttggatgt ggcagaggcg tacgccggca 11640
   gcgctggcca gacgacgcaa tccagcggtg agatgcaagc gggtagctcg tcctgcccag 11700
   aagaaatcac cagcctgcag ccggacttca gccgtgccta tcgtcgtaag atcgttgacg 11760
   atctggcgcg ctggggtctg atcgaaccac cgggtccggt tgatcagtaa taagtttaaa 11820
   cagagaagga gttctatcat gattgaaacc atcttaccag ccggcgtcga atcagcagag 11880
   ttacttgagt accctgaaga tctgaaggcg catccggctg aagagcatct gatcgcaaag 11940
   agcgtagaga agcgtcgtcg cgacttcatt ggcgcacgtc attgcgcccg tctggcactg 12000
   gcggagctgg gtgaaccgcc ggttgcgatt ggcaagggtg aacgtggtgc gccgatttgg 12060
   ccgcgtggtg tcgtgggctc tctgacccat tgcgatggct atcgcgcagc ggcggttgct 12120
   cacaaaatgc gttttcgcag catcggcatc gacgccgaac cgcacgcgac cctgccggaa 12180
   ggtgtcctgg attcggttag cctgccgcct gagcgtgagt ggctgaaaac caccgacagc 12240
   gcactgcacc tggaccgttt gctgttttgt gcgaaagaag caacttacaa agcgtggtgg 12300
   ccgctgacgg cacgttggct gggtttcgaa gaagcgcaca ttaccttcga aatcgaggat 12360
   ggtagcgccg actctggtaa tggcacgttt cacagcgaac tgctggtgcc gggtcagacc 12420
   aatgacggtg gtaccccgct gctgtctttc gacggtcgct ggctgatcgc tgatggcttc 12480
   atcctgacgg cgattgcata cgcatgataa attaacctag gctgctgcca ccgctgagca 12540
   ataactagca taaccccttg gggcctctaa acgggtcttg aggggttttt tgctgaaacc 12600
   tcaggcattt gagaagccct cgagtgaccc cagccgcccc tcatgccaac cgagcccatt 12660
   aagcgcagag tcggccgcaa ctatctcggg gacgcataaa cacgcagcga tttaccaagg 12720
   agttcggcta ggtcaatccg caattccctt tgcctggccg gagatgccgc gagcaaacaa 12780
   tgtgtccggt cgctaccaga tggactttgt gggcggagga ttgcaccaga cgcttcagcg 12840
   cagcgtcgta cgatgaatcg gaaggcccgg taagcaattg aagaaccgct gaccacctcc 12900
   catccgacaa tgcaagggtg ccccgctccc agcgcgacac ggttgcctga tccacaccca 12960
   ttaattcagc taggtgactt tgcttcatat gacgaagcaa ccgcgccctc cgcacctgcc 13020
   gccccctgtc attcgtcaac attcttcagc acctcaatgt cgttcgtagt gagcctcatt 13080
   ttttcaagac cgccgatgat gagagccagg cctcgctcga atgccgcgtc cggaccgcct 13140
   tcgtagacga ttttcatcgc gctctgtagg cgcgccggca tcgtagacgc tgaggtggtc 13200
   aactgatctt cgccccgctc ctcggcgtct gcctcgctag cttgctgctc aagaacagcg 13260
   ccgacggtga agtagctgat tgccatcaac gcataggtcg cgtcacctgc cgaaaagcca 13320
   gcatcgcaaa ggaagcgaag ctgcgcgtcg gctttttcca tctgcggcgc ggctggccgc 13380
   gtcccggcat gaatacgcgc gccatcgcga taagcgagca acgcccgtcg aaaactgcat 13440
   gcattgccct tcaggaacga acgccagtcg tcgtcatccc ttggcgtcga atgcgtgtga 13500
   tttatcgtca gcatggcttc ggcaagtgcg tcgagcaacg cacgcttgtt cttgaaatgc 13560
   cagtagagcg ctggctgttg caccccgagg cgctcagcca gtcggcgcgt cgttagacct 13620
   tccatgccca cgtcgttaag cagttcgagc gcggttcgga tcacggcctc gcgttggagc 13680
   ttgttcattc gcgaa 13695
<210> 23
   <211> 13728
   <212> DNA
   <213> Artificial Sequence
<220>
   <223> vector
<400> 23
   ttctcatgtt tgacagctta tcatcgataa gctttaatgc ggtagtttat cacagttaaa      60
   ttgctaacgc agtcaggcac cgtgtatgaa atctaacaat gcgctcatcg tcatcctcgg     120
   caccgtcacc ctggatgctg taggcatagg cttggttatg ccggtactgc cgggcctctt     180
   gcgggatatc gtccattccg acagcatcgc cagtcactat ggcgtgctgc tagcgctata     240
   tgcgttgatg caatttctat gcgcacccgt tctcggagca ctgtccgacc gctttggccg     300
   ccgcccagtc ctgctcgctt cgctacttgg agccactatc gactacgcga tcatggcgac     360
   cacacccgtc ctgtggatcc tctacgccgg acgcatcgtg gccggcatca ccggcgccac     420
   aggtgcggtt gctggcgcct atatcgccga catcaccgat ggggaagatc gggctcgcca     480
   cttcgggctc atgagcgctt gtttcggcgt gggtatggtg gcaggccccg tggccggggg     540
   actgttgggc gccatctcct tgcatgcacc attccttgcg gcggcggtgc tcaacggcct     600
   caacctacta ctgggctgct tcctaatgca ggagtcgcat aagggagagc gtcgaccgat     660
   gcccttgaga gccttcaacc cagtcagctc cttccggtgg gcgcggggca tgactatcgt     720
   cgccgcactt atgactgtct tctttatcat gcaactcgta ggacaggtgc cggcagcgct     780
   ctgggtcatt ttcggcgagg accgctttcg ctggagcgcg acgatgatcg gcctgtcgct     840
   tgcggtattc ggaatcttgc acgccctcgc tcaagccttc gtcactggtc ccgccaccaa     900
   acgtttcggc gagaagcagg ccattatcgc cggcatggcg gccgacgcgc tgggctacgt     960
   cttgctggcg ttcgcgacgc gaggctggat ggccttcccc attatgattc ttctcgcttc    1020
   cggcggcatc gggatgcccg cgttgcaggc catgctgtcc aggcaggtag atgacgacca    1080
   tcagggacag cttcaaggat cgctcgcggc tcttaccagc ctaacttcga tcactggacc    1140
   gctgatcgtc acggcgattt atgccgcctc ggcgagcaca tggaacgggt tggcatggat    1200
   tgtaggcgcc gccctatacc ttgtctgcct ccccgcgttg cgtcgcggtg catggagccg    1260
   ggccacctcg acctgaatgg aagccggcgg cacctcgcta acggattcac cactccaaga    1320
   attggagcca atcaattctt gcggagaact gtgaatgcgc aaaccaaccc ttggcagaac    1380
   atatccatcg cgtccgccat ctccagcagc cgcacgcggc gcatctcggg cagcgttggg    1440
   tcctggccac gggtgcgcat gatcgtgctc ctgtcgttga ggacccggct aggctggcgg    1500
   ggttgcctta ctggttagca gaatgaatca ccgatacgcg agcgaacgtg aagcgactgc    1560
   tgctgcaaaa cgtctgcgac ctgagcaaca acatgaatgg tcttcggttt ccgtgtttcg 1620
   taaagtctgg aaacgcggaa gtcccctacg tgctgctgaa gttgcccgca acagagagtg 1680
   gaaccaaccg gtgataccac gatactatga ctgagagtca acgccatgag cggcctcatt 1740
   tcttattctg agttacaaca gtccgcaccg ctgccggtag ctacttgact atccggctgc 1800
   actagccctg cgtcagatgg ctctgatcca aggcaaactg ccaaaatatc tgctggcacc 1860
   ggaagtcagc gccctgcacc attatgttcc ggatctgcat cgcaggatgc tgctggctac 1920
   cctgtggaac acctacatct gtattaacga agcgctggca ttgaccctga gtgatttttc 1980
   tctggtgccg ccctatccct ttgtgcagct tgccacgctc aaaggggttt gaggtccaac 2040
   cgtacgaaaa cgtacggtaa gaggaaaatt atcgtctgaa aaatcgatta gtagacaaga 2100
   aagtccgtta agtgccaatt ttcgattaaa aagacaccgt tttgatggcg ttttccaatg 2160
   tacattatgt ttcgatatat cagacagtta cttcactaac gtacgttttc gttctattgg 2220
   ccttcagacc ccatatcctt aatgtccttt atttgctggg gttatcagat ccccccgaca 2280
   cgtttaatta atgctttctc cgccggagat cgacgcacag gcttctgtgt ctatgatgtt 2340
   atttcttaat aatcatccag gtattctctt tatcaccata cgtagtgcga gtgtccacct 2400
   taacgcaggg ctttccgtca cagcgcgata tgtcagccag cggggctttc ttttgccaga 2460
   ccgcttccat cctctgcatt tcagcaatct ggctataccc gtcattcata aaccacgtaa 2520
   atgccgtcac gcaggaagcc aggacgaaga atatcgtcag tacaagataa atcgcggatt 2580
   tccacgtata gcgtgacatc tcacgacgca tttcatggat catcgctttc gccgtatcgg 2640
   cagcctgatt cagcgcttct gtcgccggtt tctgctgtgc taatccggct tgtttcagtt 2700
   ctttctcaac ctgagtgagc gcggaactca ccgatttcct gacggtgtca gtcatattac 2760
   cggacgcgct gtccagctca cgaatgaccc tgctcagcgt ttcactttgc tgctgtaatt 2820
   gtgatgaggc ggcctgaaac tgttctgtca gagaagtaac acgcttttcc agcgcctgat 2880
   gatgcccgat aagggcggca atttgtttaa tttcgtcgct catacaaaat cctgcctatc 2940
   gtgagaatga ccagccttta tccggcttct gtcgtatctg ttcggcgagt cgctgtcgtt 3000
   ctttctcctg ctgacgctgt ttttccgcca gacgttcgcg ctctctctgc ctttccatct 3060
   cctgatgtat cccctggaac tccgccatcg catcgttaac aagggactga agatcgattt 3120
   cttcctgtat atccttcatg gcatcactga ccagtgcgtt cagcttgtca ggctcttttt 3180
   caaaatcaaa cgttctgccg gaatgggatt cctgctcagg ctctgacttc agctcctgtt 3240
   ttagcgtcag agtatccctc tcgctgaggg cttcccgtaa cgaggtagtc acgtcaatta 3300
   cgctgtcacg ttcatcacgg gactgctgca cctgcctttc agcctccctg cgctcaagaa 3360
   tggcctgtag ctgctcagta tcgaatcgct gaacctgacc cgcgcccaga tgccgctcag 3420
   gctcacggtc aatgccctgc gccttcaggg aacgggaatc aacccggtca gcgtgctgat 3480
   accgttcaag gtgcttattc tggaggtcag cccagcgtct ccctctgggc aacaaggtat 3540
   tctttgcgtt cggtcggtgt ttccccgaaa cgtgcctttt ttgcgccacc gcgtccggct 3600
   ctttggtgtt agcccgttta aaatactgct cagggtcacg gtgaataccg tcattaatgc 3660
   gttcagagaa catgatatgg gcgtggggct gctcgccacc ggctatcgct gctttcggat 3720
   tatggatagc gaactgatag gcatggcggt cgccaatttc ctgttggaca aaatcgcgga 3780
   caagctcaag acgttgttcg ggttttaact cacgcggcag ggcaatctcg atttcacggt 3840
   aggtacagcc gttggcacgt tcagacgtgt cagcggcttt ccagaactcg gacggtttat 3900
   gcgctgccca cgccggcata ttgccggact ccttgtgctc aaggtcggag tctttttcac 3960
   gggcatactt tccctcacgc gcaatataat cggcatgagg agaggcactg ccttttccgc 4020
   cggtttttac gctgagatga taggatgcca tcgtgtttta tcccgctgaa gggcgcacgt 4080
   ttctgaacga agtgaagaaa gtctaagtgc gccctgataa ataaaagagt tatcagggat 4140
   tgtagtggga tttgacctcc tctgccatca tgagcgtaat cattccgtta gcattcagga 4200
   ggtaaacagc atgaataaaa gcgaaaaaac aggaacaatg ggcagcagaa agagtgcagt 4260
   atattcgcgg cttaaagtcg ccgaatgagc aacagaaact tatgctgata ctgacggata 4320
   aagcagataa aacagcacag gatatcaaaa cgctgtccct gctgatgaag gctgaacagg 4380
   cagcagagaa agcgcaggaa gccagagcga aagtcatgaa cctgatacag gcagaaaagc 4440
   gagccgaagc cagagccgcc cgtaaagccc gtgaccatgc tctgtaccag tctgccggat 4500
   tgcttatcct ggcgggtctg gttgacagta agacgggtaa gcctgttgat gataccgctg 4560
   ccttactggg tgcattagcc agtctgaatg acctgtcacg ggataatccg aagtggtcag 4620
   actggaaaat cagagggcag gaactgctga acagcaaaaa gtcagatagc accacatagc 4680
   agacccgcca taaaacgccc tgagaagccc gtgacgggct tttcttgtat tatgggtagt 4740
   ttccttgcat gaatccataa aaggcgcctg tagtgccatt tacccccatt cactgccaga 4800
   gccgtgagcg cagcgaactg aatgtcacga aaaagacagc gactcaggtg cctgatggtc 4860
   ggagacaaaa ggaatattca gcgatttgcc cgagcttgcg agggtgctac ttaagccttt 4920
   agggttttaa ggtctgtttt gtagaggagc aaacagcgtt tgcgacatcc ttttgtaata 4980
   ctgcggaact gactaaagta gtgagttata cacagggctg ggatctattc tttttatctt 5040
   tttttattct ttctttattc tataaattat aaccacttga atataaacaa aaaaaacaca 5100
   caaaggtcta gcggaattta cagagggtct agcagaattt acaagttttc cagcaaaggt 5160
   ctagcagaat ttacagatac ccacaactca aaggaaaagg actagtaatt atcattgact 5220
   agcccatctc aattggtata gtgattaaaa tcacctagac caattgagat gtatgtctga 5280
   attagttgtt ttcaaagcaa atgaactagc gattagtcgc tatgacttaa cggagcatga 5340
   aaccaagcta attttatgct gtgtggcact actcaacccc acgattgaaa accctacaag 5400
   gaaagaacgg acggtatcgt tcacttataa ccaatacgct cagatgatga acatcagtag 5460
   ggaaaatgct tatggtgtat tagctaaagc aaccagagag ctgatgacga gaactgtgga 5520
   aatcaggaat cctttggtta aaggctttga gattttccag tggacaaact atgccaagtt 5580
   ctcaagcgaa aaattagaat tagtttttag tgaagagata ttgccttatc ttttccagtt 5640
   aaaaaaattc ataaaatata atctggaaca tgttaagtct tttgaaaaca aatactctat 5700
   gaggatttat gagtggttat taaaagaact aacacaaaag aaaactcaca aggcaaatat 5760
   agagattagc cttgatgaat ttaagttcat gttaatgctt gaaaataact accatgagtt 5820
   taaaaggctt aaccaatggg ttttgaaacc aataagtaaa gatttaaaca cttacagcaa 5880
   tatgaaattg gtggttgata agcgaggccg cccgactgat acgttgattt tccaagttga 5940
   actagataga caaatggatc tcgtaaccga acttgagaac aaccagataa aaatgaatgg 6000
   tgacaaaata ccaacaacca ttacatcaga ttcctaccta cgtaacggac taagaaaaac 6060
   actacacgat gctttaactg caaaaattca gctcaccagt tttgaggcaa aatttttgag 6120
   tgacatgcaa agtaagcatg atctcaatgg ttcgttctca tggctcacgc aaaaacaacg 6180
   aaccacacta gagaacatac tggctaaata cggaaggatc tgaggttctt atggctcttg 6240
   tatctatcag tgaagcatca agactaacaa acaaaagtag aacaactgtt caccgttaga 6300
   tatcaaaggg aaaactgtcc atatgcacag atgaaaacgg tgtaaaaaag atagatacat 6360
   cagagctttt acgagttttt ggtgcattta aagctgttca ccatgaacag atcgacaatg 6420
   taacagatga acagcatgta acacctaata gaacaggtga aaccagtaaa acaaagcaac 6480
   tagaacatga aattgaacac ctgagacaac ttgttacagc tcaacagtca cacatagaca 6540
   gcctgaaaca ggcgatgctg cttatcgaat caaagctgcc gacaacacgg gagccagtga 6600
   cgcctcccgt ggggaaaaaa tcatggcaat tctggaagaa atagcgcttt cagccggcaa 6660
   acctgaagcc ggatctgcga ttctgataac aaactagcaa caccagaaca gcccgtttgc 6720
   gggcagcaaa acccgtactt ttggacgttc cggcggtttt ttgtggcgag tggtgttcgg 6780
   gcggtgcgcg caagatccat tatgttaaac gggcgagttt acatctcaaa accgcccgct 6840
   taacaccatc agaaatcctc agcgcgattt taagcaccaa cccccccccg taacacccaa 6900
   atccatactg aaagtggctt tgttgaataa atcgaacttt tgctgagttg aaggatcaga 6960
   tcacgcatcc tcccgacaac acagaccatt ccgtggcaaa gcaaaagttc agaatcacca 7020
   actggtccac ctacaacaaa gctctcatca accgtggctc cctcactttc tggctggatg 7080
   atgaggcgat tcaggcctgg tatgagtcgg caacaccttc atcacgagga aggccccagc 7140
   gctattctga tctcgccatc accaccgttc tggtgattaa acgcgtattc cggctgaccc 7200
   tgcgggctgc gcagggtttt attgattcca tttttgccct gatgaacgtt ccgttgcgct 7260
   gcccggatta caccagtgtc agtaagcggg caaagtcggt taatgtcagt ttcaaaacgt 7320
   ccacccgggg tgaaatcgca cacctggtga ttgattccac cgggctgaag gtctttggtg 7380
   aaggcgaatg gaaagtcaga aagcacggca aagagcgccg tcgtatctgg cgaaagttgc 7440
   atcttgctgt tgacagcaac acacatgaag ttgtctgtgc agacctgtcg ctgaataacg 7500
   tcacggactc agaagccttc ccgggcctta tccggcagac tcacagaaaa atcagggcag 7560
   ccgcggcaga cggggcttac gatacccggc tctgtcacga tgaactgcgc cgcaaaaaaa 7620
   tcagcgcgct tattcctccc cgaaaaggtg cgggttactg gcccggtgaa tatgcagacc 7680
   gtaaccgtgc agtggctaat cagcgaatga ccgggagtaa tgcgcggtgg aaatggacaa 7740
   cagattacaa ccgtcgctcg atagcggaaa cggcgatgta ccgggtaaaa cagctgttcg 7800
   ggggttcact gacgctgcgt gactacgatg gtcaggttgc ggaggctatg gccctggtac 7860
   gagcgctgaa caaaatgacg aaagcaggta tgcctgaaag cgtgcgtatt gcctgaaaac 7920
   acaacccgct acgggggaga cttacccgaa atctgattta ttcaacaaag ccgggtgtgg 7980
   tgaactacaa agcagacccg ttgaggttat cagttcgatg cacaatcagc agcgcataaa 8040
   atatgcacaa gaacaggagc acccttcgca ttaagctgtg gtggtaacaa gtagtgccgg 8100
   gctaccatca gcgagcatga tgcgctccca cagcattcgc cttggcagta tggaagttcc 8160
   tcgctccagt tcgggccggt atccaccgca cctggtgttt aaacggattt aaattgcagg 8220
   ggcagtgagc gcaacgcaat taatgtaagt tagctcactc attaggcacc ccaggcttga 8280
   cactttatgc ttccggctcg tataatgtgt ggaattgtga gcggataaca ataacaattt 8340
   aaatcaggat ctaggaacca aggagagtgg catatgacgg caggcgcagc agcaagagtg 8400
   gctaaattat ttgaatccga ccctcaattt cgtgcggcga tgccggaccc ggcagtgatg 8460
   gatagcctgc tggcaccggg tttgcgcctg tcccaggtgc tgcacgccct gctgtcgggt 8520
   tacgcggagc gtccggtgat gggtttccgt agccgtgaaa gcgttgtaga tacggccacc 8580
   ggccgtactg tcgaccgtct gctgccggca tttgaaacga ttacgtatgg ccagttgctg 8640
   gaagatatca gcgctatcct ggccgagtgg cagcacggtg atatcccgat gggtgctggt 8700
   gacttcatcg cgacgattgg ctttagcagc ccggactatg tcactctgga cctggcgacc 8760
   ctgatgaacg gcagcgtcag catcccgctg caacataaca ccagcgtcgc gcagctgcgt 8820
   atgatgctgg aagagacgag cccgcgtctg gttgccgcga gcgcggactg tttggatttg 8880
   gctgttgaag cggctgttgg cttgacggac ctgcgccgtg tggttgtgtt cgactaccgt 8940
   gcggagacgg acgaccatcg tgagaaactg gcgaccgcgc gtgaacgtct gcacgcagcg 9000
   ggtatggacg tggtcgtaga gccgctggcg gaagtcatcg gccgtggtcg cgatctgccg 9060
   gagccggttc tgtacaccgc cggtgatgac caacgcacgg cgttgattat gtacacgagc 9120
   ggttccacgg gtgccccgaa aggcgcgatg tttacggagt ggaccgttac ccgtttttgg 9180
   agctccggtg cggcaccgaa ccgcgatacg ccgattatca atgtcaattt cctgccgctg 9240
   aaccatctgg cgggtcgcgt cggtctgctg accgcgttca ttccgggtgg cacctgctac 9300
   ttcgtcccgg agagcgattt gtccaccctg tttgaggatt ggcagctggc tcgcccgacg 9360
   cacatgggcg tcgtgcctcg cgtcgtggac atgctgttcc agcactatca gacccgtgtg 9420
   gacgcgctga tggccggtgg taccgatgtt gacaccgccg accgtctggc taagaccgaa 9480
   ttgcgcgagg atgttttggg tggccgtgta gttgcgggta tgttggcgac cgctccgctg 9540
   agccctgaga tgaaagcgtt cctggagtcc tcgttggact ttcacctgct ggacctgtat 9600
   ggtttgaccg aggttggcgg cgttttccgt gatggcaaga tcagccgtcc gccggtgctg 9660
   gattacaaac tggtcgacgt tccggaactg ggttactata cgacggataa accgcatcca 9720
   cgtggcgaac tgctggtgaa gtctgcgacc gcgaccccag gctattacaa acgtccagac 9780
   gttacggccg aggtctttga cgccgatggt tactatcgta ctggcgacgt gatggcagag 9840
   gtcgcgccag accagctggt gtacgttgat cgtcgcaata atgtgattaa gttggctcag 9900
   ggcgagttcg ttgcggttgc gaatctggag actgtttacg tgggtgcgcc gctggtgcgt 9960
   cagatttttg tttacggtaa cagcgaacgt gcctatctgc tggccgttgt ggtgccgacc 10020
   gaagaggcgc tgcgcgcaca cccggatccg gttgagctga agaacagcat tcgtgaatcc 10080
   ctgcagcgta ccgcgcgtag caaccacctg cactcttatg agctgccggc agacttcatt 10140
   attgaaacca ccccgttcac gatcgaaagc ggcatgctgg ctgcggtggg caaaccgatc 10200
   cgcccgaaga tgattgaaca ttatggtgat cgcctggagc agctgtacgt ggatctggca 10260
   gaggcccgtg ttcaggagtt gcgtcagctg cgtgacaccg cacaacaacg cccggttttg 10320
   gataccgtta ctgaggcagc gcaggcactg ctgggcatga gcgccgacgc tgtgcgtccg 10380
   gatcatcatt tcatcgatct gggtggtgac agcctgagcg cgctgacctt cagcaatctg 10440
   ctgcgcgacc tgttcgacgt cgaggtgccg gtgggcgtga ttaccggtcc ggcagcggat 10500
   ttgcgtaaac tggcggcgta tattcaacat gaacgcgaac actctaccgc taccgcagcg 10560
   agcgttcacg gtttggacac taccgttatc agcgcaaccg agctgacgtt ggataagttc 10620
   atcgatgctg aaaccttgca caacgcgagc caactggacg tccctgccgg tgcggtcgca 10680
   acggtcctgc tgaccggtgc gaatggttat ctgggtcgct ttctgtgcct ggaatggctg 10740
   cagcgcctga gccaaactgg tggtcaactg atctgtctgg ttcgtggtga caacgcggat 10800
   caggccctgg cgcgtctggt cgcggcatat ggcgacaccg accgtaccct gctggaagaa 10860
   tttcacaccc tggcacgtcg ccatctgcgt gttattgcgg cagacattgc gcaaccgcgt 10920
   ttcggcgttg acgatgctac gtgggagcaa ctggcacgcg atgtggataa gatcgttcac 10980
   ccggctgccc tggtgaatca cgtgctgccg tacaatcaat tgtttggccc aaacgtattt 11040
   ggtaccgccg aggtgatccg tctggcgttg accacgcgca ttaagccggt tacgtacctg 11100
   agcaccatgg ccgtggcgat gaccgtcccg gacttcgatg aggacggtga catccgcacg 11160
   gtgtctccga cgcgtcatat cgacccgggc tatgccaatg gctacgcaaa ttcgaaatgg 11220
   gcaggtgaag tcctgctgcg tgaggcacat gacatttgcg gcctgccggt tagcgttttt 11280
   cgtagcgata tgattctgac ccaccgtcgt tatagcggtc agctgaacgt caccgatgcg 11340
   tttacgcgta tgctgctgtc tctggttctg accggtatcg cccctcgcag cttttatcaa 11400
   ggtgatggtt ccggtgcgcg tccacgcgcc cactacgaag gtctgccggt ggactttgtc 11460
   accgaggcca ttaccagcct gggtctgagc agctccgagg gcttccgtag ctacgatgtc 11520
   atgaatccgc acgatgatgg tattagcgtc gataccttcg tcgattggct gatggaagat 11580
   ggtcacagca ttgacatcat tgacaactac gacgagtggc tgtctcgctt cgaaactgca 11640
   ctgcgcggct tgccggatga gcaacgtcgc gcgagcgtcc tgccgttgct ggacgcgtac 11700
   cgcattccgg gcaaccctcg ccgtgctgca gcgaccccga accacgtgtt ccgcaaggca 11760
   gtgcaggaga acaatatcgg cggtgacggt gcagacatcc cgcaaattga tcgcgcgctg 11820
   atcgcaaaat acatcgcgga tctgcgtgcg catcgtctgc tgtaataagt ttaaacgaga 11880
   aggagttcta tcatgattga aaccatctta ccagccggcg tcgaatcagc agagttactt 11940
   gagtaccctg aagatctgaa ggcgcatccg gctgaagagc atctgatcgc aaagagcgta 12000
   gagaagcgtc gtcgcgactt cattggcgca cgtcattgcg cccgtctggc actggcggag 12060
   ctgggtgaac cgccggttgc gattggcaag ggtgaacgtg gtgcgccgat ttggccgcgt 12120
   ggtgtcgtgg gctctctgac ccattgcgat ggctatcgcg cagcggcggt tgctcacaaa 12180
   atgcgttttc gcagcatcgg catcgacgcc gaaccgcacg cgaccctgcc ggaaggtgtc 12240
   ctggattcgg ttagcctgcc gcctgagcgt gagtggctga aaaccaccga cagcgcactg 12300
   cacctggacc gtttgctgtt ttgtgcgaaa gaagcaactt acaaagcgtg gtggccgctg 12360
   acggcacgtt ggctgggttt cgaagaagcg cacattacct tcgaaatcga ggatggtagc 12420
   gccgactctg gtaatggcac gtttcacagc gaactgctgg tgccgggtca gaccaatgac 12480
   ggtggtaccc cgctgctgtc tttcgacggt cgctggctga tcgctgatgg cttcatcctg 12540
   acggcgattg catacgcatg ataaattaac ctaggctgct gccaccgctg agcaataact 12600
   agcataaccc cttggggcct ctaaacgggt cttgaggggt tttttgctga gctcgagtga 12660
   ccccagccgc ccctcatgcc aaccgagccc attaagcgca gagtcggccg caactatctc 12720
   ggggacgcat aaacacgcag cgatttacca aggagttcgg ctaggtcaat ccgcaattcc 12780
   ctttgcctgg ccggagatgc cgcgagcaaa caatgtgtcc ggtcgctacc agatggactt 12840
   tgtgggcgga ggattgcacc agacgcttca gcgcagcgtc gtacgatgaa tcggaaggcc 12900
   cggtaagcaa ttgaagaacc gctgaccacc tcccatccga caatgcaagg gtgccccgct 12960
   cccagcgcga cacggttgcc tgatccacac ccattaattc agctaggtga ctttgcttca 13020
   tatgacgaag caaccgcgcc ctccgcacct gccgccccct gtcattcgtc aacattcttc 13080
   agcacctcaa tgtcgttcgt agtgagcctc attttttcaa gaccgccgat gatgagagcc 13140
   aggcctcgct cgaatgccgc gtccggaccg ccttcgtaga cgattttcat cgcgctctgt 13200
   aggcgcgccg gcatcgtaga cgctgaggtg gtcaactgat cttcgccccg ctcctcggcg 13260
   tctgcctcgc tagcttgctg ctcaagaaca gcgccgacgg tgaagtagct gattgccatc 13320
   aacgcatagg tcgcgtcacc tgccgaaaag ccagcatcgc aaaggaagcg aagctgcgcg 13380
   tcggcttttt ccatctgcgg cgcggctggc cgcgtcccgg catgaatacg cgcgccatcg 13440
   cgataagcga gcaacgcccg tcgaaaactg catgcattgc ccttcaggaa cgaacgccag 13500
   tcgtcgtcat cccttggcgt cgaatgcgtg tgatttatcg tcagcatggc ttcggcaagt 13560
   gcgtcgagca acgcacgctt gttcttgaaa tgccagtaga gcgctggctg ttgcaccccg 13620
   aggcgctcag ccagtcggcg cgtcgttaga ccttccatgc ccacgtcgtt aagcagttcg 13680
   agcgcggttc ggatcacggc ctcgcgttgg agcttgttca ttcgcgaa 13728
<210> 24
   <211> 13689
   <212> DNA
   <213> Artificial Sequence
<220>
   <223> vector
<400> 24
   ttctcatgtt tgacagctta tcatcgataa gctttaatgc ggtagtttat cacagttaaa      60
   ttgctaacgc agtcaggcac cgtgtatgaa atctaacaat gcgctcatcg tcatcctcgg     120
   caccgtcacc ctggatgctg taggcatagg cttggttatg ccggtactgc cgggcctctt     180
   gcgggatatc gtccattccg acagcatcgc cagtcactat ggcgtgctgc tagcgctata     240
   tgcgttgatg caatttctat gcgcacccgt tctcggagca ctgtccgacc gctttggccg     300
   ccgcccagtc ctgctcgctt cgctacttgg agccactatc gactacgcga tcatggcgac     360
   cacacccgtc ctgtggatcc tctacgccgg acgcatcgtg gccggcatca ccggcgccac     420
   aggtgcggtt gctggcgcct atatcgccga catcaccgat ggggaagatc gggctcgcca     480
   cttcgggctc atgagcgctt gtttcggcgt gggtatggtg gcaggccccg tggccggggg     540
   actgttgggc gccatctcct tgcatgcacc attccttgcg gcggcggtgc tcaacggcct     600
   caacctacta ctgggctgct tcctaatgca ggagtcgcat aagggagagc gtcgaccgat     660
   gcccttgaga gccttcaacc cagtcagctc cttccggtgg gcgcggggca tgactatcgt     720
   cgccgcactt atgactgtct tctttatcat gcaactcgta ggacaggtgc cggcagcgct 780
   ctgggtcatt ttcggcgagg accgctttcg ctggagcgcg acgatgatcg gcctgtcgct 840
   tgcggtattc ggaatcttgc acgccctcgc tcaagccttc gtcactggtc ccgccaccaa 900
   acgtttcggc gagaagcagg ccattatcgc cggcatggcg gccgacgcgc tgggctacgt 960
   cttgctggcg ttcgcgacgc gaggctggat ggccttcccc attatgattc ttctcgcttc 1020
   cggcggcatc gggatgcccg cgttgcaggc catgctgtcc aggcaggtag atgacgacca 1080
   tcagggacag cttcaaggat cgctcgcggc tcttaccagc ctaacttcga tcactggacc 1140
   gctgatcgtc acggcgattt atgccgcctc ggcgagcaca tggaacgggt tggcatggat 1200
   tgtaggcgcc gccctatacc ttgtctgcct ccccgcgttg cgtcgcggtg catggagccg 1260
   ggccacctcg acctgaatgg aagccggcgg cacctcgcta acggattcac cactccaaga 1320
   attggagcca atcaattctt gcggagaact gtgaatgcgc aaaccaaccc ttggcagaac 1380
   atatccatcg cgtccgccat ctccagcagc cgcacgcggc gcatctcggg cagcgttggg 1440
   tcctggccac gggtgcgcat gatcgtgctc ctgtcgttga ggacccggct aggctggcgg 1500
   ggttgcctta ctggttagca gaatgaatca ccgatacgcg agcgaacgtg aagcgactgc 1560
   tgctgcaaaa cgtctgcgac ctgagcaaca acatgaatgg tcttcggttt ccgtgtttcg 1620
   taaagtctgg aaacgcggaa gtcccctacg tgctgctgaa gttgcccgca acagagagtg 1680
   gaaccaaccg gtgataccac gatactatga ctgagagtca acgccatgag cggcctcatt 1740
   tcttattctg agttacaaca gtccgcaccg ctgccggtag ctacttgact atccggctgc 1800
   actagccctg cgtcagatgg ctctgatcca aggcaaactg ccaaaatatc tgctggcacc 1860
   ggaagtcagc gccctgcacc attatgttcc ggatctgcat cgcaggatgc tgctggctac 1920
   cctgtggaac acctacatct gtattaacga agcgctggca ttgaccctga gtgatttttc 1980
   tctggtgccg ccctatccct ttgtgcagct tgccacgctc aaaggggttt gaggtccaac 2040
   cgtacgaaaa cgtacggtaa gaggaaaatt atcgtctgaa aaatcgatta gtagacaaga 2100
   aagtccgtta agtgccaatt ttcgattaaa aagacaccgt tttgatggcg ttttccaatg 2160
   tacattatgt ttcgatatat cagacagtta cttcactaac gtacgttttc gttctattgg 2220
   ccttcagacc ccatatcctt aatgtccttt atttgctggg gttatcagat ccccccgaca 2280
   cgtttaatta atgctttctc cgccggagat cgacgcacag gcttctgtgt ctatgatgtt 2340
   atttcttaat aatcatccag gtattctctt tatcaccata cgtagtgcga gtgtccacct 2400
   taacgcaggg ctttccgtca cagcgcgata tgtcagccag cggggctttc ttttgccaga 2460
   ccgcttccat cctctgcatt tcagcaatct ggctataccc gtcattcata aaccacgtaa 2520
   atgccgtcac gcaggaagcc aggacgaaga atatcgtcag tacaagataa atcgcggatt 2580
   tccacgtata gcgtgacatc tcacgacgca tttcatggat catcgctttc gccgtatcgg 2640
   cagcctgatt cagcgcttct gtcgccggtt tctgctgtgc taatccggct tgtttcagtt 2700
   ctttctcaac ctgagtgagc gcggaactca ccgatttcct gacggtgtca gtcatattac 2760
   cggacgcgct gtccagctca cgaatgaccc tgctcagcgt ttcactttgc tgctgtaatt 2820
   gtgatgaggc ggcctgaaac tgttctgtca gagaagtaac acgcttttcc agcgcctgat 2880
   gatgcccgat aagggcggca atttgtttaa tttcgtcgct catacaaaat cctgcctatc 2940
   gtgagaatga ccagccttta tccggcttct gtcgtatctg ttcggcgagt cgctgtcgtt 3000
   ctttctcctg ctgacgctgt ttttccgcca gacgttcgcg ctctctctgc ctttccatct 3060
   cctgatgtat cccctggaac tccgccatcg catcgttaac aagggactga agatcgattt 3120
   cttcctgtat atccttcatg gcatcactga ccagtgcgtt cagcttgtca ggctcttttt 3180
   caaaatcaaa cgttctgccg gaatgggatt cctgctcagg ctctgacttc agctcctgtt 3240
   ttagcgtcag agtatccctc tcgctgaggg cttcccgtaa cgaggtagtc acgtcaatta 3300
   cgctgtcacg ttcatcacgg gactgctgca cctgcctttc agcctccctg cgctcaagaa 3360
   tggcctgtag ctgctcagta tcgaatcgct gaacctgacc cgcgcccaga tgccgctcag 3420
   gctcacggtc aatgccctgc gccttcaggg aacgggaatc aacccggtca gcgtgctgat 3480
   accgttcaag gtgcttattc tggaggtcag cccagcgtct ccctctgggc aacaaggtat 3540
   tctttgcgtt cggtcggtgt ttccccgaaa cgtgcctttt ttgcgccacc gcgtccggct 3600
   ctttggtgtt agcccgttta aaatactgct cagggtcacg gtgaataccg tcattaatgc 3660
   gttcagagaa catgatatgg gcgtggggct gctcgccacc ggctatcgct gctttcggat 3720
   tatggatagc gaactgatag gcatggcggt cgccaatttc ctgttggaca aaatcgcgga 3780
   caagctcaag acgttgttcg ggttttaact cacgcggcag ggcaatctcg atttcacggt 3840
   aggtacagcc gttggcacgt tcagacgtgt cagcggcttt ccagaactcg gacggtttat 3900
   gcgctgccca cgccggcata ttgccggact ccttgtgctc aaggtcggag tctttttcac 3960
   gggcatactt tccctcacgc gcaatataat cggcatgagg agaggcactg ccttttccgc 4020
   cggtttttac gctgagatga taggatgcca tcgtgtttta tcccgctgaa gggcgcacgt 4080
   ttctgaacga agtgaagaaa gtctaagtgc gccctgataa ataaaagagt tatcagggat 4140
   tgtagtggga tttgacctcc tctgccatca tgagcgtaat cattccgtta gcattcagga 4200
   ggtaaacagc atgaataaaa gcgaaaaaac aggaacaatg ggcagcagaa agagtgcagt 4260
   atattcgcgg cttaaagtcg ccgaatgagc aacagaaact tatgctgata ctgacggata 4320
   aagcagataa aacagcacag gatatcaaaa cgctgtccct gctgatgaag gctgaacagg 4380
   cagcagagaa agcgcaggaa gccagagcga aagtcatgaa cctgatacag gcagaaaagc 4440
   gagccgaagc cagagccgcc cgtaaagccc gtgaccatgc tctgtaccag tctgccggat 4500
   tgcttatcct ggcgggtctg gttgacagta agacgggtaa gcctgttgat gataccgctg 4560
   ccttactggg tgcattagcc agtctgaatg acctgtcacg ggataatccg aagtggtcag 4620
   actggaaaat cagagggcag gaactgctga acagcaaaaa gtcagatagc accacatagc 4680
   agacccgcca taaaacgccc tgagaagccc gtgacgggct tttcttgtat tatgggtagt 4740
   ttccttgcat gaatccataa aaggcgcctg tagtgccatt tacccccatt cactgccaga 4800
   gccgtgagcg cagcgaactg aatgtcacga aaaagacagc gactcaggtg cctgatggtc 4860
   ggagacaaaa ggaatattca gcgatttgcc cgagcttgcg agggtgctac ttaagccttt 4920
   agggttttaa ggtctgtttt gtagaggagc aaacagcgtt tgcgacatcc ttttgtaata 4980
   ctgcggaact gactaaagta gtgagttata cacagggctg ggatctattc tttttatctt 5040
   tttttattct ttctttattc tataaattat aaccacttga atataaacaa aaaaaacaca 5100
   caaaggtcta gcggaattta cagagggtct agcagaattt acaagttttc cagcaaaggt 5160
   ctagcagaat ttacagatac ccacaactca aaggaaaagg actagtaatt atcattgact 5220
   agcccatctc aattggtata gtgattaaaa tcacctagac caattgagat gtatgtctga 5280
   attagttgtt ttcaaagcaa atgaactagc gattagtcgc tatgacttaa cggagcatga 5340
   aaccaagcta attttatgct gtgtggcact actcaacccc acgattgaaa accctacaag 5400
   gaaagaacgg acggtatcgt tcacttataa ccaatacgct cagatgatga acatcagtag 5460
   ggaaaatgct tatggtgtat tagctaaagc aaccagagag ctgatgacga gaactgtgga 5520
   aatcaggaat cctttggtta aaggctttga gattttccag tggacaaact atgccaagtt 5580
   ctcaagcgaa aaattagaat tagtttttag tgaagagata ttgccttatc ttttccagtt 5640
   aaaaaaattc ataaaatata atctggaaca tgttaagtct tttgaaaaca aatactctat 5700
   gaggatttat gagtggttat taaaagaact aacacaaaag aaaactcaca aggcaaatat 5760
   agagattagc cttgatgaat ttaagttcat gttaatgctt gaaaataact accatgagtt 5820
   taaaaggctt aaccaatggg ttttgaaacc aataagtaaa gatttaaaca cttacagcaa 5880
   tatgaaattg gtggttgata agcgaggccg cccgactgat acgttgattt tccaagttga 5940
   actagataga caaatggatc tcgtaaccga acttgagaac aaccagataa aaatgaatgg 6000
   tgacaaaata ccaacaacca ttacatcaga ttcctaccta cgtaacggac taagaaaaac 6060
   actacacgat gctttaactg caaaaattca gctcaccagt tttgaggcaa aatttttgag 6120
   tgacatgcaa agtaagcatg atctcaatgg ttcgttctca tggctcacgc aaaaacaacg 6180
   aaccacacta gagaacatac tggctaaata cggaaggatc tgaggttctt atggctcttg 6240
   tatctatcag tgaagcatca agactaacaa acaaaagtag aacaactgtt caccgttaga 6300
   tatcaaaggg aaaactgtcc atatgcacag atgaaaacgg tgtaaaaaag atagatacat 6360
   cagagctttt acgagttttt ggtgcattta aagctgttca ccatgaacag atcgacaatg 6420
   taacagatga acagcatgta acacctaata gaacaggtga aaccagtaaa acaaagcaac 6480
   tagaacatga aattgaacac ctgagacaac ttgttacagc tcaacagtca cacatagaca 6540
   gcctgaaaca ggcgatgctg cttatcgaat caaagctgcc gacaacacgg gagccagtga 6600
   cgcctcccgt ggggaaaaaa tcatggcaat tctggaagaa atagcgcttt cagccggcaa 6660
   acctgaagcc ggatctgcga ttctgataac aaactagcaa caccagaaca gcccgtttgc 6720
   gggcagcaaa acccgtactt ttggacgttc cggcggtttt ttgtggcgag tggtgttcgg 6780
   gcggtgcgcg caagatccat tatgttaaac gggcgagttt acatctcaaa accgcccgct 6840
   taacaccatc agaaatcctc agcgcgattt taagcaccaa cccccccccg taacacccaa 6900
   atccatactg aaagtggctt tgttgaataa atcgaacttt tgctgagttg aaggatcaga 6960
   tcacgcatcc tcccgacaac acagaccatt ccgtggcaaa gcaaaagttc agaatcacca 7020
   actggtccac ctacaacaaa gctctcatca accgtggctc cctcactttc tggctggatg 7080
   atgaggcgat tcaggcctgg tatgagtcgg caacaccttc atcacgagga aggccccagc 7140
   gctattctga tctcgccatc accaccgttc tggtgattaa acgcgtattc cggctgaccc 7200
   tgcgggctgc gcagggtttt attgattcca tttttgccct gatgaacgtt ccgttgcgct 7260
   gcccggatta caccagtgtc agtaagcggg caaagtcggt taatgtcagt ttcaaaacgt 7320
   ccacccgggg tgaaatcgca cacctggtga ttgattccac cgggctgaag gtctttggtg 7380
   aaggcgaatg gaaagtcaga aagcacggca aagagcgccg tcgtatctgg cgaaagttgc 7440
   atcttgctgt tgacagcaac acacatgaag ttgtctgtgc agacctgtcg ctgaataacg 7500
   tcacggactc agaagccttc ccgggcctta tccggcagac tcacagaaaa atcagggcag 7560
   ccgcggcaga cggggcttac gatacccggc tctgtcacga tgaactgcgc cgcaaaaaaa 7620
   tcagcgcgct tattcctccc cgaaaaggtg cgggttactg gcccggtgaa tatgcagacc 7680
   gtaaccgtgc agtggctaat cagcgaatga ccgggagtaa tgcgcggtgg aaatggacaa 7740
   cagattacaa ccgtcgctcg atagcggaaa cggcgatgta ccgggtaaaa cagctgttcg 7800
   ggggttcact gacgctgcgt gactacgatg gtcaggttgc ggaggctatg gccctggtac 7860
   gagcgctgaa caaaatgacg aaagcaggta tgcctgaaag cgtgcgtatt gcctgaaaac 7920
   acaacccgct acgggggaga cttacccgaa atctgattta ttcaacaaag ccgggtgtgg 7980
   tgaactacaa agcagacccg ttgaggttat cagttcgatg cacaatcagc agcgcataaa 8040
   atatgcacaa gaacaggagc acccttcgca ttaagctgtg gtggtaacaa gtagtgccgg 8100
   gctaccatca gcgagcatga tgcgctccca cagcattcgc cttggcagta tggaagttcc 8160
   tcgctccagt tcgggccggt atccaccgca cctggtgttt aaacggattt aaattgcagg 8220
   ggcagtgagc gcaacgcaat taatgtaagt tagctcactc attaggcacc ccaggcttga 8280
   cactttatgc ttccggctcg tataatgtgt ggaattgtga gcggataaca ataacaattt 8340
   aaatcaggat ctaggaacca aggagagtgg catatgtttg cagaggatga acaagtgaag 8400
   gcggcggtac cagatcagga agttgtggaa gcgatccgcg caccgggtct gcgcctggct 8460
   caaatcatgg cgacggtgat ggagcgctac gccgaccgtc cggccgttgg tcaacgtgct 8520
   tctgagccgg ttaccgagag cggccgtacg acgtttcgtc tgctgccgga gtttgagacg 8580
   ttgacctacc gtgaactgtg ggcgcgtgtc cgtgcagtcg ccgcagcttg gcacggtgac 8640
   gctgagcgtc cactgcgcgc aggtgacttc gtcgcgctgc tgggctttgc cggtatcgac 8700
   tacggcaccc tggacttggc gaacatccac ctgggcttgg ttaccgtccc gctgcagtct 8760
   ggcgcgaccg ctccgcaact ggcggcgatc ctggccgaaa cgaccccgcg tgtcttggcg 8820
   gcgacgccgg atcatctgga tattgcggtt gagttgctga ctggtggcgc gagcccggaa 8880
   cgcttggttg tgttcgacta ccgtccggca gatgatgacc accgtgcagc attggagagc 8940
   gcacgtcgcc gtttgagcga tgcaggcagc gcggtcgttg ttgaaacctt ggacgcggtg 9000
   cgtgctcgtg gttccgaact gccggcagca ccgctgttcg tcccggctgc ggatgaagat 9060
   ccgttggcgc tgctgattta cacgagcggt agcaccggca ccccgaaagg cgcaatgtat 9120
   accgagcgcc tgaatcgtac gacctggctg agcggtgcga agggcgtggg cctgacgttg 9180
   ggttatatgc cgatgagcca tatcgcgggt cgtgcgagct tcgcgggtgt gctggcgcgt 9240
   ggtggtaccg tctactttac cgcccgtagc gatatgagca ctctgtttga ggatttggcg 9300
   ctggtccgtc cgacggagat gttcttcgtt ccgcgcgtgt gcgacatgat ctttcagcgt 9360
   tatcaagcgg agttgtcgcg tcgtgcgcca gccgcagcgg caagccctga actggaacag 9420
   gaactgaaaa ccgagctgcg tctgtccgcg gttggcgacc gcctgctggg tgcgattgcg 9480
   ggttctgcac cgctgagcgc ggagatgcgt gagtttatgg agtccttgct ggatctggag 9540
   ttgcatgacg gctacggttc caccgaggcc ggcatcggtg tcctgcaaga caatatcgtt 9600
   caacgtccgc cggttattga ttacaagctg gtggacgttc cggagttggg ctatttccgt 9660
   accgaccagc cgcaccctcg cggtgaactg ctgctgaaaa cggaaggtat gattccgggt 9720
   tatttccgtc gcccggaagt taccgcggag attttcgatg aggacggctt ctatcgtacg 9780
   ggtgatatcg ttgcggaact ggagccagac cgcctgatct atctggaccg tcgcaataac 9840
   gtactgaagc tggcgcaagg cgagtttgtc acggtggccc atctggaagc agtgttcgcg 9900
   acgtctccgc tgatccgtca gatctacatc tatggtaaca gcgagcgtag ctttttgctg 9960
   gcagtcattg tgccgaccgc ggacgcgctg gcggacggtg tgaccgatgc cctgaacacc 10020
   gctctgactg agtcgctgcg ccagttggcg aaagaagccg gtctgcagag ctatgaactg 10080
   ccgcgtgagt tcctggtcga gacggagccg ttcaccgttg agaatggtct gctgagcggt 10140
   attgcgaaac tgctgcgtcc gaagctgaaa gaacactacg gtgaacgcct ggagcagttg 10200
   tatcgcgaca ttgaagccaa tcgcaacgat gaactgatcg agctgcgccg taccgcagcg 10260
   gagttgccgg ttctggaaac tgtgacgcgt gcggcacgca gcatgctggg tctggccgcg 10320
   agcgaactgc gtccagacgc gcattttacc gacctgggtg gtgactctct gtcggcgctg 10380
   tcctttagca ccctgctgca agatatgttg gaagttgagg tgccggtggg cgttattgtg 10440
   tctccggcaa acagcctggc tgacctggcc aagtatattg aggcggaacg tcactccggt 10500
   gtccgccgtc cgagcctgat cagcgtgcac ggcccaggta ccgagattcg cgctgcagat 10560
   ctgacgctgg acaaattcat tgacgagcgc accctggcag cggcgaaggc agttccggct 10620
   gcacctgccc aagcacagac cgtgctgctg accggtgcaa atggttacct gggtcgtttc 10680
   ttgtgcctgg aatggctgca gcgtctggat caaaccggcg gtacgctggt ctgtattgtg 10740
   cgcggcaccg atgcggcagc agcgcgtaag cgcctggatg cggttttcga cagcggtgat 10800
   cctgagctgc tggatcacta ccgtaagctg gcagccgagc acctggaagt tctggcgggt 10860
   gacatcggtg acccgaacct gggtctggat gaggccacgt ggcagcgcct ggccgcgacc 10920
   gtggatctga ttgtgcatcc ggctgctttg gttaatcacg tgctgccgta cagccagctg 10980
   ttcggtccga acgtcgtcgg caccgcagag attattcgcc tggcgattac cgagcgtcgt 11040
   aaaccggtga cctacctgag cactgtcgcg gttgccgccc aagtggaccc tgcgggcttc 11100
   gatgaagaac gtgacatccg cgagatgagc gcggtccgta gcattgacgc gggctacgcg 11160
   aacggctatg gtaatagcaa atgggctggt gaggttctgc tgcgcgaggc acacgatctg 11220
   tgtggtctgc cggtggccgt attccgcagc gatatgattc tggcacactc caaatacgtt 11280
   ggccagctga acgtgccgga cgtttttacg cgtctgattc tgagcctggc cctgacgggc 11340
   atcgctccgt atagctttta cggtactgac agcgccggtc aacgtcgtcg tgcacactat 11400
   gacggcctgc cggcagattt cgtcgccgaa gctattacca cgctgggcgc tcgcgcagaa 11460
   agcggctttc acacctacga cgtttggaac ccgtacgacg acggtatttc cctggacgaa 11520
   tttgtggact ggctgggcga ttttggcgtt ccgattcaac gtatcgatga ttatgacgag 11580
   tggttccgcc gtttcgaaac cgcgatccgt gcgctgccgg agaaacagcg cgacgcgagc 11640
   ctgctgccgc tgctggatgc ccatcgtcgt ccattgcgtg cagtgcgtgg tagcctgttg 11700
   ccggcgaaga attttcaggc tgcggtccag tcggcacgca tcggtccgga ccaggatatc 11760
   ccgcatctga gcccgcaact gatcgataag tacgtcaccg atttgcgtca tttgggcctg 11820
   ctgtaataag tttaaacgag aaggagttct atcatgattg aaaccatctt accagccggc 11880
   gtcgaatcag cagagttact tgagtaccct gaagatctga aggcgcatcc ggctgaagag 11940
   catctgatcg caaagagcgt agagaagcgt cgtcgcgact tcattggcgc acgtcattgc 12000
   gcccgtctgg cactggcgga gctgggtgaa ccgccggttg cgattggcaa gggtgaacgt 12060
   ggtgcgccga tttggccgcg tggtgtcgtg ggctctctga cccattgcga tggctatcgc 12120
   gcagcggcgg ttgctcacaa aatgcgtttt cgcagcatcg gcatcgacgc cgaaccgcac 12180
   gcgaccctgc cggaaggtgt cctggattcg gttagcctgc cgcctgagcg tgagtggctg 12240
   aaaaccaccg acagcgcact gcacctggac cgtttgctgt tttgtgcgaa agaagcaact 12300
   tacaaagcgt ggtggccgct gacggcacgt tggctgggtt tcgaagaagc gcacattacc 12360
   ttcgaaatcg aggatggtag cgccgactct ggtaatggca cgtttcacag cgaactgctg 12420
   gtgccgggtc agaccaatga cggtggtacc ccgctgctgt ctttcgacgg tcgctggctg 12480
   atcgctgatg gcttcatcct gacggcgatt gcatacgcat gataaattaa cctaggctgc 12540
   tgccaccgct gagcaataac tagcataacc ccttggggcc tctaaacggg tcttgagggg 12600
   ttttttgctg agctcgagtg accccagccg cccctcatgc caaccgagcc cattaagcgc 12660
   agagtcggcc gcaactatct cggggacgca taaacacgca gcgatttacc aaggagttcg 12720
   gctaggtcaa tccgcaattc cctttgcctg gccggagatg ccgcgagcaa acaatgtgtc 12780
   cggtcgctac cagatggact ttgtgggcgg aggattgcac cagacgcttc agcgcagcgt 12840
   cgtacgatga atcggaaggc ccggtaagca attgaagaac cgctgaccac ctcccatccg 12900
   acaatgcaag ggtgccccgc tcccagcgcg acacggttgc ctgatccaca cccattaatt 12960
   cagctaggtg actttgcttc atatgacgaa gcaaccgcgc cctccgcacc tgccgccccc 13020
   tgtcattcgt caacattctt cagcacctca atgtcgttcg tagtgagcct cattttttca 13080
   agaccgccga tgatgagagc caggcctcgc tcgaatgccg cgtccggacc gccttcgtag 13140
   acgattttca tcgcgctctg taggcgcgcc ggcatcgtag acgctgaggt ggtcaactga 13200
   tcttcgcccc gctcctcggc gtctgcctcg ctagcttgct gctcaagaac agcgccgacg 13260
   gtgaagtagc tgattgccat caacgcatag gtcgcgtcac ctgccgaaaa gccagcatcg 13320
   caaaggaagc gaagctgcgc gtcggctttt tccatctgcg gcgcggctgg ccgcgtcccg 13380
   gcatgaatac gcgcgccatc gcgataagcg agcaacgccc gtcgaaaact gcatgcattg 13440
   cccttcagga acgaacgcca gtcgtcgtca tcccttggcg tcgaatgcgt gtgatttatc 13500
   gtcagcatgg cttcggcaag tgcgtcgagc aacgcacgct tgttcttgaa atgccagtag 13560
   agcgctggct gttgcacccc gaggcgctca gccagtcggc gcgtcgttag accttccatg 13620
   cccacgtcgt taagcagttc gagcgcggtt cggatcacgg cctcgcgttg gagcttgttc 13680
   attcgcgaa 13689
<210> 25
   <211> 13803
   <212> DNA
   <213> Artificial Sequence
<220>
   <223> vector
<400> 25
   ttctcatgtt tgacagctta tcatcgataa gctttaatgc ggtagtttat cacagttaaa      60
   ttgctaacgc agtcaggcac cgtgtatgaa atctaacaat gcgctcatcg tcatcctcgg     120
   caccgtcacc ctggatgctg taggcatagg cttggttatg ccggtactgc cgggcctctt     180
   gcgggatatc gtccattccg acagcatcgc cagtcactat ggcgtgctgc tagcgctata     240
   tgcgttgatg caatttctat gcgcacccgt tctcggagca ctgtccgacc gctttggccg     300
   ccgcccagtc ctgctcgctt cgctacttgg agccactatc gactacgcga tcatggcgac     360
   cacacccgtc ctgtggatcc tctacgccgg acgcatcgtg gccggcatca ccggcgccac     420
   aggtgcggtt gctggcgcct atatcgccga catcaccgat ggggaagatc gggctcgcca     480
   cttcgggctc atgagcgctt gtttcggcgt gggtatggtg gcaggccccg tggccggggg     540
   actgttgggc gccatctcct tgcatgcacc attccttgcg gcggcggtgc tcaacggcct     600
   caacctacta ctgggctgct tcctaatgca ggagtcgcat aagggagagc gtcgaccgat     660
   gcccttgaga gccttcaacc cagtcagctc cttccggtgg gcgcggggca tgactatcgt     720
   cgccgcactt atgactgtct tctttatcat gcaactcgta ggacaggtgc cggcagcgct     780
   ctgggtcatt ttcggcgagg accgctttcg ctggagcgcg acgatgatcg gcctgtcgct     840
   tgcggtattc ggaatcttgc acgccctcgc tcaagccttc gtcactggtc ccgccaccaa     900
   acgtttcggc gagaagcagg ccattatcgc cggcatggcg gccgacgcgc tgggctacgt     960
   cttgctggcg ttcgcgacgc gaggctggat ggccttcccc attatgattc ttctcgcttc    1020
   cggcggcatc gggatgcccg cgttgcaggc catgctgtcc aggcaggtag atgacgacca    1080
   tcagggacag cttcaaggat cgctcgcggc tcttaccagc ctaacttcga tcactggacc    1140
   gctgatcgtc acggcgattt atgccgcctc ggcgagcaca tggaacgggt tggcatggat    1200
   tgtaggcgcc gccctatacc ttgtctgcct ccccgcgttg cgtcgcggtg catggagccg    1260
   ggccacctcg acctgaatgg aagccggcgg cacctcgcta acggattcac cactccaaga    1320
   attggagcca atcaattctt gcggagaact gtgaatgcgc aaaccaaccc ttggcagaac    1380
   atatccatcg cgtccgccat ctccagcagc cgcacgcggc gcatctcggg cagcgttggg    1440
   tcctggccac gggtgcgcat gatcgtgctc ctgtcgttga ggacccggct aggctggcgg    1500
   ggttgcctta ctggttagca gaatgaatca ccgatacgcg agcgaacgtg aagcgactgc    1560
   tgctgcaaaa cgtctgcgac ctgagcaaca acatgaatgg tcttcggttt ccgtgtttcg    1620
   taaagtctgg aaacgcggaa gtcccctacg tgctgctgaa gttgcccgca acagagagtg    1680
   gaaccaaccg gtgataccac gatactatga ctgagagtca acgccatgag cggcctcatt    1740
   tcttattctg agttacaaca gtccgcaccg ctgccggtag ctacttgact atccggctgc    1800
   actagccctg cgtcagatgg ctctgatcca aggcaaactg ccaaaatatc tgctggcacc 1860
   ggaagtcagc gccctgcacc attatgttcc ggatctgcat cgcaggatgc tgctggctac 1920
   cctgtggaac acctacatct gtattaacga agcgctggca ttgaccctga gtgatttttc 1980
   tctggtgccg ccctatccct ttgtgcagct tgccacgctc aaaggggttt gaggtccaac 2040
   cgtacgaaaa cgtacggtaa gaggaaaatt atcgtctgaa aaatcgatta gtagacaaga 2100
   aagtccgtta agtgccaatt ttcgattaaa aagacaccgt tttgatggcg ttttccaatg 2160
   tacattatgt ttcgatatat cagacagtta cttcactaac gtacgttttc gttctattgg 2220
   ccttcagacc ccatatcctt aatgtccttt atttgctggg gttatcagat ccccccgaca 2280
   cgtttaatta atgctttctc cgccggagat cgacgcacag gcttctgtgt ctatgatgtt 2340
   atttcttaat aatcatccag gtattctctt tatcaccata cgtagtgcga gtgtccacct 2400
   taacgcaggg ctttccgtca cagcgcgata tgtcagccag cggggctttc ttttgccaga 2460
   ccgcttccat cctctgcatt tcagcaatct ggctataccc gtcattcata aaccacgtaa 2520
   atgccgtcac gcaggaagcc aggacgaaga atatcgtcag tacaagataa atcgcggatt 2580
   tccacgtata gcgtgacatc tcacgacgca tttcatggat catcgctttc gccgtatcgg 2640
   cagcctgatt cagcgcttct gtcgccggtt tctgctgtgc taatccggct tgtttcagtt 2700
   ctttctcaac ctgagtgagc gcggaactca ccgatttcct gacggtgtca gtcatattac 2760
   cggacgcgct gtccagctca cgaatgaccc tgctcagcgt ttcactttgc tgctgtaatt 2820
   gtgatgaggc ggcctgaaac tgttctgtca gagaagtaac acgcttttcc agcgcctgat 2880
   gatgcccgat aagggcggca atttgtttaa tttcgtcgct catacaaaat cctgcctatc 2940
   gtgagaatga ccagccttta tccggcttct gtcgtatctg ttcggcgagt cgctgtcgtt 3000
   ctttctcctg ctgacgctgt ttttccgcca gacgttcgcg ctctctctgc ctttccatct 3060
   cctgatgtat cccctggaac tccgccatcg catcgttaac aagggactga agatcgattt 3120
   cttcctgtat atccttcatg gcatcactga ccagtgcgtt cagcttgtca ggctcttttt 3180
   caaaatcaaa cgttctgccg gaatgggatt cctgctcagg ctctgacttc agctcctgtt 3240
   ttagcgtcag agtatccctc tcgctgaggg cttcccgtaa cgaggtagtc acgtcaatta 3300
   cgctgtcacg ttcatcacgg gactgctgca cctgcctttc agcctccctg cgctcaagaa 3360
   tggcctgtag ctgctcagta tcgaatcgct gaacctgacc cgcgcccaga tgccgctcag 3420
   gctcacggtc aatgccctgc gccttcaggg aacgggaatc aacccggtca gcgtgctgat 3480
   accgttcaag gtgcttattc tggaggtcag cccagcgtct ccctctgggc aacaaggtat 3540
   tctttgcgtt cggtcggtgt ttccccgaaa cgtgcctttt ttgcgccacc gcgtccggct 3600
   ctttggtgtt agcccgttta aaatactgct cagggtcacg gtgaataccg tcattaatgc 3660
   gttcagagaa catgatatgg gcgtggggct gctcgccacc ggctatcgct gctttcggat 3720
   tatggatagc gaactgatag gcatggcggt cgccaatttc ctgttggaca aaatcgcgga 3780
   caagctcaag acgttgttcg ggttttaact cacgcggcag ggcaatctcg atttcacggt 3840
   aggtacagcc gttggcacgt tcagacgtgt cagcggcttt ccagaactcg gacggtttat 3900
   gcgctgccca cgccggcata ttgccggact ccttgtgctc aaggtcggag tctttttcac 3960
   gggcatactt tccctcacgc gcaatataat cggcatgagg agaggcactg ccttttccgc 4020
   cggtttttac gctgagatga taggatgcca tcgtgtttta tcccgctgaa gggcgcacgt 4080
   ttctgaacga agtgaagaaa gtctaagtgc gccctgataa ataaaagagt tatcagggat 4140
   tgtagtggga tttgacctcc tctgccatca tgagcgtaat cattccgtta gcattcagga 4200
   ggtaaacagc atgaataaaa gcgaaaaaac aggaacaatg ggcagcagaa agagtgcagt 4260
   atattcgcgg cttaaagtcg ccgaatgagc aacagaaact tatgctgata ctgacggata 4320
   aagcagataa aacagcacag gatatcaaaa cgctgtccct gctgatgaag gctgaacagg 4380
   cagcagagaa agcgcaggaa gccagagcga aagtcatgaa cctgatacag gcagaaaagc 4440
   gagccgaagc cagagccgcc cgtaaagccc gtgaccatgc tctgtaccag tctgccggat 4500
   tgcttatcct ggcgggtctg gttgacagta agacgggtaa gcctgttgat gataccgctg 4560
   ccttactggg tgcattagcc agtctgaatg acctgtcacg ggataatccg aagtggtcag 4620
   actggaaaat cagagggcag gaactgctga acagcaaaaa gtcagatagc accacatagc 4680
   agacccgcca taaaacgccc tgagaagccc gtgacgggct tttcttgtat tatgggtagt 4740
   ttccttgcat gaatccataa aaggcgcctg tagtgccatt tacccccatt cactgccaga 4800
   gccgtgagcg cagcgaactg aatgtcacga aaaagacagc gactcaggtg cctgatggtc 4860
   ggagacaaaa ggaatattca gcgatttgcc cgagcttgcg agggtgctac ttaagccttt 4920
   agggttttaa ggtctgtttt gtagaggagc aaacagcgtt tgcgacatcc ttttgtaata 4980
   ctgcggaact gactaaagta gtgagttata cacagggctg ggatctattc tttttatctt 5040
   tttttattct ttctttattc tataaattat aaccacttga atataaacaa aaaaaacaca 5100
   caaaggtcta gcggaattta cagagggtct agcagaattt acaagttttc cagcaaaggt 5160
   ctagcagaat ttacagatac ccacaactca aaggaaaagg actagtaatt atcattgact 5220
   agcccatctc aattggtata gtgattaaaa tcacctagac caattgagat gtatgtctga 5280
   attagttgtt ttcaaagcaa atgaactagc gattagtcgc tatgacttaa cggagcatga 5340
   aaccaagcta attttatgct gtgtggcact actcaacccc acgattgaaa accctacaag 5400
   gaaagaacgg acggtatcgt tcacttataa ccaatacgct cagatgatga acatcagtag 5460
   ggaaaatgct tatggtgtat tagctaaagc aaccagagag ctgatgacga gaactgtgga 5520
   aatcaggaat cctttggtta aaggctttga gattttccag tggacaaact atgccaagtt 5580
   ctcaagcgaa aaattagaat tagtttttag tgaagagata ttgccttatc ttttccagtt 5640
   aaaaaaattc ataaaatata atctggaaca tgttaagtct tttgaaaaca aatactctat 5700
   gaggatttat gagtggttat taaaagaact aacacaaaag aaaactcaca aggcaaatat 5760
   agagattagc cttgatgaat ttaagttcat gttaatgctt gaaaataact accatgagtt 5820
   taaaaggctt aaccaatggg ttttgaaacc aataagtaaa gatttaaaca cttacagcaa 5880
   tatgaaattg gtggttgata agcgaggccg cccgactgat acgttgattt tccaagttga 5940
   actagataga caaatggatc tcgtaaccga acttgagaac aaccagataa aaatgaatgg 6000
   tgacaaaata ccaacaacca ttacatcaga ttcctaccta cgtaacggac taagaaaaac 6060
   actacacgat gctttaactg caaaaattca gctcaccagt tttgaggcaa aatttttgag 6120
   tgacatgcaa agtaagcatg atctcaatgg ttcgttctca tggctcacgc aaaaacaacg 6180
   aaccacacta gagaacatac tggctaaata cggaaggatc tgaggttctt atggctcttg 6240
   tatctatcag tgaagcatca agactaacaa acaaaagtag aacaactgtt caccgttaga 6300
   tatcaaaggg aaaactgtcc atatgcacag atgaaaacgg tgtaaaaaag atagatacat 6360
   cagagctttt acgagttttt ggtgcattta aagctgttca ccatgaacag atcgacaatg 6420
   taacagatga acagcatgta acacctaata gaacaggtga aaccagtaaa acaaagcaac 6480
   tagaacatga aattgaacac ctgagacaac ttgttacagc tcaacagtca cacatagaca 6540
   gcctgaaaca ggcgatgctg cttatcgaat caaagctgcc gacaacacgg gagccagtga 6600
   cgcctcccgt ggggaaaaaa tcatggcaat tctggaagaa atagcgcttt cagccggcaa 6660
   acctgaagcc ggatctgcga ttctgataac aaactagcaa caccagaaca gcccgtttgc 6720
   gggcagcaaa acccgtactt ttggacgttc cggcggtttt ttgtggcgag tggtgttcgg 6780
   gcggtgcgcg caagatccat tatgttaaac gggcgagttt acatctcaaa accgcccgct 6840
   taacaccatc agaaatcctc agcgcgattt taagcaccaa cccccccccg taacacccaa 6900
   atccatactg aaagtggctt tgttgaataa atcgaacttt tgctgagttg aaggatcaga 6960
   tcacgcatcc tcccgacaac acagaccatt ccgtggcaaa gcaaaagttc agaatcacca 7020
   actggtccac ctacaacaaa gctctcatca accgtggctc cctcactttc tggctggatg 7080
   atgaggcgat tcaggcctgg tatgagtcgg caacaccttc atcacgagga aggccccagc 7140
   gctattctga tctcgccatc accaccgttc tggtgattaa acgcgtattc cggctgaccc 7200
   tgcgggctgc gcagggtttt attgattcca tttttgccct gatgaacgtt ccgttgcgct 7260
   gcccggatta caccagtgtc agtaagcggg caaagtcggt taatgtcagt ttcaaaacgt 7320
   ccacccgggg tgaaatcgca cacctggtga ttgattccac cgggctgaag gtctttggtg 7380
   aaggcgaatg gaaagtcaga aagcacggca aagagcgccg tcgtatctgg cgaaagttgc 7440
   atcttgctgt tgacagcaac acacatgaag ttgtctgtgc agacctgtcg ctgaataacg 7500
   tcacggactc agaagccttc ccgggcctta tccggcagac tcacagaaaa atcagggcag 7560
   ccgcggcaga cggggcttac gatacccggc tctgtcacga tgaactgcgc cgcaaaaaaa 7620
   tcagcgcgct tattcctccc cgaaaaggtg cgggttactg gcccggtgaa tatgcagacc 7680
   gtaaccgtgc agtggctaat cagcgaatga ccgggagtaa tgcgcggtgg aaatggacaa 7740
   cagattacaa ccgtcgctcg atagcggaaa cggcgatgta ccgggtaaaa cagctgttcg 7800
   ggggttcact gacgctgcgt gactacgatg gtcaggttgc ggaggctatg gccctggtac 7860
   gagcgctgaa caaaatgacg aaagcaggta tgcctgaaag cgtgcgtatt gcctgaaaac 7920
   acaacccgct acgggggaga cttacccgaa atctgattta ttcaacaaag ccgggtgtgg 7980
   tgaactacaa agcagacccg ttgaggttat cagttcgatg cacaatcagc agcgcataaa 8040
   atatgcacaa gaacaggagc acccttcgca ttaagctgtg gtggtaacaa gtagtgccgg 8100
   gctaccatca gcgagcatga tgcgctccca cagcattcgc cttggcagta tggaagttcc 8160
   tcgctccagt tcgggccggt atccaccgca cctggtgttt aaacggattt aaattgcagg 8220
   ggcagtgagc gcaacgcaat taatgtaagt tagctcactc attaggcacc ccaggcttga 8280
   cactttatgc ttccggctcg tataatgtgt ggaattgtga gcggataaca ataacaattt 8340
   aaatcaggat ctaggaacca aggagagtgg catatgacgg atacggttac ggacagcggt 8400
   cgcgagcaga gactgacgga aagagttgaa cagctgtatg cgaatgaccc gcagtttcgt 8460
   gcagccgctc cgagccctga ggttaccgag gcagcgcacc gtgccggtct gcgtctggca 8520
   gaggttgtgg acatttacct gtccggttat gcggaccgtc cggcgctggg ccaacgtgcg 8580
   tgcgaagtgg cacgcgatcc ggcgacgggt cgtgctgcca ccagcctgtt gagcggtttt 8640
   gagactatca cctaccgcga actgggtgat cgtgtcgccg ccttggcggc tgcgtggcgt 8700
   tccggcctgc cgggtggttt gcgtccgggt gatttcgtcg gtgttctggg ctttacgtcc 8760
   atcgattatg tcgtgcacta cctggcttgc atccgtctgg gtgcggtgtt tgtgccgctg 8820
   caaacgagca gcaccgccgc acaactggcg ccgattgtgg cggagacggc gcctcgcatc 8880
   ctggcggtat ctgtggagtc cctggcgacc gcagttgacg tcgttctgga agcgccgagc 8940
   gttcagcgtc tggtggtctt tgattacacc tctgatgacg atgagcagcg cggtcgttac 9000
   gatgatgcac gcgctcgtct gcgcgacgca ggtcacggtg cggagatggt ggcgttggct 9060
   gctgagttgg caagcggccg tgaacgtcct gcgccggaag cgcacgtccc aggtcagggt 9120
   gaaaacccgc tggcaactct gatctatacc agcggtagca ccggcacccc aaagggtgca 9180
   atgtacaccg cggacatgat gacgcgtatc tggcaacgtc ctcactcccc gagcgtcgac 9240
   atcggtcgtg tcattccggc gatccacctg cagtatatgc cgctgtccca cgtctacggt 9300
   ctggagtggc tgatcgcgac cctgagctcc ggtggcattg gttatttcgc agccaaaagc 9360
   gacatgagca ccctgttcga cgacattggt ctggtccgtc cgacggcgct gaacctggtt 9420
   ccgcgcgtgt gtgatatgtt tttccgtcgc taccgtaaag agctggacca acgtgcaggt 9480
   gacggtctga ccgcagagca gcgcgatgaa gccgttaaag ccgaactgcg ccaggatttc 9540
   attggtggcc gtgtcattag cgcgatgtgc ggtagcgcac cgttgagcaa gcagatgcat 9600
   gcgttcatgg agagcctgct ggatgtgacc gttgcggatg gctatggcgc caccgaaacg 9660
   ggtggcggca ttatgcgttc gggtcgcatc cgtcgtccgc cggtcactga ttacaagctg 9720
   gtggacgtgc cggaactggg ttacctgacg accgacaagc cgtacccgcg tggcgagttg 9780
   cacctgaagg cgagcaacgt tatcccgggt tactttaagc atccggaact gagcgcgcag 9840
   atttttgatg atgaaggttt ctacaaaacg ggtgacatta tggcggaact gggccctgac 9900
   catctgatgt acctggaccg tagcaacaac gtgatcaagc tgagccaggg cgagtttgtg 9960
   gcggtgagcc agctggaagc cacgttttcg accagcccgt acattcgtca gattttcctg 10020
   tatggcagct ctgaacaacc gtttctgctg gcggttatcg tcccgaatgt tgatgcagtc 10080
   ggtggtggcg atgcgcgtgc gttgatcgcc gagagcctgc agcagattgc ggcggacagc 10140
   tacctgcacc cgtatgaggt gccacgcgac ttcctgctgg agccgcaacg ttttacccgc 10200
   gataacggct tgctgtctgg tgttggtaaa ctgctgcgtc cggcactgaa agcgcgttac 10260
   ggcgaacgtt tggacgcaat gtatgatgag attgaggcat ctcacggtaa tcaactggac 10320
   gaattgcgta gcgcgagccg cgagttgccg acgattgaca ccgttcgccg tgccgctgcc 10380
   gcgaccttgg gcctgccggc tgatgccgcc ctgcgtggcg acgcgaagtt tattgagctg 10440
   ggtggcgatt ccctgagcgc attcagcttt gcgaccctgc tgtcggaaat ctttcacatc 10500
   gacgtgccgg tgcaaactat cgttagcccg accgccacct tggcgaccat cgcgaattat 10560
   gttgacggtg aacgcactag cgaatcgacg cgtccgacct tcgctagcgt gcatggtcgt 10620
   ggtgcaaccg tggcacgtgc cgctgacttg acgctggcga agttcatcga tgacgatacg 10680
   ctggcagcag cgccgcacct gccggctcca acgggtgcgg tcaacactgt gctgctgacc 10740
   ggcgcaaatg gctacctggg ccgcttcctg tgcttggact ggctggaacg cctggccccg 10800
   accggtggca ccgtaatttg tctggcgcgt ggcgccgatc cgacggcagg tcgccaacgt 10860
   attgaggccg ctatcgacag cggcgatgcg gaattgagcc gtcgtttctg tcagctggcg 10920
   gacaaacacc tgcaagttct ggtgggtgat gtgggtgctg cgaacctggg cctggacacc 10980
   ccgacgtatc aacgcctggc gcgcagcgtt gatttggtcg tccacagcgc agccctggtt 11040
   aatcatgtgc tgccgtatag ccaattgttc ggtccgaacg tggttggtac cgcagagatc 11100
   gtcaagctgg cgatttccga acgcctgaaa ccgattaact acatttccac ggttgcggtc 11160
   accaccctgc cggacggcag cttcatcggc gaggacgcgg acgtccgttc tgcatctccg 11220
   agccgtagcc tggacgagag ctacgctagc ggttatgcga ccagcaaatg ggcaggcgag 11280
   gttctgctgc gcgaggcgca cgacctgtgc ggtgtgccgg ttgcagtttt tcgtagcgac 11340
   atgattctgg cccatagcga cttcgcgggt cagttgaacg tgccggatat gttcacccgc 11400
   ctgatcttga gcctggttgc gacgggtatc gcaccacgta gcttctatca gctggacgcg 11460
   agcggtaatc gtcagcgtgc gcattacgac ggcctgccag cggatttcac tgctgaggca 11520
   atcacgacgc tgggtgcacg tacccgccaa ggctaccata cctataatgt gctgaatacc 11580
   cacgatgatg gcgtctccct ggacaccttt gttgattggc tgattgccga tggccacaaa 11640
   atcgagcgcg tggacgacta cgacgagtgg ctggcgcgtt ttaccgctgc gatgaaatct 11700
   ctgccggata atcagcgcaa aagcagcctg ctgccactga tgagcgcgta cgcgaaaccg 11760
   ggccaaccta cccatggcac cggtatgccg gctgagaagt tccgcgcagc ggtccaaagc 11820
   gctggtattg gtgccactcg tgatgtcccg catgttaccc aggccctgat tgacaagtat 11880
   gttgcggatc tgcaacgttt gggcctgctg cgtacccgcg ctggtgccca tgcaggctaa 11940
   taagtttaaa cgagaaggag ttctatcatg attgaaacca tcttaccagc cggcgtcgaa 12000
   tcagcagagt tacttgagta ccctgaagat ctgaaggcgc atccggctga agagcatctg 12060
   atcgcaaaga gcgtagagaa gcgtcgtcgc gacttcattg gcgcacgtca ttgcgcccgt 12120
   ctggcactgg cggagctggg tgaaccgccg gttgcgattg gcaagggtga acgtggtgcg 12180
   ccgatttggc cgcgtggtgt cgtgggctct ctgacccatt gcgatggcta tcgcgcagcg 12240
   gcggttgctc acaaaatgcg ttttcgcagc atcggcatcg acgccgaacc gcacgcgacc 12300
   ctgccggaag gtgtcctgga ttcggttagc ctgccgcctg agcgtgagtg gctgaaaacc 12360
   accgacagcg cactgcacct ggaccgtttg ctgttttgtg cgaaagaagc aacttacaaa 12420
   gcgtggtggc cgctgacggc acgttggctg ggtttcgaag aagcgcacat taccttcgaa 12480
   atcgaggatg gtagcgccga ctctggtaat ggcacgtttc acagcgaact gctggtgccg 12540
   ggtcagacca atgacggtgg taccccgctg ctgtctttcg acggtcgctg gctgatcgct 12600
   gatggcttca tcctgacggc gattgcatac gcatgataaa ttaacctagg ctgctgccac 12660
   cgctgagcaa taactagcat aaccccttgg ggcctctaaa cgggtcttga ggggtttttt 12720
   gctgagctcg agtgacccca gccgcccctc atgccaaccg agcccattaa gcgcagagtc 12780
   ggccgcaact atctcgggga cgcataaaca cgcagcgatt taccaaggag ttcggctagg 12840
   tcaatccgca attccctttg cctggccgga gatgccgcga gcaaacaatg tgtccggtcg 12900
   ctaccagatg gactttgtgg gcggaggatt gcaccagacg cttcagcgca gcgtcgtacg 12960
   atgaatcgga aggcccggta agcaattgaa gaaccgctga ccacctccca tccgacaatg 13020
   caagggtgcc ccgctcccag cgcgacacgg ttgcctgatc cacacccatt aattcagcta 13080
   ggtgactttg cttcatatga cgaagcaacc gcgccctccg cacctgccgc cccctgtcat 13140
   tcgtcaacat tcttcagcac ctcaatgtcg ttcgtagtga gcctcatttt ttcaagaccg 13200
   ccgatgatga gagccaggcc tcgctcgaat gccgcgtccg gaccgccttc gtagacgatt 13260
   ttcatcgcgc tctgtaggcg cgccggcatc gtagacgctg aggtggtcaa ctgatcttcg 13320
   ccccgctcct cggcgtctgc ctcgctagct tgctgctcaa gaacagcgcc gacggtgaag 13380
   tagctgattg ccatcaacgc ataggtcgcg tcacctgccg aaaagccagc atcgcaaagg 13440
   aagcgaagct gcgcgtcggc tttttccatc tgcggcgcgg ctggccgcgt cccggcatga 13500
   atacgcgcgc catcgcgata agcgagcaac gcccgtcgaa aactgcatgc attgcccttc 13560
   aggaacgaac gccagtcgtc gtcatccctt ggcgtcgaat gcgtgtgatt tatcgtcagc 13620
   atggcttcgg caagtgcgtc gagcaacgca cgcttgttct tgaaatgcca gtagagcgct 13680
   ggctgttgca ccccgaggcg ctcagccagt cggcgcgtcg ttagaccttc catgcccacg 13740
   tcgttaagca gttcgagcgc ggttcggatc acggcctcgc gttggagctt gttcattcgc 13800
   gaa 13803
<210> 26
   <211> 13704
   <212> DNA
   <213> Artificial Sequence
<220>
   <223> vector
<400> 26
   ttctcatgtt tgacagctta tcatcgataa gctttaatgc ggtagtttat cacagttaaa     60
   ttgctaacgc agtcaggcac cgtgtatgaa atctaacaat gcgctcatcg tcatcctcgg    120
   caccgtcacc ctggatgctg taggcatagg cttggttatg ccggtactgc cgggcctctt    180
   gcgggatatc gtccattccg acagcatcgc cagtcactat ggcgtgctgc tagcgctata    240
   tgcgttgatg caatttctat gcgcacccgt tctcggagca ctgtccgacc gctttggccg    300
   ccgcccagtc ctgctcgctt cgctacttgg agccactatc gactacgcga tcatggcgac    360
   cacacccgtc ctgtggatcc tctacgccgg acgcatcgtg gccggcatca ccggcgccac    420
   aggtgcggtt gctggcgcct atatcgccga catcaccgat ggggaagatc gggctcgcca    480
   cttcgggctc atgagcgctt gtttcggcgt gggtatggtg gcaggccccg tggccggggg    540
   actgttgggc gccatctcct tgcatgcacc attccttgcg gcggcggtgc tcaacggcct    600
   caacctacta ctgggctgct tcctaatgca ggagtcgcat aagggagagc gtcgaccgat    660
   gcccttgaga gccttcaacc cagtcagctc cttccggtgg gcgcggggca tgactatcgt    720
   cgccgcactt atgactgtct tctttatcat gcaactcgta ggacaggtgc cggcagcgct    780
   ctgggtcatt ttcggcgagg accgctttcg ctggagcgcg acgatgatcg gcctgtcgct    840
   tgcggtattc ggaatcttgc acgccctcgc tcaagccttc gtcactggtc ccgccaccaa    900
   acgtttcggc gagaagcagg ccattatcgc cggcatggcg gccgacgcgc tgggctacgt     960
   cttgctggcg ttcgcgacgc gaggctggat ggccttcccc attatgattc ttctcgcttc    1020
   cggcggcatc gggatgcccg cgttgcaggc catgctgtcc aggcaggtag atgacgacca    1080
   tcagggacag cttcaaggat cgctcgcggc tcttaccagc ctaacttcga tcactggacc    1140
   gctgatcgtc acggcgattt atgccgcctc ggcgagcaca tggaacgggt tggcatggat    1200
   tgtaggcgcc gccctatacc ttgtctgcct ccccgcgttg cgtcgcggtg catggagccg    1260
   ggccacctcg acctgaatgg aagccggcgg cacctcgcta acggattcac cactccaaga    1320
   attggagcca atcaattctt gcggagaact gtgaatgcgc aaaccaaccc ttggcagaac    1380
   atatccatcg cgtccgccat ctccagcagc cgcacgcggc gcatctcggg cagcgttggg    1440
   tcctggccac gggtgcgcat gatcgtgctc ctgtcgttga ggacccggct aggctggcgg    1500
   ggttgcctta ctggttagca gaatgaatca ccgatacgcg agcgaacgtg aagcgactgc    1560
   tgctgcaaaa cgtctgcgac ctgagcaaca acatgaatgg tcttcggttt ccgtgtttcg    1620
   taaagtctgg aaacgcggaa gtcccctacg tgctgctgaa gttgcccgca acagagagtg    1680
   gaaccaaccg gtgataccac gatactatga ctgagagtca acgccatgag cggcctcatt    1740
   tcttattctg agttacaaca gtccgcaccg ctgccggtag ctacttgact atccggctgc    1800
   actagccctg cgtcagatgg ctctgatcca aggcaaactg ccaaaatatc tgctggcacc    1860
   ggaagtcagc gccctgcacc attatgttcc ggatctgcat cgcaggatgc tgctggctac    1920
   cctgtggaac acctacatct gtattaacga agcgctggca ttgaccctga gtgatttttc    1980
   tctggtgccg ccctatccct ttgtgcagct tgccacgctc aaaggggttt gaggtccaac    2040
   cgtacgaaaa cgtacggtaa gaggaaaatt atcgtctgaa aaatcgatta gtagacaaga    2100
   aagtccgtta agtgccaatt ttcgattaaa aagacaccgt tttgatggcg ttttccaatg    2160
   tacattatgt ttcgatatat cagacagtta cttcactaac gtacgttttc gttctattgg    2220
   ccttcagacc ccatatcctt aatgtccttt atttgctggg gttatcagat ccccccgaca    2280
   cgtttaatta atgctttctc cgccggagat cgacgcacag gcttctgtgt ctatgatgtt    2340
   atttcttaat aatcatccag gtattctctt tatcaccata cgtagtgcga gtgtccacct    2400
   taacgcaggg ctttccgtca cagcgcgata tgtcagccag cggggctttc ttttgccaga    2460
   ccgcttccat cctctgcatt tcagcaatct ggctataccc gtcattcata aaccacgtaa    2520
   atgccgtcac gcaggaagcc aggacgaaga atatcgtcag tacaagataa atcgcggatt    2580
   tccacgtata gcgtgacatc tcacgacgca tttcatggat catcgctttc gccgtatcgg    2640
   cagcctgatt cagcgcttct gtcgccggtt tctgctgtgc taatccggct tgtttcagtt    2700
   ctttctcaac ctgagtgagc gcggaactca ccgatttcct gacggtgtca gtcatattac    2760
   cggacgcgct gtccagctca cgaatgaccc tgctcagcgt ttcactttgc tgctgtaatt 2820
   gtgatgaggc ggcctgaaac tgttctgtca gagaagtaac acgcttttcc agcgcctgat 2880
   gatgcccgat aagggcggca atttgtttaa tttcgtcgct catacaaaat cctgcctatc 2940
   gtgagaatga ccagccttta tccggcttct gtcgtatctg ttcggcgagt cgctgtcgtt 3000
   ctttctcctg ctgacgctgt ttttccgcca gacgttcgcg ctctctctgc ctttccatct 3060
   cctgatgtat cccctggaac tccgccatcg catcgttaac aagggactga agatcgattt 3120
   cttcctgtat atccttcatg gcatcactga ccagtgcgtt cagcttgtca ggctcttttt 3180
   caaaatcaaa cgttctgccg gaatgggatt cctgctcagg ctctgacttc agctcctgtt 3240
   ttagcgtcag agtatccctc tcgctgaggg cttcccgtaa cgaggtagtc acgtcaatta 3300
   cgctgtcacg ttcatcacgg gactgctgca cctgcctttc agcctccctg cgctcaagaa 3360
   tggcctgtag ctgctcagta tcgaatcgct gaacctgacc cgcgcccaga tgccgctcag 3420
   gctcacggtc aatgccctgc gccttcaggg aacgggaatc aacccggtca gcgtgctgat 3480
   accgttcaag gtgcttattc tggaggtcag cccagcgtct ccctctgggc aacaaggtat 3540
   tctttgcgtt cggtcggtgt ttccccgaaa cgtgcctttt ttgcgccacc gcgtccggct 3600
   ctttggtgtt agcccgttta aaatactgct cagggtcacg gtgaataccg tcattaatgc 3660
   gttcagagaa catgatatgg gcgtggggct gctcgccacc ggctatcgct gctttcggat 3720
   tatggatagc gaactgatag gcatggcggt cgccaatttc ctgttggaca aaatcgcgga 3780
   caagctcaag acgttgttcg ggttttaact cacgcggcag ggcaatctcg atttcacggt 3840
   aggtacagcc gttggcacgt tcagacgtgt cagcggcttt ccagaactcg gacggtttat 3900
   gcgctgccca cgccggcata ttgccggact ccttgtgctc aaggtcggag tctttttcac 3960
   gggcatactt tccctcacgc gcaatataat cggcatgagg agaggcactg ccttttccgc 4020
   cggtttttac gctgagatga taggatgcca tcgtgtttta tcccgctgaa gggcgcacgt 4080
   ttctgaacga agtgaagaaa gtctaagtgc gccctgataa ataaaagagt tatcagggat 4140
   tgtagtggga tttgacctcc tctgccatca tgagcgtaat cattccgtta gcattcagga 4200
   ggtaaacagc atgaataaaa gcgaaaaaac aggaacaatg ggcagcagaa agagtgcagt 4260
   atattcgcgg cttaaagtcg ccgaatgagc aacagaaact tatgctgata ctgacggata 4320
   aagcagataa aacagcacag gatatcaaaa cgctgtccct gctgatgaag gctgaacagg 4380
   cagcagagaa agcgcaggaa gccagagcga aagtcatgaa cctgatacag gcagaaaagc 4440
   gagccgaagc cagagccgcc cgtaaagccc gtgaccatgc tctgtaccag tctgccggat 4500
   tgcttatcct ggcgggtctg gttgacagta agacgggtaa gcctgttgat gataccgctg 4560
   ccttactggg tgcattagcc agtctgaatg acctgtcacg ggataatccg aagtggtcag 4620
   actggaaaat cagagggcag gaactgctga acagcaaaaa gtcagatagc accacatagc 4680
   agacccgcca taaaacgccc tgagaagccc gtgacgggct tttcttgtat tatgggtagt 4740
   ttccttgcat gaatccataa aaggcgcctg tagtgccatt tacccccatt cactgccaga 4800
   gccgtgagcg cagcgaactg aatgtcacga aaaagacagc gactcaggtg cctgatggtc 4860
   ggagacaaaa ggaatattca gcgatttgcc cgagcttgcg agggtgctac ttaagccttt 4920
   agggttttaa ggtctgtttt gtagaggagc aaacagcgtt tgcgacatcc ttttgtaata 4980
   ctgcggaact gactaaagta gtgagttata cacagggctg ggatctattc tttttatctt 5040
   tttttattct ttctttattc tataaattat aaccacttga atataaacaa aaaaaacaca 5100
   caaaggtcta gcggaattta cagagggtct agcagaattt acaagttttc cagcaaaggt 5160
   ctagcagaat ttacagatac ccacaactca aaggaaaagg actagtaatt atcattgact 5220
   agcccatctc aattggtata gtgattaaaa tcacctagac caattgagat gtatgtctga 5280
   attagttgtt ttcaaagcaa atgaactagc gattagtcgc tatgacttaa cggagcatga 5340
   aaccaagcta attttatgct gtgtggcact actcaacccc acgattgaaa accctacaag 5400
   gaaagaacgg acggtatcgt tcacttataa ccaatacgct cagatgatga acatcagtag 5460
   ggaaaatgct tatggtgtat tagctaaagc aaccagagag ctgatgacga gaactgtgga 5520
   aatcaggaat cctttggtta aaggctttga gattttccag tggacaaact atgccaagtt 5580
   ctcaagcgaa aaattagaat tagtttttag tgaagagata ttgccttatc ttttccagtt 5640
   aaaaaaattc ataaaatata atctggaaca tgttaagtct tttgaaaaca aatactctat 5700
   gaggatttat gagtggttat taaaagaact aacacaaaag aaaactcaca aggcaaatat 5760
   agagattagc cttgatgaat ttaagttcat gttaatgctt gaaaataact accatgagtt 5820
   taaaaggctt aaccaatggg ttttgaaacc aataagtaaa gatttaaaca cttacagcaa 5880
   tatgaaattg gtggttgata agcgaggccg cccgactgat acgttgattt tccaagttga 5940
   actagataga caaatggatc tcgtaaccga acttgagaac aaccagataa aaatgaatgg 6000
   tgacaaaata ccaacaacca ttacatcaga ttcctaccta cgtaacggac taagaaaaac 6060
   actacacgat gctttaactg caaaaattca gctcaccagt tttgaggcaa aatttttgag 6120
   tgacatgcaa agtaagcatg atctcaatgg ttcgttctca tggctcacgc aaaaacaacg 6180
   aaccacacta gagaacatac tggctaaata cggaaggatc tgaggttctt atggctcttg 6240
   tatctatcag tgaagcatca agactaacaa acaaaagtag aacaactgtt caccgttaga 6300
   tatcaaaggg aaaactgtcc atatgcacag atgaaaacgg tgtaaaaaag atagatacat 6360
   cagagctttt acgagttttt ggtgcattta aagctgttca ccatgaacag atcgacaatg 6420
   taacagatga acagcatgta acacctaata gaacaggtga aaccagtaaa acaaagcaac 6480
   tagaacatga aattgaacac ctgagacaac ttgttacagc tcaacagtca cacatagaca 6540
   gcctgaaaca ggcgatgctg cttatcgaat caaagctgcc gacaacacgg gagccagtga 6600
   cgcctcccgt ggggaaaaaa tcatggcaat tctggaagaa atagcgcttt cagccggcaa 6660
   acctgaagcc ggatctgcga ttctgataac aaactagcaa caccagaaca gcccgtttgc 6720
   gggcagcaaa acccgtactt ttggacgttc cggcggtttt ttgtggcgag tggtgttcgg 6780
   gcggtgcgcg caagatccat tatgttaaac gggcgagttt acatctcaaa accgcccgct 6840
   taacaccatc agaaatcctc agcgcgattt taagcaccaa cccccccccg taacacccaa 6900
   atccatactg aaagtggctt tgttgaataa atcgaacttt tgctgagttg aaggatcaga 6960
   tcacgcatcc tcccgacaac acagaccatt ccgtggcaaa gcaaaagttc agaatcacca 7020
   actggtccac ctacaacaaa gctctcatca accgtggctc cctcactttc tggctggatg 7080
   atgaggcgat tcaggcctgg tatgagtcgg caacaccttc atcacgagga aggccccagc 7140
   gctattctga tctcgccatc accaccgttc tggtgattaa acgcgtattc cggctgaccc 7200
   tgcgggctgc gcagggtttt attgattcca tttttgccct gatgaacgtt ccgttgcgct 7260
   gcccggatta caccagtgtc agtaagcggg caaagtcggt taatgtcagt ttcaaaacgt 7320
   ccacccgggg tgaaatcgca cacctggtga ttgattccac cgggctgaag gtctttggtg 7380
   aaggcgaatg gaaagtcaga aagcacggca aagagcgccg tcgtatctgg cgaaagttgc 7440
   atcttgctgt tgacagcaac acacatgaag ttgtctgtgc agacctgtcg ctgaataacg 7500
   tcacggactc agaagccttc ccgggcctta tccggcagac tcacagaaaa atcagggcag 7560
   ccgcggcaga cggggcttac gatacccggc tctgtcacga tgaactgcgc cgcaaaaaaa 7620
   tcagcgcgct tattcctccc cgaaaaggtg cgggttactg gcccggtgaa tatgcagacc 7680
   gtaaccgtgc agtggctaat cagcgaatga ccgggagtaa tgcgcggtgg aaatggacaa 7740
   cagattacaa ccgtcgctcg atagcggaaa cggcgatgta ccgggtaaaa cagctgttcg 7800
   ggggttcact gacgctgcgt gactacgatg gtcaggttgc ggaggctatg gccctggtac 7860
   gagcgctgaa caaaatgacg aaagcaggta tgcctgaaag cgtgcgtatt gcctgaaaac 7920
   acaacccgct acgggggaga cttacccgaa atctgattta ttcaacaaag ccgggtgtgg 7980
   tgaactacaa agcagacccg ttgaggttat cagttcgatg cacaatcagc agcgcataaa 8040
   atatgcacaa gaacaggagc acccttcgca ttaagctgtg gtggtaacaa gtagtgccgg 8100
   gctaccatca gcgagcatga tgcgctccca cagcattcgc cttggcagta tggaagttcc 8160
   tcgctccagt tcgggccggt atccaccgca cctggtgttt aaacggattt aaattgcagg 8220
   ggcagtgagc gcaacgcaat taatgtaagt tagctcactc attaggcacc ccaggcttga 8280
   cactttatgc ttccggctcg tataatgtgt ggaattgtga gcggataaca ataacaattt 8340
   aaatcaggat ctaggaacca aggagagtgg catatggaga gcactagagc aacccgcctg 8400
   agacagagaa tcgcagccct gtacgcagat gacgcgcagg tccgtgacgc acgtccagac 8460
   gaagctatct ctaccgcgct gcgcgaaccg ggcttgcgtt tgcgcgagct ggtcgcgacc 8520
   gtcgttgatg gttatcgtga ccgtccggct ttggcggcac gtagcgtcca accggctgtc 8580
   gacgcggcga ccggtgcgtg cgtggcgcgt ctgttgccgg agtacaccac gatgagctac 8640
   ggtgagctgg gcctgcgcct gcgtgccgtt gcggcagcgt ggcagcacga cgatgagacg 8700
   cgcttgcgtc caggcgaatt tgttgcgacg ttgggtttta cgtccccgga ttacgcagtg 8760
   gtggacctgg cgtgcgtgtg ggcgggtgcg gttgcagtac cgttgcaagc gagcgccagc 8820
   gtgacccagc tgacggcaat tctggcagaa accgcaccag cgatcctggc caccggtctg 8880
   gataccctgc cgcatgccgt ggattgtgtc ctggcgggtg caacgccgcg tgcgctgcac 8940
   gtgttcgact tcgatccggc aattgatgcg cagcgcaccg tttacgaagc agcatgcgca 9000
   cgcttggcgg gcaccggcgt ccgcgttcgc acgttggcgg aagttgagga ccgtggtcgt 9060
   gccctgccac cggcggttat tgatgatggt ccgggtgacg accgcctggc gttgctgatc 9120
   tatacgagcg gtagcaccgg tacgccgaag ggcgcaatgt atactgagcg cttggttgct 9180
   ctgatgtggc tgggccagcc gcaagttgca gcgctgacgg tcaattatct gccgctgagc 9240
   cacgtcgcag gccgtctggc gctgttcggc ctgctggcgc gtggcggcac cgcgtatttc 9300
   accgcccgtg cagacatgtc caccctgttt gaggatctgg ctctggcgcg tccgaccgag 9360
   ctgtttgtcg taccgcgtgt gtgcgaaatg gttctgcaac gtttccaaac tgagcgtctg 9420
   cgtcgccaag cggacgatga tcgcgtgaag gcagatctgc gcctggagct gttcggtgac 9480
   cgcctgttga gcgttgtttg tggtagcgca ccactggctc ctgaactgaa agcgttcatg 9540
   gagagcgtgc tggacctgac cttgcacgac ggttatggct ccaccgaagc aggtggtagc 9600
   gtcgtcatcg acaccaccgt gcgtcgtccg ccggttctgg actatcgtct ggcggacgtg 9660
   ccggagctgg gttactttcg tacggataaa ccgcatccgc gtggcgagct gctgctgaaa 9720
   acgaccacca tgatcccggg ctactaccgt cgcccggagc tgaatgctca aatctttgat 9780
   gaggacggtt tttaccgcac cggcgacgtg gttgcggagc tggcaccgga tcgcctggtg 9840
   tacgttgacc gtcgcaataa cgttctgaag ctggcgcagg gtgaattcgt gacgattgca 9900
   cgtttggaag cgattttcgc gaacagcccg ttggtccgcc aaatctttgt ttacggtaac 9960
   agcgaacgtg cctatctgct ggccgtgatt gttccgtctc gtcaagcaat ggctggcgac 10020
   ccggcgaccc tgaaaacccg tatcgcggag agcctgcagc tgattggtcg cgacgcggag 10080
   ctggaagcgt acgagatccc tcgcgatttc ctgatcgaga cggagccgtt caccacggaa 10140
   tccggcctgc tgtccggtat tggtaaaatc ctgcgtccgg cggtcgaagc acgctatcgc 10200
   gatcgtctgg agcagctgta cgcggacctg gcagcagcgc agcaagacga gttggcagcc 10260
   ctgcgtcgcg aggcaggtca gcgtccggtc ctggagactg tgacccgtgc cgcagcggcg 10320
   attctgggcg gtaccgctag cgacctgtcc cctgcggccc actttaccga tttgggtggt 10380
   gatagcctgg cggcgttggc gctgtcgaat ctgctgcgcg agattttcgc cgtagaggtt 10440
   ccggtcggtg tgattaccgg tccggcgacc gatctgcgtg gcctggccgc acatatcgcg 10500
   gcagaacgtg aaaaccgtac ggaaacgccg ctgttcgatc gtgtccatcc ggatcagatt 10560
   ttgatccgtg cgaccgacct ggcgctggaa aagtttttcg atgcggaaga gctggcggca 10620
   gccgcgaccg cagcaccgcc ggtagccgag ccgcgtgtgg ttttgctgac gggtgcgaac 10680
   ggttacctgg gtcgcttcct gtgcctggag tggctggagc gtctggaccg tgtcgacggc 10740
   cgtctgattt gtctggttcg tggcgcggac gaagcagcgg cactggctcg tctggaagcg 10800
   gcgttcgaca gcggcgatcc tgagttggtg cgtcgcttta aagaactggc ccaacgtcgt 10860
   ctgaccgtgg tggcgggtga tatcggcgag cctggcctgg gtctggcaac cgccacctgg 10920
   cgccgtctgg ccgctgaggt tgaacatatc gttcaccctg ctgccctggt gaaccatgtc 10980
   ctgccgtatc gccagctgtt tggcccgaac gttgcgggca cggcggagat cctgcgtctg 11040
   gccctgacgg agcgccgcaa accgatcgat ttcttgagca cggttgccgt cgctgcgcag 11100
   atcccggcag accgcttcgc cgaagatggt gacattcgtg tgatcagccc tactcgcact 11160
   gtcgatcgtg gctatgcaaa tggttacggc aatagcaagt gggcggcaga agttctgctg 11220
   cgtgcggcgc atgaccgctt cgatctgccg gtggcggtgt tccgtagcga tatgattctg 11280
   gcccatggca gctttgccgg tcaattgaat attccggatg tctttacccg tctgttgctg 11340
   tctctgttgg tgaccggcat tgccccagca tcttttcacg ccgctacggt cactggtgaa 11400
   cgtccgcgtg ctcactatga tggtctgccg gctgatttca cggctgccgc gatcaccgcc 11460
   ctgggtgccc gtacggctgg ctttcacacg tacgacgttc tgaacccgca cgatgacggt 11520
   atcagcctgg acaccttcgt ggactggctg attgaagcgg gtcacccgat cgaacgtatt 11580
   ccggagcaca gcgaatgggt cacccgcttt gagacggccc tgcacgcgct gccggagcgt 11640
   cagcgtaagc attcgctgct gccgctgctg cacgcgtatc gtcgtccggt tccggccctg 11700
   cgtggtagcg cgttgccagc tgcagagttt cgtgcagcgg tgcgtgctgc tggtattacc 11760
   gcggacggtg acattccgca cctgacccgc gctctgattg aaaagtacgt cgccgacctg 11820
   cgcttgcatg gtctgctgta ataagtttaa acgagaagga gttctatcat gattgaaacc 11880
   atcttaccag ccggcgtcga atcagcagag ttacttgagt accctgaaga tctgaaggcg 11940
   catccggctg aagagcatct gatcgcaaag agcgtagaga agcgtcgtcg cgacttcatt 12000
   ggcgcacgtc attgcgcccg tctggcactg gcggagctgg gtgaaccgcc ggttgcgatt 12060
   ggcaagggtg aacgtggtgc gccgatttgg ccgcgtggtg tcgtgggctc tctgacccat 12120
   tgcgatggct atcgcgcagc ggcggttgct cacaaaatgc gttttcgcag catcggcatc 12180
   gacgccgaac cgcacgcgac cctgccggaa ggtgtcctgg attcggttag cctgccgcct 12240
   gagcgtgagt ggctgaaaac caccgacagc gcactgcacc tggaccgttt gctgttttgt 12300
   gcgaaagaag caacttacaa agcgtggtgg ccgctgacgg cacgttggct gggtttcgaa 12360
   gaagcgcaca ttaccttcga aatcgaggat ggtagcgccg actctggtaa tggcacgttt 12420
   cacagcgaac tgctggtgcc gggtcagacc aatgacggtg gtaccccgct gctgtctttc 12480
   gacggtcgct ggctgatcgc tgatggcttc atcctgacgg cgattgcata cgcatgataa 12540
   attaacctag gctgctgcca ccgctgagca ataactagca taaccccttg gggcctctaa 12600
   acgggtcttg aggggttttt tgctgagctc gagtgacccc agccgcccct catgccaacc 12660
   gagcccatta agcgcagagt cggccgcaac tatctcgggg acgcataaac acgcagcgat 12720
   ttaccaagga gttcggctag gtcaatccgc aattcccttt gcctggccgg agatgccgcg 12780
   agcaaacaat gtgtccggtc gctaccagat ggactttgtg ggcggaggat tgcaccagac 12840
   gcttcagcgc agcgtcgtac gatgaatcgg aaggcccggt aagcaattga agaaccgctg 12900
   accacctccc atccgacaat gcaagggtgc cccgctccca gcgcgacacg gttgcctgat 12960
   ccacacccat taattcagct aggtgacttt gcttcatatg acgaagcaac cgcgccctcc 13020
   gcacctgccg ccccctgtca ttcgtcaaca ttcttcagca cctcaatgtc gttcgtagtg 13080
   agcctcattt tttcaagacc gccgatgatg agagccaggc ctcgctcgaa tgccgcgtcc 13140
   ggaccgcctt cgtagacgat tttcatcgcg ctctgtaggc gcgccggcat cgtagacgct 13200
   gaggtggtca actgatcttc gccccgctcc tcggcgtctg cctcgctagc ttgctgctca 13260
   agaacagcgc cgacggtgaa gtagctgatt gccatcaacg cataggtcgc gtcacctgcc 13320
   gaaaagccag catcgcaaag gaagcgaagc tgcgcgtcgg ctttttccat ctgcggcgcg 13380
   gctggccgcg tcccggcatg aatacgcgcg ccatcgcgat aagcgagcaa cgcccgtcga 13440
   aaactgcatg cattgccctt caggaacgaa cgccagtcgt cgtcatccct tggcgtcgaa 13500
   tgcgtgtgat ttatcgtcag catggcttcg gcaagtgcgt cgagcaacgc acgcttgttc 13560
   ttgaaatgcc agtagagcgc tggctgttgc accccgaggc gctcagccag tcggcgcgtc 13620
   gttagacctt ccatgcccac gtcgttaagc agttcgagcg cggttcggat cacggcctcg 13680
   cgttggagct tgttcattcg cgaa 13704
<210> 27
   <211> 13761
   <212> DNA
   <213> Artificial Sequence
<220>
   <223> vector
<400> 27
   ttctcatgtt tgacagctta tcatcgataa gctttaatgc ggtagtttat cacagttaaa 60
   ttgctaacgc agtcaggcac cgtgtatgaa atctaacaat gcgctcatcg tcatcctcgg      120
   caccgtcacc ctggatgctg taggcatagg cttggttatg ccggtactgc cgggcctctt      180
   gcgggatatc gtccattccg acagcatcgc cagtcactat ggcgtgctgc tagcgctata      240
   tgcgttgatg caatttctat gcgcacccgt tctcggagca ctgtccgacc gctttggccg      300
   ccgcccagtc ctgctcgctt cgctacttgg agccactatc gactacgcga tcatggcgac      360
   cacacccgtc ctgtggatcc tctacgccgg acgcatcgtg gccggcatca ccggcgccac      420
   aggtgcggtt gctggcgcct atatcgccga catcaccgat ggggaagatc gggctcgcca      480
   cttcgggctc atgagcgctt gtttcggcgt gggtatggtg gcaggccccg tggccggggg      540
   actgttgggc gccatctcct tgcatgcacc attccttgcg gcggcggtgc tcaacggcct      600
   caacctacta ctgggctgct tcctaatgca ggagtcgcat aagggagagc gtcgaccgat      660
   gcccttgaga gccttcaacc cagtcagctc cttccggtgg gcgcggggca tgactatcgt      720
   cgccgcactt atgactgtct tctttatcat gcaactcgta ggacaggtgc cggcagcgct      780
   ctgggtcatt ttcggcgagg accgctttcg ctggagcgcg acgatgatcg gcctgtcgct      840
   tgcggtattc ggaatcttgc acgccctcgc tcaagccttc gtcactggtc ccgccaccaa      900
   acgtttcggc gagaagcagg ccattatcgc cggcatggcg gccgacgcgc tgggctacgt      960
   cttgctggcg ttcgcgacgc gaggctggat ggccttcccc attatgattc ttctcgcttc     1020
   cggcggcatc gggatgcccg cgttgcaggc catgctgtcc aggcaggtag atgacgacca     1080
   tcagggacag cttcaaggat cgctcgcggc tcttaccagc ctaacttcga tcactggacc     1140
   gctgatcgtc acggcgattt atgccgcctc ggcgagcaca tggaacgggt tggcatggat     1200
   tgtaggcgcc gccctatacc ttgtctgcct ccccgcgttg cgtcgcggtg catggagccg     1260
   ggccacctcg acctgaatgg aagccggcgg cacctcgcta acggattcac cactccaaga     1320
   attggagcca atcaattctt gcggagaact gtgaatgcgc aaaccaaccc ttggcagaac     1380
   atatccatcg cgtccgccat ctccagcagc cgcacgcggc gcatctcggg cagcgttggg     1440
   tcctggccac gggtgcgcat gatcgtgctc ctgtcgttga ggacccggct aggctggcgg     1500
   ggttgcctta ctggttagca gaatgaatca ccgatacgcg agcgaacgtg aagcgactgc     1560
   tgctgcaaaa cgtctgcgac ctgagcaaca acatgaatgg tcttcggttt ccgtgtttcg     1620
   taaagtctgg aaacgcggaa gtcccctacg tgctgctgaa gttgcccgca acagagagtg     1680
   gaaccaaccg gtgataccac gatactatga ctgagagtca acgccatgag cggcctcatt     1740
   tcttattctg agttacaaca gtccgcaccg ctgccggtag ctacttgact atccggctgc     1800
   actagccctg cgtcagatgg ctctgatcca aggcaaactg ccaaaatatc tgctggcacc     1860
   ggaagtcagc gccctgcacc attatgttcc ggatctgcat cgcaggatgc tgctggctac     1920
   cctgtggaac acctacatct gtattaacga agcgctggca ttgaccctga gtgatttttc     1980
   tctggtgccg ccctatccct ttgtgcagct tgccacgctc aaaggggttt gaggtccaac 2040
   cgtacgaaaa cgtacggtaa gaggaaaatt atcgtctgaa aaatcgatta gtagacaaga 2100
   aagtccgtta agtgccaatt ttcgattaaa aagacaccgt tttgatggcg ttttccaatg 2160
   tacattatgt ttcgatatat cagacagtta cttcactaac gtacgttttc gttctattgg 2220
   ccttcagacc ccatatcctt aatgtccttt atttgctggg gttatcagat ccccccgaca 2280
   cgtttaatta atgctttctc cgccggagat cgacgcacag gcttctgtgt ctatgatgtt 2340
   atttcttaat aatcatccag gtattctctt tatcaccata cgtagtgcga gtgtccacct 2400
   taacgcaggg ctttccgtca cagcgcgata tgtcagccag cggggctttc ttttgccaga 2460
   ccgcttccat cctctgcatt tcagcaatct ggctataccc gtcattcata aaccacgtaa 2520
   atgccgtcac gcaggaagcc aggacgaaga atatcgtcag tacaagataa atcgcggatt 2580
   tccacgtata gcgtgacatc tcacgacgca tttcatggat catcgctttc gccgtatcgg 2640
   cagcctgatt cagcgcttct gtcgccggtt tctgctgtgc taatccggct tgtttcagtt 2700
   ctttctcaac ctgagtgagc gcggaactca ccgatttcct gacggtgtca gtcatattac 2760
   cggacgcgct gtccagctca cgaatgaccc tgctcagcgt ttcactttgc tgctgtaatt 2820
   gtgatgaggc ggcctgaaac tgttctgtca gagaagtaac acgcttttcc agcgcctgat 2880
   gatgcccgat aagggcggca atttgtttaa tttcgtcgct catacaaaat cctgcctatc 2940
   gtgagaatga ccagccttta tccggcttct gtcgtatctg ttcggcgagt cgctgtcgtt 3000
   ctttctcctg ctgacgctgt ttttccgcca gacgttcgcg ctctctctgc ctttccatct 3060
   cctgatgtat cccctggaac tccgccatcg catcgttaac aagggactga agatcgattt 3120
   cttcctgtat atccttcatg gcatcactga ccagtgcgtt cagcttgtca ggctcttttt 3180
   caaaatcaaa cgttctgccg gaatgggatt cctgctcagg ctctgacttc agctcctgtt 3240
   ttagcgtcag agtatccctc tcgctgaggg cttcccgtaa cgaggtagtc acgtcaatta 3300
   cgctgtcacg ttcatcacgg gactgctgca cctgcctttc agcctccctg cgctcaagaa 3360
   tggcctgtag ctgctcagta tcgaatcgct gaacctgacc cgcgcccaga tgccgctcag 3420
   gctcacggtc aatgccctgc gccttcaggg aacgggaatc aacccggtca gcgtgctgat 3480
   accgttcaag gtgcttattc tggaggtcag cccagcgtct ccctctgggc aacaaggtat 3540
   tctttgcgtt cggtcggtgt ttccccgaaa cgtgcctttt ttgcgccacc gcgtccggct 3600
   ctttggtgtt agcccgttta aaatactgct cagggtcacg gtgaataccg tcattaatgc 3660
   gttcagagaa catgatatgg gcgtggggct gctcgccacc ggctatcgct gctttcggat 3720
   tatggatagc gaactgatag gcatggcggt cgccaatttc ctgttggaca aaatcgcgga 3780
   caagctcaag acgttgttcg ggttttaact cacgcggcag ggcaatctcg atttcacggt 3840
   aggtacagcc gttggcacgt tcagacgtgt cagcggcttt ccagaactcg gacggtttat 3900
   gcgctgccca cgccggcata ttgccggact ccttgtgctc aaggtcggag tctttttcac 3960
   gggcatactt tccctcacgc gcaatataat cggcatgagg agaggcactg ccttttccgc 4020
   cggtttttac gctgagatga taggatgcca tcgtgtttta tcccgctgaa gggcgcacgt 4080
   ttctgaacga agtgaagaaa gtctaagtgc gccctgataa ataaaagagt tatcagggat 4140
   tgtagtggga tttgacctcc tctgccatca tgagcgtaat cattccgtta gcattcagga 4200
   ggtaaacagc atgaataaaa gcgaaaaaac aggaacaatg ggcagcagaa agagtgcagt 4260
   atattcgcgg cttaaagtcg ccgaatgagc aacagaaact tatgctgata ctgacggata 4320
   aagcagataa aacagcacag gatatcaaaa cgctgtccct gctgatgaag gctgaacagg 4380
   cagcagagaa agcgcaggaa gccagagcga aagtcatgaa cctgatacag gcagaaaagc 4440
   gagccgaagc cagagccgcc cgtaaagccc gtgaccatgc tctgtaccag tctgccggat 4500
   tgcttatcct ggcgggtctg gttgacagta agacgggtaa gcctgttgat gataccgctg 4560
   ccttactggg tgcattagcc agtctgaatg acctgtcacg ggataatccg aagtggtcag 4620
   actggaaaat cagagggcag gaactgctga acagcaaaaa gtcagatagc accacatagc 4680
   agacccgcca taaaacgccc tgagaagccc gtgacgggct tttcttgtat tatgggtagt 4740
   ttccttgcat gaatccataa aaggcgcctg tagtgccatt tacccccatt cactgccaga 4800
   gccgtgagcg cagcgaactg aatgtcacga aaaagacagc gactcaggtg cctgatggtc 4860
   ggagacaaaa ggaatattca gcgatttgcc cgagcttgcg agggtgctac ttaagccttt 4920
   agggttttaa ggtctgtttt gtagaggagc aaacagcgtt tgcgacatcc ttttgtaata 4980
   ctgcggaact gactaaagta gtgagttata cacagggctg ggatctattc tttttatctt 5040
   tttttattct ttctttattc tataaattat aaccacttga atataaacaa aaaaaacaca 5100
   caaaggtcta gcggaattta cagagggtct agcagaattt acaagttttc cagcaaaggt 5160
   ctagcagaat ttacagatac ccacaactca aaggaaaagg actagtaatt atcattgact 5220
   agcccatctc aattggtata gtgattaaaa tcacctagac caattgagat gtatgtctga 5280
   attagttgtt ttcaaagcaa atgaactagc gattagtcgc tatgacttaa cggagcatga 5340
   aaccaagcta attttatgct gtgtggcact actcaacccc acgattgaaa accctacaag 5400
   gaaagaacgg acggtatcgt tcacttataa ccaatacgct cagatgatga acatcagtag 5460
   ggaaaatgct tatggtgtat tagctaaagc aaccagagag ctgatgacga gaactgtgga 5520
   aatcaggaat cctttggtta aaggctttga gattttccag tggacaaact atgccaagtt 5580
   ctcaagcgaa aaattagaat tagtttttag tgaagagata ttgccttatc ttttccagtt 5640
   aaaaaaattc ataaaatata atctggaaca tgttaagtct tttgaaaaca aatactctat 5700
   gaggatttat gagtggttat taaaagaact aacacaaaag aaaactcaca aggcaaatat 5760
   agagattagc cttgatgaat ttaagttcat gttaatgctt gaaaataact accatgagtt 5820
   taaaaggctt aaccaatggg ttttgaaacc aataagtaaa gatttaaaca cttacagcaa 5880
   tatgaaattg gtggttgata agcgaggccg cccgactgat acgttgattt tccaagttga 5940
   actagataga caaatggatc tcgtaaccga acttgagaac aaccagataa aaatgaatgg 6000
   tgacaaaata ccaacaacca ttacatcaga ttcctaccta cgtaacggac taagaaaaac 6060
   actacacgat gctttaactg caaaaattca gctcaccagt tttgaggcaa aatttttgag 6120
   tgacatgcaa agtaagcatg atctcaatgg ttcgttctca tggctcacgc aaaaacaacg 6180
   aaccacacta gagaacatac tggctaaata cggaaggatc tgaggttctt atggctcttg 6240
   tatctatcag tgaagcatca agactaacaa acaaaagtag aacaactgtt caccgttaga 6300
   tatcaaaggg aaaactgtcc atatgcacag atgaaaacgg tgtaaaaaag atagatacat 6360
   cagagctttt acgagttttt ggtgcattta aagctgttca ccatgaacag atcgacaatg 6420
   taacagatga acagcatgta acacctaata gaacaggtga aaccagtaaa acaaagcaac 6480
   tagaacatga aattgaacac ctgagacaac ttgttacagc tcaacagtca cacatagaca 6540
   gcctgaaaca ggcgatgctg cttatcgaat caaagctgcc gacaacacgg gagccagtga 6600
   cgcctcccgt ggggaaaaaa tcatggcaat tctggaagaa atagcgcttt cagccggcaa 6660
   acctgaagcc ggatctgcga ttctgataac aaactagcaa caccagaaca gcccgtttgc 6720
   gggcagcaaa acccgtactt ttggacgttc cggcggtttt ttgtggcgag tggtgttcgg 6780
   gcggtgcgcg caagatccat tatgttaaac gggcgagttt acatctcaaa accgcccgct 6840
   taacaccatc agaaatcctc agcgcgattt taagcaccaa cccccccccg taacacccaa 6900
   atccatactg aaagtggctt tgttgaataa atcgaacttt tgctgagttg aaggatcaga 6960
   tcacgcatcc tcccgacaac acagaccatt ccgtggcaaa gcaaaagttc agaatcacca 7020
   actggtccac ctacaacaaa gctctcatca accgtggctc cctcactttc tggctggatg 7080
   atgaggcgat tcaggcctgg tatgagtcgg caacaccttc atcacgagga aggccccagc 7140
   gctattctga tctcgccatc accaccgttc tggtgattaa acgcgtattc cggctgaccc 7200
   tgcgggctgc gcagggtttt attgattcca tttttgccct gatgaacgtt ccgttgcgct 7260
   gcccggatta caccagtgtc agtaagcggg caaagtcggt taatgtcagt ttcaaaacgt 7320
   ccacccgggg tgaaatcgca cacctggtga ttgattccac cgggctgaag gtctttggtg 7380
   aaggcgaatg gaaagtcaga aagcacggca aagagcgccg tcgtatctgg cgaaagttgc 7440
   atcttgctgt tgacagcaac acacatgaag ttgtctgtgc agacctgtcg ctgaataacg 7500
   tcacggactc agaagccttc ccgggcctta tccggcagac tcacagaaaa atcagggcag 7560
   ccgcggcaga cggggcttac gatacccggc tctgtcacga tgaactgcgc cgcaaaaaaa 7620
   tcagcgcgct tattcctccc cgaaaaggtg cgggttactg gcccggtgaa tatgcagacc 7680
   gtaaccgtgc agtggctaat cagcgaatga ccgggagtaa tgcgcggtgg aaatggacaa 7740
   cagattacaa ccgtcgctcg atagcggaaa cggcgatgta ccgggtaaaa cagctgttcg 7800
   ggggttcact gacgctgcgt gactacgatg gtcaggttgc ggaggctatg gccctggtac 7860
   gagcgctgaa caaaatgacg aaagcaggta tgcctgaaag cgtgcgtatt gcctgaaaac 7920
   acaacccgct acgggggaga cttacccgaa atctgattta ttcaacaaag ccgggtgtgg 7980
   tgaactacaa agcagacccg ttgaggttat cagttcgatg cacaatcagc agcgcataaa 8040
   atatgcacaa gaacaggagc acccttcgca ttaagctgtg gtggtaacaa gtagtgccgg 8100
   gctaccatca gcgagcatga tgcgctccca cagcattcgc cttggcagta tggaagttcc 8160
   tcgctccagt tcgggccggt atccaccgca cctggtgttt aaacggattt aaattgcagg 8220
   ggcagtgagc gcaacgcaat taatgtaagt tagctcactc attaggcacc ccaggcttga 8280
   cactttatgc ttccggctcg tataatgtgt ggaattgtga gcggataaca ataacaattt 8340
   aaatcaggat ctaggaacca aggagagtgg catatgtccc caattacccg tgaagaacgc 8400
   ctggagcgcc gtattcaaga cctgtacgcg aatgacccgc agttcgctgc ggcgaagccg 8460
   gtgactgcga ttacggcagc aattgagcgc ccaggtctgc cgctgccgca gattatcgaa 8520
   accgtgatga ccggctacgc agatcgccca gcgttggcgc agcgctccgt cgagttcgtt 8580
   accgacgcgg gtaccggtca cacgactctg cgtctgctgc cgcactttga gacgattagc 8640
   tatggcgagc tgtgggatcg tattagcgct ttggcagacg tgctgagcac ggagcaaacc 8700
   gttaaaccgt cggatcgtgt ctgcctgctg ggtttcaaca gcgtcgatta cgccacgatc 8760
   gacatgacct tggcgcgctt gggtgcggtc gccgtgccgc tgcaaacgtc cgcagcgatc 8820
   acgcaactgc agccgatcgt ggctgagacg cagcctacca tgattgccgc gagcgtggac 8880
   gcattggcag atgcaaccga gctggcactg agcggtcaaa ccgcgacgcg tgtcctggtt 8940
   tttgaccacc accgtcaggt ggacgcgcat cgtgccgcgg tcgagtcggc ccgtgaacgc 9000
   ctggcgggca gcgcagtggt tgagactctg gcggaagcaa ttgcacgtgg cgacgttccg 9060
   cgtggcgcga gcgcgggttc tgcacctggc acggatgtta gcgatgacag cctggcactg 9120
   ctgatctaca cgagcggttc taccggtgct ccgaaaggtg cgatgtatcc gcgtcgcaac 9180
   gttgccactt tctggcgtaa acgtacgtgg tttgaaggcg gttatgagcc gagcattacc 9240
   ctgaatttca tgccgatgag ccacgttatg ggccgtcaga tcctgtatgg taccctgtgt 9300
   aatggcggta cggcgtattt cgttgttaag agcgacctga gcacgctgtt tgaagatctg 9360
   gccctggttc gcccgaccga gctgaccttt gttccgcgcg tctgggatat ggtgttcgac 9420
   gagtttcaga gcgaggtgga tcgtcgtttg gtggatggtg ctgaccgtgt ggcactggaa 9480
   gcgcaggtta aggcagagat ccgcaatgat gtcctgggtg gccgttacac cagcgcgctg 9540
   accggtagcg cgcctatttc cgacgagatg aaagcgtggg ttgaagaact gctggatatg 9600
   cacctggtgg aaggctacgg tagcaccgaa gcgggcatga ttttgatcga tggtgcgatt 9660
   cgtcgcccag ccgttctgga ctacaaactg gttgacgtcc cagacttggg ttacttcctg 9720
   acggatcgtc cgcacccgcg tggtgagctg ctggttaaga cggacagcct gttcccgggc 9780
   tactaccaac gcgctgaagt caccgcagac gtctttgacg cggatggttt ctaccgcacc 9840
   ggtgacatca tggccgaggt tggcccggag caattcgtgt atttggaccg ccgtaataac 9900
   gtcctgaaac tgagccaggg cgaattcgtg accgtttcca agctggaagc ggtctttggc 9960
   gacagcccgc tggttcgcca aatctatatc tacggtaact ccgcgcgtgc gtatctgctg 10020
   gcggtgattg tgccgaccca agaggccctg gacgccgtcc cggtggaaga gctgaaagca 10080
   cgtctgggcg acagcttgca ggaagtggcc aaggctgcgg gcctgcaatc ctacgagatc 10140
   ccgcgtgact tcatcattga gacgaccccg tggactctgc agaatggtct gctgacgggt 10200
   attcgcaagc tggcgcgtcc gcagctgaag aagcactatg gtgaattgct ggagcagatc 10260
   tacacggacc tggcgcacgg ccaagcggac gagctgcgta gcctgcgtca gagcggtgca 10320
   gacgcgcctg ttctggtcac cgtctgccgc gctgctgcag cgttgctggg tggcagcgca 10380
   agcgacgtgc agccggatgc gcattttacc gacctgggcg gtgatagcct gtctgcgctg 10440
   agctttacca atttgttgca cgagattttt gatatcgatg ttccggtggg cgtcatcgtg 10500
   tccccggcaa acgacttgca ggcgctggca gactacgtag aggccgcacg caaaccgggt 10560
   agcagccgtc cgaccttcgc ttctgtgcat ggtgccagca atgagcaggt caccgaagtc 10620
   catgcgggtg atctgagcct ggacaaattc atcgatgccg ctaccctggc agaagctccg 10680
   cgtttgccgg cagcaaacac ccaagtgcgc acggtgctgt tgaccggtgc aaccggtttt 10740
   ctgggtcgtt atttggcgct ggagtggctg gagcgtatgg acctggtgga tggcaaattg 10800
   atctgcctgg tgcgcgccaa gtccgacact gaagcgcgtg cgcgtctgga gaaaaccttc 10860
   gacagcggtg cgccggaact gttggcgcat tatcgcgccc tggctggcga tcacctggaa 10920
   gttctggcgg gtgacaaggg cgaggcggac ctgggcctgg atcgtcaaac gtggcagcgt 10980
   ctggccgaca ccgttgatct gattgttgat ccggcagcgc tggtcaacca tgtcctgccg 11040
   tacagccagc tgtttggtcc gaatgcgctg ggcaccgcgg agctgttgcg tctggccctg 11100
   accagcaaga tcaaaccgta cagctatacg agcaccattg gtgttgctga ccagatcccg 11160
   cctagcgcgt tcaccgaaga tgcagacatc cgtgttatca gcgccacccg tgccgtcgac 11220
   gatagctatg ccaacggcta cagcaactct aagtgggcag gtgaagtact gctgcgtgaa 11280
   gctcatgtgc tgtgtggtct gccggttgcc gtttttcgct gtgacatgat cttggctgat 11340
   accacctggg cgggtcaact gaacgtgccg gatatgttca cgcgcatgat tctgagcttg 11400
   gcggcgacgg gcattgcacc gggttcgttc tatgagctgg ctgcggacgg tgcccgtcaa 11460
   cgtgcgcatt acgatggcct gccggtcgag tttattgcag aagccatctc taccctgggt 11520
   gcgcagagcc aggatggttt ccacacgtat cacgtgatga acccgtacga tgatggtatc 11580
   ggtctggatg agttcgtgga ttggctgaac gagtctggct gccctattca acgcattgcg 11640
   gattatggtg attggttgca gcgctttgag actgcgctgc gtgccctgcc ggatcgtcaa 11700
   cgtcatagct cgctgctgcc gctgctgcac aattaccgcc aaccggaacg tccggtgcgt 11760
   ggttccattg ctccgaccga ccgttttcgt gcggcggttc aagaagccaa gatcggccca 11820
   gacaaagata tcccgcacgt tggtgcgccg attatcgtca aatacgtcag cgacctgcgc 11880
   ctgctgggct tgctgtaata agtttaaacg agaaggagtt ctatcatgat tgaaaccatc 11940
   ttaccagccg gcgtcgaatc agcagagtta cttgagtacc ctgaagatct gaaggcgcat 12000
   ccggctgaag agcatctgat cgcaaagagc gtagagaagc gtcgtcgcga cttcattggc 12060
   gcacgtcatt gcgcccgtct ggcactggcg gagctgggtg aaccgccggt tgcgattggc 12120
   aagggtgaac gtggtgcgcc gatttggccg cgtggtgtcg tgggctctct gacccattgc 12180
   gatggctatc gcgcagcggc ggttgctcac aaaatgcgtt ttcgcagcat cggcatcgac 12240
   gccgaaccgc acgcgaccct gccggaaggt gtcctggatt cggttagcct gccgcctgag 12300
   cgtgagtggc tgaaaaccac cgacagcgca ctgcacctgg accgtttgct gttttgtgcg 12360
   aaagaagcaa cttacaaagc gtggtggccg ctgacggcac gttggctggg tttcgaagaa 12420
   gcgcacatta ccttcgaaat cgaggatggt agcgccgact ctggtaatgg cacgtttcac 12480
   agcgaactgc tggtgccggg tcagaccaat gacggtggta ccccgctgct gtctttcgac 12540
   ggtcgctggc tgatcgctga tggcttcatc ctgacggcga ttgcatacgc atgataaatt 12600
   aacctaggct gctgccaccg ctgagcaata actagcataa ccccttgggg cctctaaacg 12660
   ggtcttgagg ggttttttgc tgagctcgag tgaccccagc cgcccctcat gccaaccgag 12720
   cccattaagc gcagagtcgg ccgcaactat ctcggggacg cataaacacg cagcgattta 12780
   ccaaggagtt cggctaggtc aatccgcaat tccctttgcc tggccggaga tgccgcgagc 12840
   aaacaatgtg tccggtcgct accagatgga ctttgtgggc ggaggattgc accagacgct 12900
   tcagcgcagc gtcgtacgat gaatcggaag gcccggtaag caattgaaga accgctgacc 12960
   acctcccatc cgacaatgca agggtgcccc gctcccagcg cgacacggtt gcctgatcca 13020
   cacccattaa ttcagctagg tgactttgct tcatatgacg aagcaaccgc gccctccgca 13080
   cctgccgccc cctgtcattc gtcaacattc ttcagcacct caatgtcgtt cgtagtgagc 13140
   ctcatttttt caagaccgcc gatgatgaga gccaggcctc gctcgaatgc cgcgtccgga 13200
   ccgccttcgt agacgatttt catcgcgctc tgtaggcgcg ccggcatcgt agacgctgag 13260
   gtggtcaact gatcttcgcc ccgctcctcg gcgtctgcct cgctagcttg ctgctcaaga 13320
   acagcgccga cggtgaagta gctgattgcc atcaacgcat aggtcgcgtc acctgccgaa    13380
   aagccagcat cgcaaaggaa gcgaagctgc gcgtcggctt tttccatctg cggcgcggct    13440
   ggccgcgtcc cggcatgaat acgcgcgcca tcgcgataag cgagcaacgc ccgtcgaaaa    13500
   ctgcatgcat tgcccttcag gaacgaacgc cagtcgtcgt catcccttgg cgtcgaatgc    13560
   gtgtgattta tcgtcagcat ggcttcggca agtgcgtcga gcaacgcacg cttgttcttg    13620
   aaatgccagt agagcgctgg ctgttgcacc ccgaggcgct cagccagtcg gcgcgtcgtt    13680
   agaccttcca tgcccacgtc gttaagcagt tcgagcgcgg ttcggatcac ggcctcgcgt    13740
   tggagcttgt tcattcgcga a                                              13761
<210> 28
   <211> 13719
   <212> DNA
   <213> Artificial Sequence
<220>
   <223> vector
<400> 28
   ttctcatgtt tgacagctta tcatcgataa gctttaatgc ggtagtttat cacagttaaa      60
   ttgctaacgc agtcaggcac cgtgtatgaa atctaacaat gcgctcatcg tcatcctcgg     120
   caccgtcacc ctggatgctg taggcatagg cttggttatg ccggtactgc cgggcctctt     180
   gcgggatatc gtccattccg acagcatcgc cagtcactat ggcgtgctgc tagcgctata     240
   tgcgttgatg caatttctat gcgcacccgt tctcggagca ctgtccgacc gctttggccg     300
   ccgcccagtc ctgctcgctt cgctacttgg agccactatc gactacgcga tcatggcgac     360
   cacacccgtc ctgtggatcc tctacgccgg acgcatcgtg gccggcatca ccggcgccac     420
   aggtgcggtt gctggcgcct atatcgccga catcaccgat ggggaagatc gggctcgcca     480
   cttcgggctc atgagcgctt gtttcggcgt gggtatggtg gcaggccccg tggccggggg     540
   actgttgggc gccatctcct tgcatgcacc attccttgcg gcggcggtgc tcaacggcct     600
   caacctacta ctgggctgct tcctaatgca ggagtcgcat aagggagagc gtcgaccgat     660
   gcccttgaga gccttcaacc cagtcagctc cttccggtgg gcgcggggca tgactatcgt     720
   cgccgcactt atgactgtct tctttatcat gcaactcgta ggacaggtgc cggcagcgct     780
   ctgggtcatt ttcggcgagg accgctttcg ctggagcgcg acgatgatcg gcctgtcgct     840
   tgcggtattc ggaatcttgc acgccctcgc tcaagccttc gtcactggtc ccgccaccaa     900
   acgtttcggc gagaagcagg ccattatcgc cggcatggcg gccgacgcgc tgggctacgt     960
   cttgctggcg ttcgcgacgc gaggctggat ggccttcccc attatgattc ttctcgcttc    1020
   cggcggcatc gggatgcccg cgttgcaggc catgctgtcc aggcaggtag atgacgacca    1080
   tcagggacag cttcaaggat cgctcgcggc tcttaccagc ctaacttcga tcactggacc 1140
   gctgatcgtc acggcgattt atgccgcctc ggcgagcaca tggaacgggt tggcatggat 1200
   tgtaggcgcc gccctatacc ttgtctgcct ccccgcgttg cgtcgcggtg catggagccg 1260
   ggccacctcg acctgaatgg aagccggcgg cacctcgcta acggattcac cactccaaga 1320
   attggagcca atcaattctt gcggagaact gtgaatgcgc aaaccaaccc ttggcagaac 1380
   atatccatcg cgtccgccat ctccagcagc cgcacgcggc gcatctcggg cagcgttggg 1440
   tcctggccac gggtgcgcat gatcgtgctc ctgtcgttga ggacccggct aggctggcgg 1500
   ggttgcctta ctggttagca gaatgaatca ccgatacgcg agcgaacgtg aagcgactgc 1560
   tgctgcaaaa cgtctgcgac ctgagcaaca acatgaatgg tcttcggttt ccgtgtttcg 1620
   taaagtctgg aaacgcggaa gtcccctacg tgctgctgaa gttgcccgca acagagagtg 1680
   gaaccaaccg gtgataccac gatactatga ctgagagtca acgccatgag cggcctcatt 1740
   tcttattctg agttacaaca gtccgcaccg ctgccggtag ctacttgact atccggctgc 1800
   actagccctg cgtcagatgg ctctgatcca aggcaaactg ccaaaatatc tgctggcacc 1860
   ggaagtcagc gccctgcacc attatgttcc ggatctgcat cgcaggatgc tgctggctac 1920
   cctgtggaac acctacatct gtattaacga agcgctggca ttgaccctga gtgatttttc 1980
   tctggtgccg ccctatccct ttgtgcagct tgccacgctc aaaggggttt gaggtccaac 2040
   cgtacgaaaa cgtacggtaa gaggaaaatt atcgtctgaa aaatcgatta gtagacaaga 2100
   aagtccgtta agtgccaatt ttcgattaaa aagacaccgt tttgatggcg ttttccaatg 2160
   tacattatgt ttcgatatat cagacagtta cttcactaac gtacgttttc gttctattgg 2220
   ccttcagacc ccatatcctt aatgtccttt atttgctggg gttatcagat ccccccgaca 2280
   cgtttaatta atgctttctc cgccggagat cgacgcacag gcttctgtgt ctatgatgtt 2340
   atttcttaat aatcatccag gtattctctt tatcaccata cgtagtgcga gtgtccacct 2400
   taacgcaggg ctttccgtca cagcgcgata tgtcagccag cggggctttc ttttgccaga 2460
   ccgcttccat cctctgcatt tcagcaatct ggctataccc gtcattcata aaccacgtaa 2520
   atgccgtcac gcaggaagcc aggacgaaga atatcgtcag tacaagataa atcgcggatt 2580
   tccacgtata gcgtgacatc tcacgacgca tttcatggat catcgctttc gccgtatcgg 2640
   cagcctgatt cagcgcttct gtcgccggtt tctgctgtgc taatccggct tgtttcagtt 2700
   ctttctcaac ctgagtgagc gcggaactca ccgatttcct gacggtgtca gtcatattac 2760
   cggacgcgct gtccagctca cgaatgaccc tgctcagcgt ttcactttgc tgctgtaatt 2820
   gtgatgaggc ggcctgaaac tgttctgtca gagaagtaac acgcttttcc agcgcctgat 2880
   gatgcccgat aagggcggca atttgtttaa tttcgtcgct catacaaaat cctgcctatc 2940
   gtgagaatga ccagccttta tccggcttct gtcgtatctg ttcggcgagt cgctgtcgtt 3000
   ctttctcctg ctgacgctgt ttttccgcca gacgttcgcg ctctctctgc ctttccatct 3060
   cctgatgtat cccctggaac tccgccatcg catcgttaac aagggactga agatcgattt 3120
   cttcctgtat atccttcatg gcatcactga ccagtgcgtt cagcttgtca ggctcttttt 3180
   caaaatcaaa cgttctgccg gaatgggatt cctgctcagg ctctgacttc agctcctgtt 3240
   ttagcgtcag agtatccctc tcgctgaggg cttcccgtaa cgaggtagtc acgtcaatta 3300
   cgctgtcacg ttcatcacgg gactgctgca cctgcctttc agcctccctg cgctcaagaa 3360
   tggcctgtag ctgctcagta tcgaatcgct gaacctgacc cgcgcccaga tgccgctcag 3420
   gctcacggtc aatgccctgc gccttcaggg aacgggaatc aacccggtca gcgtgctgat 3480
   accgttcaag gtgcttattc tggaggtcag cccagcgtct ccctctgggc aacaaggtat 3540
   tctttgcgtt cggtcggtgt ttccccgaaa cgtgcctttt ttgcgccacc gcgtccggct 3600
   ctttggtgtt agcccgttta aaatactgct cagggtcacg gtgaataccg tcattaatgc 3660
   gttcagagaa catgatatgg gcgtggggct gctcgccacc ggctatcgct gctttcggat 3720
   tatggatagc gaactgatag gcatggcggt cgccaatttc ctgttggaca aaatcgcgga 3780
   caagctcaag acgttgttcg ggttttaact cacgcggcag ggcaatctcg atttcacggt 3840
   aggtacagcc gttggcacgt tcagacgtgt cagcggcttt ccagaactcg gacggtttat 3900
   gcgctgccca cgccggcata ttgccggact ccttgtgctc aaggtcggag tctttttcac 3960
   gggcatactt tccctcacgc gcaatataat cggcatgagg agaggcactg ccttttccgc 4020
   cggtttttac gctgagatga taggatgcca tcgtgtttta tcccgctgaa gggcgcacgt 4080
   ttctgaacga agtgaagaaa gtctaagtgc gccctgataa ataaaagagt tatcagggat 4140
   tgtagtggga tttgacctcc tctgccatca tgagcgtaat cattccgtta gcattcagga 4200
   ggtaaacagc atgaataaaa gcgaaaaaac aggaacaatg ggcagcagaa agagtgcagt 4260
   atattcgcgg cttaaagtcg ccgaatgagc aacagaaact tatgctgata ctgacggata 4320
   aagcagataa aacagcacag gatatcaaaa cgctgtccct gctgatgaag gctgaacagg 4380
   cagcagagaa agcgcaggaa gccagagcga aagtcatgaa cctgatacag gcagaaaagc 4440
   gagccgaagc cagagccgcc cgtaaagccc gtgaccatgc tctgtaccag tctgccggat 4500
   tgcttatcct ggcgggtctg gttgacagta agacgggtaa gcctgttgat gataccgctg 4560
   ccttactggg tgcattagcc agtctgaatg acctgtcacg ggataatccg aagtggtcag 4620
   actggaaaat cagagggcag gaactgctga acagcaaaaa gtcagatagc accacatagc 4680
   agacccgcca taaaacgccc tgagaagccc gtgacgggct tttcttgtat tatgggtagt 4740
   ttccttgcat gaatccataa aaggcgcctg tagtgccatt tacccccatt cactgccaga 4800
   gccgtgagcg cagcgaactg aatgtcacga aaaagacagc gactcaggtg cctgatggtc 4860
   ggagacaaaa ggaatattca gcgatttgcc cgagcttgcg agggtgctac ttaagccttt 4920
   agggttttaa ggtctgtttt gtagaggagc aaacagcgtt tgcgacatcc ttttgtaata 4980
   ctgcggaact gactaaagta gtgagttata cacagggctg ggatctattc tttttatctt 5040
   tttttattct ttctttattc tataaattat aaccacttga atataaacaa aaaaaacaca 5100
   caaaggtcta gcggaattta cagagggtct agcagaattt acaagttttc cagcaaaggt 5160
   ctagcagaat ttacagatac ccacaactca aaggaaaagg actagtaatt atcattgact 5220
   agcccatctc aattggtata gtgattaaaa tcacctagac caattgagat gtatgtctga 5280
   attagttgtt ttcaaagcaa atgaactagc gattagtcgc tatgacttaa cggagcatga 5340
   aaccaagcta attttatgct gtgtggcact actcaacccc acgattgaaa accctacaag 5400
   gaaagaacgg acggtatcgt tcacttataa ccaatacgct cagatgatga acatcagtag 5460
   ggaaaatgct tatggtgtat tagctaaagc aaccagagag ctgatgacga gaactgtgga 5520
   aatcaggaat cctttggtta aaggctttga gattttccag tggacaaact atgccaagtt 5580
   ctcaagcgaa aaattagaat tagtttttag tgaagagata ttgccttatc ttttccagtt 5640
   aaaaaaattc ataaaatata atctggaaca tgttaagtct tttgaaaaca aatactctat 5700
   gaggatttat gagtggttat taaaagaact aacacaaaag aaaactcaca aggcaaatat 5760
   agagattagc cttgatgaat ttaagttcat gttaatgctt gaaaataact accatgagtt 5820
   taaaaggctt aaccaatggg ttttgaaacc aataagtaaa gatttaaaca cttacagcaa 5880
   tatgaaattg gtggttgata agcgaggccg cccgactgat acgttgattt tccaagttga 5940
   actagataga caaatggatc tcgtaaccga acttgagaac aaccagataa aaatgaatgg 6000
   tgacaaaata ccaacaacca ttacatcaga ttcctaccta cgtaacggac taagaaaaac 6060
   actacacgat gctttaactg caaaaattca gctcaccagt tttgaggcaa aatttttgag 6120
   tgacatgcaa agtaagcatg atctcaatgg ttcgttctca tggctcacgc aaaaacaacg 6180
   aaccacacta gagaacatac tggctaaata cggaaggatc tgaggttctt atggctcttg 6240
   tatctatcag tgaagcatca agactaacaa acaaaagtag aacaactgtt caccgttaga 6300
   tatcaaaggg aaaactgtcc atatgcacag atgaaaacgg tgtaaaaaag atagatacat 6360
   cagagctttt acgagttttt ggtgcattta aagctgttca ccatgaacag atcgacaatg 6420
   taacagatga acagcatgta acacctaata gaacaggtga aaccagtaaa acaaagcaac 6480
   tagaacatga aattgaacac ctgagacaac ttgttacagc tcaacagtca cacatagaca 6540
   gcctgaaaca ggcgatgctg cttatcgaat caaagctgcc gacaacacgg gagccagtga 6600
   cgcctcccgt ggggaaaaaa tcatggcaat tctggaagaa atagcgcttt cagccggcaa 6660
   acctgaagcc ggatctgcga ttctgataac aaactagcaa caccagaaca gcccgtttgc 6720
   gggcagcaaa acccgtactt ttggacgttc cggcggtttt ttgtggcgag tggtgttcgg 6780
   gcggtgcgcg caagatccat tatgttaaac gggcgagttt acatctcaaa accgcccgct 6840
   taacaccatc agaaatcctc agcgcgattt taagcaccaa cccccccccg taacacccaa 6900
   atccatactg aaagtggctt tgttgaataa atcgaacttt tgctgagttg aaggatcaga 6960
   tcacgcatcc tcccgacaac acagaccatt ccgtggcaaa gcaaaagttc agaatcacca 7020
   actggtccac ctacaacaaa gctctcatca accgtggctc cctcactttc tggctggatg 7080
   atgaggcgat tcaggcctgg tatgagtcgg caacaccttc atcacgagga aggccccagc 7140
   gctattctga tctcgccatc accaccgttc tggtgattaa acgcgtattc cggctgaccc 7200
   tgcgggctgc gcagggtttt attgattcca tttttgccct gatgaacgtt ccgttgcgct 7260
   gcccggatta caccagtgtc agtaagcggg caaagtcggt taatgtcagt ttcaaaacgt 7320
   ccacccgggg tgaaatcgca cacctggtga ttgattccac cgggctgaag gtctttggtg 7380
   aaggcgaatg gaaagtcaga aagcacggca aagagcgccg tcgtatctgg cgaaagttgc 7440
   atcttgctgt tgacagcaac acacatgaag ttgtctgtgc agacctgtcg ctgaataacg 7500
   tcacggactc agaagccttc ccgggcctta tccggcagac tcacagaaaa atcagggcag 7560
   ccgcggcaga cggggcttac gatacccggc tctgtcacga tgaactgcgc cgcaaaaaaa 7620
   tcagcgcgct tattcctccc cgaaaaggtg cgggttactg gcccggtgaa tatgcagacc 7680
   gtaaccgtgc agtggctaat cagcgaatga ccgggagtaa tgcgcggtgg aaatggacaa 7740
   cagattacaa ccgtcgctcg atagcggaaa cggcgatgta ccgggtaaaa cagctgttcg 7800
   ggggttcact gacgctgcgt gactacgatg gtcaggttgc ggaggctatg gccctggtac 7860
   gagcgctgaa caaaatgacg aaagcaggta tgcctgaaag cgtgcgtatt gcctgaaaac 7920
   acaacccgct acgggggaga cttacccgaa atctgattta ttcaacaaag ccgggtgtgg 7980
   tgaactacaa agcagacccg ttgaggttat cagttcgatg cacaatcagc agcgcataaa 8040
   atatgcacaa gaacaggagc acccttcgca ttaagctgtg gtggtaacaa gtagtgccgg 8100
   gctaccatca gcgagcatga tgcgctccca cagcattcgc cttggcagta tggaagttcc 8160
   tcgctccagt tcgggccggt atccaccgca cctggtgttt aaacggattt aaattgcagg 8220
   ggcagtgagc gcaacgcaat taatgtaagt tagctcactc attaggcacc ccaggcttga 8280
   cactttatgc ttccggctcg tataatgtgt ggaattgtga gcggataaca ataacaattt 8340
   aaatcaggat ctaggaacca aggagagtgg catatgactg taatgcctgc aaaaccacca 8400
   gccgatgtat ccccacttag ccgcgcagca cgtctggtcg ccgagttgag cgcgcacgat 8460
   ccgcagtatc gtgccgcaat gccgctgccg gcagttcgtg aagcggttcg cgaggccgca 8520
   cgcgaccaag ttctgagccg caccgttgca accgtcatgg caggttatgc ggaccgtccg 8580
   gcgctggcac gccgtgccac cgagccggtg accgatccgg ttagcggccg tactagcttg 8640
   cgtcgtctgc cggagtttac gacggtcacg tacggtgaac tgtgggcgcg tgcaggtgcg 8700
   gtgagcgcgg agtgggcagc cgacacccgc ctgccgctga gcccaggcga ctttgttgcc 8760
   atctacggtt tcacgagcgg cgactatgtt actgtcgatt tggcctgtct gcgccatggt 8820
   gcggttagcg tcccgctgca gtcgggtgcg ccggttgccg gcctggcccc gattctggcg 8880
   aaaacgggtc cgaaagtcct ggcggtgtcc ttggaattgc tggaccgtgg tgttgaactg 8940
   gcgttgagcg ccgaggttgc tccgcgtctg gtggtctttg acttccacgc cggtgacgac 9000
   gctcagcgcg aggcctttga agcggcaagc gcacgcctga ccgcggcagg ccatgcggct 9060
   ccgctgccgc tggaagcggt gattgaacgc ggtcgtgcac tgccgccagc gccactgttc 9120
   gttccgggtc cggacgaaga tccggttcgt ctgctgattt acaccagcgg tagcaccggt 9180
   accccgaaag gcgccattca aacggagcgc atgctgcatc gcgcatgggc aggcgcggtg 9240
   ccgattccgg acgacgtcgc gtccatcgtc gtgaattacc tgccgctgtc ccacgtcgct 9300
   ggtcgtagct ctctggttga gacgctgcgt cgtggcggca ttagctattt cacggcacat 9360
   agcgacctgt ctgatctgtt cgaggacatc gccctggcgc gtcctaccgc gctgttgttt 9420
   gttccgcgtg tgtgcgattt gctgttccaa gaatatcagg ctgaattggc acgtcgcgca 9480
   ggtgagtttg cggacgcggg tgcgctggac gcggctgtta aggcagatct gcgtgaacgt 9540
   ttcgttggtg gccgcctgat tcaagcgctg tacggcagcg caccgctgtc cgccgaactg 9600
   cgtgagttta tgcacacctg tctggacctg cctgtgctgg acggttatgg tagcaccgaa 9660
   acgggttccg tgttgttgaa cacccgcgtg caacgtccgc cggtcaccga tatcaagctg 9720
   gtggatgtcc cggaactggg ttacttcgcc accgattccc cgtacccacg cggcgaattg 9780
   gtgctgaaaa gcgcgaccct gacgcctggc tattaccgtc gcccagaggt caccgcagct 9840
   gctttcgacg cggatggctt ttaccgtacg ggcgacatta tggcggaagt tggcccggac 9900
   cagtacgtgt acgttgatcg tcgtaataac gttgtgaagc tggcccaggg tgagttcgtt 9960
   gcgctgtctc gtctggaagg tgtgtatgtg actcacccgc tgattcgcca gatttacgtg 10020
   tacggcaata gcgagcgtgc acacttgctg gctgtgatcg tgccgacgcg cgagaattgt 10080
   acgcacgagg atctggcagc cgcgctgcag caggcagccc gtgagagcga gttgaactct 10140
   tacgagatcc cgcgtgcgtt cctggttgaa accgagccgt tctccctggc caacggtttg 10200
   ctgagcgaca cccgtaagaa tctgccgcca cgtctgaagg cgcgctatgg cgagcgtctg 10260
   gaagcgctgt atgaagagct ggcacgtgaa caagaggatg cggttcgtgt gctgcgcgat 10320
   gaaggtacgg gtcgccctgt ttcggaaacg gtcgagcgtg cagcgcgtgc gctgctgggt 10380
   agcagcgcag cggacgcgcg ttttaccgac ttgggcggtg attctctgtc ggcgttgtcc 10440
   ttcagcaccc tgctggcaga gatcttcggt gtcgaggtgc cggtgggtac tgtcctgagc 10500
   ccggcaaacg acctgcgtgc attggcagcg cacatcgaag ctcgtcgtgc gtctggtgcg 10560
   agccgtccga ccttcgctag cgttcatggc gtgggccgta cggtcgtgcg tgcgggcgac 10620
   ctggcactgg agaagtttct ggatgccggt gcgctggcgg aagcggcgca gctgccggca 10680
   ccgggtcgtg aaacgccgcg tacggtcctg ctgaccggtg cgaacggtta tctgggtcgt 10740
   ttcatgtgcc tggactggct ggaacgtctg gcttcgggcg gtggccgtct ggtgtgcgtg 10800
   gttcgcggta aagacgacgc cgatgcccgt gcacgtctgg atgcggcgtt cgacagcggt 10860
   gatccggagc tgctgcgtcg ctaccgcgaa ctggcagcgg gtcgcctgga tgtcttggct 10920
   ggcgatatcg gtgcggagcg tctgggtctg gcgggtgaga cgtggcgtcg cctggccgag 10980
   gatgtggatc tgatcgccca cccggctgcc ctggtcaacc acgtcttgcc gtacgagcaa 11040
   ctgtttggtc cgaacgttgt aggcaccgct gaactgattc gcctggcact gaccgcgcgt 11100
   gttaaaccgt tcgtgtatgt tagcaccacc gcgattagca ccacgctgga cgagacgagc 11160
   gatattcgtg agagcatccc ggaacgcgcc ctgaccgatg catatgcagc gggctacggt 11220
   accagcaaat gggcgggtga ggtgctgctg cgcgaggcgc acgctcgcct ggcgctgccg 11280
   gttgcggtct ttcgcagcga cttgattctg gcgcacccgc atcacaccgg ccaactgaat 11340
   ccggccgacg tactgacccg tctgctgttt agcatcctga gcactggctt ggcgccgact 11400
   agcttttact ctgaagaggg tcgcgcgcac ttcgacggtc tgccggtgga ttttaccgct 11460
   gaggctatca acaccctggg cgcacagccg attagcgcgc accgtaccta taatgcggta 11520
   aatccgcatg acgacggcgt gagcctggat accttcatca cttggctgga agaggctggt 11580
   cacccgctgc gccgtttgcc gcatacgacc tggagcccgc gtctggaaac cgccttgcgc 11640
   agcctgccgg aacatcaccg tccgcacacg ttgctgcctc tgctgcatgc gtttgcaacc 11700
   cctcagccgc cgaccccgac cagcccggtt ccggccaccc attttcatga ggcagtgcgt 11760
   gaggctggca tcggcccaga taatgatatc ccgcacatca cgcaaaactt gattaccaag 11820
   tacgcgacgg atctgcgcca actgggtctg cgctaataag tttaaacgag aaggagttct 11880
   atcatgattg aaaccatctt accagccggc gtcgaatcag cagagttact tgagtaccct 11940
   gaagatctga aggcgcatcc ggctgaagag catctgatcg caaagagcgt agagaagcgt 12000
   cgtcgcgact tcattggcgc acgtcattgc gcccgtctgg cactggcgga gctgggtgaa 12060
   ccgccggttg cgattggcaa gggtgaacgt ggtgcgccga tttggccgcg tggtgtcgtg 12120
   ggctctctga cccattgcga tggctatcgc gcagcggcgg ttgctcacaa aatgcgtttt 12180
   cgcagcatcg gcatcgacgc cgaaccgcac gcgaccctgc cggaaggtgt cctggattcg 12240
   gttagcctgc cgcctgagcg tgagtggctg aaaaccaccg acagcgcact gcacctggac 12300
   cgtttgctgt tttgtgcgaa agaagcaact tacaaagcgt ggtggccgct gacggcacgt 12360
   tggctgggtt tcgaagaagc gcacattacc ttcgaaatcg aggatggtag cgccgactct 12420
   ggtaatggca cgtttcacag cgaactgctg gtgccgggtc agaccaatga cggtggtacc     12480
   ccgctgctgt ctttcgacgg tcgctggctg atcgctgatg gcttcatcct gacggcgatt     12540
   gcatacgcat gataaattaa cctaggctgc tgccaccgct gagcaataac tagcataacc     12600
   ccttggggcc tctaaacggg tcttgagggg ttttttgctg agctcgagtg accccagccg     12660
   cccctcatgc caaccgagcc cattaagcgc agagtcggcc gcaactatct cggggacgca     12720
   taaacacgca gcgatttacc aaggagttcg gctaggtcaa tccgcaattc cctttgcctg     12780
   gccggagatg ccgcgagcaa acaatgtgtc cggtcgctac cagatggact ttgtgggcgg     12840
   aggattgcac cagacgcttc agcgcagcgt cgtacgatga atcggaaggc ccggtaagca     12900
   attgaagaac cgctgaccac ctcccatccg acaatgcaag ggtgccccgc tcccagcgcg     12960
   acacggttgc ctgatccaca cccattaatt cagctaggtg actttgcttc atatgacgaa     13020
   gcaaccgcgc cctccgcacc tgccgccccc tgtcattcgt caacattctt cagcacctca     13080
   atgtcgttcg tagtgagcct cattttttca agaccgccga tgatgagagc caggcctcgc      13140
   tcgaatgccg cgtccggacc gccttcgtag acgattttca tcgcgctctg taggcgcgcc     13200
   ggcatcgtag acgctgaggt ggtcaactga tcttcgcccc gctcctcggc gtctgcctcg     13260
   ctagcttgct gctcaagaac agcgccgacg gtgaagtagc tgattgccat caacgcatag     13320
   gtcgcgtcac ctgccgaaaa gccagcatcg caaaggaagc gaagctgcgc gtcggctttt     13380
   tccatctgcg gcgcggctgg ccgcgtcccg gcatgaatac gcgcgccatc gcgataagcg     13440
   agcaacgccc gtcgaaaact gcatgcattg cccttcagga acgaacgcca gtcgtcgtca     13500
   tcccttggcg tcgaatgcgt gtgatttatc gtcagcatgg cttcggcaag tgcgtcgagc     13560
   aacgcacgct tgttcttgaa atgccagtag agcgctggct gttgcacccc gaggcgctca     13620
   gccagtcggc gcgtcgttag accttccatg cccacgtcgt taagcagttc gagcgcggtt     13680
   cggatcacgg cctcgcgttg gagcttgttc attcgcgaa                             13719
<210> 29
   <211> 13842
   <212> DNA
   <213> Artificial Sequence
<220>
   <223> vector
<400> 29
   ttctcatgtt tgacagctta tcatcgataa gctttaatgc ggtagtttat cacagttaaa        60
   ttgctaacgc agtcaggcac cgtgtatgaa atctaacaat gcgctcatcg tcatcctcgg       120
   caccgtcacc ctggatgctg taggcatagg cttggttatg ccggtactgc cgggcctctt       180
   gcgggatatc gtccattccg acagcatcgc cagtcactat ggcgtgctgc tagcgctata       240
   tgcgttgatg caatttctat gcgcacccgt tctcggagca ctgtccgacc gctttggccg       300
   ccgcccagtc ctgctcgctt cgctacttgg agccactatc gactacgcga tcatggcgac 360
   cacacccgtc ctgtggatcc tctacgccgg acgcatcgtg gccggcatca ccggcgccac 420
   aggtgcggtt gctggcgcct atatcgccga catcaccgat ggggaagatc gggctcgcca 480
   cttcgggctc atgagcgctt gtttcggcgt gggtatggtg gcaggccccg tggccggggg 540
   actgttgggc gccatctcct tgcatgcacc attccttgcg gcggcggtgc tcaacggcct 600
   caacctacta ctgggctgct tcctaatgca ggagtcgcat aagggagagc gtcgaccgat 660
   gcccttgaga gccttcaacc cagtcagctc cttccggtgg gcgcggggca tgactatcgt 720
   cgccgcactt atgactgtct tctttatcat gcaactcgta ggacaggtgc cggcagcgct 780
   ctgggtcatt ttcggcgagg accgctttcg ctggagcgcg acgatgatcg gcctgtcgct 840
   tgcggtattc ggaatcttgc acgccctcgc tcaagccttc gtcactggtc ccgccaccaa 900
   acgtttcggc gagaagcagg ccattatcgc cggcatggcg gccgacgcgc tgggctacgt 960
   cttgctggcg ttcgcgacgc gaggctggat ggccttcccc attatgattc ttctcgcttc 1020
   cggcggcatc gggatgcccg cgttgcaggc catgctgtcc aggcaggtag atgacgacca 1080
   tcagggacag cttcaaggat cgctcgcggc tcttaccagc ctaacttcga tcactggacc 1140
   gctgatcgtc acggcgattt atgccgcctc ggcgagcaca tggaacgggt tggcatggat 1200
   tgtaggcgcc gccctatacc ttgtctgcct ccccgcgttg cgtcgcggtg catggagccg 1260
   ggccacctcg acctgaatgg aagccggcgg cacctcgcta acggattcac cactccaaga 1320
   attggagcca atcaattctt gcggagaact gtgaatgcgc aaaccaaccc ttggcagaac 1380
   atatccatcg cgtccgccat ctccagcagc cgcacgcggc gcatctcggg cagcgttggg 1440
   tcctggccac gggtgcgcat gatcgtgctc ctgtcgttga ggacccggct aggctggcgg 1500
   ggttgcctta ctggttagca gaatgaatca ccgatacgcg agcgaacgtg aagcgactgc 1560
   tgctgcaaaa cgtctgcgac ctgagcaaca acatgaatgg tcttcggttt ccgtgtttcg 1620
   taaagtctgg aaacgcggaa gtcccctacg tgctgctgaa gttgcccgca acagagagtg 1680
   gaaccaaccg gtgataccac gatactatga ctgagagtca acgccatgag cggcctcatt 1740
   tcttattctg agttacaaca gtccgcaccg ctgccggtag ctacttgact atccggctgc 1800
   actagccctg cgtcagatgg ctctgatcca aggcaaactg ccaaaatatc tgctggcacc 1860
   ggaagtcagc gccctgcacc attatgttcc ggatctgcat cgcaggatgc tgctggctac 1920
   cctgtggaac acctacatct gtattaacga agcgctggca ttgaccctga gtgatttttc 1980
   tctggtgccg ccctatccct ttgtgcagct tgccacgctc aaaggggttt gaggtccaac 2040
   cgtacgaaaa cgtacggtaa gaggaaaatt atcgtctgaa aaatcgatta gtagacaaga 2100
   aagtccgtta agtgccaatt ttcgattaaa aagacaccgt tttgatggcg ttttccaatg 2160
   tacattatgt ttcgatatat cagacagtta cttcactaac gtacgttttc gttctattgg 2220
   ccttcagacc ccatatcctt aatgtccttt atttgctggg gttatcagat ccccccgaca 2280
   cgtttaatta atgctttctc cgccggagat cgacgcacag gcttctgtgt ctatgatgtt 2340
   atttcttaat aatcatccag gtattctctt tatcaccata cgtagtgcga gtgtccacct 2400
   taacgcaggg ctttccgtca cagcgcgata tgtcagccag cggggctttc ttttgccaga 2460
   ccgcttccat cctctgcatt tcagcaatct ggctataccc gtcattcata aaccacgtaa 2520
   atgccgtcac gcaggaagcc aggacgaaga atatcgtcag tacaagataa atcgcggatt 2580
   tccacgtata gcgtgacatc tcacgacgca tttcatggat catcgctttc gccgtatcgg 2640
   cagcctgatt cagcgcttct gtcgccggtt tctgctgtgc taatccggct tgtttcagtt 2700
   ctttctcaac ctgagtgagc gcggaactca ccgatttcct gacggtgtca gtcatattac 2760
   cggacgcgct gtccagctca cgaatgaccc tgctcagcgt ttcactttgc tgctgtaatt 2820
   gtgatgaggc ggcctgaaac tgttctgtca gagaagtaac acgcttttcc agcgcctgat 2880
   gatgcccgat aagggcggca atttgtttaa tttcgtcgct catacaaaat cctgcctatc 2940
   gtgagaatga ccagccttta tccggcttct gtcgtatctg ttcggcgagt cgctgtcgtt 3000
   ctttctcctg ctgacgctgt ttttccgcca gacgttcgcg ctctctctgc ctttccatct 3060
   cctgatgtat cccctggaac tccgccatcg catcgttaac aagggactga agatcgattt 3120
   cttcctgtat atccttcatg gcatcactga ccagtgcgtt cagcttgtca ggctcttttt 3180
   caaaatcaaa cgttctgccg gaatgggatt cctgctcagg ctctgacttc agctcctgtt 3240
   ttagcgtcag agtatccctc tcgctgaggg cttcccgtaa cgaggtagtc acgtcaatta 3300
   cgctgtcacg ttcatcacgg gactgctgca cctgcctttc agcctccctg cgctcaagaa 3360
   tggcctgtag ctgctcagta tcgaatcgct gaacctgacc cgcgcccaga tgccgctcag 3420
   gctcacggtc aatgccctgc gccttcaggg aacgggaatc aacccggtca gcgtgctgat 3480
   accgttcaag gtgcttattc tggaggtcag cccagcgtct ccctctgggc aacaaggtat 3540
   tctttgcgtt cggtcggtgt ttccccgaaa cgtgcctttt ttgcgccacc gcgtccggct 3600
   ctttggtgtt agcccgttta aaatactgct cagggtcacg gtgaataccg tcattaatgc 3660
   gttcagagaa catgatatgg gcgtggggct gctcgccacc ggctatcgct gctttcggat 3720
   tatggatagc gaactgatag gcatggcggt cgccaatttc ctgttggaca aaatcgcgga 3780
   caagctcaag acgttgttcg ggttttaact cacgcggcag ggcaatctcg atttcacggt 3840
   aggtacagcc gttggcacgt tcagacgtgt cagcggcttt ccagaactcg gacggtttat 3900
   gcgctgccca cgccggcata ttgccggact ccttgtgctc aaggtcggag tctttttcac 3960
   gggcatactt tccctcacgc gcaatataat cggcatgagg agaggcactg ccttttccgc 4020
   cggtttttac gctgagatga taggatgcca tcgtgtttta tcccgctgaa gggcgcacgt 4080
   ttctgaacga agtgaagaaa gtctaagtgc gccctgataa ataaaagagt tatcagggat 4140
   tgtagtggga tttgacctcc tctgccatca tgagcgtaat cattccgtta gcattcagga 4200
   ggtaaacagc atgaataaaa gcgaaaaaac aggaacaatg ggcagcagaa agagtgcagt 4260
   atattcgcgg cttaaagtcg ccgaatgagc aacagaaact tatgctgata ctgacggata 4320
   aagcagataa aacagcacag gatatcaaaa cgctgtccct gctgatgaag gctgaacagg 4380
   cagcagagaa agcgcaggaa gccagagcga aagtcatgaa cctgatacag gcagaaaagc 4440
   gagccgaagc cagagccgcc cgtaaagccc gtgaccatgc tctgtaccag tctgccggat 4500
   tgcttatcct ggcgggtctg gttgacagta agacgggtaa gcctgttgat gataccgctg 4560
   ccttactggg tgcattagcc agtctgaatg acctgtcacg ggataatccg aagtggtcag 4620
   actggaaaat cagagggcag gaactgctga acagcaaaaa gtcagatagc accacatagc 4680
   agacccgcca taaaacgccc tgagaagccc gtgacgggct tttcttgtat tatgggtagt 4740
   ttccttgcat gaatccataa aaggcgcctg tagtgccatt tacccccatt cactgccaga 4800
   gccgtgagcg cagcgaactg aatgtcacga aaaagacagc gactcaggtg cctgatggtc 4860
   ggagacaaaa ggaatattca gcgatttgcc cgagcttgcg agggtgctac ttaagccttt 4920
   agggttttaa ggtctgtttt gtagaggagc aaacagcgtt tgcgacatcc ttttgtaata 4980
   ctgcggaact gactaaagta gtgagttata cacagggctg ggatctattc tttttatctt 5040
   tttttattct ttctttattc tataaattat aaccacttga atataaacaa aaaaaacaca 5100
   caaaggtcta gcggaattta cagagggtct agcagaattt acaagttttc cagcaaaggt 5160
   ctagcagaat ttacagatac ccacaactca aaggaaaagg actagtaatt atcattgact 5220
   agcccatctc aattggtata gtgattaaaa tcacctagac caattgagat gtatgtctga 5280
   attagttgtt ttcaaagcaa atgaactagc gattagtcgc tatgacttaa cggagcatga 5340
   aaccaagcta attttatgct gtgtggcact actcaacccc acgattgaaa accctacaag 5400
   gaaagaacgg acggtatcgt tcacttataa ccaatacgct cagatgatga acatcagtag 5460
   ggaaaatgct tatggtgtat tagctaaagc aaccagagag ctgatgacga gaactgtgga 5520
   aatcaggaat cctttggtta aaggctttga gattttccag tggacaaact atgccaagtt 5580
   ctcaagcgaa aaattagaat tagtttttag tgaagagata ttgccttatc ttttccagtt 5640
   aaaaaaattc ataaaatata atctggaaca tgttaagtct tttgaaaaca aatactctat 5700
   gaggatttat gagtggttat taaaagaact aacacaaaag aaaactcaca aggcaaatat 5760
   agagattagc cttgatgaat ttaagttcat gttaatgctt gaaaataact accatgagtt 5820
   taaaaggctt aaccaatggg ttttgaaacc aataagtaaa gatttaaaca cttacagcaa 5880
   tatgaaattg gtggttgata agcgaggccg cccgactgat acgttgattt tccaagttga 5940
   actagataga caaatggatc tcgtaaccga acttgagaac aaccagataa aaatgaatgg 6000
   tgacaaaata ccaacaacca ttacatcaga ttcctaccta cgtaacggac taagaaaaac 6060
   actacacgat gctttaactg caaaaattca gctcaccagt tttgaggcaa aatttttgag 6120
   tgacatgcaa agtaagcatg atctcaatgg ttcgttctca tggctcacgc aaaaacaacg 6180
   aaccacacta gagaacatac tggctaaata cggaaggatc tgaggttctt atggctcttg 6240
   tatctatcag tgaagcatca agactaacaa acaaaagtag aacaactgtt caccgttaga 6300
   tatcaaaggg aaaactgtcc atatgcacag atgaaaacgg tgtaaaaaag atagatacat 6360
   cagagctttt acgagttttt ggtgcattta aagctgttca ccatgaacag atcgacaatg 6420
   taacagatga acagcatgta acacctaata gaacaggtga aaccagtaaa acaaagcaac 6480
   tagaacatga aattgaacac ctgagacaac ttgttacagc tcaacagtca cacatagaca 6540
   gcctgaaaca ggcgatgctg cttatcgaat caaagctgcc gacaacacgg gagccagtga 6600
   cgcctcccgt ggggaaaaaa tcatggcaat tctggaagaa atagcgcttt cagccggcaa 6660
   acctgaagcc ggatctgcga ttctgataac aaactagcaa caccagaaca gcccgtttgc 6720
   gggcagcaaa acccgtactt ttggacgttc cggcggtttt ttgtggcgag tggtgttcgg 6780
   gcggtgcgcg caagatccat tatgttaaac gggcgagttt acatctcaaa accgcccgct 6840
   taacaccatc agaaatcctc agcgcgattt taagcaccaa cccccccccg taacacccaa 6900
   atccatactg aaagtggctt tgttgaataa atcgaacttt tgctgagttg aaggatcaga 6960
   tcacgcatcc tcccgacaac acagaccatt ccgtggcaaa gcaaaagttc agaatcacca 7020
   actggtccac ctacaacaaa gctctcatca accgtggctc cctcactttc tggctggatg 7080
   atgaggcgat tcaggcctgg tatgagtcgg caacaccttc atcacgagga aggccccagc 7140
   gctattctga tctcgccatc accaccgttc tggtgattaa acgcgtattc cggctgaccc 7200
   tgcgggctgc gcagggtttt attgattcca tttttgccct gatgaacgtt ccgttgcgct 7260
   gcccggatta caccagtgtc agtaagcggg caaagtcggt taatgtcagt ttcaaaacgt 7320
   ccacccgggg tgaaatcgca cacctggtga ttgattccac cgggctgaag gtctttggtg 7380
   aaggcgaatg gaaagtcaga aagcacggca aagagcgccg tcgtatctgg cgaaagttgc 7440
   atcttgctgt tgacagcaac acacatgaag ttgtctgtgc agacctgtcg ctgaataacg 7500
   tcacggactc agaagccttc ccgggcctta tccggcagac tcacagaaaa atcagggcag 7560
   ccgcggcaga cggggcttac gatacccggc tctgtcacga tgaactgcgc cgcaaaaaaa 7620
   tcagcgcgct tattcctccc cgaaaaggtg cgggttactg gcccggtgaa tatgcagacc 7680
   gtaaccgtgc agtggctaat cagcgaatga ccgggagtaa tgcgcggtgg aaatggacaa 7740
   cagattacaa ccgtcgctcg atagcggaaa cggcgatgta ccgggtaaaa cagctgttcg 7800
   ggggttcact gacgctgcgt gactacgatg gtcaggttgc ggaggctatg gccctggtac 7860
   gagcgctgaa caaaatgacg aaagcaggta tgcctgaaag cgtgcgtatt gcctgaaaac 7920
   acaacccgct acgggggaga cttacccgaa atctgattta ttcaacaaag ccgggtgtgg 7980
   tgaactacaa agcagacccg ttgaggttat cagttcgatg cacaatcagc agcgcataaa 8040
   atatgcacaa gaacaggagc acccttcgca ttaagctgtg gtggtaacaa gtagtgccgg 8100
   gctaccatca gcgagcatga tgcgctccca cagcattcgc cttggcagta tggaagttcc 8160
   tcgctccagt tcgggccggt atccacctcg cacctggtgt ttaaacggat ttaaattgca 8220
   ggggcagtga gcgcaacgca attaatgtaa gttagctcac tcattaggca ccccaggctt 8280
   gacactttat gcttccggct cgtataatgt gtggaattgt gagcggataa caataacaat 8340
   ttaaatcagg atctaggaac caaggagagt ggcatatgac tgacgacgca aagagagcaa 8400
   aagtatccag caccggtccg atttctgcag cacgtcagca agttgcggac cgcattcgcg 8460
   atctggacgc gcgtgacgaa gaatttcgca cgaccaaacc ggactctgcg ctgcagctgg 8520
   cggcacgtga accgggtctg cgtctgccgc aaatcctgga aatctttgcc gaaggttacg 8580
   cggatcgtcc ggcgctgggc tggcgtgcgc gcagcctgac caccgatgcg gcgactggcc 8640
   gtaccagcgc tcagttgctg ccgcgcttcg acacgatgac gtaccgtgaa ctgtgggcga 8700
   atgtccgcgc aatcgccggt gcttggcgcc acgatgctac caacccggtg gcaccgggtg 8760
   atgttgtggc aactgttggt ttcgcgagcg cagaatacct gaccatcgat ctggtctgtg 8820
   cgtatctggg cctggttgcc gtgccgctgc aacataacgc gaccgcatcc cgtctgcgtc 8880
   cgatcgtcga agaggttgag ccgtcgacgt tggcggctgg cgtgggctac ctggacttgg 8940
   ccgtcgaggc ggccttgggc agctccagcc tgcgccgtgt ggttgtgttt gattatcgtc 9000
   cggaagttga cgaacagcgc gaggcggttg accgtgcgcg tagcaaactg gcgggtgcag 9060
   gtatcgcggt caccgttgag acgctgggtg acgtcattga gcgtggccgt accctgccgc 9120
   ctgagccgat gtttaccggc gatacggacg agcgtctggc aatgattatg tataccagcg 9180
   gttccaccgg tttgccgaaa ggcgccatgt ataccgagcg tatgctgtgc cgtctgtgga 9240
   ccactgaact gatgccggat ttcgccgaca ccccggttat caatgtcaac ttcatgccgc 9300
   tgaatcacct gggtggtcgt atcccgctga gcaccgcgtt ccaggccggt ggtacctctt 9360
   atttcgttcc ggagagcgat ctgagcaccc tgttcgatga ttggaatctg gtccgtccga 9420
   ccgagatggg cctggtgccg cgtgtggcag aaatgttgta ccagcgttac cagagcgcgg 9480
   tcgatcgctt tgaagttgct ggcacggaca cggcgacggc acaagcgcgt gctcaggccg 9540
   aactgcgtga gcaggtgctg ggcggtcgca ttgttactgc cttctgtggc accgcaccgc 9600
   tggctgccga gatgcgcagc ttcattgaga cgtgcctgag cgtgcatgtg ctggacggtt 9660
   acggtctgac ggaagttggt atggtcacca aggatggttt gattacgcaa ccgccggttt 9720
   tggactataa gctgatcgat gttccggagc tgggttactt cctgaccgac aaaccgtatc 9780
   cgcgtggcga gctgctggtt aaatccctga ccgcaacccc gggttacttc aagcgtccag 9840
   atgtcaccgc caatgcattc gacccagaag gctattaccg tacgggcgac gtgatggcgg 9900
   agctggaacc tagccgtctg gcctacgtgg atcgtcgcaa taacgtcctg aagctggcac 9960
   aaggtgagtt cgttgccgtc gctcgtctgg aagccgtttt tagcagcgcc gcactggtgc 10020
   gtcaaatctt tgtgtacggc aacagcgagc gcccgtactt gctggcggtc gtggtaccga 10080
   cggatgacgc agcccaaaag tatgcgggtg acccgggtgg tctgaaaagc gcgttgggtg 10140
   agagcctgcg ccaagcggct aagctggcag aactgcaaag ctacgaagtt ccggttgatt 10200
   ttgttgtgga aaccgagccg tttagcgagg ataatggctt gctgagcggt gtcggcaaac 10260
   tgctgcgtcc aaaactgaaa gagcactacg gcgcacgcct ggaacagctg tatgcagacc 10320
   tggctgaaag ccgcgttacg gagctgcgtg ccctgcgtga gggtgctgct gatcaccctg 10380
   tgatcttcac gttgacccgt gcggcggaag cgctgctggg tttggccggt ggtccgcctg 10440
   cgccggatgc gttgttcatt gaactgggtg gtgatagctt gagcgcgctg acgtttagca 10500
   atctgttgcg tgacatcttc gacgttgagg tgccggtcgg tatgattacc ggtccggcga 10560
   ccgacctggg tcagctggca gagtatgtgg aatccgaacg tgcgtccggc agccgtcgtc 10620
   cgacctttgc gaccgtccac ggtcgcggtg ccacgcaggt gcgcgctgca gacttgacgt 10680
   tggataagtt catcgacgca accaccctgg cagaagcacc ggcattgccg cgtgcgaccg 10740
   gtaccccgca cactgtgctg ctgaccggtg cgaacggcta cctgggccgt ttcttgattc 10800
   tggagtggtt ggaacgtctg gcggagacgg gtggcaagct gattagcatt attcgtgcag 10860
   cggacgctgc ggcagcagcg aaacgcctgg agagcgtgtt cgacagcggc gatccgcaac 10920
   tgctggagcg tttccgcacg ctggcagcgg atcatctgga agttatcgtc ggtgatattg 10980
   gtgagccaaa cctgggtctg caacagggca cctgggagcg cctggcgcag tcggtcgatt 11040
   tgattgtcca tccagcagca ctggtcaacc atgtgctgcc gtacgaccag ctgttcggtc 11100
   cgaatgtcgt cggcactgcg gagctgattc gtttggccat caccactcgc attaaacctg 11160
   tcacgtacat gtccaccgtg gctgtcgcgc tgagcgttga cccggctgcc ttcgccgagg 11220
   acggcgacat ccgcacggtt tcggctctgc gtccggttga tggtggctat gcgaatggtt 11280
   acgcaaactc gaagtgggcg ggtgaagtac tgctgcgcga ggcacacgat ctgtgcggcc 11340
   tgccggttgc ggtttttcgc agcgacatga tcctggcaca tagccgttac gcaggccaac 11400
   tgaacgtgcc ggacgcgttt acgcgtctga tctttagcct gctgacgacc ggcatcgcac 11460
   cgggtagctt ttatcaaacc gaccctcgcg gcaaccgtgc tattgcgcac tacgacggtc 11520
   tgccagcgga ttttgtcgct gaagcggtta ccacgctggg cgagcagatc gcgactgccg 11580
   cgcaggattc tggtgcatat cgcagctttg atgttatgaa cccgcacgac gatggcatct 11640
   ctctggacgt gttcgtggat tggctgattg cgggtggtca cgatattcgc cgtatcgatg 11700
   actatgacga gtggctgagc cgctttacca ccgccctgcg cgcactgccg gacaaacaac 11760
   gtcagcactc cgtcctgccg ctgctggacg cttatcgtaa gccggaaacc ccgctgcgtg 11820
   gtgcgcctgc gcctaccgat gtgtttcgta gcgcggttcg tgaggcgaag attggtgcgg 11880
   ccgaggatat cccacatctg agcgcggcac tgattgacaa atacgtggcc gacttgcgtt 11940
   tgctgggtct ggtgtaataa gtttaaacag agaaggagtt ctatcatgat tgaaaccatc 12000
   ttaccagccg gcgtcgaatc agcagagtta cttgagtacc ctgaagatct gaaggcgcat 12060
   ccggctgaag agcatctgat cgcaaagagc gtagagaagc gtcgtcgcga cttcattggc 12120
   gcacgtcatt gcgcccgtct ggcactggcg gagctgggtg aaccgccggt tgcgattggc 12180
   aagggtgaac gtggtgcgcc gatttggccg cgtggtgtcg tgggctctct gacccattgc 12240
   gatggctatc gcgcagcggc ggttgctcac aaaatgcgtt ttcgcagcat cggcatcgac 12300
   gccgaaccgc acgcgaccct gccggaaggt gtcctggatt cggttagcct gccgcctgag 12360
   cgtgagtggc tgaaaaccac cgacagcgca ctgcacctgg accgtttgct gttttgtgcg 12420
   aaagaagcaa cttacaaagc gtggtggccg ctgacggcac gttggctggg tttcgaagaa 12480
   gcgcacatta ccttcgaaat cgaggatggt agcgccgact ctggtaatgg cacgtttcac 12540
   agcgaactgc tggtgccggg tcagaccaat gacggtggta ccccgctgct gtctttcgac 12600
   ggtcgctggc tgatcgctga tggcttcatc ctgacggcga ttgcatacgc atgataaatt 12660
   aacctaggct gctgccaccg ctgagcaata actagcataa ccccttgggg cctctaaacg 12720
   ggtcttgagg ggttttttgc tgaaacctca ggcatttgag aagccctcga gtgaccccag 12780
   ccgcccctca tgccaaccga gcccattaag cgcagagtcg gccgcaacta tctcggggac 12840
   gcataaacac gcagcgattt accaaggagt tcggctaggt caatccgcaa ttccctttgc 12900
   ctggccggag atgccgcgag caaacaatgt gtccggtcgc taccagatgg actttgtggg 12960
   cggaggattg caccagacgc ttcagcgcag cgtcgtacga tgaatcggaa ggcccggtaa 13020
   gcaattgaag aaccgctgac cacctcccat ccgacaatgc aagggtgccc cgctcccagc 13080
   gcgacacggt tgcctgatcc acacccatta attcagctag gtgactttgc ttcatatgac 13140
   gaagcaaccg cgccctccgc acctgccgcc ccctgtcatt cgtcaacatt cttcagcacc 13200
   tcaatgtcgt tcgtagtgag cctcattttt tcaagaccgc cgatgatgag agccaggcct 13260
   cgctcgaatg ccgcgtccgg accgccttcg tagacgattt tcatcgcgct ctgtaggcgc 13320
   gccggcatcg tagacgctga ggtggtcaac tgatcttcgc cccgctcctc ggcgtctgcc 13380
   tcgctagctt gctgctcaag aacagcgccg acggtgaagt agctgattgc catcaacgca 13440
   taggtcgcgt cacctgccga aaagccagca tcgcaaagga agcgaagctg cgcgtcggct 13500
   ttttccatct gcggcgcggc tggccgcgtc ccggcatgaa tacgcgcgcc atcgcgataa    13560
   gcgagcaacg cccgtcgaaa actgcatgca ttgcccttca ggaacgaacg ccagtcgtcg    13620
   tcatcccttg gcgtcgaatg cgtgtgattt atcgtcagca tggcttcggc aagtgcgtcg    13680
   agcaacgcac gcttgttctt gaaatgccag tagagcgctg gctgttgcac cccgaggcgc    13740
   tcagccagtc ggcgcgtcgt tagaccttcc atgcccacgt cgttaagcag ttcgagcgcg    13800
   gttcggatca cggcctcgcg ttggagcttg ttcattcgcg aa                       13842

## Beschreibung

Die Erfindung betrifft einen Ganzzellkatalysator, der eine rekombinante α-Dioxygenase oder die Kombination aus einer rekombinanten Fettsäurereduktase und aus einer die Fettsäurereduktase phosphopantetheinylierenden Phosphopantetheinyl-Transferase exprimiert, und der zusätzlich eine Transaminase exprimiert, wobei die Phosphopantetheinyl-Transferase und/oder Transaminase bevorzugt rekombinant ist; sowie ein Verfahren zur Umsetzung einer Fettsäure, ω-Hydroxy-Fettsäure, ω-Oxo-Fettsäure oder eines Monoesters davon zu einem Amin, umfassend die Schritte Oxidieren der Fettsäure, ω-Hydroxy-Fettsäure, ω-Oxo-Fettsäure oder des Monoesters davon durch Kontaktieren mit einer Alkanhydroxylase und gebenenfalls einer Alkoholdehydrogenase zu einem Oxidationsprodukt Kontaktieren des Oxidationsproduktes mit einer phosphopantetheinylierten Fettsäurereduktase oder einer α-Dioxygenase zu einem Aldehyd, und Kontaktieren des Aldehyds mit einer Transaminase wobei die Enzyme phosphopantetheinylierte Fettsäurereduktase, α-Dioxygenase, Transaminase und Alkanhydroxylase in Form eines Ganzzellkatalysators nach einer erfindungsgemäßen Ausführungsform bereitgestellt werden.
Polyamide sind eine Klasse von Polymeren, die durch sich wiederholende Amidgruppen gekennzeichnet sind. Der Begriff "Polyamid" betrifft im Unterschied zu den chemisch verwandten Proteinen gewöhnlich synthetische, im Handel erhältliche, thermoplastische Kunststoffe. Polyamide werden von primären Aminen oder von sekundären Aminen abgeleitet, die herkömmlich beim Cracken von Kohlenwasserstoffen gewonnen werden. Es können jedoch auch Derivate, genauer Aminocarbonsäuren, Lactame und Diamine, zur Polymerherstellung verwendet werden. Von Interesse sind weiterhin kurzkettige, gasförmige Alkane als Edukte, die ausgehend von nachwachsenden Rohstoffen mit biotechnologischen Verfahren gewonnen werden können.
Viele kommerziell stark nachgefragte Polyamide werden ausgehend von Lactamen hergestellt. Zum Beispiel kann man "Polyamid 6" durch Polymerisation von ε-caprolactam und "Polyamid 12" durch Polymerisierung von Laurinlactam erhalten. Weitere kommerziell interessante Produkte umfassen Copolymere von Lactamen, z. B. Copolymere von ε-caprolactam und Laurinlactam.
Die konventionelle chemisch-technische Erzeugung von Aminen ist von der Versorgung mit fossilen Rohstoffen abhängig, ineffizient, und es fallen dabei große Mengen unerwünschter Nebenprodukte an, in manchen Schritten der Synthese bis zu 80 %. Ein Beispiel für einen solchen Prozess stellt die Herstellung von Laurinlactam dar. Konventionell erfolgt diese über ein vielstufiges Verfahren, das nicht nur eine geringe Ausbeute ergibt, sondern gleichzeitig die Bereitstellung einer aufwändigen Infrastruktur erfordert.

Angesichts dieser Nachteile wurden Verfahren entwickelt, um Amine unter Verwendung von Biokatalysatoren ausgehend von nachwachsenden Rohstoffen zu gewinnen. Als nachwachsende Rohstoffe kommen insbesondere Quellen für Fettsäuren in Frage, die in Form von Rapsöl, Kugeldistelöl, Palmkernöl, Kokosnussöl, Sonnenblumenkernöl und ähnlichen Naturprodukten aus einer Vielzahl von biologischen Quellen, insbesondere aus Pflanzen, gewonnen werden können.

PCT/EP 2008/067447 beschreibt ein biotechnologisches System zur Herstellung chemisch verwandter Produkte, genauer ω-Aminocarbonsäuren, unter Verwendung einer Zelle, die eine Reihe von geeigneten enzymatischen Aktivitäten aufweist und in der Lage ist, Carbonsäuren zu entsprechenden ω-Aminocarbonsäuren umzuwandeln. Das Verfahren umfasst eine Kaskade enzymatisch katalysierter Reaktionen, insbesondere die Oxidation einer Fettsäure am endständigen Kohlenstoffatom bis zum Aldehyd und die anschließende Aminierung unter Verwendung einer Transaminase und einer Aminosäure als Amindonor, die über eine Aminosäuredehydrogenase regeneriert werden kann.

Ein bekannter Nachteil des dabei verwendeten AlkBGT-Oxidasesystems aus *Pseudomonas putida* GPO1 besteht jedoch darin, dass es nicht in der Lage ist, eine selektive Oxidation von aliphatischen Alkanen zu primären Alkoholen zu leisten. Vielmehr treten eine Vielzahl von Oxidationsprodukten auf; insbesondere erhöht sich der Anteil an höher oxidierten Produkten wie dem entsprechenden Aldehyd, Keton oder der entsprechenden Carbonsäure mit zunehmender Reaktionsdauer (C. Grant, J. M. Woodley and F. Baganz (2011), Enzyme and Microbial Technology 48, 480-486), was die Ausbeute an erwünschtem Amin entsprechend verringert.

Das Problem der relativ unselektiven Oxidation ist dadurch verschärft, dass die entstehenden Oxidationsprodukte strukturell sehr ähnlich sind. Dies bedingt, dass es sehr schwierig ist, sie von den erwünschten Oxidationsprodukten effizient und ohne signifikanten Ausbeuteverlust abzutrennen.

Ein weiterer Nachteil dieses Verfahrens besteht darin, dass überoxidierte Nebenprodukte, beispielsweise die Dicarbonsäure der als Edukt eingesetzten Fettsäure, das Recycling von hydrophoben Lösungsmitteln und hydrophoben flüssigen Kationenaustauschern, die gemäß der PCT/EP2011/071491 zur Abtrennung des Produktes aus der wässrigen Reaktionsmischung verwendet werden können, auf Kosten der Effizienz bei der Ressourcennutzung gehen.

In diesem Zusammenhang ist hervorzuheben, dass die Komplexität biotechnologischer Systeme mit einer Kaskade von Reaktionen wie dem in der PCT/EP 2008/067447 beschriebenen, von denen jeweils eine Reaktion von einem bestimmten Enzym katalysiert wird, die Optimierung der Reaktionsbedingungen erschwert. So ist im Falle der grundsätzlich reaktiven ω-Aminofettsäuren als Produkt die Möglichkeit gegeben, dass sie ab einer bestimmten kritischen Konzentration im Inneren der Zelle mit essentiellen Bestandteilen des Organismus reagieren und somit toxisch wirken. Ist das der Fall, so ist die Wachstums- und Synthesefähigkeit des Organismus bis hin zum Absterben der Zelle beeinträchtigt, ohne dass der Entwickler die Toxizität unmittelbar erkennen oder gar einem bestimmten Edukt, Zwischenprodukt oder Produkt zuordnen könnte. Welcher Organismus welche Konzentration an einem chemisch reaktiven Stoff verträgt, ist ebenfalls nicht vorhersehbar.

Auch mit Hinblick auf eine zu verbessernde Produktausbeute und eine zu verringernde Entstehung von Nebenprodukten kann der Fachmann limitierende und entscheidende Faktoren in einem System wie dem in der PCT/EP2008/067447 beschriebenen nicht routinemäßig identifizieren. Ist die Ausbeute an Produkt zu niedrig, so kann dies daran liegen, dass eines der Enzyme in einer zu niedrigen Konzentration vorhanden ist, ohne dass bekannt wäre, um welches der in Frage kommenden Enzyme es sich dabei handelt, d. h. das Edukt wird im vorgesehenen Zeitrahmen oder vor dem Abbau durch konkurrierende Enzyme aufgrund unzureichender Synthesekapazität nicht umgesetzt. Alternativ ist es durchaus möglich, dass ein Enzym zwar nachweisbar in der Zelle in Form eines Polypeptids vorhanden ist, aber gerade in dieser Zelle nicht die für die Aktivität essentielle Faltung aufweist oder ein bis dato unbekannter, für die Aktivität aber essentieller Cofaktor fehlt. Gleichermaßen kann, wie bereits erwähnt, das Stoffwechselprodukt für die Zelle toxisch sein oder abgebaut werden. Schließlich ist mit interferierenden Wechselwirkungen mit endogenen, d. h. natürlich in einer als Ganzzellkatalysator verwendeten Zelle vorhandenen Enzymen zu rechnen.

WO2009/077461, US2007/032428 und Coop, J.N. (2006), Applied and Environmental Microbiology; 72 (4):2298-2305 beschreiben den Stand der Technik.

Es besteht somit Bedarf an Verfahren zur Herstellung von ω-Aminofettsäuren aus Fettsäuren, bei denen die enzymatisch katalysierten Reaktionen selektiver verlaufen und die Bildung unerwünschter Nebenprodukte minimiert ist.
Vor diesem Hintergrund besteht die der Erfindung zu Grunde liegende Aufgabe darin, ein mit Hinblick auf Ausbeute, Kohlenstoff- und/oder Stickstoffbilanz und/oder Reinheit möglichst effizientes biotechnologisches Verfahren zur Herstellung von ω-Aminofettsäuren bereitzustellen.
Eine weitere der Erfindung zu Grunde liegende Aufgabe besteht darin, ein mit Hinblick auf Ausbeute, Kohlenstoff- und/oder Stickstoffbilanz, Wiederverwendbarkeit verwendeter Agenzien und/oder Reinheit des Produktes möglichst effizientes biotechnologisches Verfahren zur Umsetzung von Carbonsäureestern zu aminierten Carbonsäureestern bereitzustellen. In diesem Zusammenhang wird unter einer effizienten Kohlenstoff- und/oder Stickstoffbilanz bevorzugt verstanden, dass sich ein möglichst hoher Anteil des zur Umsetzung eines Carbonsäureesters in Form von geeigneten Substraten an eine Zelle verfütterten Kohlenstoffs und/oder Stickstoffs im erwünschten Endprodukt wiederfindet, anstatt beispielsweise zu anderen Produkten als den erwünschten umgesetzt zu werden.
Eine weitere der Erfindung zu Grunde liegende Aufgabe besteht darin, die Aufarbeitbarkeit einer mehrphasigen Reaktionsmischung aus der Umsetzung eines Carbonsäureesters zu verbessern, besonders mit Hinblick auf die Wiederverwendbarkeit zur Aufarbeitung verwendeter hydrophober Lösungsmittel und flüssiger Kationenaustauscher, sowie mit Hinblick auf die Phasenbildung und -trennung bei einem biphasischen System umfassend eine wässrige Phase, in der die Umsetzung des Carbonsäureesters abläuft, und eine organische Phase mit organischen Lösungsmitteln und/oder flüssigen Kationenaustauschern.
Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

Das der Erfindung zu Grunde liegende Problem wird in einem ersten Aspekt gelöst durch einen Ganzzellkatalysator, der eine rekombinante α-Dioxygenase oder die Kombination aus einer rekombinanten Fettsäurereduktase und aus einer die Fettsäurereduktase phosphopantetheinylierenden Phosphopantetheinyl-Transferase exprimiert, und der zusätzlich eine Transaminase exprimiert, wobei die Phosphopantetheinyl-Transferase und/oder Transaminase bevorzugt rekombinant ist.

In einer ersten Ausführungsform des ersten Aspekts wird das Problem durch einen Ganzzellkatalysator gelöst, der zusätzlich eine Aminosäuredehydrogenase exprimiert, die bevorzugt rekombinant ist.

In einer zweiten Ausführungsform, die auch eine Ausführungsform der ersten Ausführungsform darstellt, wird das Problem durch einen Ganzzellkatalysator gelöst, der zusätzlich eine Alkanhydroxylase exprimiert, die bevorzugt rekombinant ist.

In einer dritten Ausführungsform, die auch eine Ausführungsform der ersten bis zweiten Ausführungsform darstellt, wird das Problem durch einen Ganzzellkatalysator gelöst, der zusätzlich ein Polypeptid der AlkL-Familie exprimiert, das bevorzugt rekombinant ist.

In einer vierten Ausführungsform, die auch eine Ausführungsform der ersten bis dritten Ausführungsform darstellt, wird das Problem durch einen Ganzzellkatalysator gelöst, der zusätzlich eine Alkoholdehydrogenase exprimiert, die bevorzugt rekombinant ist.

In einer fünften Ausführungsform, die auch eine Ausführungsform der ersten bis vierten Ausführungsform darstellt, wird das Problem durch einen Ganzzellkatalysator gelöst, wobei die Aktivität wenigstens eines an der β-Oxidation beteiligten Enzyms gegenüber dem Wildtyp des Ganzzellkatalysators verringert ist.

In einer sechsten Ausführungsform, die auch eine Ausführungsform der ersten bis fünften Ausführungsform darstellt, wird das Problem durch einen Ganzzellkatalysator gelöst, wobei die Aktivität von BioH oder einer Variante davon gegenüber dem Wildtyp des Ganzzellkatalysators verringert ist.

In einer siebten Ausführungsform, die auch eine Ausführungsform der ersten bis sechsten Ausführungsform darstellt, wird das Problem durch einen Ganzzellkatalysator gelöst, wobei die Aktivität von FadL oder einer Variante davon gegenüber dem Wildtyp des Ganzzellkatalysators erhöht ist.

In einem zweiten Aspekt wird das der Erfindung zu Grunde liegende Problem gelöst durch ein Verfahren zur Umsetzung einer Fettsäure, ω-Hydroxy-Fettsäure, ω-Oxo-Fettsäure oder eines Monoesters davon zu einem Amin, umfassend die Schritte
a) Oxidieren der Fettsäure, ω-Hydroxy-Fettsäure, ω-Oxo-Fettsäure oder des Monoesters davon durch Kontaktieren mit einer Alkanhydroxylase und gegebenenfalls einer Alkoholdehydrogenase zu einem Oxidationsprodukt
b) Kontaktieren des Oxidationsproduktes mit einer phosphopantetheinylierten Fettsäurereduktase oder einer α-Dioxygenase zu einem Aldehyd, und
c) Kontaktieren des Aldehyds mit einer Transaminase
wobei die Enzyme phosphopantetheinylierte Fettsäurereduktase, α-Dioxygenase, Transaminase und Alkanhydroxylase in Form eines Ganzzellkatalysators gemäß dem ersten Aspekt der Erfindung bereitgestellt werden.
In einem zweiten Aspekt wird das der Erfindung zu Grunde liegende Problem gelöst durch ein Verfahren, wobei bei Schritt c) eine Aminosäuredehydrogenase anwesend ist.
In einer ersten Ausführungsform des zweiten Aspekts wird das Problem gelöst durch ein Verfahren, wobei die Enzyme phosphopantetheinylierte Fettsäurereduktase, α-Dioxygenase, Transaminase, Aminosäuredehydrogenase und Alkanhydroxylase in Form eines Ganzzellkatalysators gemäß dem ersten Aspekt der Erfindung bereitgestellt werden.
In einer zweiten Ausführungsform, die auch eine Ausführungsform der ersten Ausführungsform darstellt, wird das Problem gelöst durch ein Verfahren, wobei es sich bei der Fettsäure, ω-Hydroxy-Fettsäure, ω-Oxo-Fettsäure oder dem Monoester davon um eine Verbindung der Formel (I)

R¹ - A - COOR² (I)

handelt, wobei R¹ aus der Gruppe ausgewählt ist, die -H, -CHO, -OH und COOR³ umfasst,
wobei R² und R³ jeweils und unabhängig voneinander aus der Gruppe ausgewählt sind, die H, Methyl, Ethyl und Propyl umfasst,
mit der Maßgabe, dass wenigstens einer der Reste R² und R³ H ist,
wobei A eine unverzweigte, verzweigte, lineare, zyklische, substituierte oder unsubstituierte Kohlenwasserstoffgruppe mit wenigstens vier Kohlenstoffatomen darstellt.

In einer dritten Ausführungsform, die auch eine Ausführungsform der ersten bis zweiten Ausführungsform darstellt, wird das Problem gelöst durch ein Verfahren, wobei A die Formel - (CH₂)ₙ - aufweist, wobei n wenigstens 4, bevorzugt wenigstens 10 ist.

In einem dritten Aspekt wird das der Erfindung zu Grunde liegende Problem gelöst durch eine Verwendung des Ganzzellkatalysators nach dem ersten Aspekt oder des Verfahrens nach dem zweiten Aspekt zur Aminierung einer Fettsäure, ω-Hydroxy-Fettsäure, ω-Oxo-Fettsäure oder einem Monoester davon.

In einem vierten Aspekt wird das der Erfindung zu Grunde liegende Problem gelöst durch eine Reaktionsmischung umfassend den Ganzzellkatalysator nach dem ersten Aspekt in wässriger Lösung sowie eine Fettsäure, ω-Hydroxy-Fettsäure, ω-Oxo-Fettsäure oder einen Monoester davon der Formel (I)

R¹ -A - COOR² (I),

wobei R¹ aus der Gruppe ausgewählt ist, die -H, -CHO, -OH und COOR³ umfasst,
wobei R² und R³ jeweils und unabhängig voneinander aus der Gruppe ausgewählt sind, die H, Methyl, Ethyl und Propyl umfasst,
mit der Maßgabe, dass wenigstens einer der Reste R² und R³ H ist,
wobei A eine unverzweigte, verzweigte, lineare, zyklische, substituierte oder unsubstituierte Kohlenwasserstoffgruppe mit wenigstens vier Kohlenstoffatomen darstellt, bevorzugt die Formel - (CH₂)ₙ -, wobei n wenigstens 4, besonders bevorzugt wenigstens 10 ist.

Die vorliegende Erfindung beruht auf der Erkenntnis der Erfinder, dass eine funktionell coexprimierte rekombinante Fettsäurereduktase oder α-Dioxygenase in einem Ganzzellkatalysator, der zur Herstellung von ω-Aminofettsäuren aus Fettsäuren verwendet wird und eine entsprechende Enzymausstattung aufweist, überraschend die Ausbeute an ω-Aminofettsäuren erhöht.

Weiterhin beruht die vorliegende Erfindung auf der Erkenntnis der Erfinder, dass eine funktionell coexprimierte rekombinante Fettsäurereduktase oder α-Dioxygenase in einem Ganzzellkatalysator, der zur Herstellung von ω-Aminofettsäuren aus Fettsäuren verwendet wird und eine entsprechende Enzymausstattung aufweist, überraschend die Konzentration störender Nebenprodukte, insbesondere von überoxidierten Fettsäuren in Form von Dicarbonsäuren und Estern davon, im anfallenden Produkt verringert.

Weiterhin beruht die vorliegende Erfindung auf der Erkenntnis der Erfinder, dass eine funktionell coexprimierte rekombinante Fettsäurereduktase oder α-Dioxygenase in einem Ganzzellkatalysator, der zur Herstellung von ω-Aminofettsäuren aus Fettsäuren verwendet wird und eine entsprechende Enzymausstattung aufweist, die Reinheit und Wiederverwendbarkeit von flüssigen Kationenaustauschern wie Ölsäure verbessert, die zur Entfernung einer ω-Aminofettsäure aus einem Fermentationslösung umfassend den Ganzzellkatalysator verwendet werden.

Die vorliegende Erfindung betrifft ein verbessertes Verfahren zur Umsetzung einer Fettsäure, ω-Hydroxy-Fettsäure, ω-Oxo-Fettsäure oder eines Monoesters davon zu einem Amin, das sich dadurch auszeichnet, dass neben den Enzymen, die die Überführung der Fettsäure über ihre verschiedenen Oxidationsstufen zum Amin katalysieren, auch wenigstens eine Fettsäurereduktase oder α-Dioxygenase, oder die Kombination beider Enzyme, anwesend ist, wenn ein Ganzzellkatalysator zur Durchführung des Verfahrens eingesetzt wird. In einer bevorzugten Ausführungsform wird unter dem Begriff "Fettsäurereduktase", wie hierin verwendet, ein Enzym verstanden, das die Umwandlung einer ω-Carboxysäure, auch als Dicarbonsäure oder ω-Carboxy-Fettsäure bezeichnet, zur entsprechenden ω-Oxofettsäure unter Verbrauch von ATP und NAD(P)H katalysiert. Im Stand der Technik, beispielsweise in der WO/2010/135624, sind Fettsäurereduktasen zur Herstellung von ω-Hydroxyfettsäuren beschrieben, nicht jedoch als Teil eines Systems zu Herstellung von ω-Aminofettsäuren. In einer noch bevorzugteren Ausführungsform ist die Fettsäurereduktase aus der Gruppe von Fettsäurereduktasen ausgewählt, die die Aminosäuresequenzen YP_887275.1, ZP_11001941.1, ZP_06852401.1, NP_959974.1, YP_001070587.1, ZP_05217435.1, YP_882653.1, YP_639435.1, ZP_10800193.1, YP_006452763.1, YP_006730440.1, ZP_11196216.1, YP_005349252.1, ZP_05224908.1, YP_005338837.1, YP_006307000.1, YP_005343991.1, ZP_11001942.1, ZP_09979565.1, YP_005003162.1, YP_953393.1, YP_001850422.1, ZP_11011489.1, P_12689264.1, YP_905678.1, ZP_09976919.1, YP_004746059.1, N_217106.1, YP_004525443.1, NP_337166.1, ZP_09685823.1, YP_978699.1, ZP_06437984.1, ZP_06514086.1, NP_856267.1, CAA19077.1, N_301424.1, ZP_06522140.1, ZP_06518098.1, ZP_11008938.1, ZP_07432374.2, AAR91681.1, YP_006808747.1, YP_001851230.1, ZP_15327751.1, ZP_15455857.1, ZP_12874284.1, ZP_15332534.1, ZP_15512956.1, ZP_14244106.1, ZP_15470899.1, ZP_11439367.1, YP_001703694.1, ZP_15446742.1, YP_006808978.1, ZP_07964926.1, YP_006521379.1, WP_007769435.1, ZP_15512957.1, ZP_12874283.1, YP_005350955.1, ZP_14243341.1, YP_001705436.1, ZP_15329649.1, YP_006522325.1, YP_006732197.1, YP_003658971.1, ZP_05227804.1, YP_001703695.1, YP_006308707.1, ZP_15342047.1, YP_006521380.1, ZP_15327752.1, YP_005340557.1, ZP_11439578.1, ZP_15392943.1, ZP_15514789.1, ZP_12996178.1, ZP_09412214.1, ZP_06849686.1, YP_889972.1, YP_006570321.1, ZP_15375693.1, YP_006308219.1, YP_006521600.1, YP_005340029.1, YP_005350457.1, ZP_11439836.1, ZP_12994664.1, ZP_14240588.1, ZP_14236860.1, ZP_09410830.1, YP_006731697.1, YP_005264225.1, YP_001704097.1, ZP_15328186.1, ZP_09402885.1, ZP_12690463.1, AFO59871.1, ZP_07966879.1, YP_118225.1, YP_001828302.1, YP_006566873.1, YP_003660169.1, ZP_15337407.1, ZP_08240521.1, ZP_10456477.1, YP_001537947.1, YP_004016539.1, ZP_07664024.1, ZP_14244107.1, ZP_09794557.1, ZP_09274211.1, ZP_05224899.1, ZP_15484175.1, AAA17105.1, ZP_11437924.1, ZP_15446621.1, YP_003646340.1, ZP_15382134.1, ZP_14237669.1, ZP_09165547.1, YP_004019203.1, ZP_14240225.1, YP_001220863.1, CBA74242.1, ZP_12994240.1, EIE27140.1, ZP_15354547.1, ZP_15432557.1, ZP_15500132.1, ZP_15478632.1, ZP_06846978.1, AAA17108.1, ZP_15333767.1, ZP_05217205.1, AAD44234.1, YP_005348984.1, YP_006306749.1, ZP_05224611.1, YP_005343772.1, YP_006730188.1, YP_882425.1, ZP_10799956.1, ZP_05045132.1, NP_960176.1, ZP_12398880.1, ZP_11192735.1, ZP_11440091.1, ZP_05217203.1, ZP_06846979.1, ZP_10800936.1, ZP_06523596.1, YP_882421.1, YP_006306748.1, YP_006522017.1, ZP_15432556.1, ZP_15354095.1, ZP_05227781.1, ZP_09684639.1, YP_006730187.1, YP_005343770.1, YP_005338616.1, YP_005348983.1, ZP_15472813.1, ZP_15457007.1, ZP_15421152.1, ZP_15488933.1, ZP_14240030.1, YP_001704825.1, ZP_15328982.1, YP_005911512.1, ZP_09411638.1, ZP_12876400.1, ZP_12995435.1, ZP_07667680.1, YP_001281387.1, EIE21044.1, ZP_15375054.1, NP_334518.1, 4DQV_A, ZP_06435375.1, YP_003030020.1, YP_976237.1, ZP_04926822.1, YP_004998149.1, YP_004743589.1, YP_005907921.1, NP_214615.1, YP_001286047.1, ZP_06515541.1, ZP_05139482.1, YP_888016.1, ZP_06452908.1, ZP_06519578.1, YP_004721827.1, CAJ77696.1, ZP_09680854.1, ZP_09686453.1, YP_884815.1, YP_884815.1, CAB55600.1, ZP_09081423.1, YP_006521568.1, ZP_11440626.1, ZP_15513309.1, ZP_09410778.1, ZP_15374248.1, ZP_15405954.1, YP_001704047.1, ZP_14236911.1, ZP_12873916.1, ZP_14242094.1, ZP_12994610.1, ZP_07664023.1, ZP_15446620.1, ZP_15484174.1, ZP_14240245.1, YP_005358845.1 und XP_002669159.1, insbesondere YP_006731697.1, ZP_09839660.1, YP_001704097.1, YP_889972.1, ZP_05045132.1, NP_959974.1, ZP_10456477.1, YP_118225.1, YP_905678.1, YP_887275.1, ZP_11001941.1, WP_007769435.1 und YP_005349252.1
und Varianten davon umfassen.

Bei Fettsäurereduktasen handelt es sich um eine Gruppe von Enzymen, die für ihre Aktivität eine Phosphopantetheinylierung, d. h. die kovalente Befestigung eines Phosphopantetheinyl-Cofaktors am Enzym, benötigen. Dementsprechend ist die erfindungsgemäß verwendete Fettsäurereduktase phosphopantetheinyliert, und eine die Fettsäurereduktase exprimierender Ganzzellkatalysator exprimiert, entweder als Teil seiner Ausstattung endogen exprimierter Enzyme oder in rekombinanter Form, eine die Fettsäurereduktase phosphopantetheinylierende Phosphopantetheinyl-Transferase. In einer bevorzugten Ausführungsform wird unter dem Begriff "Phosphopantetheinyl-Transferase", wie hierin verwendet, ein Enzym verstanden, dass einen Phosphopantetheinyl-Rest von einem Phosphopantetheinyl-CoA auf ein Enzym, bevorzugt auf die Fettsäurereduktase, überträgt. In einer besonders bevorzugten Ausführungsform ist die Phosphopantetheinyl-Transferase aus der Gruppe von Phosphopantetheinyl-Transferasen ausgewählt, die die Aminosäuresequenzen ABI83656.1, YP_006811024.1, YP_120266.1, YP_005265173.1, YP_004006671.1, ZP_08152482.1, ZP_11104141.1, ZP_14482198.1, YP_706581.1, ZP_10002626.1, ZP_09308410.1, YP_002783881.1, ZP_18276502.1, ZP_09271851.1, ZP_08204640.1, YP_002766085.1, ZP_09788717.1, ZP_09799863.1, ZP_10961877.1, YP_003273299.1, GAB86168.1, YP_006668875.1, ZP_08766535.1, ZP_09793386.1, ZP_09212827.1, ZP_09276344.1, ZP_09213870.1, ZP_09081490.1, ZP_10947586.1, YP_003658841.1, ZP_06852853.1, YP_953148.1, ZP_11011170.1, YP_639258.1, YP_886985.1, ZP_11194383.1, ZP_09681094.1, ZP_06455719.1, NP_337369.1, YP_004077819.1, NP_217310.1, YP_006452521.1, YP_005339056.1, ZP_05226335.1, ZP_07965127.1, ZP_07419314.2, NP_302077.1, YP_005003342.1, YP_005349465.1, ZP_10800435.1, ZP_06564430.1, YP_882860.1, YP_001135287.1, YP_001850220.1, ZP_05217634.1, YP_003646683.1, YP_004746246.1, ZP_15327906.1, ZP_09979035.1, YP_001703848.1, YP_906028.1, ZP_15395499.1, ZP_11438833.1, ZP_11005955.1, ZP_09410582.1, N_961833.1, YP_001106197.1, ZP_14237113.1, YP_004085491.1, YP_003835595.1, ZP_12994399.1, YP_004523804.1, ZP_12690887.1, YP_003339468.1, ZP_06589331.1, YP_004801334.1, ZP_09974565.1, ZP_04608379.1, ZP_13037142.1, YP_712537.1, ZP_11236665.1, NP_630748.1, ZP_06527138.1, YP_003835167.1, CCH33620.1, ZP_10309401.1, ZP_08881396.1, YP_003102953.1, YP_003487252.1, ZP_08881565.1, YP_006263961.1, NP_822924.1, YP_004914569.1, ZP_09400366.1, AFV71333.1, ZP_07309518.1, ZP_09172171.1, ZP_06710898.1, CAN89630.1, ZP_06921116.1, ZP_08804003.1, ZP_19189663.1, ZP_10545589.1, YP_006248725.1, ZP_10455557.1, YP_004015869.1, ZP_08801530.1, ZP_10550999.1, YP_004492879.1, ZP_09958730.1, ZP_08286666.1, ZP_11212856.1, AAL15597.1, AAZ94407.1, ZP_19188802.1, AFF18625.1, ZP_06575404.1, AAK06801.1, ADC79635.1, YP_004080528.1, YP_004921314.1, ACY01405.1, YP_004584022.1, YP_003114157.1, YP_003203177.1, AFB69911.1, YP_006876460.1, ZP_08024798.1, YP_006269867.1, YP_006881814.1, CCK26150.1, ZP_07307765.1, ZP_07315112.1, YP_005466392.1, NP_824081.1, YP_003493882.1, ZP_06412387.1, ZP_10068239.1, ZP_08234258.1, YP_001822177.1, ZP_03979107.1, ZP_07979043.1, BAA22407.1, ZP_09402950.1, YP_003112617.1, NP_738483.1, YP_480609.1, EKX90208.1, BAE93744.1, BAB69186.1, ZP_04713061.1, YP_006881735.1, ZP_07274901.1, ZP_11379052.1, ZP_06581115.1, YP_006437406.1, ZP_12871839.1, N_601186.1, ZP_08451808.1, YP_005057339.1, YP_005303909.1, ZP_07090824.1, YP_003783676.1, YP_004630011.1, ZP_06588772.1, AAX98203.1, AFK80329.1, ZP_08124665.1, ZP_03710365.1, AAB17877.1, ZP_07403633.1, ZP_11268660.1, ZP_07288841.1, ABV83217.1, ZP_16178576.1, AAG43513.1, ZP_09155938.1, YP_004605750.1, ZP_03918977.1, AAF71762.1, ZP_05007864.1, ZP_06836265.1, ZP_03934882.1, YP_001508477.1, ZP_06043756.1, ZP_05366306.1, YP_002835056.1, ZP_03933464.1, ZP_07469321.1, ZP_07713507.1, YP_005160553.1, NP_939820.1, AAU93794.1, ZP_14659796.1, ZP_14383679.1, YP_005058606.1, YP_001221073.1, ZP_08231568.1, YP_250920.1, ZP_11383249.1, YP_003916320.1, ZP_08681170.1, YP_001800249.1, YP_001157632.1, YP_166099.1, ZP_10088015.1, YP_004760065.1, ZP_07947675.1, YP_001603066.1, YP_003812683.1, YP_004403402.1, ZP_08292153.1, ZP_09471260.1, YP_004018108.1, ZP_05115352.1, AAD13565.1, ZP_09295321.1, YP_001535629.1, ZP_04607273.1, YP_006561753.1, ZP_00960958.1, YP_006571985.1, ZP_08862188.1, YP_002906426.1, CCK30433.1, ZP_13042493.1, ZP_09090153.1, YP_614397.1, ZP_11163860.1, YP_003983492.1, YP_004080668.1, ZP_09420475.1, ZP_05914565.1, ZP_01101149.1, ZP_14743088.1, YP_001239694.1, ZP_09127532.1, YP_003833873.1, ZP_08516197.1, ZP_10160483.1, ZP_01987188.1, ZP_01755304.1, ZP_08825027.1, ZP_05077116.1, YP_001444606.1, ZP_03392800.1, ZP_01057781.1, AFB69889.1, ZP_08815097.1 und AAO17175.1 und Varianten davon umfassen. In einer besonders bevorzugten Ausführungsform handelt es sich dabei um die Phosphopantetheinyl-Transferase mit dem Datenbankcode ABI83656.1 oder eine Variante davon.

Alternativ oder zusätzlich zur Kombination aus Fettsäurereduktase und Phosphopantetheinyl-Transferase kann der Ganzzellkatalysator auch eine α-Dioxygenase aufweisen. In einer bevorzugten Ausführungsform wird unter dem Begriff "α-Dioxygenase", wie hierin verwendet, ein Enzym verstanden, dass die Umwandlung einer Fettsäure unter Verbrauch eines Moleküls Sauerstoff und unter Abspaltung eines Kohlendioxid-Moleküls zu einer am endständigen ω-Kohlenstoffatom gegenüber der als Edukt eingesetzten Fettsäure um ein Kohlenstoffatom verkürzten, am endständigen ω-Kohlenstoffatom eine Aldehydgruppe tragenden Fettsäure katalysiert. In einer besonders bevorzugten Ausführungsform ist die α-Dioxygenase aus der Gruppe von α-Dioxygenasen ausgewählt, die die Aminosäuresequenzen NP_001066718.1, EAY82977.1, BAH79993.1, ABG22011.1, BAJ90503.1, AFD04418.1, AFD04417.1, BAJ87736.1, AFW75180.1, ABG22012.1, XP_002311389.1, CAH05011.1, XP_002279884.1, CBI34957.3, AAG59584.1, NP_001234414.1, NP_001234410.1, XP_003553942.1, XP_002275161.1, XP_003553937.1, CBI34960.3, CAA07589.1, XP_003543402.1, XP_002517402.1, XP_002882184.1, NP_186791.1, AAK85133.1, CAN77070.1, XP_002529555.1, CAH64542.1, NP_001234061.1, XP_002281357.1, ADM21465.1, XP_002318527.1, NP_177509.1, CAN74266.1, XP_002888940.1, NP_001185393.1, XP_003631072.1, BAJ33800.1, XP_002517377.1, XP_003530944.1, BAJ34623.1, ABG22013.1, ABP02610.1, XP_001773135.1, XP_002960339.1, ABK95279.1, ABD73303.1, ABD73304.1, YP_001805721.1, ZP_08971815.1, ZP_08430366.1, YP_823013.1, ZP_05026427.1, ZP_11003953.1, YP_007064484.1, YP_007113008.1, YP_633369.1, ZP_18906570.1, ZP_09251410.1, ZP_10050808.1, ZP_01306662.1, YP_001516886.1, ZP_05042862.1, AAC49625.1, ZP_09648375.1, ZP_09792714.1, ZP_09788527.1, XP_001728273.1, AAC83355.1, YP_890542.1, ZP_11000891.1, XP_002605323.1, EGO58341.1, YP_006249145.1, YP_001507004.1, YP_001704637.1, ZP_12876141.1, ZP_11150830.1, ZP_14236257.1, ZP_09411385.1, ZP_14243118.1, EKD16664.1, ZP_15416799.1, ZP_15338016.1, ZP_10080295.1, ZP_11438929.1, ZP_12995210.1, ZP_10946648.1, YP_003409541.1, XP_001637870.1, YP_005451221.1, XP_001212758.1, ZP_07290489.1, ZP_05781329.1, ZP_19187748.1, ZP_06574534.1, XP_002605322.1, NP_822950.1, YP_006366425.1, EJP63377.1, EKD21217.1, XP_001795927.1, XP_003042615.1, ZP_06566152.1, EGU88116.1, EFY94417.1, XP_388327.1, EKJ68934.1, ZP_07290463.1, CCC10458.1, YP_001107201.1, XP_003348248.1, T49753, CAD31840.1, XP_001229975.1, CBN77040.1, YP_004813753.1, XP_002513273.1, XP_001627136.1, AFG52858.1, AFG52857.1, AEW08450.1, NP_841291.1, YP_004512343.1, ACG75701.1 und ZP_03500906.1 und Varianten davon umfassen. In einer besonders bevorzugten Ausführungsform handelt es sich um die α-Dioxygenase mit dem Datenbankcode NP_001066718.1 oder eine Variante davon.

Neben der α-Dioxygenase oder der Kombination aus Fettsäurereduktase und der Phosphopantetheinyl-Transferase weist der erfindungsgemäße Ganzzellkatalysator notwendigerweise eine Transaminase auf, die die ω-Oxofettsäure aminiert. In einer bevorzugten Ausführungsform wird unter dem Begriff "Transaminase", wie hierin verwendet, ein Enzym verstanden, das die Übertragung von α-Aminogruppen von einem Donor-, bevorzugt einer Aminosäure, auf ein Akzeptormolekül, bevorzugt eine α-Ketocarbonsäure, katalysiert. In einer besonders bevorzugten Ausführungsform ist die Transaminase aus der Gruppe von Transaminasen ausgewählt, die die Aminosäuresequenzen 3HMU_A, AAD41041.1, AAK15486.1, ABE03917.1, ADR60699.1, ADR61066.1, ADR62525.1, AEL07495.1, CAZ86955.1, EFW82310.1, EFW87681.1, EGC99983.1, EGD03176.1, EGE58369.1, EGH06681.1, EGH08331.1, EGH24301.1, EGH32343.1, EGH46412.1, EGH55033.1, EGH62152.1, EGH67339.1, EGH70821.1, EGH71404.1, EGH78772.1, EGH85312.1, EGH97105.1, EGP57596.1, NP_102850.1, NP_106560.1, NP_248912.1, NP_248990.1, NP_354026.2, NP_421926.1, NP_637699.1, NP_642792.1, NP_744329.1, NP_744732.1, NP_747283.1, NP_795039.1, NP_901695.1 (, XP_002943905.1, YP_001021095.1, YP_001059677.1, YP_001061726.1, YP_001066961.1, YP_001074671.1, YP_001120907.1, YP_001140117.1, YP_001170616.1, YP_001185848.1, YP_001188121.1, YP_001233688.1, YP_001268866.1, YP_001270391.1, YP_001345703.1, YP_001412573.1, YP_001417624.1, YP_001526058.1, YP_001579295.1, YP_001581170.1, YP_001668026.1, YP_001669478.1, YP_001671460.1, YP_001685569.1, YP_001747156.1, YP_001749732.1, YP_001765463.1, YP_001766294.1, YP_001790770.1, YP_001808775.1, YP_001809596.1, YP_001859758.1, YP_001888405.1, YP_001903233.1, YP_001977571.1, YP_002229759.1, YP_002231363.1, YP_002280472.1, YP_002297678.1, YP_002543874.1, YP_002549011.1, YP_002796201.1, YP_002801960.1, YP_002875335.1, YP_002897523.1, YP_002912290.1, YP_002974935.1, YP_003060891.1, YP_003264235.1, YP_003552364.1, YP_003578319.1, YP_003591946.1, YP_003607814.1, YP_003641922.1, YP_003674025.1, YP_003692877.1, YP_003755112.1, YP_003896973.1, YP_003907026.1, YP_003912421.1, YP_004086766.1, YP_004142571.1, YP_004147141.1, YP_004228105.1, YP_004278247.1, YP_004305252.1, YP_004356916.1, YP_004361407.1, YP_004378186.1, YP_004379856.1, YP_004390782.1, YP_004472442.1, YP_004590892.1, YP_004612414.1, YP_004676537.1, YP_004693233.1, YP_004701580.1, YP_004701637.1, YP_004704442.1, YP_108931.1, YP_110490.1, YP_168667.1, YP_237931.1, YP_260624.1, YP_262985.1, YP_271307.1, YP_276987.1, YP_334171.1, YP_337172.1, YP_350660.1, YP_351134.1, YP_364386.1, YP_366340.1, YP_369710.1, YP_370582.1, YP_426342.1, YP_440141.1, YP_442361.1, YP_468848.1, YP_521636.1, YP_554363.1, YP_608454.1, YP_610700.1, YP_614980.1, YP_622254.1, YP_625753.1, YP_680590.1, YP_751687.1, YP_767071.1, YP_774090.1, YP_774932.1, YP_788372.1, YP_858562.1, YP_928515.1, YP_983084.1, YP_995622.1, ZP_00948889.1, ZP_00954344.1, ZP_00959736.1, ZP_00998881.1, ZP_01011725.1, ZP_01037109.1, ZP_01058030.1, ZP_01076707.1, ZP_01103959.1, ZP_01167926.1, ZP_01224713.1, ZP_01442907.1, ZP_01446892.1, ZP_01550953.1, ZP_01625518.1, ZP_01745731.1, ZP_01750280.1, ZP_01754305.1, ZP_01763880.1, ZP_01769626.1, ZP_01865961.1, ZP_01881393.1, ZP_01901558.1, ZP_02145337.1, ZP_02151268.1, ZP_02152332.1, ZP_02167267.1, ZP_02190082.1, ZP_02242934.1, ZP_02360937.1, ZP_02367056.1, ZP_02385477.1, ZP_02456487.1, ZP_02883670.1, ZP_03263915.1, ZP_03263990.1, ZP_03400081.1, ZP_03452573.1, ZP_03456092.1, ZP_03517291.1, ZP_03529055.1, ZP_03571515.1, ZP_03572809.1, ZP_03587785.1, ZP_03588560.1, ZP_03697266.1, ZP_03697962.1, ZP_04521092.1, ZP_04590693.1, ZP_04890914.1, ZP_04891982.1, ZP_04893793.1, ZP_04902131.1, ZP_04905327.1, ZP_04941068.1, ZP_04944536.1, ZP_04945255.1, ZP_04959332.1, ZP_04964181.1, ZP_05053721.1, ZP_05063588.1, ZP_05073059.1, ZP_05077806.1, ZP_05082750.1, ZP_05091128.1, ZP_05095488.1, ZP_05101701.1, ZP_05116783.1, ZP_05121836.1, ZP_05127756.1, ZP_05637806.1, ZP_05742087.1, ZP_05783548.1, ZP_05786246.1, ZP_05843149.1, ZP_05945960.1, ZP_06459045.1, ZP_06487195.1, ZP_06492453.1, ZP_06493162.1, ZP_06703644.1, ZP_06731146.1, ZP_06839371.1, ZP_07007312.1, ZP_07266194.1, ZP_07374050.1, ZP_07662787.1, ZP_07778196.1, ZP_07797983.1, ZP_08099459.1, ZP_08138203.1, ZP_08141719.1, ZP_08142973.1, ZP_08177102.1, ZP_08185821.1, ZP_08186468.1, ZP_08208888.1, ZP_08266590.1, ZP_08402041.1, ZP_08406891.1, ZP_08522175.1, ZP_08527488.1, ZP_08631252.1, ZP_08636687 und Varianten davon umfassen.

Bei der erfindungsgemäß verwendeten Fettsäurereduktase und bevorzugt auch bei anderen erfindungsgemäß verwendeten Enzymen handelt es sich um rekombinante Enzyme. In einer bevorzugten Ausführungsform wird unter dem Begriff "rekombinant", wie hierin verwendet, verstanden, dass das für das entsprechende Enzym kodierende Nukleinsäuremolekül in der natürlichen Zelle nicht vorkommt und/oder es unter Verwendung von gentechnischen Methoden hergestellt wurde. In einer bevorzugten Ausführungsform spricht man von einem rekombinanten Protein, wenn das entsprechende Polypeptid von einer rekombinanten Nukleinsäure kodiert ist. In einer bevorzugten Ausführungsform wird unter einer rekombinanten Zelle, wie hierin verwendet, eine Zelle verstanden, die wenigstens eine rekombinante Nukleinsäure oder ein rekombinantes Polypeptid aufweist. Dem Fachmann sind zum Herstellen rekombinanter Moleküle oder Zellen geeignete Verfahren bekannt, beispielsweise die in Sambrook/Fritsch/Maniatis (1989): Molecular Cloning: A Laboratory Manual, Cold Spring Harbor Laboratory Press, 2nd edition, beschriebenen. Rekombinante Enzyme werden bevorzugt überexprimiert, beispielsweise unter Verwendung von pET- oder pGEX-Vektor-Systemen, die dem Fachmann bekannt sind.

Mit Hinblick auf die Wahl des Organismus unterliegt der erfindungsgemäß einsetzbare Ganzzellkatalysator keinen Einschränkungen, sofern er kultivierbar, stabil und ggf. gentechnisch einführbaren Modifikationen, z. B. Verfahren zur Abschwächung von Enzymaktivitäten, beispielsweise Knock outs, zugänglich ist. So kann es sich gleichermaßen um eine prokaryontische oder eukaryontische Zelle handeln. Im Falle einer eukaryontischen Zelle sind unizelluläre Eukaryonten besonders bevorzugt, besonders Hefen wie *Saccharomyces cerevisiae, Candida tropicalis, Candida albicans* und *Pichia pastoris.* Im Falle von prokaryontischen Zellen kann es sich beispielsweise um ein Bakterium handeln, das aus der Gruppe ausgewählt ist, die *Magnetococcus, Mariprofundus, Acetobacter, Acetobacterium, Acidiphilium, Afipia, Ahrensia, Asticcacaulis, Aurantimonas, Azorhizobium, Azospirillum, Bacillus, Bartonella, tribocorum, Beijerinckia, Bradyrhizobium, Brevundimonas, subvibrioides, Brucella, Caulobacter, Chelativorans, Citreicella, Citromicrobium, Clostridium, Corynebacterium, Dinoroseobacter, Erythrobacter, Fulvimarina, Gluconacetobacter, Granulibacter, Hirschia, Hoeflea, Hyphomicrobium, Hyphomonas, Ketogulonicigenium, Labrenzia, Loktanella, Magnetospirillum, Maricaulis, Maritimibacter, Mesorhizobium, Methylobacterium, Methylocystis, Methylosinus, Nitrobacter, Novosphingobium, Oceanibulbus, Oceanicaulis, Oceanicola, Ochrobactrum, Octadecabacter, Oligotropha, Paracoccus, Parvibaculum, Parvularcula, Pelagibaca, Phaeobacter, Phenylobacterium, Polymorphum, Pseudovibrio, Rhodobacter, Rhodomicrobium, Rhodopseudomonas, Rhodospirillum, Roseibium, Roseobacter, Roseomonas, Roseovarius, Ruegeria, Sagittula, Silicibacter, Sphingobium, Sphingomonas, Sphingopyxis, Starkeya, Sulfitobacter, Thalassiobium, Xanthobacter, Zymomonas, Agrobacterium, Rhizobium, Sinorhizobium, Anaplasma, Ehrlichia, Neorickettsia, Orientia, Rickettsia, Wolbachia, Bordetella, Burkholderia, Cupriavidus, Taiwanensis, Lautropia, Limnobacter, Polynucleobacter, Ralstonia, Chromobacterium, Eikenella, corrodens, Basfia, Kingella, Laribacter, Lutiella, Neisseria, Simonsiella, Achromobacter, Acidovorax, Alicycliphilus, Aromatoleum, Azoarcus, Comamonas, Dechloromonas, Delftia, Gallionella, Herbaspirillum, Herminiimonas, Hylemonella, Janthinobacterium, Leptothrix, Methylibium, Methylobacillus, Methylophilales, Methyloversatilis, Methylovorus, Nitrosomonas, Nitrosospira, Oxalobacter, Parasutterella, Polaromonas, Polaromonas, Pusillimonas, Rhodoferax, Rubrivivax, Sideroxydans, Sutterella, wadsworthensis, Taylorella, Thauera, Thiobacillus, Thiomonas, Variovorax, Verminephrobacter, Anaeromyxobacter, Bdellovibrio, bacteriovorus, Bilophila, Desulfarculus, Desulfatibacillum, Desulfobacca, Desulfobacterium, Desulfobulbus, Desulfococcus, Desulfohalobium, Desulfitobacterium, Desulfomicrobium, Desulfonatronospira, Desulfotalea, Desulfovibrio, Desulfuromonas, Geobacter, Haliangium, Hippea, Lawsonia, Myxococcus, Pelobacter, Plesiocystis, Sorangium, Stigmatella, Syntrophobacter, Syntrophus, Arcobacter, Caminibacter, Campylobacter, Helicobacter, Nitratifractor, Nitratiruptor, Sulfuricurvum, Sulfurimonas, Sulfurospirillum, Sulfurovum, Wolinella, Buchnera, Blochmannia, Hamiltonella, Regiella, Riesia, Citrobacter, Cronobacter, Dickeya, Edwardsiella, Enterobacter, Erwinia, Escherichia, Klebsiella, Pantoea, Pectobacterium, Proteus, Providencia, Rahnella, Salmonella, Serratia, Shigella, Sodalis, Wigglesworthia, Glossina, Xenorhabdus, Yersinia, Acidithiobacillus, Acinetobacter, Aeromonas, Alcanivorax, Alkalilimnicola, Allochromatium, Alteromonadales, Alteromonas, Baumannia, Beggiatoa, Bermanella, Carsonella, Ruthia, Vesicomyosocius, Cardiobacterium, Chromohalobacter, Colwellia, Congregibacter, Coxiella, Dichelobacter, Endoriftia, Enhydrobacter, Ferrimonas, Francisella, Glaciecola, Hahella, Halomonas, Halorhodospira, Halothiobacillus, Idiomarina, Kangiella, Legionella, Marinobacter, Marinomonas, Methylobacter, Methylococcus, Methylomicrobium, Methylophaga, Moraxella, Moritella, Neptuniibacter, Nitrococcus, Pseudoalteromonas, Psychrobacter, Psychromonas, Reinekea, Rickettsiella, Saccharophagus, Shewanella, Succinatimonas, Teredinibacter, Thioalkalimicrobium, Thioalkalivibrio, Thiomicrospira, Tolumonas, Vibrionales, Actinobacillus, Aggregatibacter, Gallibacterium, Haemophilus, Histophilus, Mannheimia, Pasteurella, Azotobacter, Cellvibrio, Pseudomonas, Aliivibrio, Grimontia, Photobacterium, Photobacterium, Vibrio, Pseudoxanthomonas, Stenotrophomonas, Xanthomonas, Xylella, Borrelia, Brachyspira, Leptospira, Spirochaeta, Treponema, Hodgkinia, Puniceispirillum, Liberibacter, Pelagibacter, Odyssella, Accumulibacter,* insbesondere *B. subtilis, B. megaterium, C. glutamicum, E. coli, Pseudomonas sp., Pseudomonas fluorescens, Pseudomonas putida, Pseudomonas stutzeri" Acinetobacter* sp., *Burkholderia* sp., *Burkholderia thailandensis,* Cyanobakterien, *Klebsiella* sp., *Klebsiella oxytoca, Salmonella sp., Rhizobium sp.* und *Rhizobium meliloti,* umfasst. In einer besonders bevorzugten Ausführungsform handelt es sich um ein Enterobakterium, am bevorzugtesten um *Escherichia coli.*

Es ist vorteilhaft, wenn der erfindungsgemäße Ganzzellkatalysator neben der Fettsäurereduktase, Phosphopantetheinyl-Transferase und der Transaminase auch eine Alanindehydrogenase aufweist, um das von der Transaminase bei der Aminierung der ω-Oxofettsäure verbrauchte Alanin aus anorganischen stickstoffhaltigen Molekülen zu regenerieren. In einer bevorzugten Ausführungsform wird unter dem Begriff "Alanindehydrogenase", wie hierein verwendet, ein Enzym verstanden, das die Umwandlung von L-Alanin unter Verbrauch von Wasser und NAD⁺ zu Pyruvat, Ammoniak und NADH und die umgekehrte Reaktion katalysiert. In einer besonders bevorzugten Ausführungsform ist die Alanindehydrogenase aus der Gruppe von Alanindehydrogenasen ausgewählt, die die Aminosäuresequenz der Alanindehydrogenase aus *Bacillus subtilis* (Datenbankcode L20916), *Rhizobium leguminosarum* (Datenbankcode CP001622), *Vibrio proteolyticus* (Datenbankcode AF070716), *Mycobacterium tuberculosis* (Datenbankcode X63069), *Enterobacter aerogenes* (Datenbankcode AB013821), EGR93259.1, YP_003654745.1, YP_003651439.1, YP_003637111.1, YP_003631815.1, YP_001327051.1, YP_001262560.1, YP_886996.1, YP_882850.1, YP_704410.1, YP_703508.1, ZP_08624689.1, YP_001230376.1, P17557.1, P17556.1, CCB94892.1, CCB73698.1, YP_001168635.1, YP_004668736.1, YP_004569425.1, YP_003513168.1, YP_004561169.1, ZP_08554945.1, YP_400777.1, ZP_08311476.1, ZP_08310170.1, ZP_08267322.1, ZP_08263846.1, ZP_07898723.1, YP_149301.1, YP_148605.1, YP_004340432.1, EFT09946.1, EFS80513.1, EFS51332.1, EFS42459.1, YP_003060895.1, YP_003059033.1, ZP_03305373.1, YP_847214.1, YP_004095847.1, YP_003338282.1, YP_003337256.1, YP_355846.1, YP_253131.1, ZP_08197563.1, ZP_08196283.1, ADW06447.1, YP_734091.1, NP_372233.1, NP_102173.1, ZP_08170259.1, EGD36706.1, EGD32748.1, ZP_08155540.1, YP_004142849.1, YP_002417649.1, YP_001301040.1, YP_002992892.1, YP_081348.1, YP_080482.1, YP_002476349.1, ZP_08115025.1, ZP_08114403.1, YP_003552869.1, YP_002358112.1, YP_575010.1, YP_477594.1, YP_474564.1, YP_130399.1, YP_129373.1, YP_123314.1, NP_810467.1, NP_646469.1, NP_626044.1, NP_391071.1, ZP_08086822.1, ZP_08084776.1, ZP_08083119.1, ZP_08020768.1, ZP_08013590.1, ZP_08011832.1, YP_003783744.1, YP_002781576.1, YP_002780533.1, ZP_02195873.1, NP_797482.1, ZP_07645051.1, ZP_07643260.1, ZP_06611917.1, AAT40119.1, ZP_07864946.1, YP_004068409.1, YP_002796203.1, YP_002774420.1, YP_003600348.1, YP_003599946.1, YP_003565624.1, YP_003565223.1, YP_335198.1, YP_423850.1, YP_155059.1, ZP_07843538.1, ZP_07841226.1, ZP_06928932.1, ZP_05692073.1, ZP_05687006.1, ZP_04867480.1, YP_775531.1, CBE70214.1, ZP_07721182.1, ZP_04302850.1, ZP_04298961.1, ZP_04287684.1, ZP_04277177.1, ZP_04248389.1, ZP_04235899.1, ZP_02159718.1, ZP_02152178.1, YP_003974610.1, YP_003546595.1, YP_002317127.1, ZP_07313778.1, ZP_07302778.1, ZP_07298850.1, CBK69442.1, YP_003413835.1, YP_003595089.1, ZP_ 06807811.1, YP_003582455.1, YP_003464731.1, YP_003496397.1, YP_003421918.1, CBL07274.1, CBK64956.1, YP_003508515.1, AAL87460.1, AAC23579.1, AAC23578.1, AAC23577.1, ACU78652.1, YP_003471439.1, YP_003452777.1, ZP_06384971.1, ACY25368.1, ABC26869.1, AAP44334.1, EEZ80018.1, ZP_05110458.1, 1PJB_A, ZP_04717201.1, ZP_04689103.1, CAO90307.1, CAM75354.1, CAA44791.1, BAA77513.1, EGR96638.1, EGL90046.1, YP_004510847.1, ZP_08450330.1, YP_003387804.1, YP_003058152.1, EFS74272.1, EFS67128.1, ZP_06844564.1, YP_826658.1, YP_001195249.1, YP_003095978.1, YP_469292.1, YP_004442054.1, YP_004461174.1, YP_004055616.1, YP_003576656.1, YP_003094537.1, YP_001295973.1, AEE71143.1, YP_004447480.1, YP_003761844.1, YP_040853.1, YP_003154888.1, YP_003142045.1, YP_002280953.1, NP_371963.1, NP_422368.1, EGC98966.1, EGC76398.1, YP_004263661.1, YP_004252039.1, YP_679036.1, YP_499973.1, ZP_08054972.1, ZP_08053009.1, ZP_04067276.1, ZP_03968868.1, ZP_03963857.1, ZP_03933079.1, ZP_03497046.1, ZP_06668924.1, ZP_06667106.1, ZP_06324464.1, ZP_06196777.1, ZP_05114159.1, ZP_05083968.1, ZP_05070370.1, ZP_05030022.1, ZP_04673064.1, ZP_03517011.1, ZP_03505783.1, XP_001310698.1, ABK27691.1 und CAB59281.2 und Varianten davon umfassen. Für die von der Alanindehydrogenase katalysierte Reaktion ist die Anwesenheit nicht nur von Pyruvat, das als Teil des Primärstoffwechsels von jeder als Ganzzellkatalysator in Frage kommenden Zelle gebildet wird, erforderlich, sondern auch von Ammonium. Letzteres wird typischerweise in Form von anorganischen Stickstoffsalzen, beispielsweise Ammoniumsalzen, Nitraten o. ä. bereitgestellt. Bevorzugt wird dem wässrigen Reaktionsmedium ein Ammoniumsalz zugesetzt, z.B. Ammoniumchlorid.

Weiterhin ist es vorteilhaft, wenn der erfindungsgemäße Ganzzellkatalysator eine Alkanhydroxylase und optional weitere, für die Aktivität der Alkanhydroxylase essentielle Enzyme exprimiert, insbesondere für den Fall, dass als Substrate zur Herstellung der ω-Aminofettsäure eine Fettsäure mit einer Oxidationsstufe am endständigen ω-Kohlenstoffatom eingesetzt wird, die unterhalb der Oxidationsstufe des Aldehyds liegt. Die Alkanhydroxylase und/oder eine zusätzlich exprimierte Alkoholdehydrogenase oxidieren das endständige Kohlenstoffatom dann bis zur Aldehydgruppe, die anschließend von der Transaminase aminiert werden kann. In einer bevorzugten Ausführungsform wird unter dem Begriff "Alkanhydroxylase", wie hierin verwendet, ein Enzym verstanden, das die Hydroxylierung von unsubstituierten linearen Alkylresten umfassend wenigstens sechs, bevorzugt zwölf Kohlenstoffstoffreste katalysiert.

Als Alkanhydroxylasen sind erfindungsgemäß zahlreiche Oxidationssysteme geeignet, wie sie u. a. in der PCT/EP 2008/067447 beschrieben sind. In einer bevorzugten Ausführungsform handelt es sich bei der Alkanhydroxylase um eine Cytochrom P450-Monooxygenase der CYP153-Familie. In einer bevorzugten Ausführungsform wird unter dem Begriff "Cytochrom P450-Monooxygenase der CYP153-Familie" eine cytosolische Oxidase verstanden, die Teil eines 3 Komponenten-Systems ist, das weiterhin ein Ferredoxin und eine Ferredoxin-Reduktase umfasst, mit einer Alkan-Bindestelle und der Fähigkeit, Alkane zu hydroxylieren. In einer besonders bevorzugten Ausführungsform handelt es sich um ein Enzym, das zu wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist oder um ein Enzym, das eine Polypeptidsequenz umfasst, die wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist und darüber hinaus Alkanhydroxylase-Aktivität aufweist. Die genannten Datenbankcodes beziehen sich dabei, wie durchgehend in dieser Anmeldung, auf die NCBI (National Center for Biotechnology Information, Bethesda, USA)-Datenbanken, genauer gesagt die am 21. November 2012 online verfügbare Version. In einer bevorzugten Ausführungsform wird unter dem Begriff "Cytochrom P450-Monooxygenase der CYP153-Familie" eine nicht membrangebundene Oxidase verstanden, die eine Bindestelle für Alkane, unsubstituierte lineare Alkylreste umfassend wenigstens fünf, bevorzugt zwölf Kohlenstoffstoffreste oder einfach hydroxylierte Alkane und deren Polypeptidkette das Motiv LL(I/L)(V/I)GGNDTTRN umfasst. In einer bevorzugten Ausführungsform handelt es sich bei einer "Cytochrom P450-Monooxygenase der CYP153-Familie", wie hierin verwendet, um eine Cytochrom P450-Monooxygenase der CYP153-Familie aus *Alcanivorax borkumensis* SK2 (Datenbankcode YP_691921) oder eine Variante davon, die bevorzugt Alkanhydroxylaseaktivität aufweist.

Zur optimalen Versorgung der Cytochrom P450-Monooxygenase der CYP153-Familie mit Elektronen aus dem Reduktionsmittel, bevorzugt NADH, ist es bevorzugt, dass die Zelle die Monooxygenase zusammen mit funktionell mit ihr wechselwirkender Ferredoxin-Reduktase und funktionell mit ihr wechselwirkendem Ferredoxin exprimiert wird. Dabei kann es sich um isolierte oder bei der Verwendung eines Ganzzellkatalysators um co-exprimierte Polypeptide oder um N-oder C-terminal mit der Cytochrom P450-Monooxygenase der CYP153-Familie fusionierte Polypeptide handeln. Ob eine Ferredoxin-Reduktase oder ein Ferredoxin mit einer gegebenen Cytochrom P450-Monooxygenase der CYP153-Familie mit einander funktionell wechselwirken, kann der Fachmann leicht dadurch feststellen, ob das Reduktionsmittel in Gegenwart eines Alkansubstrates und der drei Polypeptide effizienter als für den Fall, dass wenigstens eines der drei fehlt, oxidiert wird. Alternativ kann der von Scheps, D., Malca, H., Hoffmann, B., Nestl, B. M, und Hauer, B. (2011) Org. Biomol. Chem., 9, 6727 beschriebene Enzymtest verwendet werden, der im Fall funktionell wechselwirkender Polypeptide eine deutliche Erhöhung der Reaktionsgeschwindigkeit zeigt. In einer besonders bevorzugten Ausführungsform stammen die Cytochrom P450-Monooxygenase der CYP153-Familie, das Ferredoxin und die Ferredoxin-Reduktase aus dem gleichen Organismus. In einer besonders bevorzugten Ausführungsform handelt es sich um die Ferredoxin-Reduktase aus *Alcanivorax borkumensis* SK2 (Datenbankcode YP_691923) oder eine Variante davon, das Ferredoxin aus *Alcanivorax borkumensis* SK2 (Datenbankcode YP_691920) oder eine Variante davon und die Cytochrom P450-Monooxygenase der CYP153-Familie aus *Alcanivorax borkumensis* SK2 (Datenbankcode YP_691921) oder eine Variante davon.

In einer weiteren bevorzugten Ausführungsform handelt es sich bei der Alkanhydroxylase um eine AlkB-Monooxygenase. AlkB stellt eine zunächst aus dem AlkBGT-System aus *Pseudomonas putida* Gpo1 bekannt gewordene Oxidoreduktase dar, die von zwei weiteren Polypeptiden, AlkG und AlkT, abhängig ist. AlkT wird als FAD-abhängige Rubredoxin-Reduktase charakterisiert, die Elektronen aus NADH an AlkG weitergibt. Bei AlkG handelt es sich um ein Rubredoxin, ein eisenhaltiges Redoxprotein, das als direkter Elektronendonor für AlkB fungiert. In einer bevorzugten Ausführungsform wird unter dem Begriff "AlkB-Monooxygenase" ein Polypeptid mit einer Sequenzhomologie von wenigstens, in der Reihenfolge zunehmender Bevorzugung angegeben, 75, 80, 85, 90, 92, 94, 96, 98 oder 99 % zur Sequenz des AlkB von *Pseudomonas putida* Gpo1 (Datenbankcode: CAB54050.1; dieser Datenbankcode stammt wie alle weiteren in der Anmeldung verwendeten aus dem Stand der Technik, nämlich aus der NCBI Datenbank, genauer dem am 15. Oktober 2012 online verfügbaren Release) mit der Fähigkeit, Alkane zu oxidieren, verstanden. In einer besonders bevorzugten Ausführungsform handelt es sich bei der AlkB-Monooxygenase um eine mit den AlkG (CAB54052.1)- und AlkT (CAB54063.1)-Polypeptiden aus *Pseudomonas putida* Gpo1 funktionell zusammenwirkende, Alkane oxidierende Oxidoreduktase. Zur optimalen Versorgung der AlkB-Alkanhydroxylase mit Elektronen ist es bevorzugt, dass die Zelle die Monooxygenase zusammen mit funktionell mit ihr wechselwirkenden Hilfsproteinen, bevorzugt AlkG und/oder AlkT oder jeweils Varianten davon exprimiert wird, wobei es sich in einer besonders bevorzugten Ausführungsform wiederum um AlkG (CAB54052.1)- und AlkT (CAB54063.1)-Polypeptide aus *Pseudomonas putida* Gpo1 handelt.

Bei der Verwendung eines Ganzzellkatalysators kann sich das Problem stellen, dass ein Substrat mit einem intrazellulär lokalisierten Enzym in Kontakt gebracht werden muss, damit es zur erwünschten Reaktion kommt. Im Falle langkettiger Alkane und Derivate davon ist bevorzugt, dass der Ganzzellkatalysator ein Polypeptid der AlkL-Familie aufweist. AlkL ist ein Membranprotein aus *Pseudomonas putida,* das langkettige Fettsäuren und Derivate davon in bakterielle Zellen importieren kann. In einer bevorzugten Ausführungsform handelt es sich bei einem "Polypeptid der AlkL-Familie", wie hierin verwendet, um ein Polypeptid, das über eine Länge von 230 aufeinander abfolgenden Aminosäuren eine wenigstens 80, bevorzugt 90, noch bevorzugter 90%ige Sequenzidentität zu AlkL aus *Pseudomonas putida* (Datenbankcode CAB69081) oder eine Variante von AlkL aus *Pseudomonas putida* und bevorzugt die Fähigkeit aufweist, den Import von langkettigen Alkanen ins Innere einer Zelle zu unterstützen. In einer weiteren Ausführungsform handelt es sich bei einem "Polypeptid der AlkL-Familie", wie hierin verwendet, um eine in der äußeren Membran eines Gram-negativen Bakteriums lokalisiertes Polypeptid, welches das Sequenzmotiv DXWAPAXQ(V/A)GXR, aufweist, wobei X eine proteinogene Aminosäure darstellt, und bevorzugt zusätzlich AlkL aus *Pseudomonas putida* (Datenbankcode CAB69081) oder eine Variante davon ist. Beispielhafte Mitglieder der AlkL-Familie umfassen AlkL aus *Pseudomonas putida* (Datenbankcode CAB69081), *Marinobacter aquaeolei* VT8 (Datenbankcode YP_957722), *Oceanicaulis alexandrii* HTCC2633 (Datenbankcode ZP_00953584), *Marinobacter manganoxydans* MnI7-9 (Datenbankcode ZP_09158756), *Caulobacter* sp. K31 (Datenbankcode YP_001672217), *Pseudomonas oleovorans* (Datenbankcode Q00595) und Varianten davon.

Die Lehre der vorliegenden Erfindung kann nicht nur unter Verwendung von Makromolekülen mit der exakten Aminosäure- oder Nukleinsäuresequenz, auf die hierin Bezug genommen wird, bzw. nicht nur unter Verwendung von einer Zelle mit relativ zum jeweiligen Wildtyp verringerter Aktivität eines Polypeptides mit der exakten Aminosäuresequenz, auf die hierin Bezug genommen wird, ausgeführt werden, sondern auch unter Verwendung einer Variante derartiger Makromoleküle oder von einer Zelle mit einer relativ zum jeweiligen Wildtyp der jeweiligen Zelle verringerten Aktivität einer Variante des Polypeptids, die durch Deletion, Addition oder Substitution einer oder mehr als einer Aminosäure oder Nukleinsäure erhalten werden kann. In einer bevorzugten Ausführungsform bedeutet der Begriff "Variante" einer Nukleinsäuresequenz oder Aminosäuresequenz, im Folgenden gleichbedeutend und austauschbar mit dem Begriff "Homologon" gebraucht, wie hierin verwendet, eine andere Nukleinsäure- oder Aminosäuresequenz, die eine Sequenz umfasst oder darstellt, welche mit Hinblick auf die entsprechende ursprüngliche Wildtyp-Nukleinsäure- oder -aminosäuresequenz eine Homologie, hier gleichbedeutend mit Identität verwendet, von 70, 75, 80, 85, 90, 92, 94, 96, 98, 99 % oder mehr Prozent aufweist, wobei bevorzugt andere als die das katalytisch aktive Zentrum ausbildende Aminosäuren oder für die Struktur oder Faltung essentielle Aminosäuren deletiert oder substituiert sind oder solche lediglich konservativ substituiert sind, beispielsweise ein Glutamat statt einem Aspartat oder ein Leucin statt einem Valin. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3rd edition. In einer weiteren bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche enzymatische Aktivität des Wildtypmoleküls bzw. des ursprünglichen Moleküls auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. In einer besonderen Ausführungsform bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, bevorzugter 2, noch bevorzugter eine Größenordnung von den K_{M}- und/oder k_{cat}-Werten unterscheidet, die das Wildtyppolypeptid mit Hinblick auf die gleichen Substrate aufweist. In einer weiteren bevorzugten Ausführungsform umfasst der Begriff "Variante" einer Nukleinsäure- oder Aminosäuresequenz wenigstens einen aktiven Teil/oder Fragment der Nukleinsäure- bzw. Aminosäuresequenz. In einer weiteren bevorzugten Ausführungsform bedeutet der Begriff "aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist, beispielsweise als Protease. In einer besonderen Ausführungsform umfasst der Begriff "Variante" einer Nukleinsäure eine Nukleinsäure, deren komplementärer Strang, bevorzugt unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Proben mit geringen Temperaturen auskommen. Ob Hybridisierung stattfindet, hängt im Allgemeinen von der Fähigkeit der denaturierten DNA ab, an komplementäre Stränge zu annellieren, die in ihrer Umgebung vorhanden sind, und zwar unterhalb der Schmelztemperatur. Die Stringenz von Hybridisierungsreaktion und entsprechende Bedingungen sind ausführlicher in F M Ausubel (1995), Current Protocols in Molecular Biology. John Wiley & Sons, Inc. beschrieben. Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet in einer bevorzugten Ausführungsform unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996). Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von in der Reihenfolge zunehmender Bevorzugung ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2 x SSC oder 0,1 x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise in der Reihenfolge zunehmender Bevorzugung mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz des eingesetzten Nukleinsäuremoleküls aufweisen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). In einer bevorzugten Ausführungsform umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

In einer bevorzugten Ausführungsform weist die erfindungsgemäß verwendete Zelle eine ihrem Wildtyp gegenüber verringerte Aktivität wenigstens eines Enzyms aufweist, das eine der Reaktionen der β-Oxidation von Fettsäuren katalysiert, wobei es sich bevorzugt um ein Enzym aus der Gruppe handelt, die Fettsäure-CoA-Ligase, Acyl-CoA-Dehydrogenase, 2,4-Dienoyl-CoA-Reduktase, Enoyl-CoA-Hydratase and 3-Ketoacyl-CoA-Thiolase, einen Fettsäureimporter oder Varianten davon umfasst. Die β-Oxidation von Fettsäuren ist ein weit verbreiteter Stoffwechselweg, der es prokaryontischen und eukaryontischen Organismen gleichermaßen erlaubt, Fettsäuren zu oxidieren und die darin enthaltene chemische Energie dem Stoffwechsel verfügbar zu machen. Im weiteren Sinn beginnt sie mit der Aufnahme einer Fettsäure in die Zelle. Dort wird die Fettsäure, sofern die Bedingungen es erfordern, zunächst an der β-Position des CoA-Fettsäureesters durch eine Acyl-CoA-Dehydrogenase, im Falle von *E. coli* FadE, oxidiert. Ein ähnliches Molekül kann alternativ auch aus einer doppelt ungesättigten Fettsäure durch Reduktion mittels einer 2,4-dienoyl-CoA-Reduktase, bei *E. coli* FadH, gebildet werden. Ein multifunktionelles Enzym, die Enoyl-CoA-Hydratase/3-Hydroxyacyl-CoA-Dehydrogenase, bei *E. coli* FadB, katalysiert anschließend die Hydratisierung unter Bildung des sekundären Alkohols und dessen anschließende Oxidation zum Keton. Im letzten Schritt katalysiert eine 3-Ketoacyl-CoA-Thiolase, im Falle von *E. coli* FadA, die Spaltung des Ketoacyl-CoA mit dem Ergebnis, dass Acetyl-CoA und ein im Vergleich zum Ausgangsmolekül um zwei Kohlenstoffatome verkürzter CoA-Ester der Fettsäure freigesetzt werden. Sofern es sich nicht ebenfalls um Acetyl-CoA handelt, kann letzterer erneut in den β-Oxidationszyklus eingespeist und unter Oxidation verkürzt werden. An der Regulation der β-Oxidation von Fettsäuren ist auch FadR beteiligt, ein Regulator des Fad-Operons, der die für den Abbau von Fettsäuren erforderlichen Gene umfasst, ohne dass FadR eine Reaktion der β-Oxidation katalysieren würde. In einer bevorzugten Ausführungsform wird unter dem Begriff "Enzym, das eine der Reaktionen der β-Oxidation von Fettsäuren katalysiert" ein jegliches Enzym verstanden, das direkt mit dem Fettsäuresubstrat oder einem auf dem Weg zum Acetyl-CoA daraus entstehenden Molekül wechselwirkt, bevorzugt es als Substrat erkennt, und seine Umwandlung zu einem auf diesem Abbauweg näher am Acetyl-CoA liegenden Stoffwechselprodukt katalysiert, bevorzugt einschließlich des Fettsäureimporters, der die Aufnahme der Fettsäure in die Zelle bewerkstelligt. Beispielsweise zählt zu diesen Enzymen nach der vorangegangenen Definition die Acyl-CoA-Dehydrogenase, da sie mit dem Fettsäure-CoA-Ester wechselwirkt und dessen Umwandlung zum Enyol-CoA katalysiert, das auf dem Stoffwechselweg der β-Oxidation näher am Acetyl-CoA liegt als der Fettsäure-CoA-Ester. In einer besonders bevorzugten Ausführungsform wird unter dem Begriff "Enzym, das eine der Reaktionen der β-Oxidation von Fettsäuren katalysiert", wie hierin verwendet, jedes Enzym aus der Gruppe verstanden, die die Genprodukte FadA, FadB, FadD, FadL und FadE aus *E*. *coli* und/oder deren Varianten oder Homologa aus anderen Organismen umfasst. Die Genprodukte FadA, FadB, FadD, FadL und FadE aus *E*. *coli* ebenso wie Varianten und Homologa aus zahlreichen weiteren biotechnologisch nutzbaren Organismen und ihre Nukleinsäure- und Polypeptidssequenzen sind im Stand der Technik beschrieben, beispielsweise FadA unter Zugangsnummer AP009048.1, FadB unter Zugangsnummer BAE77457.1, FadD unter Zugangsnummer BAA15609.1, und FadE unter Zugangsnummer BAA77891.2. Der Stand der Technik offenbart zahlreiche Tests, die speziell für die Messung der Aktivität von Enzymen geeignet sind, die eine der Reaktionen der β-Oxidation von Fettsäuren katalysieren, beispielsweise in K Kameda & W D Nunn (1981) J. Biol. Chem. 256, 5702-5707, Hi Marrakchi, W E DeWolf, C Quinn, J West, B J Polizzi, C Y So et al. (2003) Biochem. J. 370, 1055-1062, Lobo *et al.* (2001) und X Yu, T Liu, F Zhu, and C Khosla (2011) PNAS, elektronische Veröffentlichung vor Drucklegung*.*

Für die Effektivität des erfindungsgemäßen Ganzzellkatalysators ist es von Vorteil, wenn das umzusetzende Substrat, bevorzugt die Fettsäure, ω-Hydroxy- oder ω-Oxo-Fettsäure, leicht mit den erfindungsgemäß erforderlichen Enzymen, die im Inneren des Ganzzellkatalysators lokalisiert sind, in Kontakt treten kann. Entscheidend ist daher, dass das Substrat ins Innere der Zelle gelangen kann. Um dies zu erleichtern, ist bevorzugt, dass der Ganzzellkatalysator einen Fettsäureimporter, im Falle eines bakteriellen, insbesondere Gram-negativen Ganzzellkatalysators, besonders bevorzugt den Fettsäureimporter FadL (Datenbankcode: BAA16205.1) oder eine Variante exprimiert, bevorzugt in einer Konzentration und mit einer Aktivität, die gegenüber der Aktivität des Wildtyps des entsprechenden Ganzzellkatalysators erhöht ist. Der Erhöhung der Aktivität eines Polypeptids gegenüber dem Wildtyp der Zelle kann über verschiedene, dem Fachmann routinemäßig zugängliche Wege erreicht werden, beispielsweise den Einbau zusätzlicher, funktionell mit einem Promotor verbundener Kopien der für das Polypeptid kodierenden Nukleotidsequenz oder den Austausch des natürlichen gegen einen stärkeren Promotor.

Es hat sich gezeigt, dass die ω-Aminofettsäuren erfindungsgemäß in höherer Ausbeute und Reinheit produziert werden, wenn der Hintergrund an im Ganzzellkatalysator endogen exprimierten Enzymen derart optimiert ist, dass die Aktivität von endogenen Enzymen verringert oder ausgeschaltet ist, die Edukte, Zwischenprodukte oder Produkte des erfindungsgemäßen Verfahrens oder unter Verwendung der erfindungsgemäßen Zelle, bevorzugt ω-Aminofettsäuren, auf Stoffwechselwegen abbauen oder anderweitig modifizieren, die von der Entstehung des erwünschten Produktes wegführen. Vor diesem Hintergrund ist es vorteilhaft, wenn es sich bei dem erfindungsgemäßen Ganzzellkatalysator um eine Zelle handelt, die ihrem Wildtyp gegenüber eine verringerte Aktivität der Esterase BioH [Datenbankcode YP_492020.1] oder einer Variante davon aufweist. Derartige Zellen mit verringerter BioH-Aktivität, ihre Herstellung und Tests zur Aktivitätsbestimmung sind in der Europäischen Patentanmeldung EP 12007663.3 beschrieben.

Der erfindungsgemäße Ganzzellkatalysator kann in einem Verfahren zur Umsetzung von einer Fettsäure, ω-Hydroxy- oder ω-Oxo-Fettsäure zum entsprechenden Amin, bevorzugt einer ω-Aminofettsäure, eingesetzt werden, wobei es sich bei der Fettsäure, ω-Hydroxy-Fettsäure, ω-Oxo-Fettsäure oder dem Monoester davon um eine Verbindung der Formel (I)

R¹ - A - COOR² (I)

handelt, wobei R¹ aus der Gruppe ausgewählt ist, die -H, -CHO, -OH und COOR³ umfasst, wobei R² und R³ jeweils und unabhängig voneinander aus der Gruppe ausgewählt sind, die H, Methyl, Ethyl und Propyl umfasst, mit der Maßgabe, dass wenigstens einer der Reste R² und R³ H ist, wobei A eine unverzweigte, verzweigte, lineare, zyklische, substituierte oder unsubstituierte Kohlenwasserstoffgruppe mit wenigstens vier Kohlenstoffatomen darstellt. In einer bevorzugten Ausführungsform handelt es sich bei A um eine Struktur der Formel -(CH₂)ₙ-, wobei n 4, 5, 6, 7, 8, 9, 10, 11, 12, 13, 14, 15, 16, 17, 18, 19, 20, 21, 22, 23, 24, 25, 26, 27, 28, 29 oder 30 ist. In einer bevorzugtesten Ausführungsform handelt es sich bei der Fettsäure, ω-Hydroxy- oder ω-Oxo-Fettsäure um Laurinsäure, ω-Hydroxy- bzw. ω-Oxo-Laurinsäure. In einer weiteren bevorzugtesten Ausführungsform handelt es sich bei der Fettsäure, ω-Hydroxy- oder ω-Oxo-Fettsäure um Hexansäure, ω-Hydroxy- bzw. ω-Oxo-Hexansäure. In einer weiteren bevorzugtesten Ausführungsform handelt es sich bei der Fettsäure, ω-Hydroxy- oder ω-Oxo-Fettsäure um Decansäure, ω-Hydroxy- bzw. ω-Oxo-Decansäure.

Mit Hinblick auf die Fettsäure wie auf jede in dieser Anmeldung beschriebene chemische Verbindung gilt, dass die jeweilige angegebene Formel sämtliche Salze, protonierte oder deprotonierte der jeweiligen Verbindung umfasst. Beispielsweise umfasst die Laurinsäure nicht nur die protonierte Form, sondern auch das Salz Laureat mit sämtlichen Kationen, beispielsweise Natriumlaureat.

Das erfindungsgemäße Verfahren erfordert, dass die für das erfindungsgemäße Verfahren eingesetzten Enzyme, optional in Form des erfindungsgemäßen Ganzzellkatalysators bereitgestellt, mit Fettsäure, ω-Hydroxy- oder ω-Oxo-Fettsäure in einer wässrigen Lösung kontaktiert werden. In einer bevorzugten Ausführungsform wird unter dem Begriff "Kontaktieren", wie hierin verwendet, verstanden, dass das jeweilige Enzym in unmittelbaren Kontakt mit seinem Substrat gelangt, insbesondere ohne dass physikalische Barrieren wie impermeable Membranen oder dergleichen zwischengeschaltet sind. Das Kontaktieren geschieht im einfachsten Fall dadurch, dass das Substrat zu einer wässrigen Lösung gegeben wird, in der sich das Enzym oder der Ganzzellkatalysator befindet.

Zur Ausführung der erfindungsgemäßen Lehre eignet sich eine Reaktionsmischung umfassend den erfindungsgemäßen Ganzzellkatalysator in wässriger Lösung sowie eine Fettsäure, ω-Hydroxy-Fettsäure, ω-Oxo-Fettsäure oder einen Monoester davon der Formel (I), wobei R¹ aus der Gruppe ausgewählt ist, die -H, -CHO, -OH und COOR³ umfasst, wobei R² und R³ jeweils und unabhängig voneinander aus der Gruppe ausgewählt sind, die H, Methyl, Ethyl und Propyl umfasst, mit der Maßgabe, dass wenigstens einer der Reste R² und R³ H ist, wobei A eine unverzweigte, verzweigte, lineare, zyklische, substituierte oder unsubstituierte Kohlenwasserstoffgruppe mit wenigstens vier Kohlenstoffatomen darstellt, bevorzugt die Formel - (CH₂)ₙ -, wobei n wenigstens 4, besonders bevorzugt wenigstens 10 ist. Die wässrige Lösung muss, beispielsweise mit Hinblick auf Zusammensetzung, pH-Wert und Temperatur, dabei derart beschaffen sein, dass sie die Lebensfähigkeit oder zumindest die katalytische Fähigkeit des Ganzzellkatalysators wenigstens zeitweilig unterstützt. Dem Fachmann sind zahlreiche als wässrige Lösung geeignete wässrige Kulturmedien bekannt, die für die Erhaltung oder Kultivierung von Zellen, insbesondere biotechnologisch bedeutsamer Zellen, geeignet sind. Darunter fallen gleichermaßen Vollmedien wie LB-Medien, Minimalmedien wie M9-Medien sowie Selektivmedien, beispielsweise solche, die eine hohe Salzkonzentration enthalten und daher nur das Wachstum halophiler oder zumindest halotoleranter Organismen ermöglichen. In einer bevorzugten Ausführungsform wird unter dem Begriff "wässriges Kulturmedium", wie hierin verwendet, ein Reaktionsmedium auf Wasserbasis verstanden, das mit Hinblick auf sämtliche relevante Faktoren, insbesondere pH-Wert, Salzgehalt und Temperatur, derart beschaffen ist, das es die Lebensfähigkeit darin enthaltener Zellen, bevorzugt Mikroorganismen, erhält oder fördert und sowohl wässriges Kulturmedium als auch hydrophobe organische Phase in flüssiger Form vorliegen. Die Temperaturansprüche verschiedener biotechnologisch bedeutsamer Zellen können mikrobiologischen und molekularbiologischen Lehrbüchern entnommen werden, z.B. Fuchs/Schlegl, 2008. In einer bevorzugten Ausführungsform liegt der pH-Wert des wässrigen Kulturmediums zum Zeitpunkt des Kontaktierens zwischen 4 bis 9, bevorzugter zwischen 4,5 bis 8,5, am bevorzugtesten zwischen 6,5 und 7,5. In einer weiteren bevorzugten Ausführungsform liegt die Temperatur zwischen 0 und 45 °C, bevorzugter zwischen 15 und 40 °C, am bevorzugtesten zwischen 20 und 37 °C. Die Reaktionsmischung ist typischerweise in einem Fermenter enthalten. Als Fermenter kann ein jedes Reaktionsgefäß fungieren, das sterilisiert, bevorzugt autoklaviert werden kann und die Anzucht des Ganzzellkatalysators, Belüftung und Steuerung der Reaktionsbedingungen, beispielsweise des Sauerstoffgehalts und der Temperatur erlaubt.

In einer bevorzugten Ausführungsform umfasst die Reaktionsmischung zusätzlich zur wässrigen Lösung eine hydrophobe organische Phase. Diese kann ein organisches Lösungsmittel und/oder einen hydrophoben flüssigen Kationenaustauscher zur Entfernung der ω-Aminofettsäure aus der wässrigen Lösung umfassen. Geeignete Lösungsmittel und Kationenaustauscher sind in der EP11191520.3 beschrieben.

Die vorliegende Erfindung wird weiterhin durch die folgenden Figuren und nicht beschränkenden Beispiele veranschaulicht, denen weitere Merkmale, Ausführungsformen, Aspekte und Vorteile der vorliegenden Erfindung entnommen werden können.

### Beispiel 1

### Herstellung von Expressionsvektoren für Co-Expression von Genen codierend für eine Fettsäurereduktase mit npt aus Nocardia sp.

Zur Herstellung von Vektoren für die Co-Expression der Gene codierend für eine Fettsäurereduktase mit *npt* (SEQ ID Nr. 1, kodiert für ABI83656.1) aus *Nocardia sp.,* welches für eine Phosphopantetheinyltransferase kodiert, wurden die Gene für die Expression in *Escherichia coli* codonoptimiert und zusammen mit einem lacuv5-Promotor (SEQ ID Nr. 2) synthetisiert und gleichzeitig je eine Restriktionsschnittstelle stromaufwärts und stromabwärts eingeführt. Folgende Fettsäurereduktase-Gene wurden eingesetzt:
- MSMEG_2956 aus *Mycobacterium smegmatis* (carA, SEQ ID Nr. 3, kodiert für YP_887275.1)
- MSMEG_5739 aus *Mycobacterium smegmatis* (carB, SEQ ID Nr. 4, kodiert für YP_889972.1)
- fadD9 aus *Mycobacterium intracellulare* MOTT-64 (car_Mint, SEQ ID Nr. 5, kodiert für YP_005349252.1)
- MFORT_07381 aus *Mycobacterium fortuitum subsp. fortuitum* DSM 46621 (car_Mfort, SEQ ID Nr. 6, kodiert für ZP_11001941.1)
- FadD9 aus *Mycobacterium avium subsp. paratuberculosis* K-10 (car_Mavi, SEQ ID Nr. 7, kodiert für NP_959974.1)
- CPCC7001_1320 aus *Cyanobium sp.* PCC 7001 (car_Cs, SEQ ID Nr. 8, kodiert für ZP_05045132.1)
- MAB_3367 aus *Mycobacterium abscessus* ATCC 19977 (car_Mab, SEQ ID Nr. 9, kodiert für YP_001704097.1)
- FadD9 aus *Nocardia brasiliensis* ATCC 700358 (car_nbr, SEQ ID Nr. 10, kodiert für ZP_09839660.1)
- MIP_06852 aus *Mycobacterium indicus pranii* MTCC 9506 (car_Mip, SEQ ID Nr. 11, kodiert für YP_006731697.1)
- nfa20150 aus *Nocardia farcinica* IFM 10152 (car_nfa, SEQ ID Nr. 12, kodiert für YP_118225.1)
- MUL_1722 aus *Mycobacterium ulcerans* Agy99 (car_Mul, SEQ ID Nr. 13, kodiert für YP_905678.1)
- Saci8_010100039603 aus *Streptomyces acidiscabies* 84-104 (car_Sac, SEQ ID Nr. 14, kodiert für ZP_10456477.1)
- MCOL_V203220 aus *Mycobacterium colombiense* CECT 3035 (car_Mcol, SEQ ID Nr. 15, kodiert für WP_007769435.1)

Das synthetisierte DNA-Fragment wurde mit den Restriktionsendonukleasen *Nde*I und *Avr*II verdaut und in den entsprechend geschnittenen Vektor pSC101 (SEQ ID Nr. 16) ligiert. Der Vektor pSC101 ist ein very low copy Vektor, der Tetrazyklin-Resistenz vermittelt sowie eine niedrige Kopienzahl von ca. 4 Kopien pro Zelle aufweist. Auf diese Weise konnten folgende Expressionsvektoren hergestellt wurden:
- pSC101{Placuv5}[carA_Ms(co_Ec)-npt_Noc(co_Ec)] (SEQ ID Nr. 17)
- pSC101{Placuv5}[carB_Ms(co_Ec)-npt_Noc(co_Ec)] (SEQ ID Nr. 18)
- pSC101{Placuv5}[car_Mint(co_Ec)-npt_Noc(co_Ec)] (SEQ ID Nr. 19)
- pSC101{Placuv5}[car_Mfort(co_Ec)-npt_Noc(co_Ec)] (SEQ ID Nr. 20)
- pSC101{Placuv5}[car_Mavi(co_Ec)-npt_Noc(co_Ec)] (SEQ ID Nr. 21)
- pSC101{Placuv5}[car-Csp(co_Ec)-npt_Noc(co-Ec)] (SEQ ID Nr. 22)
- pSC101{Placuv5}[car_Mab(co_Ec)-npt_Noc(co_Ec)] (SEQ ID Nr. 23)
- pSC101{Placuv5}[car_nbr(co_Ec)-npt_Noc(co_Ec)] (SEQ ID Nr. 24)
- pSC101{Placuv5}[car_Mip(co_Ec)-npt_Noc(co_Ec)] (SEQ ID Nr. 25)
- pSC101{Placuv5}[car_nfa(co_Ec)-npt_Noc(co_Ec)] (SEQ ID Nr. 26)
- pSC101{Placuv5}[car_Mul(co_Ec)-npt_Noc(co_Ec)] (SEQ ID Nr. 27)
- pSC101{Placuv5}[car_Sac(co_Ec)-npt_Noc(co_Ec)] (SEQ ID Nr. 28)
- pSC101{Placuv5}[car_Mcol(co_Ec)-npt_Noc(co_Ec)] (SEQ ID Nr. 29)

Das Genprodukt *npt* überträgt zur Aktivierung der Fettsäurereduktase einen Phosphopantetheinyl-Rest von Coenzym A auf das Fettsäurereduktase-Enzym.

### Beispiel 2

### Herstellung von E.coli-Stämmen mit Deletion im Gen bioH mit verstärkter Fettsäurereduktase- und Phosphopantetheinyltransferase-Aktivität für die Herstellung von Aminolaurinsäuremethylester aus Laurinsäuremethylester

Zur Erzeugung von *E.coli*-Stämmen mit Deletion des Gens *bioH,* welches für eine Esterase kodiert, und verstärkter Fettsäurereduktase-Aktivität zur Herstellung von Aminolaurinsäuremethylester wurde der Stamm *Escherichia coli* W3110 ΔbioH (Herstellung siehe EP12007663) mit den Plasmiden pBT10_alkL (Sequenz und Herstellung vergleiche Beispiel 1 der WO/2011/131420 bzw. die dort aufgeführte Seq ID NR: 8), pJ294_alaDH_B.s._TA_C.v.(Ct) (vergleiche Beispiel 1 der WO/2013/024114 und die dort aufgeführte SEQ ID Nr. 17) und einem der in Beispiel 1 erzeugten Vektoren für die Expression einer Fettsäurereduktase mittels Elektroporation transformiert und auf LB-Agar-Platten mit Kanamycin (50 µg/ml), Ampicillin (100 µg/ml) und Tetrazyklin (5 µg/ml) ausplattiert. Transformanten wurden durch Plasmidpräparation und Restriktionsanalyse bezüglich der Präsenz der korrekten Plasmide überprüft. So wurden folgende Stämme erzeugt:
- *E.coli* W3110 ΔbioH pBT10_alkL / pJ294_alaDH_B.s._TA_C.v.(Ct) / pSC101{Placuv5}[carA_Ms(co_Ec)-npt_Noc(co_Ec)]
- *E.coli* W3110 ΔbioH pBT10_alkL / pJ294_alaDH_B.s._TA_C.v.(Ct) / pSC101{Placuv5}[carB_Ms(co_Ec)-npt_Noc(co_Ec)]
- *E.coli* W3110 ΔbioH pBT10_alkL / pJ294_alaDH_B.s._TA_C.v.(Ct) / pSC101{Placuv5}[car_Mint(co_Ec)-npt_Noc(co_Ec)]
- *E.coli* W3110 ΔbioH pBT10_alkL / pJ294_alaDH_B.s._TA_C.v.(Ct) / pSC101{Placuv5}[car_Mfort(co_Ec)-npt_Noc(co_Ec)]
- *E.coli* W3110 ΔbioH pBT10_alkL / pJ294_alaDH_B.s._TA_C.v.(Ct) / pSC101{Placuv5}[car_Mavi(co_Ec)-npt_Noc(co_Ec)]
- *E.coli* W3110 ΔbioH pBT10_alkL / pJ294_alaDH_B.s._TA_C.v.(Ct) / pSC101{Placuv5}[car_Csp(co_Ec)-npt_Noc(co_Ec)]
- *E.coli* W3110 ΔbioH pBT10_alkL / pJ294_alaDH_B.s._TA_C.v.(Ct) / pSC101{Placuv5}[car_Mab(co_Ec)-npt_Noc(co_Ec)]
- *E.coli* W3110 ΔbioH pBT10_alkL / pJ294_alaDH_B.s._TA_C.v.(Ct) / pSC101{Placuv5}[car_nbr(co_Ec)-npt_Noc(co_Ec)]
- *E.coli* W3110 ΔbioH pBT10_alkL / pJ294_alaDH_B.s._TA_C.v.(Ct) / pSC101{Placuv5}[car_Mip(co_Ec)-npt_Noc(co_Ec)]
- *E.coli* W3110 ΔbioH pBT10_alkL / pJ294_alaDH_B.s._TA_C.v.(Ct) / pSC101{Placuv5}[car_nfa(co_Ec)-npt_Noc(co_Ec)]
- *E.coli* W3110 ΔbioH pBT10_alkL / pJ294_alaDH_B.s._TA_C.v.(Ct) / pSC101{Placuv5}[car_Mul(co_Ec)-npt_Noc(co_Ec)]
- *E.coli* W3110 ΔbioH pBT10_alkL / pJ294_alaDH_B.s._TA_C.v.(Ct) / pSC101{Placuv5}[car_Sac(co_Ec)-npt_Noc(co_Ec)]
- *E.coli* W3110 ΔbioH pBT10_alkL / pJ294_alaDH_B.s._TA_C.v.(Ct) / pSC101{Placuv5}[car_Mcol(co_Ec)-npt_Noc(co_Ec)]

Diese Stämme wurden verwendet, um ihre Fähigkeit zur Produktion von Aminolaurinsäuremethylester ausgehend von Laurinsäuremethylester zu untersuchen.

Der Expressionsvektor pBT10_alkL enthält die Gene *alkB, alkG*, *alkT*, *alkS* und *alkL* aus dem alk-Operon von *Pseudomonas putida.* Durch die Genprodukte wird die Oxidation des Substrates Laurinsäuremethylester über Hydroxylaurinsäuremethylester zu Oxolaurinsäuremethylester katalysiert. Der Vektor pJ294_alaDH_B.s_TA_C.v.(Ct) enthält die Gene *ald* aus *Bacillus subtilis* (kodierend für eine Alanindehydrogenase, NP_391071.1) und Cv_2505 aus *Chromobacterium violaceum* (kodierend für eine ω-Transaminase, NP_901695.1). Das Genprodukt Cv_2505 ist dazu in der Lage Oxolaurinsäuremethylester zu Aminolaurinsäuremethylester umzusetzen, der dazu benötigte Aminodonor Alanin wird von dem Genprodukt *ald* aus Pyruvat zur Verfügung gestellt. Anhand einer zusätzlich exprimierten Fettsäurereduktase, die durch die ebenfalls überexprimierte Phosphopantetheinyltransferase *npt* aktiviert wurde, sollen bei der Produktion von Aminolaurinsäuremethylester entstehende Nebenprodukte, insbesondere Dodecandisäuremethylester, reduziert werden um das Verhältnis von Aminolaurinsäuremethylester zu Dodecandisäuremethylester zu erhöhen.

### Beispiel 3

### Herstellung von E.coli-Stämmen mit verstärkter Fettsäurereduktase- und Phosphopantetheinyltransferase-Aktivität für die Herstellung von Aminolaurinsäuremethylester aus Laurinsäuremethylester

Der verwendete Wirtsstamm *E.coli* W3110 wurde mit den Expressionsvektoren pBT10_alkL (Sequenz und Herstellung vergleiche Beispiel 1 der WO/2011/131420 bzw. die dort aufgeführte Seq ID NR: 8), pJ294_alaDH_B.s._TA_C.v.(Ct) (vergleiche Beispiel 1 der WO/2013/024114 und die dort aufgeführte SEQ ID Nr. 17) und pSC101{Placuv5}[carB_Ms(co_Ec)-npt_Noc(co_Ec)] oder pSC101, Leervektor, mittels Elektroporation transformiert und auf LB-Agar-Platten mit Kanamycin (50 µg/ml), Ampicillin (100 µg/ml) und Tetrazyklin (5 µg/ml) ausplattiert. Transformanten wurden durch Plasmidpräparation und Restriktionsanalyse bezüglich der Präsenz der korrekten Plasmide überprüft. So wurden die Stämme *E.coli* W3110 pBT10_alkL / pJ294_alaDH_B.s_TA_C.v.(Ct) / pSC101{Placuv5}[carB_Ms(co_Ec)-npt_Noc(co_Ec)] und *E.coli* W3110 pBT10_alkL / pJ294_alaDH_B.s._TA_C.v.(Ct) / pSC101 erzeugt.

### Beispiel 4

### Produktion von Aminolaurinsäure durch E.coli Stämme mit einem Expressionsvektor für ein Fettsäurereduktase-Gen und das Gen npt aus Nocardia sp. und Expressionsvektoren für die Gene ald aus Bacillus subtilis, Cv_2025 aus Chromobacterium violaceum in Kombination mit einem Expressionsvektor für die Gene alkB, alkG, alkT und alkL aus dem alk-Operon von Pseudomonas putida.

Die in Beispiel 2 und 3 erzeugten Stämme wurden verwendet, um ihre Fähigkeit zur Produktion von Aminolaurinsäuremethylester zu untersuchen.

Die Biotransformation von Laurinsäuremethylester zu Aminolaurinsäuremethylester wurde im 8-fach Parallelfermentationssystem von DASGIP durchgeführt. Dabei wurde wie folgt vorgegangen:

Für die Fermentation wurden 1L-Reaktoren verwendet. Die pH-Sonden wurden mittels einer Zwei-Punkt-Kalibration mit Maßlösungen von pH 4,0 und pH 7,0 kalibriert. Die Reaktoren wurden mit 300 mL Trinkwasser befüllt und 20 min bei 121°C autoklaviert, um Sterilität zu gewährleisten. Anschließend wurden die pO2-Sonden über Nacht (mindestens 6 h lang) am DASGIP-System polarisiert. Am nächsten Morgen wurde das Wasser unter der Clean Bench entnommen und durch 300 mL Hochzelldichtemedium mit 100 mg/L Ampicillin, 50 mg/L Kanamycin und 5 mg/L Tetrazyklin ersetzt. Im Folgenden wurden die pO2-Sonden mit einer Einpunkt-Kalibration (Rührer: 400 rpm / Begasung: 10 sL/h Luft) kalibriert und die Feed-, Korrekturmittel- und Induktionsmittelstrecken mittels Clean-in-Place gereinigt. Dazu wurden die Schläuche mit 70% Ethanol, anschließend mit 1 M NaOH, dann mit sterilem VE-Wasser gespült und zuletzt mit den jeweiligen Medien befüllt.

Die ALS und ALSME produzierenden *E*. *coli-*Stämme wurden zuerst aus den jeweiligen Cryokulturen in LB-Medium (25 mL in einem 100 mL Schikanekolben) mit 100 mg/L Ampicillin über Nacht bei 37°C und 200 rpm ca. 18 h lang angezogen. Anschließend wurden je 2 mL der Kulturen in Hochzelldichtemedium (Glucose 15 g/L (30 mL / L einer separat autoklavierten 500 g/L Stammlösung mit 1% MgSO₄*7H₂O und 2,2% NH₄Cl), (NH₄)₂SO4 1,76 g/L, K₂HPO₄ 19,08 g/L, KH₂PO₄ 12,5 g/L, Hefeextrakt 6,66 g/L, Trinatriumcitrat-Dihydrat 2,24 g/L, Ammoniumeisencitrat-Lösung 17 mL/L einer separat autoklavierten 1% igen Stammlösung, Spurenelementlösung 5 mL/L separat autoklavierten Stammlösung (HCl (37 %) 36,50 g/L, MnCl₂*4H₂O 1,91 g/L, ZnSO₄*7H₂O 1,87 g/L, Ethylendiamintetraessigsäure-Dihydrat 0,84 g/L, H₃BO₃ 0,30 g/L. Na₂MoO₄*2H₂O 0,25 g/L, CaCl₂*2H₂O 4,70 g/L, FeSO₄*7H₂O 17,80 g/L, CuCl₂*2H₂O 0,15 g/L)) (Je Stamm 25mL in einem 100 mL Schikanekolben) mit 100 mg/L Ampicillin, 50 mg/L Kanamycin und 5 mg/L Tetrazyklin überimpft und bei 37°C / 200 rpm weitere 5,5 h lang inkubiert.

Die Reaktoren wurden mit einer optischen Dichte von 0,1 angeimpft indem ein entsprechendes Volumen der Vorkultur in einer 5 mL Spritze (unter sterilen Bedingungen) aufgezogen wurde und die Reaktoren mittels Kanüle über ein mit 70% Ethanol überschichtetes Septum beimpft wurde.

Folgendes Standardprogramm wurde verwendet:

| DO-Regler | | pH-Regler | |
|---|---|---|---|
| Preset | 0% | Preset | 0 ml/h |
| P | 0,1 | P | 5 |
| Ti | 300 s | Ti | 200 s |
| Min | 0% | Min | 0 mlL/h |
| Max | 100% | Max | 40 mL/h |

| N (Rotation) | von | bis | XO2 (Gasmischung) | von | bis | F (Gasfluss) | von | bis |
|---|---|---|---|---|---|---|---|---|
| Wachstum und Biotransformation | 0% | 30% | Wachstum und Biotransformation | 0% | 100% | Wachstum und Biotransformation | 15% | 80% |
| | 400 rpm | 1500 rpm | | 21 % | 21% | | 6 sL/h | 72 sL/h |

| Script | |
|---|---|
| Trigger scharf | 31% DO (1/60h) |
| Induktion IPTG | 2 h nach Feedstart |
| Feedtrigger | 50% DO |
| Feedrate | 3 [mL/h] |

Das durchgeführte Experiment lässt sich in zwei Phasen gliedern, die Anzucht, bei der die Zellen eine bestimmte optische Dichte erreichen sollen, und die nachfolgende Biotransformation, in der nach Zugabe des Substrates Laurinsäuremethylester eine Umsetzung zu Aminolaurinsäureester von in der Expression gebildeten Enzyme stattfinden soll. Die pH-Werte wurden einseitig mit Ammoniak (12,5 %) auf pH 6,8 geregelt. Während Anzucht und Biotransformation wurde der gelöste Sauerstoff (DO, dissolved oxygen) in der Kultur über Rührerdrehzahl und Begasungsrate bei 30% geregelt. Die Fermentation wurde als Fed-Batch durchgeführt, wobei der Feedstart, 5 g/Lh Glucosefeed (500 g/L Glucose mit 1% MgSO₄*7H₂O und 2,2% NH₄Cl), über einen DO-Peak getriggert wurde. Mit Feedstart wurde auch die Temperatur von vorher 37°C auf 30°C gesenkt. Die Expression der Transaminase, Alanindehydrogenase und Fettsäurereduktase wurde 2 h nach Feedstart durch die automatische Zugabe von IPTG (1 mM) induziert. Die Induktion der alk-Gene erfolgte durch die manuelle Zugabe von DCPK (0,025 % v/v) 10 h nach Feedstart. Vor Start der Biotransformation wurde die optische Dichte der Kulturbrühen bestimmt.

Der Start der Biotransformationsphase erfolgte 14 h nach Feedstart. Dazu wurden 150 mL eines Gemisches aus Laurinsäuremethylesther und dem Ionentauscher Ölsäure (techn. 90%) als Batch zu der Fermentationsbrühe zugegeben. Um einen Aminogruppendonor für die Transaminase zur Verfügung zu stellen, wurde eine halbe Stunde vor Biotransformationsstart 5 mL einer 3M Ammoniumsulfatlösung zu der Fermentationsbrühe zugegeben. Zur Probennahme wurden 2 mL Fermentationsbrühe aus dem Kessel entnommen und ein Teil davon 1/20 in einem Aceton-HCl-Gemisch (c(HCl) = 0,1 mol/L) verdünnt und extrahiert. Proben wurden bei 1 h, 2 h, 3 h, 4 h, 5 h, 7.5 h, 10.5 h, 19.5 h und 21 h nach Start der Biotransformation von allen Reaktoren genommen. Die Umsatzraten für Sauerstoff (OTR = oxygen transfer rate) und Kohlenstoff (CTR = carbon transfer rate) wurden während der Fermentation über die Abgasanalytik an den DASGIP-Systemen bestimmt. Die Fermentation wurde 21 h nach Start der Biotransformation beendet. Der Rührer, die Begasung, die Temperaturregelung und pH-Regelung wurden ausgestellt und die Kessel 5-10 Minuten ruhig stehen gelassen.

Zur Quantifizierung von DDS (C12-Di-Carbonsäure), DDSME (C12-Di-Carbonsäure-Metylester), LS (Laurinsäure), LSME (Laurinsäure-Methylester), HLS (omega-Hydroxy-Laurinsäure), HLSME(omega-Hydroxy-Laurinsäure-Methylester), OLS (omega-Oxo-Laurinsäure), OLSME OLS (omega-Oxo-Laurinsäure-Methylester), ALS (omega-Amino-Laurinsäure)und ALSME (omega-Amino-Laurinsäure-Methylester) in Fermentationsproben wurden während der Kultivierung Proben entnommen. Diese Proben wurden für die Analytik vorbereitet. (siehe LC-ESI/MS²-basierte Quantifizierung von Produkten).

### LC-ESI/MS²-basierte Quantifizierung von Produkten

Die Quantifizierung von ALS, ALSME, DDS, DDSME, LS, LSME, HLS, HLSME, OLS und OLSME in Fermentationsproben erfolgte mittels LC-ESI/MS² anhand einer externen Kalibrierung für alle Analyten (0,1 - 50 mg/L) und unter Verwendung der internen Standards Aminoundekansäure (AUD für HLS, DDS, OLS, HLSME, OLSME), d4-ALSME (für ALSME), ¹³C-DDSME (für DDSME), d3-LS (für LS) und d3-LSME (für LSME).

Dabei kommen folgende Geräte zum Einsatz:
- HPLC Anlage 1260 (Agilent; Böblingen) mit Autosampler (G1367E), binärer Pumpe (G1312B) und Säulenofen (G1316A)
- Massenspektrometer TripelQuad 6410 (Agilent; Böblingen) mit ESI-Quelle
- HPLC-Säule: Kinetex C18, 100 x 2,1 mm, Partikelgröße: 2,6 µm, Porengröße 100 Å (Phenomenex; Aschaffenburg)
- Vorsäule: KrudKatcher Ultra HPLC In-Line Filter; 0,5 µm Filtertiefe und 0,004 mm Innendurchmesser (Phenomenex; Aschaffenburg)

Die Proben wurden vorbereitet, indem 1900 µL Lösungsmittel (80% (v/v) ACN, 20% bidest. H₂O (v/v), + 0.1% Ameisensäure) und 100 µL Probe in ein 2-mL-Reaktionsgefäß pipettiert wurden. Das Gemisch wurde ca. 10 Sekunden gevortext und anschließend bei ca. 13000 rpm für 5 min zentrifugiert. Der klare Überstand wurde mit einer Pipette entnommen und nach entsprechender Verdünnung mit Diluent (80% (v/v) ACN, 20% bidest. H₂O (v/v), + 0.1% Ameisensäure) analysiert. Zu je 900 µL Probe wurden 100 µL ISTD hinzupipettiert (10 µL bei einem Probenvolumen von 90 µL).

Die HPLC-Trennung erfolgte mit oben genannter Säule bzw. Vorsäule. Das Injektionsvolumen beträgt 0,7 µL, die Säulentemperatur 50°C, die Flussrate 0,6 mL/min. Die mobile Phase besteht aus Eluent A (0,1 %ige (v/v) wässrige Ameisensäure) und Eluent B (Acetonitril mit 0,1 % (v/v) Ameisensäure). Folgendes Gradientenprofil wurde genutzt:

| **Zeit [min]** | **Eluent A [%]** | **Eluent B [%]** |
|---|---|---|
| 0 | 77 | 23 |
| 0.3 | 77 | 23 |
| 0.4 | 40 | 60 |
| 2.5 | 40 | 60 |
| 2.6 | 2 | 98 |
| 5.5 | 2 | 98 |
| 5.6 | 77 | 23 |
| 9 | 77 | 23 |

Die ESI-MS²-Analyse erfolgte im positiven Modus mit folgenden Parametern der ESI-Quelle:
- Gastemperatur 280°C
- Gasfluss 11 L/min
- Nebulizerdruck 50 psi
- Kapillarspannung 4000 V

Die Detektion und Quantifizierung der Verbindungen ALS, ALSME, DDS, DDSME, HLS, HLSME, OLS, OLSME erfolgte mit den folgenden MRM Parametern, wobei jeweils ein Produkt-Ion als *Qualifier* und eines als *Quantifier* genutzt wurde:

| **Analyt** | **Vorläufer-Ion [m/z]** | **Produkt-Ion [m/z]** | **Verweilzeit [ms]** | **Kollisionsenergie [eV]** |
|---|---|---|---|---|
| DDSME | 245,2 | 167,1 | 25 | 6 |
| DDSME | 245,2 | 149,1 | 50 | 8 |
| HLSME | 231,3 | 181,2 | 15 | 2 |
| HLSME | 231,3 | 163,2 | 25 | 5 |
| DDS | 231,2 | 213,2 | 50 | 0 |
| DDS | 231,2 | 149,1 | 25 | 9 |
| ALSME | 230,3 | 198,1 | 25 | 10 |
| ALSME | 230,3 | 163,2 | 15 | 10 |
| OLSME | 229,2 | 197,2 | 50 | 0 |
| OLSME | 229,2 | 161,1 | 25 | 5 |
| HLS | 217,2 | 181,2 | 35 | 0 |
| HLS | 217,2 | 163,1 | 20 | 4 |
| OLS | 215,2 | 161,2 | 25 | 0 |
| OLS | 215,2 | 95,2 | 60 | 13 |

Die Analyten LS und LSME wurden im SIM Modus detektiert (m/z 201 und 215).

Es wurde gezeigt, dass die Stämme in der Lage sind aus Laurinsäuremethylester über die Zwischenstufen Hydroxylaurinsäuremethylester und Oxolaurinsäuremethylester Aminolaurinsäuremethylester herzustellen und dabei gebildete Nebenprodukte wie Dodecandisäuremethylester und Dodecandisäure mit Hilfe einer Fettsäurereduktase-Aktivität zu reduzieren und somit wieder in den Stoffwechsel zur Bildung von Aminolaurinsäuremethylester einzubringen (Tab. 1 und 2). Es wurde weiterhin gezeigt, dass durch die Einbringung der verschiedenen Fettsäurereduktasen das Verhältnis von gewünschten Produkten (Aminolaurinsäuremethylester und Aminolaurinsäure) zu Nebenprodukten (Dodecandisäuremethylester und Dodecandisäure) erhöht wurde (Tab. 1 und 2). Es wurde auch gezeigt, dass durch die Einbringung der verschiedenen Fettsäurereduktasen, die finale Produktkonzentration von Aminolaurinsäuremethylester erhöht wurde (Tab. 1 und 2). Schließlich wurde gezeigt, dass durch die Einbringung einer Fettsäurereduktase die Raum-ZeitAusbeute der Aminolaurinsäuremethylester-Bildung (gemessen zwischen 1 und 19 h nach Beginn der Biotransformation) erhöht und der zugehörige produktspezifische Glukoseverbrauch verringert wurde (Tab. 2).

**Tab. 1: Produkt- und Nebenproduktbildung der in Beispiel 2 generierten Stämme mit überexprimierter Fettsäurereduktase und dem Gen npt aus Nocardia sp. relativ zu dem Stamm ohne Fettsäurereduktase-Aktivität.**

| **Stamm** | relative ALSME-Bildung [%] | relative DDSME-Bildung [%] | Verhältnis ALSME/DDSME |
|---|---|---|---|
| *E.coli* W3110 ΔbioH pBT10_alkL / pJ294_alaDH_B.s._TA_C.v.(Ct) | 100 | 100 | 4,5 |
| *E.coli* W3110 ΔbioH pBT10_alkL / pJ294_alaDH_B.s._TA_C.v.(Ct) / pSC101{Placuv5}[carA_Ms(co_Ec)-npt_Noc(co_Ec)] | 103 | 12 | 38,8 |
| *E.coli* W3110 ΔbioH pBT10_alkL / pJ294_alaDH_B.s._TA_C.v.(Ct) / pSC101{Placuv5}[carB_Ms(co_Ec)-npt_Noc(co_Ec)] | 105 | 26 | 18,9 |
| *E.coli* W3110 ΔbioH pBT10_alkL / pJ294_alaDH_B.s._TA_C.v.(Ct) / pSC101{Placuv5}[car_Mint(co_Ec)-npt_Noc(co_Ec)] | 106 | 27 | 17,7 |
| *E.coli* W3110 ΔbioH pBT10_alkL / pJ294_alaDH_B.s._TA_C.v.(Ct) / pSC101{Placuv5}[car_Mfort(co_Ec)-npt_Noc(co_Ec)] | 103 | 37 | 12,3 |
| *E.coli* W3110 ΔbioH pBT10_alkL / pJ294_alaDH_B.s._TA_C.v.(Ct) / pSC101{Placuv5}[car_Mavi(co_Ec)-npt_Noc(co_Ec)] | 112 | 21 | 23,3 |
| *E.coli* W3110 ΔbioH pBT10_alkL / pJ294_alaDH_B.s._TA_C.v.(Ct) / pSC101 {Placuv5}[car_Csp(co_Ec)-npt_Noc(co_Ec)] | 101 | 87 | 5,2 |
| *E.coli* W3110 ΔbioH pBT10_alkL / pJ294_alaDH_B.s._TA_C.v.(Ct) / pSC101{Placuv5}[car_Mab(co_Ec)-npt_Noc(co_Ec)] | 111 | 20 | 24,9 |
| *E.coli* W3110 ΔbioH pBT10_alkL / pJ294_alaDH_B.s._TA_C.v.(Ct) / pSC101{Placuv5}[car_nbr(co_Ec)-npt_Noc(co_Ec)] | 109 | 24 | 20,4 |
| *E.coli* W3110 ΔbioH pBT10_alkL / pJ294_alaDH_B.s._TA_C.v.(Ct) / pSC101{Placuv5}[car_Mip(co_Ec)-npt_Noc(co_Ec)] | 101 | 66 | 6,8 |
| *E.coli* W3110 ΔbioH pBT10_alkL / pJ294_alaDH_B.s._TA_C.v.(Ct) / pSC101{Placuv5}[car_nfa(co_Ec)-npt_Noc(co_Ec)] | 110 | 25 | 19,6 |
| *E.coli* W3110 ΔbioH pBT10_alkL / pJ294_alaDH_B.s._TA_C.v.(Ct) / pSC101{Placuv5}[car_Mul(co_Ec)-npt_Noc(co_Ec)] | 107 | 35 | 13,6 |
| *E.coli* W3110 ΔbioH pBT10_alkL / pJ294_alaDH_B.s._TA_C.v.(Ct) / pSC101{Placuv5}[car_Sac(co_Ec)-npt_Noc(co_Ec)] | 102 | 87 | 5,3 |
| *E.coli* W3110 ΔbioH pBT10_alkL / pJ294_alaDH_B.s._TA_C.v.(Ct) / pSC101{Placuv5}[car_Mcol(co_Ec)-npt_Noc(co_Ec)] | 107 | 31 | 15,4 |

**Tab. 2: Produkt- und Nebenproduktbildung, Produktbildungsrate und Ausbeute durch den in Beispiel 3 generierten Stamm, der eine Fettsäurereduktase und das Gen npt aus Nocardia sp. überexprimiert relativ zu dem Stamm ohne Fettsäurereduktase-Aktivität.**

| Stamm | relative ALS(ME)-Bildung [%] | relative DDS(ME)-Bildung [%] | Verhältnis ALS(ME)/ DDS(ME) | relative Raum-ZeitAusbeute [%] | relative Ausbeute ALS(ME)/ Glucose [%] |
|---|---|---|---|---|---|
| *E.coli* W3110 pBT10_alkL / pJ294_alaDH_B.s._TA_C.v.(Ct) / pSC101{Placuv5}[carB_Ms(co_Ec)-npt_Noc(co_Ec)] | 121 | 40 | 37,02 | 133 | 123 |
| *E.coli* W3110 pBT10_alkL / pJ294_alaDH_B.s._TA_C.v.(Ct) / pSC101 | 100 | 100 | 12,15 | 100 | 100 |

## Patentansprüche

1. Ganzzellkatalysator, der eine rekombinante α-Dioxygenase oder die Kombination aus einer rekombinanten Fettsäurereduktase und aus einer die Fettsäurereduktase phosphopantetheinylierenden Phosphopantetheinyl-Transferase exprimiert, und der zusätzlich eine Transaminase exprimiert, wobei die Phosphopantetheinyl-Transferase und/oder Transaminase bevorzugt rekombinant ist.

2. Ganzzellkatalysator nach Anspruch 2, wobei der Ganzzellkatalysator zusätzlich eine Aminosäuredehydrogenase exprimiert, die bevorzugt rekombinant ist.

3. Ganzzellkatalysator nach einem der Ansprüche 1 bis 2, wobei der Ganzzellkatalysator zusätzlich eine Alkanhydroxylase exprimiert, die bevorzugt rekombinant ist.

4. Ganzzellkatalysator nach einem der Ansprüche 1 bis 3, wobei der Ganzzellkatalysator zusätzlich ein Polypeptid der AlkL-Familie exprimiert, das bevorzugt rekombinant ist.

5. Ganzzellkatalysator nach einem der Ansprüche 1 bis 4, der zusätzlich eine Alkoholdehydrogenase exprimiert, die bevorzugt rekombinant ist.

6. Ganzzellkatalysator nach einem der Ansprüche 1 bis 5, wobei die Aktivität wenigstens eines an der β-Oxidation beteiligten Enzyms gegenüber dem Wildtyp des Ganzzellkatalysators verringert ist.

7. Ganzzellkatalysator nach einem der Ansprüche 1 bis 6, wobei die Aktivität von BioH oder einer Variante davon gegenüber dem Wildtyp des Ganzzellkatalysators verringert ist.

8. Ganzzellkatalysator nach einem der Ansprüche 1 bis 6, wobei die Aktivität von FadL oder einer Variante davon gegenüber dem Wildtyp des Ganzzellkatalysators erhöht ist.

9. Verfahren zur Umsetzung einer Fettsäure, ω-Hydroxy-Fettsäure, ω-Oxo-Fettsäure oder eines Monoesters davon zu einem Amin, umfassend die Schritte
a) Oxidieren der Fettsäure, ω-Hydroxy-Fettsäure, ω-Oxo-Fettsäure oder des Monoesters davon durch Kontaktieren mit einer Alkanhydroxylase und gegebenenfalls einer Alkoholdehydrogenase zu einem Oxidationsprodukt
b) Kontaktieren des Oxidationsproduktes mit einer phosphopantetheinylierten Fettsäurereduktase oder einer α-Dioxygenase zu einem Aldehyd, und
c) Kontaktieren des Aldehyds mit einer Transaminase
wobei die Enzyme phosphopantetheinylierte Fettsäurereduktase, α-Dioxygenase, Transaminase und Alkanhydroxylase in Form eines Ganzzellkatalysators nach einem der Ansprüche 1 bis 8 bereitgestellt werden.

10. Verfahren nach Anspruch 9, wobei in Schritt c) eine Aminosäuredehydrogenase anwesend ist.

11. Verfahren nach einem der Ansprüche 9 bis 10, wobei die Enzyme phosphopantetheinylierte Fettsäurereduktase, α-Dioxygenase, Transaminase, Aminosäuredehydrogenase und Alkanhydroxylase in Form eines Ganzzellkatalysators nach einem der Ansprüche 2 bis 8 bereitgestellt werden.

12. Verfahren nach einem der Ansprüche 9 bis 11, wobei es sich bei der Fettsäure, ω-Hydroxy-Fettsäure, ω-Oxo-Fettsäure oder dem Monoester davon um eine Verbindung der Formel (I)
R¹ - A - COOR² (I)
handelt, wobei R¹ aus der Gruppe ausgewählt ist, die -H, -CHO, -OH und COOR³ umfasst,
wobei R² und R³ jeweils und unabhängig voneinander aus der Gruppe ausgewählt sind, die H, Methyl, Ethyl und Propyl umfasst,
mit der Maßgabe, dass wenigstens einer der Reste R² und R³ H ist,
wobei A eine unverzweigte, verzweigte, lineare, zyklische, substituierte oder unsubstituierte Kohlenwasserstoffgruppe mit wenigstens vier Kohlenstoffatomen darstellt.

13. Verfahren nach einem der Ansprüche 9 bis 12, wobei A die Formel - (CH₂)ₙ - aufweist, wobei n wenigstens 4, bevorzugt wenigstens 10 ist.

14. Verwendung des Ganzzellkatalysators nach einem der Ansprüche 1 bis 8 oder des Verfahrens nach einem der Ansprüche 8 bis 12 zur Aminierung einer Fettsäure, ω-Hydroxy-Fettsäure, ω-Oxo-Fettsäure oder einem Monoester davon.

15. Reaktionsmischung umfassend den Ganzzellkatalysator nach einem der Ansprüche 1 bis 8 in wässriger Lösung sowie eine Fettsäure, ω-Hydroxy-Fettsäure, ω-Oxo-Fettsäure oder einen Monoester davon der Formel (I)
R¹-A-COOR² (I),
wobei R¹ aus der Gruppe ausgewählt ist, die -H, -CHO, -OH und COOR³ umfasst,
wobei R² und R³ jeweils und unabhängig voneinander aus der Gruppe ausgewählt sind, die H, Methyl, Ethyl und Propyl umfasst,
mit der Maßgabe, dass wenigstens einer der Reste R² und R³ H ist,
wobei A eine unverzweigte, verzweigte, lineare, zyklische, substituierte oder unsubstituierte Kohlenwasserstoffgruppe mit wenigstens vier Kohlenstoffatomen darstellt, bevorzugt die Formel - (CH₂)ₙ -, wobei n wenigstens 4, besonders bevorzugt wenigstens 10 ist.

## Claims

1. Whole cell catalyst which expresses a recombinant α-dioxygenase or the combination of a recombinant fatty acid reductase and of a phosphopantetheinyl transferase phosphopantetheinyl -pantheteinylating the fatty acid reductase, and which additionally expresses a transaminase, wherein the phosphopantetheinyl transferase and/or transaminase is preferably recombinant.

2. Whole cell catalyst according to Claim 2, wherein the whole cell catalyst additionally expresses an amino acid dehydrogenase which is preferably recombinant.

3. Whole cell catalyst according to one of Claims 1 to 2, wherein the whole cell catalyst additionally expresses an alkane hydroxylase which is preferably recombinant.

4. Whole cell catalyst according to one of Claims 1 to 3, wherein the whole cell catalyst additionally expresses a polypeptide of the AlkL family which is preferably recombinant.

5. Whole cell catalyst according to one of Claims 1 to 4, which additionally expresses an alcohol dehydrogenase which is preferably recombinant.

6. Whole cell catalyst according to one of Claims 1 to 5, wherein the activity of at least one of the enzymes involved in β-oxidation is decreased compared to the wild type of the whole cell catalyst.

7. Whole cell catalyst according to one of Claims 1 to 6, wherein the activity of BioH or a variant thereof is decreased compared to the wild type of the whole cell catalyst.

8. Whole cell catalyst according to one of Claims 1 to 6, wherein the activity of FadL or a variant thereof compared to the wild type of the whole cell catalyst is increased.

9. Method for converting a fatty acid, ω-hydroxy fatty acid, ω-oxo fatty acid or a monoester thereof to an amine, comprising the steps
a) oxidizing the fatty acid, ω-hydroxy fatty acid, ω-oxo fatty acid or the monoester thereof to an oxidation product by contacting with an alkane hydroxylase and optionally an alcohol dehydrogenase,
b) contacting the oxidation product with a phosphopantetheinylated fatty acid reductase or an α-dioxygenase to give an aldehyde, and
c) contacting the aldehyde with a transaminase, wherein the enzymes phosphopantetheinylated fatty acid reductase, α-dioxygenase, transaminase and alkane hydroxylase are provided in the form of a whole cell catalyst according to one of Claims 1 to 8.

10. Method according to Claim 9, wherein in step c) an amino acid dehydrogenase is present.

11. Method according to one of Claims 9 to 10, wherein the enzymes phosphopantetheinylated fatty acid reductase, α-dioxygenase, transaminase, amino acid dehydrogenase and alkane hydroxylase are provided in the form of a whole cell catalyst according to one of Claims 2 to 8.

12. Method according to one of Claims 9 to 11, wherein the fatty acid, ω-hydroxy fatty acid, ω-oxo fatty acid or the monoester thereof is a compound of the formula (I)
R¹ - A - COOR² (I)
wherein R¹ is selected from the group which comprises -H, -CHO, -OH and COOR³,
wherein R² and R³ each and independently of one another are selected from the group which comprises H, methyl, ethyl and propyl,
with the proviso that at least one of the residues R² and R³ is H, and
wherein A represents an unbranched, branched, linear, cyclic, substituted or unsubstituted hydrocarbon group with at least four carbon atoms.

13. Method according to one of Claims 9 to 12, wherein A has the formula - (CH₂)ₙ -, wherein n is at least 4, preferably at least 10.

14. Use of the whole cell catalyst according to one of Claims 1 to 8 or of the method according to one of Claims 8 to 12 for the amination of a fatty acid, ω-hydroxy fatty acid, ω-oxo fatty acid or a monoester thereof.

15. Reaction mixture comprising the whole cell catalyst according to one of Claims 1 to 8 in aqueous solution and a fatty acid, ω-hydroxy fatty acid, ω-oxo fatty acid or a monoester thereof of the formula (I)
R¹ - A - COOR² (I),
wherein R¹ is selected from the group which comprises -H, -CHO, -OH and COOR³,
wherein R² and R³ each and independently of one another are selected from the group which comprises H, methyl, ethyl and propyl,
with the proviso that at least one of the residues R² and R³ is H,
wherein A represents an unbranched, branched, linear, cyclic, substituted or unsubstituted hydrocarbon group with at least four carbon atoms, preferably the formula - (CH₂)ₙ -, and wherein n is at least 4, particularly preferably at least 10.

## Revendications

1. Catalyseur à cellules entières, qui exprime une α-dioxygénase recombinante ou la combinaison d'une réductase d'acide gras recombinante et d'une transférase de phosphopanthétéinyle qui phosphopanthétéinylise la réductase d'acide gras, et qui exprime également une transaminase, la transférase de phosphopanthétéinyle et/ou la transaminase étant de préférence recombinantes.

2. Catalyseur à cellules entières selon la revendication 2, dans lequel le catalyseur à cellules entières exprime également une déshydrogénase d'acide aminé, qui est de préférence recombinante.

3. Catalyseur à cellules entières selon l'une quelconque des revendications 1 à 2, dans lequel le catalyseur à cellules entières exprime également une hydroxylase d'alcane, qui est de préférence recombinante.

4. Catalyseur à cellules entières selon l'une quelconque des revendications 1 à 3, dans lequel le catalyseur à cellules entières exprime également un polypeptide de la famille AlkL, qui est de préférence recombinant.

5. Catalyseur à cellules entières selon l'une quelconque des revendications 1 à 4, qui exprime également une déshydrogénase d'alcool, qui est de préférence recombinante.

6. Catalyseur à cellules entières selon l'une quelconque des revendications 1 à 5, dans lequel l'activité d'au moins une enzyme participant à l'oxydation β est réduite par rapport au type sauvage du catalyseur à cellules entières.

7. Catalyseur à cellules entières selon l'une quelconque des revendications 1 à 6, dans lequel l'activité de BioH ou d'une variante de celle-ci est réduite par rapport au type sauvage du catalyseur à cellules entières.

8. Catalyseur à cellules entières selon l'une quelconque des revendications 1 à 6, dans lequel l'activité de FadL ou d'une variante de celle-ci est augmentée par rapport au type sauvage du catalyseur à cellules entières.

9. Procédé de transformation d'un acide gras, d'un acide ω-hydroxy-gras, d'un acide ω-oxo-gras ou d'un monoester de ceux-ci en une amine, comprenant les étapes suivantes :
a) l'oxydation de l'acide gras, de l'acide ω-hydroxy-gras, de l'acide ω-oxo-gras ou du monoester de ceux-ci par mise en contact avec une hydroxylase d'alcane et éventuellement une déshydrogénase d'alcool pour obtenir un produit d'oxydation,
b) la mise en contact du produit d'oxydation avec une réductase d'acide gras phosphopanthétéinylisée ou une α-dioxygénase pour obtenir un aldéhyde, et
c) la mise en contact de l'aldéhyde avec une transaminase,
les enzymes réductase d'acide gras phosphopanthétéinylisée, α-dioxygénase, transaminase et hydroxylase d'alcane étant préparées sous la forme d'un catalyseur à cellules entières selon l'une quelconque des revendications 1 à 8.

10. Procédé selon la revendication 9, dans lequel une déshydrogénase d'acide aminé est présente à l'étape c).

11. Procédé selon l'une quelconque des revendications 9 à 10, dans lequel les enzymes réductase d'acide gras phosphopanthétéinylisée, α-dioxygénase, transaminase, déshydrogénase d'acide aminé et hydroxylase d'alcane sont préparées sous la forme d'un catalyseur à cellules entières selon l'une quelconque des revendications 2 à 8.

12. Procédé selon l'une quelconque des revendications 9 à 11, dans lequel l'acide gras, l'acide ω-hydroxy-gras, l'acide ω-oxo-gras ou le monoester de ceux-ci est un composé de formule (I)
R¹-A-COOR² (I)
R¹ étant choisi dans le groupe comprenant -H, -CHO, -OH et COOR³,
R² et R³ étant choisis à chaque fois et indépendamment l'un de l'autre dans le groupe comprenant H, méthyle, éthyle et propyle,
à condition qu'au moins un des radicaux R² et R³ représente H,
A représentant un groupe hydrocarboné non ramifié, ramifié, linéaire, cyclique, substitué ou non substitué, contenant au moins quatre atomes de carbone.

13. Procédé selon l'une quelconque des revendications 9 à 12, dans lequel A présente la formule - (CH₂)ₙ-, n valant au moins 4, de préférence au moins 10.

14. Utilisation du catalyseur à cellules entières selon l'une quelconque des revendications 1 à 8 ou du procédé selon l'une quelconque des revendications 8 à 12 pour l'amination d'un acide gras, d'un acide ω-hydroxy-gras, d'un acide ω-oxo-gras ou d'un monoester de ceux-ci.

15. Mélange réactionnel comprenant le catalyseur à cellules entières selon l'une quelconque des revendications 1 à 8 en solution aqueuse, ainsi qu'un acide gras, un acide ω-hydroxy-gras, un acide ω-oxo-gras ou un monoester de ceux-ci de formule (I)
R¹-A-COOR² (I)
R¹ étant choisi dans le groupe comprenant -H, -CHO, -OH et COOR³,
R² et R³ étant choisis à chaque fois et indépendamment l'un de l'autre dans le groupe comprenant H, méthyle, éthyle et propyle,
à condition qu'au moins un des radicaux R² et R³ représente H,
A représentant un groupe hydrocarboné non ramifié, ramifié, linéaire, cyclique, substitué ou non substitué, contenant au moins quatre atomes de carbone, de préférence la formule - (CH₂)ₙ-, n valant au moins 4, de préférence au moins 10.
